# EUROPEAN PATENT SPECIFICATION

(11) **EP 2 261 358 B1**
(45) Date of publication and mention of the grant of the patent: **14.05.2014**
(21) Application number: 10180372.4
(22) Date of filing: 20.12.1999
(51) Int. Cl.: A61K 39/09, C12N 15/62, C07K 14/315

(54) **Novel streptococcus antigen**
Streptokokken-Antigene
Antigènes de Streptococcus

(30) Priority: 23.12.1998 US 113800 P
(43) Date of publication of application: 15.12.2010
(62) Divisional of application: 08155317.4
(73) Proprietor: ID Biomedical Corporation of Quebec, Laval, QC H7V 3S8 (CA)
(72) Inventor: Brodeur, Bernard R, Sillery G1T 1N6 (CA); Charland, Nathalie, Breakeyville Québec G0S 1E3 (CA); Hamel, Josee, Sillery Québec G1T 1N6 (CA); Martin, Denis, St-Augustin-Des Maures Québec G3A 1E9 (CA); Pineau, Isabelle, Sainte-Foy Québec G1V 3Y7 (CA); Rioux, Clement, Ile Bizard Québec H9C 2T4 (CA)
(74) Representative: Johnston, Caroline Louise

(56) References cited:
- EP-A2- 0 846 766
- WO-A-00/06738
- WO-A-00/17370
- WO-A-98/18930
- WO-A-99/15675
- WO-A1-99/53940
- WO-A2-00/37105

### SEQUENCE LISTING

<110> BIOCHEM PHARMA INC. HAMEL, Josée BRODEUR, Bernard R. PINEAU, Isabelle MARTIN, Denis RIOUX, Clément CHARLAND, Nathalie
<120> NOVEL STREPTOCOCCUS ANTIGENS
<130> 12806-11PCT
<150> US 60/113,800
   <151> 1998-12-23
<160> 102
<170> FastSEQ for Windows Version 3.0
<210> 1
   <211> 3120
   <212> DNA
   <213> S. pneumoniae
<220>
<400> 1
   atg aaa ttt agt aaa aaa tat ata gca gct gga tca gct gtt atc gta     48
   tcc ttg agt cta tgt gcc tat gca cta aac cag cat cgt tcg cag gaa     96
   aat aag gac aat aat cgt gtc tct tat gtg gat ggc agc cag tca agt    144
   cag aaa agt gaa aac ttg aca cca gac cag gtt agc cag aaa gaa gga    192
   att cag gct gag caa att gta atc aaa att aca gat cag ggc tat gta    240
   acg tca cac ggt gac cac tat cat tac tat aat ggg aaa gtt cct tat    288
   gat gcc ctc ttt agt gaa gaa ctc ttg atg aag gat cca aac tat caa    336
   ctt aaa gac gct gat att gtc aat gaa gtc aag ggt ggt tat atc atc    384
   aag gtc gat gga aaa tat tat gtc tac ctg aaa gat gca gct cat gct    432
   gat aat gtt cga act aaa gat gaa atc aat cgt caa aaa caa gaa cat    480
   gtc aaa gat aat gag aag gtt aac tct aat gtt gct gta gca agg tct    528
   cag gga cga tat acg aca aat gat ggt tat gtc ttt aat cca gct gat    576
   att atc gaa gat acg ggt aat gct tat atc gtt cct cat gga ggt cac    624
   tat cac tac att ccc aaa agc gat tta tct gct agt gaa tta gca gca    672
   gct aaa gca cat ctg gct gga aaa aat atg caa ccg agt cag tta agc    720
   tat tct tca aca gct agt gac aat aac acg caa tct gta gca aaa gga    768
   tca act agc aag cca gca aat aaa tct gaa aat ctc cag agt ctt ttg    816
   aag gaa ctc tat gat tca cct agc gcc caa cgt tac agt gaa tca gat    864
   ggc ctg gtc ttt gac cct gct aag att atc agt cgt aca cca aat gga    912
   gtt gcg att ccg cat ggc gac cat tac cac ttt att cct tac agc aag    960
   ctt tct gct tta gaa gaa aag att gcc aga atg gtg cct atc agt gga   1008
   act ggt tct aca gtt tct aca aat gca aaa cct aat gaa gta gtg tct   1056
   agt cta ggc agt ctt tca agc aat cct tct tct tta acg aca agt aag   1104
   gag ctc tct tca gca tct gat ggt tat att ttt aat cca aaa gat atc   1152
   gtt gaa gaa acg gct aca gct tat att gta aga cat ggt gat cat ttc   1200
   cat tac att cca aaa tca aat caa att ggg caa ccg act ctt cca aac   1248
   aat agt cta gca aca cct tct cca tct ctt cca atc aat cca gga act 1296
   tca cat gag aaa cat gaa gaa gat gga tac gga ttt gat gct aat cgt 1344
   att atc gct gaa gat gaa tca ggt ttt gtc atg agt cac gga gac cac 1392
   aat cat tat ttc ttc aag aag gac ttg aca gaa gag caa att aag gct 1440
   gcg caa aaa cat tta gag gaa gtt aaa act agt cat aat gga tta gat 1488
   tct ttg tca tct cat gaa cag gat tat cca ggt aat gcc aaa gaa atg 1536
   aaa gat tta gat aaa aaa atc gaa gaa aaa att gct ggc att atg aaa 1584
   caa tat ggt gtc aaa cgt gaa agt att gtc gtg aat aaa gaa aaa aat 1632
   gcg att att tat ccg cat gga gat cac cat cat gca gat ccg att gat 1680
   gaa cat aaa ccg gtt gga att ggt cat tct cac agt aac tat gaa ctg 1728
   ttt aaa ccc gaa gaa gga gtt gct aaa aaa gaa ggg aat aaa gtt tat 1776
   act gga gaa gaa tta acg aat gtt gtt aat ttg tta aaa aat agt acg 1824
   ttt aat aat caa aac ttt act cta gcc aat ggt caa aaa cgc gtt tct 1872
   ttt agt ttt ccg cct gaa ttg gag aaa aaa tta ggt atc aat atg cta 1920
   gta aaa tta ata aca cca gat gga aaa gta ttg gag aaa gta tct ggt 1968
   aaa gta ttt gga gaa gga gta ggg aat att gca aac ttt gaa tta gat 2016
   caa cct tat tta cca gga caa aca ttt aag tat act atc gct tca aaa 2064
   gat tat cca gaa gta agt tat gat ggt aca ttt aca gtt cca acc tct 2112
   tta gct tac aaa atg gcc agt caa acg att ttc tat cct ttc cat gca 2160
   ggg gat act tat tta aga gtg aac cct caa ttt gca gtg cct aaa gga 2208
   act gat gct tta gtc aga gtg ttt gat gaa ttt cat gga aat gct tat 2256
   tta gaa aat aac tat aaa gtt ggt gaa atc aaa tta ccg att ccg aaa 2304
   tta aac caa gga aca acc aga acg gcc gga aat aaa att cct gta acc 2352
   ttc atg gca aat gct tat ttg gac aat caa tcg act tat att gtg gaa 2400
   gta cct atc ttg gaa aaa gaa aat caa act gat aaa cca agt att cta 2448
   cca caa ttt aaa agg aat aaa gca caa gaa aac tca aaa ctt gat gaa 2496
   aag gta gaa gaa cca aag act agt gag aag gta gaa aaa gaa aaa ctt 2544
   tct gaa act ggg aat agt act agt aat tca acg tta gaa gaa gtt cct 2592
   aca gtg gat cct gta caa gaa aaa gta gca aaa ttt gct gaa agt tat 2640
   ggg atg aag cta gaa aat gtc ttg ttt aat atg gac gga aca att gaa 2688
   tta tat tta cca tca gga gaa gtc att aaa aag aat atg gca gat ttt 2736
   aca gga gaa gca cct caa gga aat ggt gaa aat aaa cca tct gaa aat 2784
   gga aaa gta tct act gga aca gtt gag aac caa cca aca gaa aat aaa 2832
   cca gca gat tct tta cca gag gca cca aac gaa aaa cct gta aaa cca 2880
   gaa aac tca acg gat aat gga atg ttg aat cca gaa ggg aat gtg ggg 2928
   agt gac cct atg tta gat cca gca tta gag gaa gct cca gca gta gat 2976
   cct gta caa gaa aaa tta gaa aaa ttt aca gct agt tac gga tta ggc 3024
   tta gat agt gtt ata ttc aat atg gat gga acg att gaa tta aga ttg 3072
   cca agt gga gaa gtg ata aaa aag aat tta tct gat ttc ata gcg 3117
   taa 3120
<210> 2
   <211> 1039
   <212> PRT
   <213> S. pneumoniae
<400> 2
   Met Lys Phe Ser Lys Lys Tyr Ile Ala Ala Gly Ser Ala Val Ile Val
    1               5                  10                  15
   Ser Leu Ser Leu Cys Ala Tyr Ala Leu Asn Gln His Arg Ser Gln Glu
               20                  25                  30
   Asn Lys Asp Asn Asn Arg Val Ser Tyr Val Asp Gly Ser Gln Ser Ser
           35                  40                  45
   Gln Lys Ser Glu Asn Leu Thr Pro Asp Gln Val Ser Gln Lys Glu Gly
       50                  55                  60
   Ile Gln Ala Glu Gln Ile Val Ile Lys Ile Thr Asp Gln Gly Tyr Val
   65                  70                  75                  80
   Thr Ser His Gly Asp His Tyr His Tyr Tyr Asn Gly Lys Val Pro Tyr
                       85                  90                  95
   Asp Ala Leu Phe Ser Glu Glu Leu Leu Met Lys Asp Pro Asn Tyr Gln
                   100                 105                 110
   Leu Lys Asp Ala Asp Ile Val Asn Glu Val Lys Gly Gly Tyr Ile Ile
               115                 120                 125
   Lys Val Asp Gly Lys Tyr Tyr Val Tyr Leu Lys Asp Ala Ala His Ala
           130                 135                 140
   Asp Asn Val Arg Thr Lys Asp Glu Ile Asn Arg Gln Lys Gln Glu His
   145                 150                 155                 160
   Val Lys Asp Asn Glu Lys Val Asn Ser Asn Val Ala Val Ala Arg Ser
                   165                 170                 175
   Gln Gly Arg Tyr Thr Thr Asn Asp Gly Tyr Val Phe Asn Pro Ala Asp
               180                 185                 190
   Ile Ile Glu Asp Thr Gly Asn Ala Tyr Ile Val Pro His Gly Gly His
           195                 200                 205
   Tyr His Tyr Ile Pro Lys Ser Asp Leu Ser Ala Ser Glu Leu Ala Ala
       210                 215                 220
   Ala Lys Ala His Leu Ala Gly Lys Asn Met Gln Pro Ser Gln Leu Ser
   225                 230                 235                 240
   Tyr Ser Ser Thr Ala Ser Asp Asn Asn Thr Gln Ser Val Ala Lys Gly
                   245                 250                 255
   Ser Thr Ser Lys Pro Ala Asn Lys Ser Glu Asn Leu Gln Ser Leu Leu
               260                 265                 270
   Lys Glu Leu Tyr Asp Ser Pro Ser Ala Gln Arg Tyr Ser Glu Ser Asp
           275                 280                 285
   Gly Leu Val Phe Asp Pro Ala Lys Ile Ile Ser Arg Thr Pro Asn Gly
       290                 295                 300
   Val Ala Ile Pro His Gly Asp His Tyr His Phe Ile Pro Tyr Ser Lys
   305                 310                 315                 320
   Leu Ser Ala Leu Glu Glu Lys Ile Ala Arg Met Val Pro Ile Ser Gly
                   325                 330                 335
   Thr Gly Ser Thr Val Ser Thr Asn Ala Lys Pro Asn Glu Val Val Ser
               340                 345                 350
   Ser Leu Gly Ser Leu Ser Ser Asn Pro Ser Ser Leu Thr Thr Ser Lys
           355                 360                 365
   Glu Leu Ser Ser Ala Ser Asp Gly Tyr Ile Phe Asn Pro Lys Asp Ile
       370                 375                 380
   Val Glu Glu Thr Ala Thr Ala Tyr Ile Val Arg His Gly Asp His Phe
   385                 390                 395                 400
   His Tyr Ile Pro Lys Ser Asn Gln Ile Gly Gln Pro Thr Leu Pro Asn
                   405                 410                 415
   Asn Ser Leu Ala Thr Pro Ser Pro Ser Leu Pro Ile Asn Pro Gly Thr
               420                 425                 430
   Ser His Glu Lys His Glu Glu Asp Gly Tyr Gly Phe Asp Ala Asn Arg
           435                 440                 445
   Ile Ile Ala Glu Asp Glu Ser Gly Phe Val Met Ser His Gly Asp His
       450                 455                 460
   Asn His Tyr Phe Phe Lys Lys Asp Leu Thr Glu Glu Gln Ile Lys Ala
   465                 470                 475                 480
   Ala Gln Lys His Leu Glu Glu Val Lys Thr Ser His Asn Gly Leu Asp
                   485                 490                 495
   Ser Leu Ser Ser His Glu Gln Asp Tyr Pro Gly Asn Ala Lys Glu Met
               500                 505                 510
   Lys Asp Leu Asp Lys Lys Ile Glu Glu Lys Ile Ala Gly Ile Met Lys
           515                 520                 525
   Gln Tyr Gly Val Lys Arg Glu Ser Ile Val Val Asn Lys Glu Lys Asn
       530                 535                 540
   Ala Ile Ile Tyr Pro His Gly Asp His His His Ala Asp Pro Ile Asp
   545                 550                 555                 560
   Glu His Lys Pro Val Gly Ile Gly His Ser His Ser Asn Tyr Glu Leu
                   565                 570                 575
   Phe Lys Pro Glu Glu Gly Val Ala Lys Lys Glu Gly Asn Lys Val Tyr
               580                 585                 590
   Thr Gly Glu Glu Leu Thr Asn Val Val Asn Leu Leu Lys Asn Ser Thr
           595                 600                 605
   Phe Asn Asn Gln Asn Phe Thr Leu Ala Asn Gly Gln Lys Arg Val Ser
       610                 615                 620
   Phe Ser Phe Pro Pro Glu Leu Glu Lys Lys Leu Gly Ile Asn Met Leu
   625                 630                 635                 640
   Val Lys Leu Ile Thr Pro Asp Gly Lys Val Leu Glu Lys Val Ser Gly
                   645                 650                 655
   Lys Val Phe Gly Glu Gly Val Gly Asn Ile Ala Asn Phe Glu Leu Asp
               660                 665                 670
   Gln Pro Tyr Leu Pro Gly Gln Thr Phe Lys Tyr Thr Ile Ala Ser Lys
           675                 680                 685
   Asp Tyr Pro Glu Val Ser Tyr Asp Gly Thr Phe Thr Val Pro Thr Ser
       690                 695                 700
   Leu Ala Tyr Lys Met Ala Ser Gln Thr Ile Phe Tyr Pro Phe His Ala
   705                 710                 715                 720
   Gly Asp Thr Tyr Leu Arg Val Asn Pro Gln Phe Ala Val Pro Lys Gly
                   725                 730                 735
   Thr Asp Ala Leu Val Arg Val Phe Asp Glu Phe His Gly Asn Ala Tyr
               740                 745                 750
   Leu Glu Asn Asn Tyr Lys Val Gly Glu Ile Lys Leu Pro Ile Pro Lys
           755                 760                 765
   Leu Asn Gln Gly Thr Thr Arg Thr Ala Gly Asn Lys Ile Pro Val Thr
       770                 775                 780
   Phe Met Ala Asn Ala Tyr Leu Asp Asn Gln Ser Thr Tyr Ile Val Glu
   785                 790                 795                 800
   Val Pro Ile Leu Glu Lys Glu Asn Gln Thr Asp Lys Pro Ser Ile Leu
                   805                 810                 815
   Pro Gln Phe Lys Arg Asn Lys Ala Gln Glu Asn Ser Lys Leu Asp Glu
               820                 825                 830
   Lys Val Glu Glu Pro Lys Thr Ser Glu Lys Val Glu Lys Glu Lys Leu
           835                 840                 845
   Ser Glu Thr Gly Asn Ser Thr Ser Asn Ser Thr Leu Glu Glu Val Pro
       850                 855                 860
   Thr Val Asp Pro Val Gln Glu Lys Val Ala Lys Phe Ala Glu Ser Tyr
   865                 870                 875                 880
   Gly Met Lys Leu Glu Asn Val Leu Phe Asn Met Asp Gly Thr Ile Glu
                   885                 890                 895
   Leu Tyr Leu Pro Ser Gly Glu Val Ile Lys Lys Asn Met Ala Asp Phe
               900                 905                 910
   Thr Gly Glu Ala Pro Gln Gly Asn Gly Glu Asn Lys Pro Ser Glu Asn
           915                 920                 925
   Gly Lys Val Ser Thr Gly Thr Val Glu Asn Gln Pro Thr Glu Asn Lys
       930                 935                 940
   Pro Ala Asp Ser Leu Pro Glu Ala Pro Asn Glu Lys Pro Val Lys Pro
   945                 950                 955                 960
   Glu Asn Ser Thr Asp Asn Gly Met Leu Asn Pro Glu Gly Asn Val Gly
                   965                 970                 975
   Ser Asp Pro Met Leu Asp Pro Ala Leu Glu Glu Ala Pro Ala Val Asp
               980                 985                 990
   Pro Val Gln Glu Lys Leu Glu Lys Phe Thr Ala Ser Tyr Gly Leu Gly
           995                 1000                1005
   Leu Asp Ser Val Ile Phe Asn Met Asp Gly Thr Ile Glu Leu Arg Leu
       1010                1015                1020
   Pro Ser Gly Glu Val Ile Lys Lys Asn Leu Ser Asp Phe Ile Ala
   1025                1030                1035
<210> 3
   <211> 2523
   <212> DNA
   <213> S. pneumoniae
<220>
   <221> CDS
   <222> (1)...(2520)
   <223> Coding region of BVH-11 gene
<400> 3
   atg aaa atc aat aaa aaa tat cta gct ggg tca gta gct aca ctt gtt      48
   Met Lys Ile Asn Lys Lys Tyr Leu Ala Gly Ser Val Ala Thr Leu Val
    1               5                  10                  15
   tta agt gtc tgt gct tat gaa cta ggt ttg cat caa gct caa act gta      96
   Leu Ser Val Cys Ala Tyr Glu Leu Gly Leu His Gln Ala Gln Thr Val
               20                  25                  30
   aaa gaa aat aat cgt gtt tcc tat ata gat gga aaa caa gcg acg caa     144
   Lys Glu Asn Asn Arg Val Ser Tyr Ile Asp Gly Lys Gln Ala Thr Gln
           35                  40                  45
   aaa acg gag aat ttg act cct gat gag gtt agc aag cgt gaa gga atc     192
   Lys Thr Glu Asn Leu Thr Pro Asp Glu Val Ser Lys Arg Glu Gly Ile
       50                  55                  60
   aac gcc gaa caa atc gtc atc aag att acg gat caa ggt tat gtg acc     240
   Asn Ala Glu Gln Ile Val Ile Lys Ile Thr Asp Gln Gly Tyr Val Thr
   65                  70                  75                  80
   tct cat gga gac cat tat cat tac tat aat ggc aag gtc cct tat gat     288
   Ser His Gly Asp His Tyr His Tyr Tyr Asn Gly Lys Val Pro Tyr Asp
                   85                  90                  95
   gcc atc atc agt gaa gag ctc ctc atg aaa gat ccg aat tat cag ttg     336
   Ala Ile Ile Ser Glu Glu Leu Leu Met Lys Asp Pro Asn Tyr Gln Leu
               100                 105                 110
   aag gat tca gac att gtc aat gaa atc aag ggt ggt tat gtc att aag     384
   Lys Asp Ser Asp Ile Val Asn Glu Ile Lys Gly Gly Tyr Val Ile Lys
           115                 120                 125
   gta aac ggt aaa tac tat gtt tac ctt aag gat gca gct cat gcg gat     432
   Val Asn Gly Lys Tyr Tyr Val Tyr Leu Lys Asp Ala Ala His Ala Asp
       130                 135                 140
   aat gtc cgt aca aaa gaa gaa atc aat cgg caa aaa caa gaa cat agt     480
   Asn Val Arg Thr Lys Glu Glu Ile Asn Arg Gln Lys Gln Glu His Ser
   145                 150                 155                 160
   cag cat cgt gaa gga ggg act tca gca aac gat ggt gcg gta gcc ttt     528
   Gln His Arg Glu Gly Gly Thr Ser Ala Asn Asp Gly Ala Val Ala Phe
                   165                 170                 175
   gca cgt tca cag gga cgc tac acc aca gat gat ggt tat atc ttc aat      576
   Ala Arg Ser Gln Gly Arg Tyr Thr Thr Asp Asp Gly Tyr Ile Phe Asn
               180                 185                 190
   gca tct gat atc atc gaa gat acg ggc gat gcc tat atc gtt cct cat      624
   Ala Ser Asp Ile Ile Glu Asp Thr Gly Asp Ala Tyr Ile Val Pro His
           195                 200                 205
   gga gat cat tac cat tac att cct aag aat gag tta tca gct agc gag      672
   Gly Asp His Tyr His Tyr Ile Pro Lys Asn Glu Leu Ser Ala Ser Glu
       210                 215                 220
   ttg gct gct gca gaa gcc ttc cta tct ggt cgg gaa aat ctg tca aat      720
   Leu Ala Ala Ala Glu Ala Phe Leu Ser Gly Arg Glu Asn Leu Ser Asn
   225                 230                 235                 240
   tta aga acc tat cgc cga caa aat agc gat aac act cca aga aca aac      768
   Leu Arg Thr Tyr Arg Arg Gln Asn Ser Asp Asn Thr Pro Arg Thr Asn
                   245                 250                 255
   tgg gta cct tct gta agc aat cca gga act aca aat act aac aca agc      816
   Trp Val Pro Ser Val Ser Asn Pro Gly Thr Thr Asn Thr Asn Thr Ser
               260                 265                 270
   aac aac agc aac act aac agt caa gca agt caa agt aat gac att gat      864
   Asn Asn Ser Asn Thr Asn Ser Gln Ala Ser Gln Ser Asn Asp Ile Asp
           275                 280                 285
   agt ctc ttg aaa cag ctc tac aaa ctg cct ttg agt caa cgc cat gta      912
   Ser Leu Leu Lys Gln Leu Tyr Lys Leu Pro Leu Ser Gln Arg His Val
       290                 295                 300
   gaa tct gat ggc ctt att ttc gac cca gcg caa atc aca agt cga acc      960
   Glu Ser Asp Gly Leu Ile Phe Asp Pro Ala Gln Ile Thr Ser Arg Thr
   305                 310                 315                 320
   gcc aga ggt gta gct gtc cct cat ggt aac cat tac cac ttt atc cct     1008
   Ala Arg Gly Val Ala Val Pro His Gly Asn His Tyr His Phe Ile Pro
                   325                 330                 335
   tat gaa caa atg tct gaa ttg gaa aaa cga att gct cgt att att ccc     1056
   Tyr Glu Gln Met Ser Glu Leu Glu Lys Arg Ile Ala Arg Ile Ile Pro
               340                 345                 350
   ctt cgt tat cgt tca aac cat tgg gta cca gat tca aga cca gaa gaa     1104
   Leu Arg Tyr Arg Ser Asn His Trp Val Pro Asp Ser Arg Pro Glu Glu
           355                 360                 365
   cca agt cca caa ccg act cca gaa cct agt cca agt ccg caa cct gca     1152
   Pro Ser Pro Gln Pro Thr Pro Glu Pro Ser Pro Ser Pro Gln Pro Ala
       370                 375                 380
   cca aat cct caa cca gct cca agc aat cca att gat gag aaa ttg gtc     1200
   Pro Asn Pro Gln Pro Ala Pro Ser Asn Pro Ile Asp Glu Lys Leu Val
   385                 390                 395                 400
   aaa gaa gct gtt cga aaa gta ggc gat ggt tat gtc ttt gag gag aat     1248
   Lys Glu Ala Val Arg Lys Val Gly Asp Gly Tyr Val Phe Glu Glu Asn
                   405                 410                 415
   gga gtt tct cgt tat atc cca gcc aag aat ctt tca gca gaa aca gca      1296
   Gly Val Ser Arg Tyr Ile Pro Ala Lys Asn Leu Ser Ala Glu Thr Ala
               420                 425                 430
   gca ggc att gat agc aaa ctg gcc aag cag gaa agt tta tct cat aag      1344
   Ala Gly Ile Asp Ser Lys Leu Ala Lys Gln Glu Ser Leu Ser His Lys
           435                 440                 445
   cta gga gct aag aaa act gac ctc cca tct agt gat cga gaa ttt tac      1392
   Leu Gly Ala Lys Lys Thr Asp Leu Pro Ser Ser Asp Arg Glu Phe Tyr
       450                 455                 460
   aat aag gct tat gac tta cta gca aga att cac caa gat tta ctt gat      1440
   Asn Lys Ala Tyr Asp Leu Leu Ala Arg Ile His Gln Asp Leu Leu Asp
   465                 470                 475                 480
   aat aaa ggt cga caa gtt gat ttt gag gct ttg gat aac ctg ttg gaa      1488
   Asn Lys Gly Arg Gln Val Asp Phe Glu Ala Leu Asp Asn Leu Leu Glu
                   485                 490                 495
   cga ctc aag gat gtc tca agt gat aaa gtc aag tta gtg gat gat att      1536
   Arg Leu Lys Asp Val Ser Ser Asp Lys Val Lys Leu Val Asp Asp Ile
               500                 505                 510
   ctt gcc ttc tta gct ccg att cgt cat cca gaa cgt tta gga aaa cca      1584
   Leu Ala Phe Leu Ala Pro Ile Arg His Pro Glu Arg Leu Gly Lys Pro
           515                 520                 525
   aat gcg caa att acc tac act gat gat gag att caa gta gcc aag ttg      1632
   Asn Ala Gln Ile Thr Tyr Thr Asp Asp Glu Ile Gln Val Ala Lys Leu
       530                 535                 540
   gca ggc aag tac aca aca gaa gac ggt tat atc ttt gat cct cgt gat      1680
   Ala Gly Lys Tyr Thr Thr Glu Asp Gly Tyr Ile Phe Asp Pro Arg Asp
   545                 550                 555                 560
   ata acc agt gat gag ggg gat gcc tat gta act cca cat atg acc cat      1728
   Ile Thr Ser Asp Glu Gly Asp Ala Tyr Val Thr Pro His Met Thr His
                   565                 570                 575
   agc cac tgg att aaa aaa gat agt ttg tct gaa gct gag aga gcg gca      1776
   Ser His Trp Ile Lys Lys Asp Ser Leu Ser Glu Ala Glu Arg Ala Ala
               580                 585                 590
   gcc cag gct tat gct aaa gag aaa ggt ttg acc cct cct tcg aca gac      1824
   Ala Gln Ala Tyr Ala Lys Glu Lys Gly Leu Thr Pro Pro Ser Thr Asp
           595                 600                 605
   cat cag gat tca gga aat act gag gca aaa gga gca gaa gct atc tac      1872
   His Gln Asp Ser Gly Asn Thr Glu Ala Lys Gly Ala Glu Ala Ile Tyr
       610                 615                 620
   aac cgc gtg aaa gca gct aag aag gtg cca ctt gat cgt atg cct tac      1920
   Asn Arg Val Lys Ala Ala Lys Lys Val Pro Leu Asp Arg Met Pro Tyr
   625                 630                 635                 640
   aat ctt caa tat act gta gaa gtc aaa aac ggt agt tta atc ata cct      1968
   Asn Leu Gln Tyr Thr Val Glu Val Lys Asn Gly Ser Leu Ile Ile Pro
                   645                 650                 655
   cat tat gac cat tac cat aac atc aaa ttt gag tgg ttt gac gaa ggc      2016
   His Tyr Asp His Tyr His Asn Ile Lys Phe Glu Trp Phe Asp Glu Gly
               660                 665                 670
   ctt tat gag gca cct aag ggg tat act ctt gag gat ctt ttg gcg act      2064
   Leu Tyr Glu Ala Pro Lys Gly Tyr Thr Leu Glu Asp Leu Leu Ala Thr
           675                 680                 685
   gtc aag tac tat gtc gaa cat cca aac gaa cgt ccg cat tca gat aat      2112
   Val Lys Tyr Tyr Val Glu His Pro Asn Glu Arg Pro His Ser Asp Asn
       690                 695                 700
   ggt ttt ggt aac gct agc gac cat gtt caa aga aac aaa aat ggt caa      2160
   Gly Phe Gly Asn Ala Ser Asp His Val Gln Arg Asn Lys Asn Gly Gln
   705                 710                 715                 720
   gct gat acc aat caa acg gaa aaa cca agc gag gag aaa cct cag aca      2208
   Ala Asp Thr Asn Gln Thr Glu Lys Pro Ser Glu Glu Lys Pro Gln Thr
                   725                 730                 735
   gaa aaa cct gag gaa gaa acc cct cga gaa gag aaa cca caa agc gag      2256
   Glu Lys Pro Glu Glu Glu Thr Pro Arg Glu Glu Lys Pro Gln Ser Glu
               740                 745                 750
   aaa cca gag tct cca aaa cca aca gag gaa cca gaa gaa gaa tca cca      2304
   Lys Pro Glu Ser Pro Lys Pro Thr Glu Glu Pro Glu Glu Glu Ser Pro
           755                 760                 765
   gag gaa tca gaa gaa cct cag gtc gag act gaa aag gtt gaa gaa aaa      2352
   Glu Glu Ser Glu Glu Pro Gln Val Glu Thr Glu Lys Val Glu Glu Lys
       770                 775                 780
   ctg aga gag gct gaa gat tta ctt gga aaa atc cag gat cca att atc      2400
   Leu Arg Glu Ala Glu Asp Leu Leu Gly Lys Ile Gln Asp Pro Ile Ile
   785                 790                 795                 800
   aag tcc aat gcc aaa gag act ctc aca gga tta aaa aat aat tta cta      2448
   Lys Ser Asn Ala Lys Glu Thr Leu Thr Gly Leu Lys Asn Asn Leu Leu
                   805                 810                 815
   ttt ggc acc cag gac aac aat act att atg gca gaa gct gaa aaa cta      2496
   Phe Gly Thr Gln Asp Asn Asn Thr Ile Met Ala Glu Ala Glu Lys Leu
               820                 825                 830
   ttg gct tta tta aag gag agt aag taa                                  2523
   Leu Ala Leu Leu Lys Glu Ser Lys
           835                 840
<210> 4
   <211> 840
   <212> PRT
   <213> S. pneumoniae
<400> 4
   Met Lys Ile Asn Lys Lys Tyr Leu Ala Gly Ser Val Ala Thr Leu Val
   1               5                   10                  15
   Leu Ser Val Cys Ala Tyr Glu Leu Gly Leu His Gln Ala Gln Thr Val
               20                  25                  30
   Lys Glu Asn Asn Arg Val Ser Tyr Ile Asp Gly Lys Gln Ala Thr Gln
           35                  40                  45
   Lys Thr Glu Asn Leu Thr Pro Asp Glu Val Ser Lys Arg Glu Gly Ile
       50                  55                  60
   Asn Ala Glu Gln Ile Val Ile Lys Ile Thr Asp Gln Gly Tyr Val Thr
   65                  70                  75                  80
   Ser His Gly Asp His Tyr His Tyr Tyr Asn Gly Lys Val Pro Tyr Asp
                   85                  90                  95
   Ala Ile Ile Ser Glu Glu Leu Leu Met Lys Asp Pro Asn Tyr Gln Leu
               100                 105                 110
   Lys Asp Ser Asp Ile Val Asn Glu Ile Lys Gly Gly Tyr Val Ile Lys
           115                 120                 125
   Val Asn Gly Lys Tyr Tyr Val Tyr Leu Lys Asp Ala Ala His Ala Asp
       130                 135                 140
   Asn Val Arg Thr Lys Glu Glu Ile Asn Arg Gln Lys Gln Glu His Ser
   145                 150                 155                 160
   Gln His Arg Glu Gly Gly Thr Ser Ala Asn Asp Gly Ala Val Ala Phe
                   165                 170                 175
   Ala Arg Ser Gln Gly Arg Tyr Thr Thr Asp Asp Gly Tyr Ile Phe Asn
               180                 185                 190
   Ala Ser Asp Ile Ile Glu Asp Thr Gly Asp Ala Tyr Ile Val Pro His
           195                 200                 205
   Gly Asp His Tyr His Tyr Ile Pro Lys Asn Glu Leu Ser Ala Ser Glu
       210                 215                 220
   Leu Ala Ala Ala Glu Ala Phe Leu Ser Gly Arg Glu Asn Leu Ser Asn
   225                 230                 235                 240
   Leu Arg Thr Tyr Arg Arg Gln Asn Ser Asp Asn Thr Pro Arg Thr Asn
                   245                 250                 255
   Trp Val Pro Ser Val Ser Asn Pro Gly Thr Thr Asn Thr Asn Thr Ser
               260                 265                 270
   Asn Asn Ser Asn Thr Asn Ser Gln Ala Ser Gln Ser Asn Asp Ile Asp
           275                 280                 285
   Ser Leu Leu Lys Gln Leu Tyr Lys Leu Pro Leu Ser Gln Arg His Val
       290                 295                 300
   Glu Ser Asp Gly Leu Ile Phe Asp Pro Ala Gln Ile Thr Ser Arg Thr
   305                 310                 315                 320
   Ala Arg Gly Val Ala Val Pro His Gly Asn His Tyr His Phe Ile Pro
                   325                 330                 335
   Tyr Glu Gln Met Ser Glu Leu Glu Lys Arg Ile Ala Arg Ile Ile Pro
               340                 345                 350
   Leu Arg Tyr Arg Ser Asn His Trp Val Pro Asp Ser Arg Pro Glu Glu
           355                 360                 365
   Pro Ser Pro Gln Pro Thr Pro Glu Pro Ser Pro Ser Pro Gln Pro Ala
       370                 375                 380
   Pro Asn Pro Gln Pro Ala Pro Ser Asn Pro Ile Asp Glu Lys Leu Val
   385                 390                 395                 400
   Lys Glu Ala Val Arg Lys Val Gly Asp Gly Tyr Val Phe Glu Glu Asn
                   405                 410                 415
   Gly Val Ser Arg Tyr Ile Pro Ala Lys Asn Leu Ser Ala Glu Thr Ala
               420                 425                 430
   Ala Gly Ile Asp Ser Lys Leu Ala Lys Gln Glu Ser Leu Ser His Lys
           435                 440                 445
   Leu Gly Ala Lys Lys Thr Asp Leu Pro Ser Ser Asp Arg Glu Phe Tyr
       450                 455                 460
   Asn Lys Ala Tyr Asp Leu Leu Ala Arg Ile His Gln Asp Leu Leu Asp
   465                 470                 475                 480
   Asn Lys Gly Arg Gln Val Asp Phe Glu Ala Leu Asp Asn Leu Leu Glu
                   485                 490                 495
   Arg Leu Lys Asp Val Ser Ser Asp Lys Val Lys Leu Val Asp Asp Ile
               500                 505                 510
   Leu Ala Phe Leu Ala Pro Ile Arg His Pro Glu Arg Leu Gly Lys Pro
           515                 520                 525
   Asn Ala Gln Ile Thr Tyr Thr Asp Asp Glu Ile Gln Val Ala Lys Leu
       530                 535                 540
   Ala Gly Lys Tyr Thr Thr Glu Asp Gly Tyr Ile Phe Asp Pro Arg Asp
   545                 550                 555                 560
   Ile Thr Ser Asp Glu Gly Asp Ala Tyr Val Thr Pro His Met Thr His
                   565                 570                 575
   Ser His Trp Ile Lys Lys Asp Ser Leu Ser Glu Ala Glu Arg Ala Ala
               580                 585                 590
   Ala Gln Ala Tyr Ala Lys Glu Lys Gly Leu Thr Pro Pro Ser Thr Asp
           595                 600                 605
   His Gln Asp Ser Gly Asn Thr Glu Ala Lys Gly Ala Glu Ala Ile Tyr
       610                 615                 620
   Asn Arg Val Lys Ala Ala Lys Lys Val Pro Leu Asp Arg Met Pro Tyr
   625                 630                 635                 640
   Asn Leu Gln Tyr Thr Val Glu Val Lys Asn Gly Ser Leu Ile Ile Pro
                   645                 650                 655
   His Tyr Asp His Tyr His Asn Ile Lys Phe Glu Trp Phe Asp Glu Gly
               660                 665                 670
   Leu Tyr Glu Ala Pro Lys Gly Tyr Thr Leu Glu Asp Leu Leu Ala Thr
           675                 680                 685
   Val Lys Tyr Tyr Val Glu His Pro Asn Glu Arg Pro His Ser Asp Asn
       690                 695                 700
   Gly Phe Gly Asn Ala Ser Asp His Val Gln Arg Asn Lys Asn Gly Gln
   705                 710                 715                 720
   Ala Asp Thr Asn Gln Thr Glu Lys Pro Ser Glu Glu Lys Pro Gln Thr
                   725                 730                 735
   Glu Lys Pro Glu Glu Glu Thr Pro Arg Glu Glu Lys Pro Gln Ser Glu
               740                 745                 750
   Lys Pro Glu Ser Pro Lys Pro Thr Glu Glu Pro Glu Glu Glu Ser Pro
           755                 760                 765
   Glu Glu Ser Glu Glu Pro Gln Val Glu Thr Glu Lys Val Glu Glu Lys
       770                 775                 780
   Leu Arg Glu Ala Glu Asp Leu Leu Gly Lys Ile Gln Asp Pro Ile Ile
   785                 790                 795                 800
   Lys Ser Asn Ala Lys Glu Thr Leu Thr Gly Leu Lys Asn Asn Leu Leu
                   805                 810                 815
   Phe Gly Thr Gln Asp Asn Asn Thr Ile Met Ala Glu Ala Glu Lys Leu
               820                 825                 830
   Leu Ala Leu Leu Lys Glu Ser Lys
           835                 840
<210> 5
   <211> 1581
   <212> DNA
   <213> S. pneumoniae
<220>
   <221> CDS
   <222> (1)...(1578)
<400> 5
   atg gag aat ata gac atg ttt aaa tca aat cat gag cga aga atg cgt      48
   Met Glu Asn Ile Asp Met Phe Lys Ser Asn His Glu Arg Arg Met Arg
    1               5                   10                  15
   tat tcc att cgt aaa ttt agt gta gga gta gct agc gta gct gtt gcc      96
   Tyr Ser Ile Arg Lys Phe Ser Val Gly Val Ala Ser Val Ala Val Ala
               20                  25                  30
   agt ctt ttt atg gga agt gtt gta cat gcg aca gag aaa gag gga agt     144
   Ser Leu Phe Met Gly Ser Val Val His Ala Thr Glu Lys Glu Gly Ser
           35                  40                  45
   acc caa gca gcc act tct ttt aat agg gga aat gga agt cag gca gaa     192
   Thr Gln Ala Ala Thr Ser Phe Asn Arg Gly Asn Gly Ser Gln Ala Glu
       50                  55                  60
   caa cgt gga gaa ctc gat tta gaa cga gat aag gca atg aaa gcg gtc     240
   Gln Arg Gly Glu Leu Asp Leu Glu Arg Asp Lys Ala Met Lys Ala Val
   65                  70                  75                  80
   agt gaa tat gta gga aaa atg gtg aga gat gcc tat gta aaa tca gat     288
   Ser Glu Tyr Val Gly Lys Met Val Arg Asp Ala Tyr Val Lys Ser Asp
                   85                  90                  95
   aga aaa cga cat aaa aat act gta gct cta gtt aac cag ttg gga aac     336
   Arg Lys Arg His Lys Asn Thr Val Ala Leu Val Asn Gln Leu Gly Asn
               100                 105                 110
   att aag aac agg tat ttg aat gaa ata gtt cat tca acc tca aaa agc     384
   Ile Lys Asn Arg Tyr Leu Asn Glu Ile Val His Ser Thr Ser Lys Ser
           115                 120                 125
   caa cta cag gaa ctg atg atg aag agt caa tca gaa gta gat gaa gct     432
   Gln Leu Gln Glu Leu Met Met Lys Ser Gln Ser Glu Val Asp Glu Ala
       130                 135                 140
   gtg tct aaa ttt gaa aag gac tca ttt tct tcg tca agt tca gga tcc     480
   Val Ser Lys Phe Glu Lys Asp Ser Phe Ser Ser Ser Ser Ser Gly Ser
   145                 150                 155                 160
   tcc act aaa cca gaa act ccg cag ccg gaa aat cca gag cat caa aaa     528
   Ser Thr Lys Pro Glu Thr Pro Gln Pro Glu Asn Pro Glu His Gln Lys
                   165                 170                 175
   cca aca act cca tct ccg gat acc aaa cca agc cct caa cca gaa ggc     576
   Pro Thr Thr Pro Ser Pro Asp Thr Lys Pro Ser Pro Gln Pro Glu Gly
               180                 185                 190
   aag aaa cca agc gta cca gac att aat cag gaa aaa gaa aaa gct aag     624
   Lys Lys Pro Ser Val Pro Asp Ile Asn Gln Glu Lys Glu Lys Ala Lys
           195                 200                 205
   ctt gct gta gta acc tac atg agc aag att tta gat gat ata caa aaa     672
   Leu Ala Val Val Thr Tyr Met Ser Lys Ile Leu Asp Asp Ile Gln Lys
       210                 215                 220
   cat cat ctg cag aaa gaa aaa cat cgt cag att gtt gct ctt att aag      720
   His His Leu Gln Lys Glu Lys His Arg Gln Ile Val Ala Leu Ile Lys
   225                 230                 235                 240
   gag ctt gat gag ctt aaa aag caa gct ctt tct gaa att gat aat gta      768
   Glu Leu Asp Glu Leu Lys Lys Gln Ala Leu Ser Glu Ile Asp Asn Val
                   245                 250                 255
   aat acc aaa gta gaa att gaa aat aca gtc cac aag ata ttt gca gac      816
   Asn Thr Lys Val Glu Ile Glu Asn Thr Val His Lys Ile Phe Ala Asp
               260                 265                 270
   atg gat gca gtt gtg act aaa ttc aaa aaa ggc tta act cag gac aca      864
   Met Asp Ala Val Val Thr Lys Phe Lys Lys Gly Leu Thr Gln Asp Thr
           275                 280                 285
   cca aaa gaa cca ggt aac aaa aaa cca tct gct cca aaa cca ggt atg      912
   Pro Lys Glu Pro Gly Asn Lys Lys Pro Ser Ala Pro Lys Pro Gly Met
       290                 295                 300
   caa cca agt cct caa cca gag gtt aaa ccg cag ctg gaa aaa cca aaa      960
   Gln Pro Ser Pro Gln Pro Glu Val Lys Pro Gln Leu Glu Lys Pro Lys
   305                 310                 315                 320
   cca gag gtt aaa ccg caa cca gaa aaa cca aaa cca gag gtt aaa ccg     1008
   Pro Glu Val Lys Pro Gln Pro Glu Lys Pro Lys Pro Glu Val Lys Pro
                   325                 330                 335
   cag ccg gaa aaa cca aaa cca gag gtt aaa ccg cag ccg gaa aaa cca     1056
   Gln Pro Glu Lys Pro Lys Pro Glu Val Lys Pro Gln Pro Glu Lys Pro
               340                 345                 350
   aaa cca gag gtt aaa ccg cag ccg gaa aaa cca aaa cca gag gtt aaa     1104
   Lys Pro Glu Val Lys Pro Gln Pro Glu Lys Pro Lys Pro Glu Val Lys
           355                 360                 365
   ccg cag ccg gaa aaa cca aaa cca gag gtt aaa ccg cag ccg gaa aaa     1152
   Pro Gln Pro Glu Lys Pro Lys Pro Glu Val Lys Pro Gln Pro Glu Lys
       370                 375                 380
   cca aaa cca gag gtt aaa ccg cag ccg gaa aaa cca aaa cca gag gtt     1200
   Pro Lys Pro Glu Val Lys Pro Gln Pro Glu Lys Pro Lys Pro Glu Val
   385                 390                 395                 400
   aaa ccg cag ccg gaa aaa cca aaa cca gag gtt aaa ccg cag ccg gaa     1248
   Lys Pro Gln Pro Glu Lys Pro Lys Pro Glu Val Lys Pro Gln Pro Glu
                   405                 410                 415
   aaa cca aaa cca gag gtt aaa ccg cag ccg gaa aaa cca aaa cca gag     1296
   Lys Pro Lys Pro Glu Val Lys Pro Gln Pro Glu Lys Pro Lys Pro Glu
               420                 425                 430
   gtt aaa ccg caa cca gaa aaa cca aaa cca gag gtt aaa ccg caa cca     1344
   Val Lys Pro Gln Pro Glu Lys Pro Lys Pro Glu Val Lys Pro Gln Pro
           435                 440                 445
   gaa aaa cca aaa cca gat aat agc aag cca caa gca gat gat aag aag     1392
   Glu Lys Pro Lys Pro Asp Asn Ser Lys Pro Gln Ala Asp Asp Lys Lys
       450                 455                 460
   cca tca act aca aat aat tta agc aag gac aag caa cct tct aac caa      1440
   Pro Ser Thr Thr Asn Asn Leu Ser Lys Asp Lys Gln Pro Ser Asn Gln
   465                 470                 475                 480
   gct tca aca aac gaa aaa gca aca aat aaa ccg aag aag tca ttg cca      1488
   Ala Ser Thr Asn Glu Lys Ala Thr Asn Lys Pro Lys Lys Ser Leu Pro
                   485                 490                 495
   tca act gga tct att tca aat cta gca ctt gaa att gca ggt ctt ctt      1536
   Ser Thr Gly Ser Ile Ser Asn Leu Ala Leu Glu Ile Ala Gly Leu Leu
               500                 505                 510
   acc ttg gcg ggg gca acc att ctt gct aag aaa aga atg aaa              1578
   Thr Leu Ala Gly Ala Thr Ile Leu Ala Lys Lys Arg Met Lys
           515                 520                 525
   tag                                                                  1581
<210> 6
   <211> 526
   <212> PRT
   <213> S. pneumoniae
<400> 6
   Met Glu Asn Ile Asp Met Phe Lys Ser Asn His Glu Arg Arg Met Arg
    1              5                   10                  15
   Tyr Ser Ile Arg Lys Phe Ser Val Gly Val Ala Ser Val Ala Val Ala
               20                  25                  30
   Ser Leu Phe Met Gly Ser Val Val His Ala Thr Glu Lys Glu Gly Ser
           35                  40                  45
   Thr Gln Ala Ala Thr Ser Phe Asn Arg Gly Asn Gly Ser Gln Ala Glu
       50                  55                  60
   Gln Arg Gly Glu Leu Asp Leu Glu Arg Asp Lys Ala Met Lys Ala Val
   65                  70                  75                  80
   Ser Glu Tyr Val Gly Lys Met Val Arg Asp Ala Tyr Val Lys Ser Asp
                   85                  90                  95
   Arg Lys Arg His Lys Asn Thr Val Ala Leu Val Asn Gln Leu Gly Asn
               100                 105                 110
   Ile Lys Asn Arg Tyr Leu Asn Glu Ile Val His Ser Thr Ser Lys Ser
           115                 120                 125
   Gln Leu Gln Glu Leu Met Met Lys Ser Gln Ser Glu Val Asp Glu Ala
       130                 135                 140
   Val Ser Lys Phe Glu Lys Asp Ser Phe Ser Ser Ser Ser Ser Gly Ser
   145                 150                 155                 160
   Ser Thr Lys Pro Glu Thr Pro Gln Pro Glu Asn Pro Glu His Gln Lys
                   165                 170                 175
   Pro Thr Thr Pro Ser Pro Asp Thr Lys Pro Ser Pro Gln Pro Glu Gly
               180                 185                 190
   Lys Lys Pro Ser Val Pro Asp Ile Asn Gln Glu Lys Glu Lys Ala Lys
           195                 200                 205
   Leu Ala Val Val Thr Tyr Met Ser Lys Ile Leu Asp Asp Ile Gln Lys
       210                 215                 220
   His His Leu Gln Lys Glu Lys His Arg Gln Ile Val Ala Leu Ile Lys
   225                 230                 235                 240
   Glu Leu Asp Glu Leu Lys Lys Gln Ala Leu Ser Glu Ile Asp Asn Val
                           245                     250                     255
   Asn Thr Lys Val Glu Ile Glu Asn Thr Val His Lys Ile Phe Ala Asp
                   260                     265                     270
   Met Asp Ala Val Val Thr Lys Phe Lys Lys Gly Leu Thr Gln Asp Thr
               275                     280                     285
   Pro Lys Glu Pro Gly Asn Lys Lys Pro Ser Ala Pro Lys Pro Gly Met
       290                     295                     300
   Gln Pro Ser Pro Gln Pro Glu Val Lys Pro Gln Leu Glu Lys Pro Lys
   305                     310                     315                     320
   Pro Glu Val Lys Pro Gln Pro Glu Lys Pro Lys Pro Glu Val Lys Pro
                   325                     330                     335
   Gln Pro Glu Lys Pro Lys Pro Glu Val Lys Pro Gln Pro Glu Lys Pro
                   340                     345                     350
   Lys Pro Glu Val Lys Pro Gln Pro Glu Lys Pro Lys Pro Glu Val Lys
               355                     360                     365
   Pro Gln Pro Glu Lys Pro Lys Pro Glu Val Lys Pro Gln Pro Glu Lys
       370                     375                     380
   Pro Lys Pro Glu Val Lys Pro Gln Pro Glu Lys Pro Lys Pro Glu Val
   385                     390                     395                     400
   Lys Pro Gln Pro Glu Lys Pro Lys Pro Glu Val Lys Pro Gln Pro Glu
                   405                     410                     415
   Lys Pro Lys Pro Glu Val Lys Pro Gln Pro Glu Lys Pro Lys Pro Glu
                   420                     425                     430
   Val Lys Pro Gln Pro Glu Lys Pro Lys Pro Glu Val Lys Pro Gln Pro
               435                     440                     445
   Glu Lys Pro Lys Pro Asp Asn Ser Lys Pro Gln Ala Asp Asp Lys Lys
       450                     455                     460
   Pro Ser Thr Thr Asn Asn Leu Ser Lys Asp Lys Gln Pro Ser Asn Gln
   465                     470                     475                     480
   Ala Ser Thr Asn Glu Lys Ala Thr Asn Lys Pro Lys Lys Ser Leu Pro
                   485                     490                     495
   Ser Thr Gly Ser Ile Ser Asn Leu Ala Leu Glu Ile Ala Gly Leu Leu
                   500                     505                     510
   Thr Leu Ala Gly Ala Thr Ile Leu Ala Lys Lys Arg Met Lys
               515                     520                     525
<210> 7
   <211> 1455
   <212> DNA
   <213> S. pneumoniae
<220>
   <221> CDS
   <222> (1)...(1452)
<400> 7
   atg aaa ttt agt aaa aaa tat ata gca gct gga tca gct gtt atc gta      48
   Met Lys Phe Ser Lys Lys Tyr Ile Ala Ala Gly Ser Ala Val Ile Val
    1              5                  10                  15
   tcc ttg agt cta tgt gcc tat gca cta aac cag cat cgt tcg cag gaa      96
   Ser Leu Ser Leu Cys Ala Tyr Ala Leu Asn Gln His Arg Ser Gln Glu
               20                  25                  30
   aat aag gac aat aat cgt gtc tct tat gtg gat ggc agc cag tca agt     144
   Asn Lys Asp Asn Asn Arg Val Ser Tyr Val Asp Gly Ser Gln Ser Ser
           35                  40                  45
   cag aaa agt gaa aac ttg aca cca gac cag gtt agc cag aaa gaa gga      192
   Gln Lys Ser Glu Asn Leu Thr Pro Asp Gln Val Ser Gln Lys Glu Gly
           50                  55                  60
   att cag gct gag caa att gta atc aaa att aca gat cag ggc tat gta      240
   Ile Gln Ala Glu Gln Ile Val Ile Lys Ile Thr Asp Gln Gly Tyr Val
   65                  70                  75                  80
   acg tca cac ggt gac cac tat cat tac tat aat ggg aaa gtt cct tat      288
   Thr Ser His Gly Asp His Tyr His Tyr Tyr Asn Gly Lys Val Pro Tyr
                   85                  90                  95
   gat gcc ctc ttt agt gaa gaa ctc ttg atg aag gat cca aac tat caa      336
   Asp Ala Leu Phe Ser Glu Glu Leu Leu Met Lys Asp Pro Asn Tyr Gln
               100                 105                 110
   ctt aaa gac gct gat att gtc aat gaa gtc aag ggt ggt tat atc atc      384
   Leu Lys Asp Ala Asp Ile Val Asn Glu Val Lys Gly Gly Tyr Ile Ile
           115                 120                 125
   aag gtc gat gga aaa tat tat gtc tac ctg aaa gat gca gct cat gct      432
   Lys Val Asp Gly Lys Tyr Tyr Val Tyr Leu Lys Asp Ala Ala His Ala
       130                 135                 140
   gat aat gtt cga act aaa gat gaa atc aat cgt caa aaa caa gaa cat      480
   Asp Asn Val Arg Thr Lys Asp Glu Ile Asn Arg Gln Lys Gln Glu His
   145                 150                 155                 160
   gtc aaa gat aat gag aag gtt aac tct aat gtt gct gta gca agg tct      528
   Val Lys Asp Asn Glu Lys Val Asn Ser Asn Val Ala Val Ala Arg Ser
                   165                 170                 175
   cag gga cga tat acg aca aat gat ggt tat gtc ttt aat cca gct gat      576
   Gln Gly Arg Tyr Thr Thr Asn Asp Gly Tyr Val Phe Asn Pro Ala Asp
               180                 185                 190
   att atc gaa gat acg ggt aat gct tat atc gtt cct cat gga ggt cac      624
   Ile Ile Glu Asp Thr Gly Asn Ala Tyr Ile Val Pro His Gly Gly His
           195                 200                 205
   tat cac tac att ccc aaa agc gat tta tct gct agt gaa tta gca gca      672
   Tyr His Tyr Ile Pro Lys Ser Asp Leu Ser Ala Ser Glu Leu Ala Ala
       210                 215                 220
   gct aaa gca cat ctg gct gga aaa aat atg caa ccg agt cag tta agc      720
   Ala Lys Ala His Leu Ala Gly Lys Asn Met Gln Pro Ser Gln Leu Ser
   225                 230                 235                 240
   tat tct tca aca gct agt gac aat aac acg caa tct gta gca aaa gga      768
   Tyr Ser Ser Thr Ala Ser Asp Asn Asn Thr Gln Ser Val Ala Lys Gly
                   245                 250                 255
   tca act agc aag cca gca aat aaa tct gaa aat ctc cag agt ctt ttg      816
   Ser Thr Ser Lys Pro Ala Asn Lys Ser Glu Asn Leu Gln Ser Leu Leu
               260                 265                 270
   aag gaa ctc tat gat tca cct agc gcc caa cgt tac agt gaa tca gat      864
   Lys Glu Leu Tyr Asp Ser Pro Ser Ala Gln Arg Tyr Ser Glu Ser Asp
           275                 280                 285
   ggc ctg gtc ttt gac cct gct aag att atc agt cgt aca cca aat gga      912
   Gly Leu Val Phe Asp Pro Ala Lys Ile Ile Ser Arg Thr Pro Asn Gly
       290                 295                 300
   gtt gcg att ccg cat ggc gac cat tac cac ttt att cct tac agc aag      960
   Val Ala Ile Pro His Gly Asp His Tyr His Phe Ile Pro Tyr Ser Lys
   305                 310                 315                 320
   ctt tct gct tta gaa gaa aag att gcc aga atg gtg cct atc agt gga     1008
   Leu Ser Ala Leu Glu Glu Lys Ile Ala Arg Met Val Pro Ile Ser Gly
                   325                 330                 335
   act ggt tct aca gtt tct aca aat gca aaa cct aat gaa gta gtg tct     1056
   Thr Gly Ser Thr Val Ser Thr Asn Ala Lys Pro Asn Glu Val Val Ser
               340                 345                 350
   agt cta ggc agt ctt tca agc aat cct tct tct tta acg aca agt aag     1104
   Ser Leu Gly Ser Leu Ser Ser Asn Pro Ser Ser Leu Thr Thr Ser Lys
           355                 360                 365
   gag ctc tct tca gca tct gat ggt tat att ttt aat cca aaa gat atc     1152
   Glu Leu Ser Ser Ala Ser Asp Gly Tyr Ile Phe Asn Pro Lys Asp Ile
       370                 375                 380
   gtt gaa gaa acg gct aca gct tat att gta aga cat ggt gat cat ttc     1200
   Val Glu Glu Thr Ala Thr Ala Tyr Ile Val Arg His Gly Asp His Phe
   385                 390                 395                 400
   cat tac att cca aaa tca aat caa att ggg caa ccg act ctt cca aac     1248
   His Tyr Ile Pro Lys Ser Asn Gln Ile Gly Gln Pro Thr Leu Pro Asn
                   405                 410                 415
   aat agt cta gca aca cct tct cca tct ctt cca atc aat cca gga act     1296
   Asn Ser Leu Ala Thr Pro Ser Pro Ser Leu Pro Ile Asn Pro Gly Thr
               420                 425                 430
   tca cat gag aaa cat gaa gaa gat gga tac gga ttt gat gct aat cgt     1344
   Ser His Glu Lys His Glu Glu Asp Gly Tyr Gly Phe Asp Ala Asn Arg
           435                 440                 445
   att atc gct gaa gat gaa tca ggt ttt gtc atg agt cac gga gac cac     1392
   Ile Ile Ala Glu Asp Glu Ser Gly Phe Val Met Ser His Gly Asp His
       450                 455                 460
   aat cat tat ttc ttc aag aag gac ttg aca gaa gag caa att aag gtg     1440
   Asn His Tyr Phe Phe Lys Lys Asp Leu Thr Glu Glu Gln Ile Lys Val
   465                 470                 475                 480
   cgc aaa aac att tag                                                 1455
   Arg Lys Asn Ile
<210> 8
   <211> 484
   <212> PRT
   <213> S. pneumoniae
<400> 8
   Met Lys Phe Ser Lys Lys Tyr Ile Ala Ala Gly Ser Ala Val Ile Val
   1               5                   10                  15
   Ser Leu Ser Leu Cys Ala Tyr Ala Leu Asn Gln His Arg Ser Gln Glu
               20                  25                  30
   Asn Lys Asp Asn Asn Arg Val Ser Tyr Val Asp Gly Ser Gln Ser Ser
           35                  40                  45
   Gln Lys Ser Glu Asn Leu Thr Pro Asp Gln Val Ser Gln Lys Glu Gly
       50                  55                  60
   Ile Gln Ala Glu Gln Ile Val Ile Lys Ile Thr Asp Gln Gly Tyr Val
   65                  70                  75                  80
   Thr Ser His Gly Asp His Tyr His Tyr Tyr Asn Gly Lys Val Pro Tyr
                   85                  90                  95
   Asp Ala Leu Phe Ser Glu Glu Leu Leu Met Lys Asp Pro Asn Tyr Gln
               100                 105                 110
   Leu Lys Asp Ala Asp Ile Val Asn Glu Val Lys Gly Gly Tyr Ile Ile
           115                 120                 125
   Lys Val Asp Gly Lys Tyr Tyr Val Tyr Leu Lys Asp Ala Ala His Ala
       130                 135                 140
   Asp Asn Val Arg Thr Lys Asp Glu Ile Asn Arg Gln Lys Gln Glu His
   145                 150                 155                 160
   Val Lys Asp Asn Glu Lys Val Asn Ser Asn Val Ala Val Ala Arg Ser
                   165                 170                 175
   Gln Gly Arg Tyr Thr Thr Asn Asp Gly Tyr Val Phe Asn Pro Ala Asp
               180                 185                 190
   Ile Ile Glu Asp Thr Gly Asn Ala Tyr Ile Val Pro His Gly Gly His
           195                 200                 205
   Tyr His Tyr Ile Pro Lys Ser Asp Leu Ser Ala Ser Glu Leu Ala Ala
       210                 215                 220
   Ala Lys Ala His Leu Ala Gly Lys Asn Met Gln Pro Ser Gln Leu Ser
   225                 230                 235                 240
   Tyr Ser Ser Thr Ala Ser Asp Asn Asn Thr Gln Ser Val Ala Lys Gly
                   245                 250                 255
   Ser Thr Ser Lys Pro Ala Asn Lys Ser Glu Asn Leu Gln Ser Leu Leu
               260                 265                 270
   Lys Glu Leu Tyr Asp Ser Pro Ser Ala Gln Arg Tyr Ser Glu Ser Asp
           275                 280                 285
   Gly Leu Val Phe Asp Pro Ala Lys Ile Ile Ser Arg Thr Pro Asn Gly
       290                 295                 300
   Val Ala Ile Pro His Gly Asp His Tyr His Phe Ile Pro Tyr Ser Lys
   305                 310                 315                 320
   Leu Ser Ala Leu Glu Glu Lys Ile Ala Arg Met Val Pro Ile Ser Gly
                   325                 330                 335
   Thr Gly Ser Thr Val Ser Thr Asn Ala Lys Pro Asn Glu Val Val Ser
               340                 345                 350
   Ser Leu Gly Ser Leu Ser Ser Asn Pro Ser Ser Leu Thr Thr Ser Lys
           355                 360                 365
   Glu Leu Ser Ser Ala Ser Asp Gly Tyr Ile Phe Asn Pro Lys Asp Ile
       370                 375                 380
   Val Glu Glu Thr Ala Thr Ala Tyr Ile Val Arg His Gly Asp His Phe
   385                 390                 395                 400
   His Tyr Ile Pro Lys Ser Asn Gln Ile Gly Gln Pro Thr Leu Pro Asn
                   405                 410                 415
   Asn Ser Leu Ala Thr Pro Ser Pro Ser Leu Pro Ile Asn Pro Gly Thr
               420                 425                 430
   Ser His Glu Lys His Glu Glu Asp Gly Tyr Gly Phe Asp Ala Asn Arg
           435                 440                 445
   Ile Ile Ala Glu Asp Glu Ser Gly Phe Val Met Ser His Gly Asp His
       450                 455                 460
   Asn His Tyr Phe Phe Lys Lys Asp Leu Thr Glu Glu Gln Ile Lys Val
   465                 470                 475                 480
   Arg Lys Asn Ile
<210> 9
   <211> 1587
   <212> DNA
   <213> S pneumoniae
<220>
   <221> CDS
   <222> (1)...(1584)
<400> 9
   atg aaa gat tta gat aaa aaa atc gaa gaa aaa att gct ggc att atg     48
   Met Lys Asp Leu Asp Lys Lys Ile Glu Glu Lys Ile Ala Gly Ile Met
    1               5                  10                  15
   aaa caa tat ggt gtc aaa cgt gaa agt att gtc gtg aat aaa gaa aaa     96
   Lys Gln Tyr Gly Val Lys Arg Glu Ser Ile Val Val Asn Lys Glu Lys
               20                  25                  30
   aat gcg att att tat ccg cat gga gat cac cat cat gca gat ccg att    144
   Asn Ala Ile Ile Tyr Pro His Gly Asp His His His Ala Asp Pro Ile
           35                  40                  45
   gat gaa cat aaa ccg gtt gga att ggt cat tct cac agt aac tat gaa    192
   Asp Glu His Lys Pro Val Gly Ile Gly His Ser His Ser Asn Tyr Glu
       50                  55                  60
   ctg ttt aaa ccc gaa gaa gga gtt gct aaa aaa gaa ggg aat aaa gtt    240
   Leu Phe Lys Pro Glu Glu Gly Val Ala Lys Lys Glu Gly Asn Lys Val
   65                  70                  75                  80
   tat act gga gaa gaa tta acg aat gtt gtt aat ttg tta aaa aat agt    288
   Tyr Thr Gly Glu Glu Leu Thr Asn Val Val Asn Leu Leu Lys Asn Ser
                   85                  90                  95
   acg ttt aat aat caa aac ttt act cta gcc aat ggt caa aaa cgc gtt    336
   Thr Phe Asn Asn Gln Asn Phe Thr Leu Ala Asn Gly Gln Lys Arg Val
               100                 105                 110
   tct ttt agt ttt ccg cct gaa ttg gag aaa aaa tta ggt atc aat atg    384
   Ser Phe Ser Phe Pro Pro Glu Leu Glu Lys Lys Leu Gly Ile Asn Met
           115                 120                 125
   cta gta aaa tta ata aca cca gat gga aaa gta ttg gag aaa gta tct    432
   Leu Val Lys Leu Ile Thr Pro Asp Gly Lys Val Leu Glu Lys Val Ser
       130                 135                 140
   ggt aaa gta ttt gga gaa gga gta ggg aat att gca aac ttt gaa tta    480
   Gly Lys Val Phe Gly Glu Gly Val Gly Asn Ile Ala Asn Phe Glu Leu
   145                 150                 155                 160
   gat caa cct tat tta cca gga caa aca ttt aag tat act atc gct tca      528
   Asp Gln Pro Tyr Leu Pro Gly Gln Thr Phe Lys Tyr Thr Ile Ala Ser
                   165                 170                 175
   aaa gat tat cca gaa gta agt tat gat ggt aca ttt aca gtt cca acc      576
   Lys Asp Tyr Pro Glu Val Ser Tyr Asp Gly Thr Phe Thr Val Pro Thr
               180                 185                 190
   tct tta gct tac aaa atg gcc agt caa acg att ttc tat cct ttc cat      624
   Ser Leu Ala Tyr Lys Met Ala Ser Gln Thr Ile Phe Tyr Pro Phe His
           195                 200                 205
   gca ggg gat act tat tta aga gtg aac cct caa ttt gca gtg cct aaa      672
   Ala Gly Asp Thr Tyr Leu Arg Val Asn Pro Gln Phe Ala Val Pro Lys
       210                 215                 220
   gga act gat gct tta gtc aga gtg ttt gat gaa ttt cat gga aat gct      720
   Gly Thr Asp Ala Leu Val Arg Val Phe Asp Glu Phe His Gly Asn Ala
   225                 230                 235                 240
   tat tta gaa aat aac tat aaa gtt ggt gaa atc aaa tta ccg att ccg      768
   Tyr Leu Glu Asn Asn Tyr Lys Val Gly Glu Ile Lys Leu Pro Ile Pro
                   245                 250                 255
   aaa tta aac caa gga aca acc aga acg gcc gga aat aaa att cct gta      816
   Lys Leu Asn Gln Gly Thr Thr Arg Thr Ala Gly Asn Lys Ile Pro Val
               260                 265                 270
   acc ttc atg gca aat gct tat ttg gac aat caa tcg act tat att gtg      864
   Thr Phe Met Ala Asn Ala Tyr Leu Asp Asn Gln Ser Thr Tyr Ile Val
           275                 280                 285
   gaa gta cct atc ttg gaa aaa gaa aat caa act gat aaa cca agt att      912
   Glu Val Pro Ile Leu Glu Lys Glu Asn Gln Thr Asp Lys Pro Ser Ile
       290                 295                 300
   cta cca caa ttt aaa agg aat aaa gca caa gaa aac tca aaa ctt gat      960
   Leu Pro Gln Phe Lys Arg Asn Lys Ala Gln Glu Asn Ser Lys Leu Asp
   305                 310                 315                 320
   gaa aag gta gaa gaa cca aag act agt gag aag gta gaa aaa gaa aaa      1008
   Glu Lys Val Glu Glu Pro Lys Thr Ser Glu Lys Val Glu Lys Glu Lys
                   325                 330                 335
   ctt tct gaa act ggg aat agt act agt aat tca acg tta gaa gaa gtt      1056
   Leu Ser Glu Thr Gly Asn Ser Thr Ser Asn Ser Thr Leu Glu Glu Val
               340                 345                 350
   cct aca gtg gat cct gta caa gaa aaa gta gca aaa ttt gct gaa agt      1104
   Pro Thr Val Asp Pro Val Gln Glu Lys Val Ala Lys Phe Ala Glu Ser
           355                 360                 365
   tat ggg atg aag cta gaa aat gtc ttg ttt aat atg gac gga aca att      1152
   Tyr Gly Met Lys Leu Glu Asn Val Leu Phe Asn Met Asp Gly Thr Ile
       370                 375                 380
   gaa tta tat tta cca tca gga gaa gtc att aaa aag aat atg gca gat      1200
   Glu Leu Tyr Leu Pro Ser Gly Glu Val Ile Lys Lys Asn Met Ala Asp
   385                 390                 395                 400
   ttt aca gga gaa gca cct caa gga aat ggt gaa aat aaa cca tct gaa      1248
   Phe Thr Gly Glu Ala Pro Gln Gly Asn Gly Glu Asn Lys Pro Ser Glu
                   405                 410                 415
   aat gga aaa gta tct act gga aca gtt gag aac caa cca aca gaa aat      1296
   Asn Gly Lys Val Ser Thr Gly Thr Val Glu Asn Gln Pro Thr Glu Asn
               420                 425                 430
   aaa cca gca gat tct tta cca gag gca cca aac gaa aaa cct gta aaa      1344
   Lys Pro Ala Asp Ser Leu Pro Glu Ala Pro Asn Glu Lys Pro Val Lys
           435                 440                 445
   cca gaa aac tca acg gat aat gga atg ttg aat cca gaa ggg aat gtg      1392
   Pro Glu Asn Ser Thr Asp Asn Gly Met Leu Asn Pro Glu Gly Asn Val
       450                 455                 460
   ggg agt gac cct atg tta gat cca gca tta gag gaa gct cca gca gta      1440
   Gly Ser Asp Pro Met Leu Asp Pro Ala Leu Glu Glu Ala Pro Ala Val
   465                 470                 475                 480
   gat cct gta caa gaa aaa tta gaa aaa ttt aca gct agt tac gga tta      1488
   Asp Pro Val Gln Glu Lys Leu Glu Lys Phe Thr Ala Ser Tyr Gly Leu
                   485                 490                 495
   ggc tta gat agt gtt ata ttc aat atg gat gga acg att gaa tta aga      1536
   Gly Leu Asp Ser Val Ile Phe Asn Met Asp Gly Thr Ile Glu Leu Arg
               500                 505                 510
   ttg cca agt gga gaa gtg ata aaa aag aat tta tct gat ttc ata gcg      1584
   Leu Pro Ser Gly Glu Val Ile Lys Lys Asn Leu Ser Asp Phe Ile Ala
           515                 520                 525
   taa                                                                  1587
<210> 10
   <211> 528
   <212> PRT
   <213> S pneumoniae
<400> 10
   Met Lys Asp Leu Asp Lys Lys Ile Glu Glu Lys Ile Ala Gly Ile Met
    1               5                   10                  15
   Lys Gln Tyr Gly Val Lys Arg Glu Ser Ile Val Val Asn Lys Glu Lys
               20                  25                  30
   Asn Ala Ile Ile Tyr Pro His Gly Asp His His His Ala Asp Pro Ile
           35                  40                  45
   Asp Glu His Lys Pro Val Gly Ile Gly His Ser His Ser Asn Tyr Glu
       50                  55                  60
   Leu Phe Lys Pro Glu Glu Gly Val Ala Lys Lys Glu Gly Asn Lys Val
   65                  70                  75                  80
   Tyr Thr Gly Glu Glu Leu Thr Asn Val Val Asn Leu Leu Lys Asn Ser
                   85                  90                  95
   Thr Phe Asn Asn Gln Asn Phe Thr Leu Ala Asn Gly Gln Lys Arg Val
               100                 105                 110
   Ser Phe Ser Phe Pro Pro Glu Leu Glu Lys Lys Leu Gly Ile Asn Met
           115                 120                 125
   Leu Val Lys Leu Ile Thr Pro Asp Gly Lys Val Leu Glu Lys Val Ser
       130                 135                 140
   Gly Lys Val Phe Gly Glu Gly Val Gly Asn Ile Ala Asn Phe Glu Leu
   145                 150                 155                 160
   Asp Gln Pro Tyr Leu Pro Gly Gln Thr Phe Lys Tyr Thr Ile Ala Ser
                   165                 170                 175
   Lys Asp Tyr Pro Glu Val Ser Tyr Asp Gly Thr Phe Thr Val Pro Thr
               180                 185                 190
   Ser Leu Ala Tyr Lys Met Ala Ser Gln Thr Ile Phe Tyr Pro Phe His
           195                 200                 205
   Ala Gly Asp Thr Tyr Leu Arg Val Asn Pro Gln Phe Ala Val Pro Lys
       210                 215                 220
   Gly Thr Asp Ala Leu Val Arg Val Phe Asp Glu Phe His Gly Asn Ala
   225                 230                 235                 240
   Tyr Leu Glu Asn Asn Tyr Lys Val Gly Glu Ile Lys Leu Pro Ile Pro
                   245                 250                 255
   Lys Leu Asn Gln Gly Thr Thr Arg Thr Ala Gly Asn Lys Ile Pro Val
               260                 265                 270
   Thr Phe Met Ala Asn Ala Tyr Leu Asp Asn Gln Ser Thr Tyr Ile Val
           275                 280                 285
   Glu Val Pro Ile Leu Glu Lys Glu Asn Gln Thr Asp Lys Pro Ser Ile
       290                 295                 300
   Leu Pro Gln Phe Lys Arg Asn Lys Ala Gln Glu Asn Ser Lys Leu Asp
   305                 310                 315                 320
   Glu Lys Val Glu Glu Pro Lys Thr Ser Glu Lys Val Glu Lys Glu Lys
                   325                 330                 335
   Leu Ser Glu Thr Gly Asn Ser Thr Ser Asn Ser Thr Leu Glu Glu Val
               340                 345                 350
   Pro Thr Val Asp Pro Val Gln Glu Lys Val Ala Lys Phe Ala Glu Ser
           355                 360                 365
   Tyr Gly Met Lys Leu Glu Asn Val Leu Phe Asn Met Asp Gly Thr Ile
       370                 375                 380
   Glu Leu Tyr Leu Pro Ser Gly Glu Val Ile Lys Lys Asn Met Ala Asp
   385                 390                 395                 400
   Phe Thr Gly Glu Ala Pro Gln Gly Asn Gly Glu Asn Lys Pro Ser Glu
                   405                 410                 415
   Asn Gly Lys Val Ser Thr Gly Thr Val Glu Asn Gln Pro Thr Glu Asn
               420                 425                 430
   Lys Pro Ala Asp Ser Leu Pro Glu Ala Pro Asn Glu Lys Pro Val Lys
           435                 440                 445
   Pro Glu Asn Ser Thr Asp Asn Gly Met Leu Asn Pro Glu Gly Asn Val
       450                 455                 460
   Gly Ser Asp Pro Met Leu Asp Pro Ala Leu Glu Glu Ala Pro Ala Val
   465                 470                 475                 480
   Asp Pro Val Gln Glu Lys Leu Glu Lys Phe Thr Ala Ser Tyr Gly Leu
                   485                 490                 495
   Gly Leu Asp Ser Val Ile Phe Asn Met Asp Gly Thr Ile Glu Leu Arg
               500                 505                 510
   Leu Pro Ser Gly Glu Val Ile Lys Lys Asn Leu Ser Asp Phe Ile Ala
           515                 520                 525
<210> 11
   <211> 5048
   <212> DNA
   <213> S. pneumoniae
<400> 11
   aattccttgt cgggtaagtt ccgacccgca cgaaaggcgt aatgatttgg gcactgtctc 60
   aacgagagac tcggtgaaat tttagtacct gtgaagatgc aggttacccg cgacaggacg 120
   gaaagacccc atggagcttt actgcagttt gatattgagt gtctgtacca catgtacagg 180
   ataggtagga gtctaagaga tcgggacgcc agtttcgaag gagacgctgt tgggatacta 240
   cccttgtgtt atggccactc taacccagat aggtgatccc tatcggagac agtgtctgac 300
   gggcagtttg actggggcgg tcgcctccta aaaggtaacg gaggcgccca aaggttccct 360
   cagaatggtt ggaaatcatt cgcagagtgt aaaggtataa gggagcttga ctgcgagagc 420
   tacaactcga gcagggacga aagtcgggct tagtgatccg gtggttccgt atggaagggc 480
   catcgctcaa cggataaaag ctaccctggg gataacaggc ttatctcccc caagagttca 540
   catcgacggg gaggtttggc acctcgatgt cggctcgtcg catcctgggg ctgtagtcgg 600
   tcccaagggt tgggctgttc gcccattaaa gcggcacgcg agctgggttc agaacgtcgt 660
   gagacagttc ggtccctatc cgtcgcgggc gtaggaaatt tgagaggatc tgctcctagt 720
   acgagaggac cagagtggac ttaccgctgg tgtaccagtt gtcttgccaa aggcatcgct 780
   gggtagctat gtagggaagg gataaacgct gaaagcatct aagtgtgaaa cccacctcaa 840
   gatgagattt cccatgatta tatatcagta agagccctga gagatgatca ggtagatagg 900
   ttagaagtgg aagtgtggcg acacatgtag cggactaata ctaatagctc gaggacttat 960
   ccaaagtaac tgagaatatg aaagcgaacg gttttcttaa attgaataga tattcaattt 1020
   tgagtaggta ttactcagag ttaagtgacg atagcctagg agatacacct gtacccatgc 1080
   cgaacacaga agttaagccc tagaacgccg gaagtagttg ggggttgccc cctgtgagat 1140
   agggaagtcg cttagctcta gggagtttag ctcagctggg agagcatctg ccttacaagc 1200
   agagggtcag cggttcgatc ccgttaactc ccaaaggtcc cgtagtgtag cggttatcac 1260
   gtcgccctgt cacggcgaag atcgcgggtt cgattcccgt cgggaccgtt taaggtaacg 1320
   caagttattt tagactcgtt agctcagttg gtagagcaat tgacttttaa tcaatgggtc 1380
   actggttcga gcccagtacg ggtcatatat gcgggtttgg cggaattcta atctctttga 1440
   aatcatcttc tctcactttc caaaactcta ttacctctta ttataccaca tttcaatctt 1500
   caacttccca gtaatataag cacctctggc gaaagaagtt tcaatgtcct aaagtaataa 1560
   gtgaatccaa ttcaggaact ccaagaacaa aagaaacatc tggtgtcaca agtattggat 1620
   ggcacagagt cacgtggtag tctgacccta gcagaaattt taaatagtaa actatttact 1680
   ggttaattaa atggttaaat aaccggttta gaaaactatt taataaagta aaagaagttg 1740
   agaaaaaact tcatcattta ttgaaatgag ggatttatga aatttagtaa aaaatatata 1800
   gcagctggat cagctgttat cgtatccttg agtctatgtg cctatgcact aaaccagcat 1860
   cgttcgcagg aaaataagga caataatcgt gtctcttatg tggatggcag ccagtcaagt 1920
   cagaaaagtg aaaacttgac accagaccag gttagccaga aagaaggaat tcaggctgag 1980
   caaattgtaa tcaaaattac agatcagggc tatgtaacgt cacacggtga ccactatcat 2040
   tactataatg ggaaagttcc ttatgatgcc ctctttagtg aagaactctt gatgaaggat 2100
   ccaaactatc aacttaaaga cgctgatatt gtcaatgaag tcaagggtgg ttatatcatc 2160
   aaggtcgatg gaaaatatta tgtctacctg aaagatgcag ctcatgctga taatgttcga 2220
   actaaagatg aaatcaatcg tcaaaaacaa gaacatgtca aagataatga gaaggttaac 2280
   tctaatgttg ctgtagcaag gtctcaggga cgatatacga caaatgatgg ttatgtcttt 2340
   aatccagctg atattatcga agatacgggt aatgcttata tcgttcctca tggaggtcac 2400
   tatcactaca ttcccaaaag cgatttatct gctagtgaat tagcagcagc taaagcacat 2460
   ctggctggaa aaaatatgca accgagtcag ttaagctatt cttcaacagc tagtgacaat 2520
   aacacgcaat ctgtagcaaa aggatcaact agcaagccag caaataaatc tgaaaatctc 2580
   cagagtcttt tgaaggaact ctatgattca cctagcgccc aacgttacag tgaatcagat 2640
   ggcctggtct ttgaccctgc taagattatc agtcgtacac caaatggagt tgcgattccg 2700
   catggcgacc attaccactt tattccttac agcaagcttt ctgctttaga agaaaagatt 2760
   gccagaatgg tgcctatcag tggaactggt tctacagttt ctacaaatgc aaaacctaat 2820
   gaagtagtgt ctagtctagg cagtctttca agcaatcctt cttctttaac gacaagtaag 2880
   gagctctctt cagcatctga tggttatatt tttaatccaa aagatatcgt tgaagaaacg 2940
   gctacagctt atattgtaag acatggtgat catttccatt acattccaaa atcaaatcaa 3000
   attgggcaac cgactcttcc aaacaatagt ctagcaacac cttctccatc tcttccaatc 3060
   aatccaggaa cttcacatga gaaacatgaa gaagatggat acggatttga tgctaatcgt 3120
   attatcgctg aagatgaatc aggttttgtc atgagtcacg gagaccacaa tcattatttc 3180
   ttcaagaagg acttgacaga agagcaaatt aaggctgcgc aaaaacattt agaggaagtt 3240
   aaaactagtc ataatggatt agattctttg tcatctcatg aacaggatta tccaggtaat 3300
   gccaaagaaa tgaaagattt agataaaaaa atcgaagaaa aaattgctgg cattatgaaa 3360
   caatatggtg tcaaacgtga aagtattgtc gtgaataaag aaaaaaatgc gattatttat 3420
   ccgcatggag atcaccatca tgcagatccg attgatgaac ataaaccggt tggaattggt 3480
   cattctcaca gtaactatga actgtttaaa cccgaagaag gagttgctaa aaaagaaggg 3540
   aataaagttt atactggaga agaattaacg aatgttgtta atttgttaaa aaatagtacg   3600
   tttaataatc aaaactttac tctagccaat ggtcaaaaac gcgtttcttt tagttttccg   3660
   cctgaattgg agaaaaaatt aggtatcaat atgctagtaa aattaataac accagatgga   3720
   aaagtattgg agaaagtatc tggtaaagta tttggagaag gagtagggaa tattgcaaac   3780
   tttgaattag atcaacctta tttaccagga caaacattta agtatactat cgcttcaaaa   3840
   gattatccag aagtaagtta tgatggtaca tttacagttc caacctcttt agcttacaaa   3900
   atggccagtc aaacgatttt ctatcctttc catgcagggg atacttattt aagagtgaac   3960
   cctcaatttg cagtgcctaa aggaactgat gctttagtca gagtgtttga tgaatttcat   4020
   ggaaatgctt atttagaaaa taactataaa gttggtgaaa tcaaattacc gattccgaaa   4080
   ttaaaccaag gaacaaccag aacggccgga aataaaattc ctgtaacctt catggcaaat   4140
   gcttatttgg acaatcaatc gacttatatt gtggaagtac ctatcttgga aaaagaaaat   4200
   caaactgata aaccaagtat tctaccacaa tttaaaagga ataaagcaca agaaaactca   4260
   aaacttgatg aaaaggtaga agaaccaaag actagtgaga aggtagaaaa agaaaaactt   4320
   tctgaaactg ggaatagtac tagtaattca acgttagaag aagttcctac agtggatcct   4380
   gtacaagaaa aagtagcaaa atttgctgaa agttatggga tgaagctaga aaatgtcttg   4440
   tttaatatgg acggaacaat tgaattatat ttaccatcag gagaagtcat taaaaagaat   4500
   atggcagatt ttacaggaga agcacctcaa ggaaatggtg aaaataaacc atctgaaaat   4560
   ggaaaagtat ctactggaac agttgagaac caaccaacag aaaataaacc agcagattct   4620
   ttaccagagg caccaaacga aaaacctgta aaaccagaaa actcaacgga taatggaatg   4680
   ttgaatccag aagggaatgt ggggagtgac cctatgttag atccagcatt agaggaagct   4740
   ccagcagtag atcctgtaca agaaaaatta gaaaaattta cagctagtta cggattaggc   4800
   ttagatagtg ttatattcaa tatggatgga acgattgaat taagattgcc aagtggagaa   4860
   gtgataaaaa agaatttatc tgatttcata gcgtaaggaa tagcagtaga aaaagtctga   4920
   atcaaaaatg aagttctctc aaaagttaga aataaaactc tgactttggg agaatttcat   4980
   tttattatta atatataaaa tttcttgaca tacaacttaa aaagaggtgg aatatttact   5040
   agttaatt                                                            5048
<210> 12
   <211> 2647
   <212> DNA
   <213> S. pneumoniae
<400> 12
   cagagatctt agtgaatcaa atatacttaa gaaaagagga aagaatgaaa atcaataaaa     60
   aatatctagc tgggtcagta gctacacttg ttttaagtgt ctgtgcttat gaactaggtt    120
   tgcatcaagc tcaaactgta aaagaaaata atcgtgtttc ctatatagat ggaaaacaag    180
   cgacgcaaaa aacggagaat ttgactcctg atgaggttag caagcgtgaa ggaatcaacg    240
   ccgaacaaat cgtcatcaag attacggatc aaggttatgt gacctctcat ggagaccatt    300
   atcattacta taatggcaag gtcccttatg atgccatcat cagtgaagag ctcctcatga    360
   aagatccgaa ttatcagttg aaggattcag acattgtcaa tgaaatcaag ggtggttatg    420
   tcattaaggt aaacggtaaa tactatgttt accttaagga tgcagctcat gcggataatg    480
   tccgtacaaa agaagaaatc aatcggcaaa aacaagaaca tagtcagcat cgtgaaggag    540
   ggacttcagc aaacgatggt gcggtagcct ttgcacgttc acagggacgc tacaccacag    600
   atgatggtta tatcttcaat gcatctgata tcatcgaaga tacgggcgat gcctatatcg    660
   ttcctcatgg agatcattac cattacattc ctaagaatga gttatcagct agcgagttgg    720
   ctgctgcaga agccttccta tctggtcggg aaaatctgtc aaatttaaga acctatcgcc    780
   gacaaaatag cgataacact ccaagaacaa actgggtacc ttctgtaagc aatccaggaa    840
   ctacaaatac taacacaagc aacaacagca acactaacag tcaagcaagt caaagtaatg    900
   acattgatag tctcttgaaa cagctctaca aactgccttt gagtcaacgc catgtagaat    960
   ctgatggcct tattttcgac ccagcgcaaa tcacaagtcg aaccgccaga ggtgtagctg   1020
   tccctcatgg taaccattac cactttatcc cttatgaaca aatgtctgaa ttggaaaaac   1080
   gaattgctcg tattattccc cttcgttatc gttcaaacca ttgggtacca gattcaagac   1140
   cagaagaacc aagtccacaa ccgactccag aacctagtcc aagtccgcaa cctgcaccaa   1200
   atcctcaacc agctccaagc aatccaattg atgagaaatt ggtcaaagaa gctgttcgaa   1260
   aagtaggcga tggttatgtc tttgaggaga atggagtttc tcgttatatc ccagccaaga   1320
   atctttcagc agaaacagca gcaggcattg atagcaaact ggccaagcag gaaagtttat   1380
   ctcataagct aggagctaag aaaactgacc tcccatctag tgatcgagaa ttttacaata   1440
   aggcttatga cttactagca agaattcacc aagatttact tgataataaa ggtcgacaag   1500
   ttgattttga ggctttggat aacctgttgg aacgactcaa ggatgtctca agtgataaag   1560
   tcaagttagt ggatgatatt cttgccttct tagctccgat tcgtcatcca gaacgtttag   1620
   gaaaaccaaa tgcgcaaatt acctacactg atgatgagat tcaagtagcc aagttggcag   1680
   gcaagtacac aacagaagac ggttatatct ttgatcctcg tgatataacc agtgatgagg   1740
   gggatgccta tgtaactcca catatgaccc atagccactg gattaaaaaa gatagtttgt   1800
   ctgaagctga gagagcggca gcccaggctt atgctaaaga gaaaggtttg acccctcctt   1860
   cgacagacca tcaggattca ggaaatactg aggcaaaagg agcagaagct atctacaacc   1920
   gcgtgaaagc agctaagaag gtgccacttg atcgtatgcc ttacaatctt caatatactg   1980
   tagaagtcaa aaacggtagt ttaatcatac ctcattatga ccattaccat aacatcaaat   2040
   ttgagtggtt tgacgaaggc ctttatgagg cacctaaggg gtatactctt gaggatcttt   2100
   tggcgactgt caagtactat gtcgaacatc caaacgaacg tccgcattca gataatggtt   2160
   ttggtaacgc tagcgaccat gttcaaagaa acaaaaatgg tcaagctgat accaatcaaa   2220
   cggaaaaacc aagcgaggag aaacctcaga cagaaaaacc tgaggaagaa acccctcgag   2280
   aagagaaacc acaaagcgag aaaccagagt ctccaaaacc aacagaggaa ccagaagaag   2340
   aatcaccaga ggaatcagaa gaacctcagg tcgagactga aaaggttgaa gaaaaactga   2400
   gagaggctga agatttactt ggaaaaatcc aggatccaat tatcaagtcc aatgccaaag   2460
   agactctcac aggattaaaa aataatttac tatttggcac ccaggacaac aatactatta   2520
   tggcagaagc tgaaaaacta ttggctttat taaaggagag taagtaaagg tagcagcatt   2580
   ttctaactcc taaaaacagg ataggagaac gggaaaacga aaaatgagag cagaatgtga   2640
   gttctag                                                             2647
<210> 13
   <211> 2639
   <212> DNA
   <213> S. pneumoniae
<220>
   <221> CDS
   <222> (114) ... (2627)
<400> 13
   gggtcttaaa actctgaatc ctttagaggc agacccacaa aatgacaaga cctatttaga      60
   aaatctggaa gaaaatatga gtgttctagc agaagaatta aagtgaggaa aga atg        116
                                                              Met
                                                              1
   aaa atc aat aaa aaa tat cta gca ggt tca gtg gca gtc ctt gcc cta      164
   Lys Ile Asn Lys Lys Tyr Leu Ala Gly Ser Val Ala Val Leu Ala Leu
               5                   10                  15
   agt gtt tgt tcc tat gaa ctt ggt cgt cac caa gct ggt cag gtt aag      212
   Ser Val Cys Ser Tyr Glu Leu Gly Arg His Gln Ala Gly Gln Val Lys
           20                  25                  30
   aaa gag tct aat cga gtt tct tat ata gat ggt gat cag gct ggt caa      260
   Lys Glu Ser Asn Arg Val Ser Tyr Ile Asp Gly Asp Gln Ala Gly Gln
       35                  40                  45
   aag gca gaa aat ttg aca cca gat gaa gtc agt aag aga gag ggg atc      308
   Lys Ala Glu Asn Leu Thr Pro Asp Glu Val Ser Lys Arg Glu Gly Ile
   50                  55                  60                  65
   aac gcc gaa caa att gtt atc aag att acg gat caa ggt tat gtg acc      356
   Asn Ala Glu Gln Ile Val Ile Lys Ile Thr Asp Gln Gly Tyr Val Thr
                   70                  75                  80
   tct cat gga gac cat tat cat tac tat aat ggc aag gtt cct tat gat      404
   Ser His Gly Asp His Tyr His Tyr Tyr Asn Gly Lys Val Pro Tyr Asp
               85                  90                  95
   gcc atc atc agt gaa gaa ctt ctc atg aaa gat ccg aat tat cag ttg      452
   Ala Ile Ile Ser Glu Glu Leu Leu Met Lys Asp Pro Asn Tyr Gln Leu
           100                 105                 110
   aag gat tca gac att gtc aat gaa atc aag ggt ggc tat gtg att aag      500
   Lys Asp Ser Asp Ile Val Asn Glu Ile Lys Gly Gly Tyr Val Ile Lys
       115                 120                 125
   gta gac gga aaa tac tat gtt tac ctt aaa gat gcg gcc cat gcg gac      548
   Val Asp Gly Lys Tyr Tyr Val Tyr Leu Lys Asp Ala Ala His Ala Asp
   130                 135                 140                 145
   aat att cgg aca aaa gaa gag att aaa cgt cag aag cag gaa cac agt      596
   Asn Ile Arg Thr Lys Glu Glu Ile Lys Arg Gln Lys Gln Glu His Ser
                   150                 155                 160
   cat aat cat aac tca aga gca gat aat gct gtt gct gca gcc aga gcc      644
   His Asn His Asn Ser Arg Ala Asp Asn Ala Val Ala Ala Ala Arg Ala
               165                 170                 175
   caa gga cgt tat aca acg gat gat ggg tat atc ttc aat gca tct gat      692
   Gln Gly Arg Tyr Thr Thr Asp Asp Gly Tyr Ile Phe Asn Ala Ser Asp
           180                 185                 190
   atc att gag gac acg ggt gat gct tat atc gtt cct cac ggc gac cat      740
   Ile Ile Glu Asp Thr Gly Asp Ala Tyr Ile Val Pro His Gly Asp His
       195                 200                 205
   tac cat tac att cct aag aat gag tta tca gct agc gag tta gct gct      788
   Tyr His Tyr Ile Pro Lys Asn Glu Leu Ser Ala Ser Glu Leu Ala Ala
   210                 215                 220                 225
   gca gaa gcc tat tgg aat ggg aag cag gga tct cgt cct tct tca agt      836
   Ala Glu Ala Tyr Trp Asn Gly Lys Gln Gly Ser Arg Pro Ser Ser Ser
                   230                 235                 240
   tct agt tat aat gca aat cca gtt caa cca aga ttg tca gag aac cac      884
   Ser Ser Tyr Asn Ala Asn Pro Val Gln Pro Arg Leu Ser Glu Asn His
               245                 250                 255
   aat ctg act gtc act cca act tat cat caa aat caa ggg gaa aac att      932
   Asn Leu Thr Val Thr Pro Thr Tyr His Gln Asn Gln Gly Glu Asn Ile
           260                 265                 270
   tca agc ctt tta cgt gaa ttg tat gct aaa ccc tta tca gaa cgc cat      980
   Ser Ser Leu Leu Arg Glu Leu Tyr Ala Lys Pro Leu Ser Glu Arg His
       275                 280                 285
   gta gaa tct gat ggc ctt att ttc gac cca gcg caa atc aca agt cga     1028
   Val Glu Ser Asp Gly Leu Ile Phe Asp Pro Ala Gln Ile Thr Ser Arg
   290                 295                 300                 305
   acc gcc aga ggt gta gct gtc cct cat ggt aac cat tac cac ttt atc     1076
   Thr Ala Arg Gly Val Ala Val Pro His Gly Asn His Tyr His Phe Ile
                   310                 315                 320
   cct tat gaa caa atg tct gaa ttg gaa aaa cga att gct cgt att att     1124
   Pro Tyr Glu Gln Met Ser Glu Leu Glu Lys Arg Ile Ala Arg Ile Ile
               325                 330                 335
   ccc ctt cgt tat cgt tca aac cat tgg gta cca gat tca aga cca gaa     1172
   Pro Leu Arg Tyr Arg Ser Asn His Trp Val Pro Asp Ser Arg Pro Glu
           340                 345                 350
   caa cca agt cca caa tcg act ccg gaa cct agt cca agt ctg caa cct     1220
   Gln Pro Ser Pro Gln Ser Thr Pro Glu Pro Ser Pro Ser Leu Gln Pro
       355                 360                 365
   gca cca aat cct caa cca gct cca agc aat cca att gat gag aaa ttg     1268
   Ala Pro Asn Pro Gln Pro Ala Pro Ser Asn Pro Ile Asp Glu Lys Leu
   370                 375                 380                 385
   gtc aaa gaa gct gtt cga aaa gta ggc gat ggt tat gtc ttt gag gag     1316
   Val Lys Glu Ala Val Arg Lys Val Gly Asp Gly Tyr Val Phe Glu Glu
                   390                 395                 400
   aat gga gtt tct cgt tat atc cca gcc aag gat ctt tca gca gaa aca     1364
   Asn Gly Val Ser Arg Tyr Ile Pro Ala Lys Asp Leu Ser Ala Glu Thr
               405                 410                 415
   gca gca ggc att gat agc aaa ctg gcc aag cag gaa agt tta tct cat     1412
   Ala Ala Gly Ile Asp Ser Lys Leu Ala Lys Gln Glu Ser Leu Ser His
           420                 425                 430
   aag cta gga gct aag aaa act gac ctc cca tct agt gat cga gaa ttt     1460
   Lys Leu Gly Ala Lys Lys Thr Asp Leu Pro Ser Ser Asp Arg Glu Phe
       435                 440                 445
   tac aat aag gct tat gac tta cta gca aga att cac caa gat tta ctt     1508
   Tyr Asn Lys Ala Tyr Asp Leu Leu Ala Arg Ile His Gln Asp Leu Leu
   450                 455                 460                 465
   gat aat aaa ggt cga caa gtt gat ttt gag gtt ttg gat aac ctg ttg     1556
   Asp Asn Lys Gly Arg Gln Val Asp Phe Glu Val Leu Asp Asn Leu Leu
                   470                 475                 480
   gaa cga ctc aag gat gtc tca agt gat aaa gtc aag tta gtg gat gat     1604
   Glu Arg Leu Lys Asp Val Ser Ser Asp Lys Val Lys Leu Val Asp Asp
               485                 490                 495
   att ctt gcc ttc tta gct ccg att cgt cat cca gaa cgt tta gga aaa     1652
   Ile Leu Ala Phe Leu Ala Pro Ile Arg His Pro Glu Arg Leu Gly Lys
           500                 505                 510
   cca aat gcg caa att acc tac act gat gat gag att caa gta gcc aag     1700
   Pro Asn Ala Gln Ile Thr Tyr Thr Asp Asp Glu Ile Gln Val Ala Lys
       515                 520                 525
   ttg gca ggc aag tac aca aca gaa gac ggt tat atc ttt gat cct cgt     1748
   Leu Ala Gly Lys Tyr Thr Thr Glu Asp Gly Tyr Ile Phe Asp Pro Arg
   530                 535                 540                 545
   gat ata acc agt gat gag ggg gat gcc tat gta act cca cat atg acc     1796
   Asp Ile Thr Ser Asp Glu Gly Asp Ala Tyr Val Thr Pro His Met Thr
                   550                 555                 560
   cat agc cac tgg att aaa aaa gat agt ttg tct gaa gct gag aga gcg      1844
   His Ser His Trp Ile Lys Lys Asp Ser Leu Ser Glu Ala Glu Arg Ala
                   565                 570                 575
   gca gcc cag gct tat gct aaa gag aaa ggt ttg acc cct cct tcg aca      1892
   Ala Ala Gln Ala Tyr Ala Lys Glu Lys Gly Leu Thr Pro Pro Ser Thr
               580                 585                 590
   gac cac cag gat tca gga aat act gag gca aaa gga gca gaa gct atc      1940
   Asp His Gln Asp Ser Gly Asn Thr Glu Ala Lys Gly Ala Glu Ala Ile
           595                 600                 605
   tac aac cgc gtg aaa gca gct aag aag gtg cca ctt gat cgt atg cct      1988
   Tyr Asn Arg Val Lys Ala Ala Lys Lys Val Pro Leu Asp Arg Met Pro
   610                 615                 620                 625
   tac aat ctt caa tat act gta gaa gtc aaa aac ggt agt tta atc ata      2036
   Tyr Asn Leu Gln Tyr Thr Val Glu Val Lys Asn Gly Ser Leu Ile Ile
                   630                 635                 640
   cct cat tat gac cat tac cat aac atc aaa ttt gag tgg ttt gac gaa      2084
   Pro His Tyr Asp His Tyr His Asn Ile Lys Phe Glu Trp Phe Asp Glu
               645                 650                 655
   ggc ctt tat gag gca cct aag ggg tat agt ctt gag gat ctt ttg gcg      2132
   Gly Leu Tyr Glu Ala Pro Lys Gly Tyr Ser Leu Glu Asp Leu Leu Ala
           660                 665                 670
   act gtc aag tac tat gtc gaa cat cca aac gaa cgt ccg cat tca gat      2180
   Thr Val Lys Tyr Tyr Val Glu His Pro Asn Glu Arg Pro His Ser Asp
       675                 680                 685
   aat ggt ttt ggt aac gct agt gac cat gtt cgt aaa aat aag gca gac      2228
   Asn Gly Phe Gly Asn Ala Ser Asp His Val Arg Lys Asn Lys Ala Asp
   690                 695                 700                 705
   caa gat agt aaa cct gat gaa gat aag gaa cat gat gaa gta agt gag      2276
   Gln Asp Ser Lys Pro Asp Glu Asp Lys Glu His Asp Glu Val Ser Glu
                   710                 715                 720
   cca act cac cct gaa tct gat gaa aaa gag aat cac gct ggt tta aat      2324
   Pro Thr His Pro Glu Ser Asp Glu Lys Glu Asn His Ala Gly Leu Asn
               725                 730                 735
   cct tca gca gat aat ctt tat aaa cca agc act gat acg gaa gag aca      2372
   Pro Ser Ala Asp Asn Leu Tyr Lys Pro Ser Thr Asp Thr Glu Glu Thr
           740                 745                 750
   gag gaa gaa gct gaa gat acc aca gat gag gct gaa att cct caa gta      2420
   Glu Glu Glu Ala Glu Asp Thr Thr Asp Glu Ala Glu Ile Pro Gln Val
       755                 760                 765
   gag aat tct gtt att aac gct aag ata gca gat gcg gag gcc ttg cta      2468
   Glu Asn Ser Val Ile Asn Ala Lys Ile Ala Asp Ala Glu Ala Leu Leu
   770                 775                 780                 785
   gaa aaa gta aca gat cct agt att aga caa aat gct atg gag aca ttg      2516
   Glu Lys Val Thr Asp Pro Ser Ile Arg Gln Asn Ala Met Glu Thr Leu
                   790                 795                 800
   act ggt cta aaa agt agt ctt ctt ctc gga acg aaa gat aat aac act      2564
   Thr Gly Leu Lys Ser Ser Leu Leu Leu Gly Thr Lys Asp Asn Asn Thr
               805                 810                 815
   att tca gca gaa gta gat agt ctc ttg gct ttg tta aaa gaa agt caa      2612
   Ile Ser Ala Glu Val Asp Ser Leu Leu Ala Leu Leu Lys Glu Ser Gln
           820                 825                 830
   ccg gct cct ata cag tagtaaaatg aa                                    2639
   Pro Ala Pro Ile Gln
       835
<210> 14
   <211> 838
   <212> PRT
   <213> S. pneumoniae
<400> 14
   Met Lys Ile Asn Lys Lys Tyr Leu Ala Gly Ser Val Ala Val Leu Ala
   1               5                   10                  15
   Leu Ser Val Cys Ser Tyr Glu Leu Gly Arg His Gln Ala Gly Gln Val
               20                  25                  30
   Lys Lys Glu Ser Asn Arg Val Ser Tyr Ile Asp Gly Asp Gln Ala Gly
           35                  40                  45
   Gln Lys Ala Glu Asn Leu Thr Pro Asp Glu Val Ser Lys Arg Glu Gly
       50                  55                  60
   Ile Asn Ala Glu Gln Ile Val Ile Lys Ile Thr Asp Gln Gly Tyr Val
   65                  70                  75                  80
   Thr Ser His Gly Asp His Tyr His Tyr Tyr Asn Gly Lys Val Pro Tyr
                   85                  90                  95
   Asp Ala Ile Ile Ser Glu Glu Leu Leu Met Lys Asp Pro Asn Tyr Gln
               100                 105                 110
   Leu Lys Asp Ser Asp Ile Val Asn Glu Ile Lys Gly Gly Tyr Val Ile
           115                 120                 125
   Lys Val Asp Gly Lys Tyr Tyr Val Tyr Leu Lys Asp Ala Ala His Ala
       130                 135                 140
   Asp Asn Ile Arg Thr Lys Glu Glu Ile Lys Arg Gln Lys Gln Glu His
   145                 150                 155                 160
   Ser His Asn His Asn Ser Arg Ala Asp Asn Ala Val Ala Ala Ala Arg
                   165                 170                 175
   Ala Gln Gly Arg Tyr Thr Thr Asp Asp Gly Tyr Ile Phe Asn Ala Ser
               180                 185                 190
   Asp Ile Ile Glu Asp Thr Gly Asp Ala Tyr Ile Val Pro His Gly Asp
           195                 200                 205
   His Tyr His Tyr Ile Pro Lys Asn Glu Leu Ser Ala Ser Glu Leu Ala
       210                 215                 220
   Ala Ala Glu Ala Tyr Trp Asn Gly Lys Gln Gly Ser Arg Pro Ser Ser
   225                 230                 235                 240
   Ser Ser Ser Tyr Asn Ala Asn Pro Val Gln Pro Arg Leu Ser Glu Asn
                   245                 250                 255
   His Asn Leu Thr Val Thr Pro Thr Tyr His Gln Asn Gln Gly Glu Asn
               260                 265                 270
   Ile Ser Ser Leu Leu Arg Glu Leu Tyr Ala Lys Pro Leu Ser Glu Arg
           275                 280                 285
   His Val Glu Ser Asp Gly Leu Ile Phe Asp Pro Ala Gln Ile Thr Ser
   290 295 300
   Arg Thr Ala Arg Gly Val Ala Val Pro His Gly Asn His Tyr His Phe
   305 310 315 320
   Ile Pro Tyr Glu Gln Met Ser Glu Leu Glu Lys Arg Ile Ala Arg Ile
   325 330 335
   Ile Pro Leu Arg Tyr Arg Ser Asn His Trp Val Pro Asp Ser Arg Pro
   340 345 350
   Glu Gln Pro Ser Pro Gln Ser Thr Pro Glu Pro Ser Pro Ser Leu Gln
   355 360 365
   Pro Ala Pro Asn Pro Gln Pro Ala Pro Ser Asn Pro Ile Asp Glu Lys
   370 375 380
   Leu Val Lys Glu Ala Val Arg Lys Val Gly Asp Gly Tyr Val Phe Glu
   385 390 395 400
   Glu Asn Gly Val Ser Arg Tyr Ile Pro Ala Lys Asp Leu Ser Ala Glu
   405 410 415
   Thr Ala Ala Gly Ile Asp Ser Lys Leu Ala Lys Gln Glu Ser Leu Ser
   420 425 430
   His Lys Leu Gly Ala Lys Lys Thr Asp Leu Pro Ser Ser Asp Arg Glu
   435 440 445
   Phe Tyr Asn Lys Ala Tyr Asp Leu Leu Ala Arg Ile His Gln Asp Leu
   450 455 460
   Leu Asp Asn Lys Gly Arg Gln Val Asp Phe Glu Val Leu Asp Asn Leu
   465 470 475 480
   Leu Glu Arg Leu Lys Asp Val Ser Ser Asp Lys Val Lys Leu Val Asp
   485 490 495
   Asp Ile Leu Ala Phe Leu Ala Pro Ile Arg His Pro Glu Arg Leu Gly
   500 505 510
   Lys Pro Asn Ala Gln Ile Thr Tyr Thr Asp Asp Glu Ile Gln Val Ala
   515 520 525
   Lys Leu Ala Gly Lys Tyr Thr Thr Glu Asp Gly Tyr Ile Phe Asp Pro
   530 535 540
   Arg Asp Ile Thr Ser Asp Glu Gly Asp Ala Tyr Val Thr Pro His Met
   545 550 555 560
   Thr His Ser His Trp Ile Lys Lys Asp Ser Leu Ser Glu Ala Glu Arg
   565 570 575
   Ala Ala Ala Gln Ala Tyr Ala Lys Glu Lys Gly Leu Thr Pro Pro Ser
   580 585 590
   Thr Asp His Gln Asp Ser Gly Asn Thr Glu Ala Lys Gly Ala Glu Ala
   595 600 605
   Ile Tyr Asn Arg Val Lys Ala Ala Lys Lys Val Pro Leu Asp Arg Met
   610 615 620
   Pro Tyr Asn Leu Gln Tyr Thr Val Glu Val Lys Asn Gly Ser Leu Ile
   625 630 635 640
   Ile Pro His Tyr Asp His Tyr His Asn Ile Lys Phe Glu Trp Phe Asp
   645 650 655
   Glu Gly Leu Tyr Glu Ala Pro Lys Gly Tyr Ser Leu Glu Asp Leu Leu
   660 665 670
   Ala Thr Val Lys Tyr Tyr Val Glu His Pro Asn Glu Arg Pro His Ser
   675 680 685
   Asp Asn Gly Phe Gly Asn Ala Ser Asp His Val Arg Lys Asn Lys Ala
   690 695 700
   Asp Gln Asp Ser Lys Pro Asp Glu Asp Lys Glu His Asp Glu Val Ser
   705 710 715 720
   Glu Pro Thr His Pro Glu Ser Asp Glu Lys Glu Asn His Ala Gly Leu
   725 730 735
   Asn Pro Ser Ala Asp Asn Leu Tyr Lys Pro Ser Thr Asp Thr Glu Glu
   740 745 750
   Thr Glu Glu Glu Ala Glu Asp Thr Thr Asp Glu Ala Glu Ile Pro Gln
           755                 760                 765
   Val Glu Asn Ser Val Ile Asn Ala Lys Ile Ala Asp Ala Glu Ala Leu
       770                 775                 780
   Leu Glu Lys Val Thr Asp Pro Ser Ile Arg Gln Asn Ala Met Glu Thr
   785                 790                 795                 800
   Leu Thr Gly Leu Lys Ser Ser Leu Leu Leu Gly Thr Lys Asp Asn Asn
                   805                 810                 815
   Thr Ile Ser Ala Glu Val Asp Ser Leu Leu Ala Leu Leu Lys Glu Ser
               820                 825                 830
   Gln Pro Ala Pro Ile Gln
           835
<210> 15
   <211> 2528
   <212> DNA
   <213> S. pneumoniae
<220>
   <221> CDS
   <222> (1)...(2520)
<400> 15
   tgt gcc tat gca cta aac cag cat cgt tcg cag gaa aat aag gac aat      48
   Cys Ala Tyr Ala Leu Asn Gln His Arg Ser Gln Glu Asn Lys Asp Asn
    1               5                  10                  15
   aat cgt gtc tct tat gtg gat ggc agc cag tca agt cag aaa agt gaa      96
   Asn Arg Val Ser Tyr Val Asp Gly Ser Gln Ser Ser Gln Lys Ser Glu
               20                  25                  30
   aac ttg aca cca gac cag gtt agc cag aaa gaa gga att cag gct gag     144
   Asn Leu Thr Pro Asp Gln Val Ser Gln Lys Glu Gly Ile Gln Ala Glu
           35                  40                  45
   caa att gta atc aaa att aca gat cag ggc tat gta acg tca cac ggt     192
   Gln Ile Val Ile Lys Ile Thr Asp Gln Gly Tyr Val Thr Ser His Gly
       50                  55                  60
   gat cac tat cat tac tat aat ggg aaa gtt cct tat gat gcc ctc ttt     240
   Asp His Tyr His Tyr Tyr Asn Gly Lys Val Pro Tyr Asp Ala Leu Phe
   65                  70                  75                  80
   agt gaa gaa ctc ttg atg aag gat cca aac tat caa ctt aaa gac gct     288
   Ser Glu Glu Leu Leu Met Lys Asp Pro Asn Tyr Gln Leu Lys Asp Ala
                   85                  90                  95
   gat att gtc aat gaa gtc aag ggt ggt tat atc atc aag gtc gat gga     336
   Asp Ile Val Asn Glu Val Lys Gly Gly Tyr Ile Ile Lys Val Asp Gly
               100                 105                 110
   aaa tat tat gtc tac ctg aaa gat gca gct cat gct gat aat gtt cga     384
   Lys Tyr Tyr Val Tyr Leu Lys Asp Ala Ala His Ala Asp Asn Val Arg
           115                 120                 125
   act aaa gat gaa atc aat cgt caa aaa caa gaa cat gtc aaa gat aat     432
   Thr Lys Asp Glu Ile Asn Arg Gln Lys Gln Glu His Val Lys Asp Asn
       130                 135                 140
   gag aag gtt aac tct aat gtt gct gta gca agg tct cag gga cga tat      480
   Glu Lys Val Asn Ser Asn Val Ala Val Ala Arg Ser Gln Gly Arg Tyr
   145                 150                 155                 160
   acg aca aat gat ggt tat gtc ttt aat cca gct gat att atc gaa gat      528
   Thr Thr Asn Asp Gly Tyr Val Phe Asn Pro Ala Asp Ile Ile Glu Asp
                   165                 170                 175
   acg ggt aat gct tat atc gtt cct cat gga ggt cac tat cac tac att      576
   Thr Gly Asn Ala Tyr Ile Val Pro His Gly Gly His Tyr His Tyr Ile
               180                 185                 190
   ccc aaa agc gat tta tct gct agt gaa tta gca gca gct aaa gca cat      624
   Pro Lys Ser Asp Leu Ser Ala Ser Glu Leu Ala Ala Ala Lys Ala His
           195                 200                 205
   ctg gct gga aaa aat atg caa ccg agt cag tta agc tat tct tca aca      672
   Leu Ala Gly Lys Asn Met Gln Pro Ser Gln Leu Ser Tyr Ser Ser Thr
       210                 215                 220
   cct tct cca tct ctt cca atc aat cca gga act tca cat gag aaa cat      720
   Pro Ser Pro Ser Leu Pro Ile Asn Pro Gly Thr Ser His Glu Lys His
   225                 230                 235                 240
   gaa gaa gat gga tac gga ttt gat gct aat cgt att atc gct gaa gat      768
   Glu Glu Asp Gly Tyr Gly Phe Asp Ala Asn Arg Ile Ile Ala Glu Asp
                   245                 250                 255
   gaa tca ggt ttt gtc atg agt cac gga gac cac aat cat tat ttc ttc      816
   Glu Ser Gly Phe Val Met Ser His Gly Asp His Asn His Tyr Phe Phe
               260                 265                 270
   aag aag gac ttg aca gaa gag caa att aag gct gcg caa aaa cat tta      864
   Lys Lys Asp Leu Thr Glu Glu Gln Ile Lys Ala Ala Gln Lys His Leu
           275                 280                 285
   gag gaa gtt aaa act agt cat aat gga tta gat tct ttg tca tct cat      912
   Glu Glu Val Lys Thr Ser His Asn Gly Leu Asp Ser Leu Ser Ser His
       290                 295                 300
   gaa cag gat tat cca agt aat gcc aaa gaa atg aaa gat tta gat aaa      960
   Glu Gln Asp Tyr Pro Ser Asn Ala Lys Glu Met Lys Asp Leu Asp Lys
   305                 310                 315                 320
   aaa atc gaa gaa aaa att gct ggc att atg aaa caa tat ggt gtc aaa     1008
   Lys Ile Glu Glu Lys Ile Ala Gly Ile Met Lys Gln Tyr Gly Val Lys
                   325                 330                 335
   cgt gaa agt att gtc gtg aat aaa gaa aaa aat gcg att att tat ccg     1056
   Arg Glu Ser Ile Val Val Asn Lys Glu Lys Asn Ala Ile Ile Tyr Pro
               340                 345                 350
   cat gga gat cac cat cat gca gat ccg att gat gaa cat aaa ccg gtt     1104
   His Gly Asp His His His Ala Asp Pro Ile Asp Glu His Lys Pro Val
           355                 360                 365
   gga att ggt cat tct cac agt aac tat gaa ctg ttt aaa ccc gaa gaa     1152
   Gly Ile Gly His Ser His Ser Asn Tyr Glu Leu Phe Lys Pro Glu Glu
       370                 375                 380
   gga gtt gct aaa aaa gaa ggg aat aaa gtt tat act gga gaa gaa tta      1200
   Gly Val Ala Lys Lys Glu Gly Asn Lys Val Tyr Thr Gly Glu Glu Leu
   385                 390                 395                 400
   acg aat gtt gtt aat ttg tta aaa aat agt acg ttt aat aat caa aac      1248
   Thr Asn Val Val Asn Leu Leu Lys Asn Ser Thr Phe Asn Asn Gln Asn
                   405                 410                 415
   ttt act cta gcc aat ggt caa aaa cgc gtt tct ttt agt ttt ccg cct      1296
   Phe Thr Leu Ala Asn Gly Gln Lys Arg Val Ser Phe Ser Phe Pro Pro
               420                 425                 430
   gaa ttg gag aaa aaa tta ggt atc aat atg cta gta aaa tta ata aca      1344
   Glu Leu Glu Lys Lys Leu Gly Ile Asn Met Leu Val Lys Leu Ile Thr
           435                 440                 445
   cca gat gga aaa gta ttg gag aaa gta tct ggt aaa gta ttt gga gaa      1392
   Pro Asp Gly Lys Val Leu Glu Lys Val Ser Gly Lys Val Phe Gly Glu
       450                 455                 460
   gga gta ggg aat att gca aac ttt gaa tta gat caa cct tat tta cca      1440
   Gly Val Gly Asn Ile Ala Asn Phe Glu Leu Asp Gln Pro Tyr Leu Pro
   465                 470                 475                 480
   gga caa aca ttt aag tat act atc gct tca aaa gat tat cca gaa gta      1488
   Gly Gln Thr Phe Lys Tyr Thr Ile Ala Ser Lys Asp Tyr Pro Glu Val
                   485                 490                 495
   agt tat gat ggt aca ttt aca gtt cca acc tct tta gct tac aaa atg      1536
   Ser Tyr Asp Gly Thr Phe Thr Val Pro Thr Ser Leu Ala Tyr Lys Met
               500                 505                 510
   gcc agt caa acg att ttc tat cct ttc cat gca ggg gat act tat tta      1584
   Ala Ser Gln Thr Ile Phe Tyr Pro Phe His Ala Gly Asp Thr Tyr Leu
           515                 520                 525
   aga gtg aac cct caa ttt gca gtg cct aaa gga act gat gct tta gtc      1632
   Arg Val Asn Pro Gln Phe Ala Val Pro Lys Gly Thr Asp Ala Leu Val
       530                 535                 540
   aga gtg ttt gat gaa ttt cat gga aat gct tat tta gaa aat aac tat      1680
   Arg Val Phe Asp Glu Phe His Gly Asn Ala Tyr Leu Glu Asn Asn Tyr
   545                 550                 555                 560
   aaa gtt ggt gaa atc aaa tta ccg att ccg aaa tta aac caa gga aca      1728
   Lys Val Gly Glu Ile Lys Leu Pro Ile Pro Lys Leu Asn Gln Gly Thr
                   565                 570                 575
   acc aga acg gcc gga aat aaa att cct gta acc ttc atg gca aat gct      1776
   Thr Arg Thr Ala Gly Asn Lys Ile Pro Val Thr Phe Met Ala Asn Ala
               580                 585                 590
   tat ttg gac aat caa tcg act tat att gtg gaa gta cct atc ttg gaa      1824
   Tyr Leu Asp Asn Gln Ser Thr Tyr Ile Val Glu Val Pro Ile Leu Glu
           595                 600                 605
   aaa gaa aat caa act gat aaa cca agt att cta cca caa ttt aaa agg      1872
   Lys Glu Asn Gln Thr Asp Lys Pro Ser Ile Leu Pro Gln Phe Lys Arg
       610                 615                 620
   aat aaa gca caa gaa aac tca aaa ctt gat gaa aag gta gaa gaa cca      1920
   Asn Lys Ala Gln Glu Asn Ser Lys Leu Asp Glu Lys Val Glu Glu Pro
   625                 630                 635                 640
   aag act agt gag aag gta gaa aaa gaa aaa ctt tct gaa act ggg aat      1968
   Lys Thr Ser Glu Lys Val Glu Lys Glu Lys Leu Ser Glu Thr Gly Asn
                   645                 650                 655
   agt act agt aat tca acg tta gaa gaa gtt cct aca gtg gat cct gta      2016
   Ser Thr Ser Asn Ser Thr Leu Glu Glu Val Pro Thr Val Asp Pro Val
               660                 665                 670
   caa gaa aaa gta gca aaa ttt gct gaa agt tat ggg atg aag cta gaa      2064
   Gln Glu Lys Val Ala Lys Phe Ala Glu Ser Tyr Gly Met Lys Leu Glu
           675                 680                 685
   aat gtc ttg ttt aat atg gac gga aca att gaa tta tat tta cca tcg      2112
   Asn Val Leu Phe Asn Met Asp Gly Thr Ile Glu Leu Tyr Leu Pro Ser
       690                 695                 700
   gga gaa gtc att aaa aag aat atg gca gat ttt aca gga gaa gca cct      2160
   Gly Glu Val Ile Lys Lys Asn Met Ala Asp Phe Thr Gly Glu Ala Pro
   705                 710                 715                 720
   caa gga aat ggt gaa aat aaa cca tct gaa aat gga aaa gta tct act      2208
   Gln Gly Asn Gly Glu Asn Lys Pro Ser Glu Asn Gly Lys Val Ser Thr
                   725                 730                 735
   gga aca gtt gag aac caa cca aca gaa aat aaa cca gca gat tct tta      2256
   Gly Thr Val Glu Asn Gln Pro Thr Glu Asn Lys Pro Ala Asp Ser Leu
               740                 745                 750
   cca gag gca cca aac gaa aaa cct gta aaa cca gaa aac tca acg gat      2304
   Pro Glu Ala Pro Asn Glu Lys Pro Val Lys Pro Glu Asn Ser Thr Asp
           755                 760                 765
   aat gga atg ttg aat cca gaa ggg aat gtg ggg agt gac cct atg tta      2352
   Asn Gly Met Leu Asn Pro Glu Gly Asn Val Gly Ser Asp Pro Met Leu
       770                 775                 780
   gat tca gca tta gag gaa gct cca gca gta gat cct gta caa gaa aaa      2400
   Asp Ser Ala Leu Glu Glu Ala Pro Ala Val Asp Pro Val Gln Glu Lys
   785                 790                 795                 800
   tta gaa aaa ttt aca gct agt tac gga tta ggc tta gat agt gtt ata      2448
   Leu Glu Lys Phe Thr Ala Ser Tyr Gly Leu Gly Leu Asp Ser Val Ile
                   805                 810                 815
   ttc aat atg gat gga acg att gaa tta aga ttg cca agt gga gaa gtg      2496
   Phe Asn Met Asp Gly Thr Ile Glu Leu Arg Leu Pro Ser Gly Glu Val
               820                 825                 830
   ata aaa aag aat tta ttg atc tca tagcgtaa                             2528
   Ile Lys Lys Asn Leu Leu Ile Ser
           835                 840
<210> 16
   <211> 840
   <212> PRT
   <213> S. pneumoniae
<400> 16
   Cys Ala Tyr Ala Leu Asn Gln His Arg Ser Gln Glu Asn Lys Asp Asn
    1               5                  10                  15
   Asn Arg Val Ser Tyr Val Asp Gly Ser Gln Ser Ser Gln Lys Ser Glu
               20                  25                  30
   Asn Leu Thr Pro Asp Gln Val Ser Gln Lys Glu Gly Ile Gln Ala Glu
           35                  40                  45
   Gln Ile Val Ile Lys Ile Thr Asp Gln Gly Tyr Val Thr Ser His Gly
       50                  55                  60
   Asp His Tyr His Tyr Tyr Asn Gly Lys Val Pro Tyr Asp Ala Leu Phe
   65                  70                  75                  80
   Ser Glu Glu Leu Leu Met Lys Asp Pro Asn Tyr Gln Leu Lys Asp Ala
                   85                  90                  95
   Asp Ile Val Asn Glu Val Lys Gly Gly Tyr Ile Ile Lys Val Asp Gly
               100                 105                 110
   Lys Tyr Tyr Val Tyr Leu Lys Asp Ala Ala His Ala Asp Asn Val Arg
           115                 120                 125
   Thr Lys Asp Glu Ile Asn Arg Gln Lys Gln Glu His Val Lys Asp Asn
       130                 135                 140
   Glu Lys Val Asn Ser Asn Val Ala Val Ala Arg Ser Gln Gly Arg Tyr
   145                 150                 155                 160
   Thr Thr Asn Asp Gly Tyr Val Phe Asn Pro Ala Asp Ile Ile Glu Asp
                   165                 170                 175
   Thr Gly Asn Ala Tyr Ile Val Pro His Gly Gly His Tyr His Tyr Ile
               180                 185                 190
   Pro Lys Ser Asp Leu Ser Ala Ser Glu Leu Ala Ala Ala Lys Ala His
           195                 200                 205
   Leu Ala Gly Lys Asn Met Gln Pro Ser Gln Leu Ser Tyr Ser Ser Thr
       210                 215                 220
   Pro Ser Pro Ser Leu Pro Ile Asn Pro Gly Thr Ser His Glu Lys His
   225                 230                 235                 240
   Glu Glu Asp Gly Tyr Gly Phe Asp Ala Asn Arg Ile Ile Ala Glu Asp
                   245                 250                 255
   Glu Ser Gly Phe Val Met Ser His Gly Asp His Asn His Tyr Phe Phe
               260                 265                 270
   Lys Lys Asp Leu Thr Glu Glu Gln Ile Lys Ala Ala Gln Lys His Leu
           275                 280                 285
   Glu Glu Val Lys Thr Ser His Asn Gly Leu Asp Ser Leu Ser Ser His
       290                 295                 300
   Glu Gln Asp Tyr Pro Ser Asn Ala Lys Glu Met Lys Asp Leu Asp Lys
   305                 310                 315                 320
   Lys Ile Glu Glu Lys Ile Ala Gly Ile Met Lys Gln Tyr Gly Val Lys
                   325                 330                 335
   Arg Glu Ser Ile Val Val Asn Lys Glu Lys Asn Ala Ile Ile Tyr Pro
               340                 345                 350
   His Gly Asp His His His Ala Asp Pro Ile Asp Glu His Lys Pro Val
           355                 360                 365
   Gly Ile Gly His Ser His Ser Asn Tyr Glu Leu Phe Lys Pro Glu Glu
       370                 375                 380
   Gly Val Ala Lys Lys Glu Gly Asn Lys Val Tyr Thr Gly Glu Glu Leu
   385 390 395 400
   Thr Asn Val Val Asn Leu Leu Lys Asn Ser Thr Phe Asn Asn Gln Asn
   405 410 415
   Phe Thr Leu Ala Asn Gly Gln Lys Arg Val Ser Phe Ser Phe Pro Pro
   420 425 430
   Glu Leu Glu Lys Lys Leu Gly Ile Asn Met Leu Val Lys Leu Ile Thr
   435 440 445
   Pro Asp Gly Lys Val Leu Glu Lys Val Ser Gly Lys Val Phe Gly Glu
   450 455 460
   Gly Val Gly Asn Ile Ala Asn Phe Glu Leu Asp Gln Pro Tyr Leu Pro
   465 470 475 480
   Gly Gln Thr Phe Lys Tyr Thr Ile Ala Ser Lys Asp Tyr Pro Glu Val
   485 490 495
   Ser Tyr Asp Gly Thr Phe Thr Val Pro Thr Ser Leu Ala Tyr Lys Met
   500 505 510
   Ala Ser Gln Thr Ile Phe Tyr Pro Phe His Ala Gly Asp Thr Tyr Leu
   515 520 525
   Arg Val Asn Pro Gln Phe Ala Val Pro Lys Gly Thr Asp Ala Leu Val
   530 535 540
   Arg Val Phe Asp Glu Phe His Gly Asn Ala Tyr Leu Glu Asn Asn Tyr
   545 550 555 560
   Lys Val Gly Glu Ile Lys Leu Pro Ile Pro Lys Leu Asn Gln Gly Thr
   565 570 575
   Thr Arg Thr Ala Gly Asn Lys Ile Pro Val Thr Phe Met Ala Asn Ala
   580 585 590
   Tyr Leu Asp Asn Gln Ser Thr Tyr Ile Val Glu Val Pro Ile Leu Glu
   595 600 605
   Lys Glu Asn Gln Thr Asp Lys Pro Ser Ile Leu Pro Gln Phe Lys Arg
   610 615 620
   Asn Lys Ala Gln Glu Asn Ser Lys Leu Asp Glu Lys Val Glu Glu Pro
   625 630 635 640
   Lys Thr Ser Glu Lys Val Glu Lys Glu Lys Leu Ser Glu Thr Gly Asn
   645 650 655
   Ser Thr Ser Asn Ser Thr Leu Glu Glu Val Pro Thr Val Asp Pro Val
   660 665 670
   Gln Glu Lys Val Ala Lys Phe Ala Glu Ser Tyr Gly Met Lys Leu Glu
   675 680 685
   Asn Val Leu Phe Asn Met Asp Gly Thr Ile Glu Leu Tyr Leu Pro Ser
   690 695 700
   Gly Glu Val Ile Lys Lys Asn Met Ala Asp Phe Thr Gly Glu Ala Pro
   705 710 715 720
   Gln Gly Asn Gly Glu Asn Lys Pro Ser Glu Asn Gly Lys Val Ser Thr
   725 730 735
   Gly Thr Val Glu Asn Gln Pro Thr Glu Asn Lys Pro Ala Asp Ser Leu
   740 745 750
   Pro Glu Ala Pro Asn Glu Lys Pro Val Lys Pro Glu Asn Ser Thr Asp
   755 760 765
   Asn Gly Met Leu Asn Pro Glu Gly Asn Val Gly Ser Asp Pro Met Leu
   770 775 780
   Asp Ser Ala Leu Glu Glu Ala Pro Ala Val Asp Pro Val Gln Glu Lys
   785 790 795 800
   Leu Glu Lys Phe Thr Ala Ser Tyr Gly Leu Gly Leu Asp Ser Val Ile
   805 810 815
   Phe Asn Met Asp Gly Thr Ile Glu Leu Arg Leu Pro Ser Gly Glu Val
   820 825 830
   Ile Lys Lys Asn Leu Leu Ile Ser
   835 840
<210> 17
   <211> 27
   <212> DNA
   <213> Artificial Sequence
<220>
   <223> PCR oligonucleotide primer
<400> 17
   cagtagatct gtgcctatgc actaaac 27
<210> 18
   <211> 33
   <212> DNA
   <213> Artificial Sequence
<220>
   <223> PCR oligonucleotide primer
<400> 18
   gatctctaga ctactgctat tccttacgct atg 33
<210> 19
   <211> 30
   <212> DNA
   <213> Artificial Sequence
<220>
   <223> PCR oligonucleotide primer
<400> 19
   atcactcgag cattacctgg ataatcctgt 30
<210> 20
   <211> 26
   <212> DNA
   <213> Artificial Sequence
<220>
   <223> PCR oligonucleotide primer
<400> 20
   ctgctaagct tatgaaagat ttagat 26
<210> 21
   <211> 24
   <212> DNA
   <213> Artificial Sequence
<220>
   <223> PCR oligonucleotide primer
<400> 21
   gatactcgag ctgctattcc ttac 24
<210> 22
   <211> 28
   <212> DNA
   <213> Artificial Sequence
<220>
   <223> PCR oligonucleotide primer
<400> 22
   gaatctcgag ttaagctgct gctaattc 28
<210> 23
   <211> 32
   <212> DNA
   <213> Artificial Sequence
<220>
   <223> PCR oligonucleotide primer
<400> 23
   gacgctcgag cgctatgaaa tcagataaat tc 32
<210> 24
   <211> 37
   <212> DNA
   <213> Artificial Sequence
<220>
   <223> PCR oligonucleotide primer
<400> 24
   gacgctcgag ggcattacct ggataatcct gttcatg 37
<210> 25
   <211> 33
   <212> DNA
   <213> Artificial Sequence
<220>
   <223> PCR oligonucleotide primer
<400> 25
   cagtagatct cttcatcatt tattgaaaag agg 33
<210> 26
   <211> 40
   <212> DNA
   <213> Artificial Sequence
<220>
   <223> PCR oligonucleotide primer
<400> 26
   ttatttcttc catatggact tgacagaaga gcaaattaag 40
<210> 27
   <211> 32
   <212> DNA
   <213> Artificial Sequence
<220>
   <223> PCR oligonucleotide primer
<400> 27
   cgccaagctt cgctatgaaa tcagataaat tc 32
<210> 28
   <211> 34
   <212> DNA
   <213> Artificial Sequence
<220>
   <223> PCR oligonucleotide primer
<400> 28
   cgccaagctt ttccacaata taagtcgatt gatt 34
<210> 29
   <211> 40
   <212> DNA
   <213> Artificial Sequence
<220>
   <223> PCR oligonucleotide primer
<400> 29
   ttatttcttc catatggaag tacctatctt ggaaaaagaa 40
<210> 30
   <211> 37
   <212> DNA
   <213> Artificial Sequence
<220>
   <223> PCR oligonucleotide primer
<400> 30
   ttatttcttc catatggtgc ctatgcacta aaccagc 37
<210> 31
   <211> 40
   <212> DNA
   <213> Artificial Sequence
<220>
   <223> PCR oligonucleotide primer
<400> 31
   ataagaatgc ggccgcttcc acaatataag tcgattgatt 40
<210> 32
   <211> 31
   <212> DNA
   <213> Artificial Sequence
<220>
   <223> PCR oligonucleotide primer
<400> 32
   cagtagatct gtgcttatga actaggtttg c 31
<210> 33
   <211> 32
   <212> DNA
   <213> Artificial Sequence
<220>
   <223> PCR oligonucleotide primer
<400> 33
   gatcaagctt gctgctacct ttacttactc tc 32
<210> 34
   <211> 26
   <212> DNA
   <213> Artificial Sequence
<220>
   <223> PCR oligonucleotide primer
<400> 34
   ctgagatatc cgttatcgtt caaacc 26
<210> 35
   <211> 28
   <212> DNA
   <213> Artificial Sequence
<220>
   <223> PCR oligonucleotide primer
<400> 35
   ctgcaagctt ttaaagggga ataatacg 28
<210> 36
   <211> 29
   <212> DNA
   <213> Artificial Sequence
<220>
   <223> PCR oligonucleotide primer
<400> 36
   cagtagatct gcagaagcct tcctatctg 29
<210> 37
   <211> 33
   <212> DNA
   <213> Artificial Sequence
<220>
   <223> PCR oligonucleotide primer
<400> 37
   tcgccaagct tcgttatcgt tcaaaccatt ggg 33
<210> 38
   <211> 45
   <212> DNA
   <213> Artificial Sequence
<220>
   <223> PCR oligonucleotide primer
<400> 38
   ataagaatgc ggccgcctta ctctccttta ataaagccaa tagtt 45
<210> 39
   <211> 34
   <212> DNA
   <213> Artificial Sequence
<220>
   <223> PCR oligonucleotide primer
<400> 39
   catgccatgg acattgatag tctcttgaaa cagc 34
<210> 40
   <211> 37
   <212> DNA
   <213> Artificial Sequence
<220>
   <223> PCR oligonucleotide primer
<400> 40
   cgccaagctt cttactctcc tttaataaag ccaatag 37
<210> 41
   <211> 31
   <212> DNA
   <213> Artificial Sequence
<220>
   <223> PCR oligonucleotide primer
<400> 41
   cgacaagctt aacatggtcg ctagcgttac c 31
<210> 42
   <211> 30
   <212> DNA
   <213> Artificial Sequence
<220>
   <223> PCR oligonucleotide primer
<400> 42
   cataccatgg gcctttatga ggcacctaag 30
<210> 43
   <211> 34
   <212> DNA
   <213> Artificial Sequence
<220>
   <223> PCR oligonucleotide primer
<400> 43
   cgacaagctt aagtaaatct tcagcctctc tcag 34
<210> 44
   <211> 31
   <212> DNA
   <213> Artificial Sequence
<220>
   <223> PCR oligonucleotide primer
<400> 44
   gataccatgg ctagcgacca tgttcaaaga a 31
<210> 45
   <211> 36
   <212> DNA
   <213> Artificial Sequence
<220>
   <223> PCR oligonucleotide primer
<400> 45
   cgccaagctt atcatccact aacttgactt tatcac 36
<210> 46
   <211> 33
   <212> DNA
   <213> Artificial Sequence
<220>
   <223> PCR oligonucleotide primer
<400> 46
   cataccatgg atattcttgc cttcttagct ccg 33
<210> 47
   <211> 30
   <212> DNA
   <213> Artificial Sequence
<220>
   <223> PCR oligonucleotide primer
<400> 47
   catgccatgg tgcttatgaa ctaggtttgc 30
<210> 48
   <211> 32
   <212> DNA
   <213> Artificial Sequence
<220>
   <223> PCR oligonucleotide primer
<400> 48
   cgccaagctt tagcgttacc aaaaccatta tc 32
<210> 49
   <211> 36
   <212> DNA
   <213> Artificial Sequence
<220>
   <223> PCR oligonucleotide primer
<400> 49
   gtattagatc tgttcctatg aacttggtcg tcacca 36
<210> 50
   <211> 28
   <212> DNA
   <213> Artificial Sequence
<220>
   <223> PCR oligonucleotide primer
<400> 50
   cgcctctaga ctactgtata ggagccgg 28
<210> 51
   <211> 34
   <212> DNA
   <213> Artificial Sequence
<220>
   <223> PCR oligonucleotide primer
<400> 51
   catgccatgg aaaacatttc aagcctttta cgtg 34
<210> 52
   <211> 34
   <212> DNA
   <213> Artificial Sequence
<220>
   <223> PCR oligonucleotide primer
<400> 52
   cgacaagctt ctgtatagga gccggttgac tttc 34
<210> 53
   <211> 34
   <212> DNA
   <213> Artificial Sequence
<220>
   <223> PCR oligonucleotide primer
<400> 53
   catgccatgg ttcgtaaaaa taaggcagac caag 34
<210> 54
   <211> 30
   <212> DNA
   <213> Artificial Sequence
<220>
   <223> PCR oligonucleotide primer
<400> 54
   catgccatgg aagcctattg gaatgggaag 30
<210> 55
   <211> 1019
   <212> PRT
   <213> S. pneumoniae
<400> 55
   Cys Ala Tyr Ala Leu Asn Gln His Arg Ser Gln Glu Asn Lys Asp Asn
   1               5                   10                  15
   Asn Arg Val Ser Tyr Val Asp Gly Ser Gln Ser Ser Gln Lys Ser Glu
               20                  25                  30
   Asn Leu Thr Pro Asp Gln Val Ser Gln Lys Glu Gly Ile Gln Ala Glu
           35                  40                  45
   Gln Ile Val Ile Lys Ile Thr Asp Gln Gly Tyr Val Thr Ser His Gly
       50                  55                  60
   Asp His Tyr His Tyr Tyr Asn Gly Lys Val Pro Tyr Asp Ala Leu Phe
   65                  70                  75                  80
   Ser Glu Glu Leu Leu Met Lys Asp Pro Asn Tyr Gln Leu Lys Asp Ala
                   85                  90                  95
   Asp Ile Val Asn Glu Val Lys Gly Gly Tyr Ile Ile Lys Val Asp Gly
               100                 105                 110
   Lys Tyr Tyr Val Tyr Leu Lys Asp Ala Ala His Ala Asp Asn Val Arg
           115                 120                 125
   Thr Lys Asp Glu Ile Asn Arg Gln Lys Gln Glu His Val Lys Asp Asn
       130                 135                 140
   Glu Lys Val Asn Ser Asn Val Ala Val Ala Arg Ser Gln Gly Arg Tyr
   145                 150                 155                 160
   Thr Thr Asn Asp Gly Tyr Val Phe Asn Pro Ala Asp Ile Ile Glu Asp
                   165                 170                 175
   Thr Gly Asn Ala Tyr Ile Val Pro His Gly Gly His Tyr His Tyr Ile
               180                 185                 190
   Pro Lys Ser Asp Leu Ser Ala Ser Glu Leu Ala Ala Ala Lys Ala His
           195                 200                 205
   Leu Ala Gly Lys Asn Met Gln Pro Ser Gln Leu Ser Tyr Ser Ser Thr
       210                 215                 220
   Ala Ser Asp Asn Asn Thr Gln Ser Val Ala Lys Gly Ser Thr Ser Lys
   225                 230                 235                 240
   Pro Ala Asn Lys Ser Glu Asn Leu Gln Ser Leu Leu Lys Glu Leu Tyr
                   245                 250                 255
   Asp Ser Pro Ser Ala Gln Arg Tyr Ser Glu Ser Asp Gly Leu Val Phe
               260                 265                 270
   Asp Pro Ala Lys Ile Ile Ser Arg Thr Pro Asn Gly Val Ala Ile Pro
           275                 280                 285
   His Gly Asp His Tyr His Phe Ile Pro Tyr Ser Lys Leu Ser Ala Leu
       290                 295                 300
   Glu Glu Lys Ile Ala Arg Met Val Pro Ile Ser Gly Thr Gly Ser Thr
   305                 310                 315                 320
   Val Ser Thr Asn Ala Lys Pro Asn Glu Val Val Ser Ser Leu Gly Ser
                   325                 330                 335
   Leu Ser Ser Asn Pro Ser Ser Leu Thr Thr Ser Lys Glu Leu Ser Ser
               340                 345                 350
   Ala Ser Asp Gly Tyr Ile Phe Asn Pro Lys Asp Ile Val Glu Glu Thr
           355                 360                 365
   Ala Thr Ala Tyr Ile Val Arg His Gly Asp His Phe His Tyr Ile Pro
       370                 375                 380
   Lys Ser Asn Gln Ile Gly Gln Pro Thr Leu Pro Asn Asn Ser Leu Ala
   385                 390                 395                 400
   Thr Pro Ser Pro Ser Leu Pro Ile Asn Pro Gly Thr Ser His Glu Lys
                   405                 410                 415
   His Glu Glu Asp Gly Tyr Gly Phe Asp Ala Asn Arg Ile Ile Ala Glu
               420                 425                 430
   Asp Glu Ser Gly Phe Val Met Ser His Gly Asp His Asn His Tyr Phe
           435                 440                 445
   Phe Lys Lys Asp Leu Thr Glu Glu Gln Ile Lys Ala Ala Gln Lys His
       450                 455                 460
   Leu Glu Glu Val Lys Thr Ser His Asn Gly Leu Asp Ser Leu Ser Ser
   465                 470                 475                 480
   His Glu Gln Asp Tyr Pro Gly Asn Ala Lys Glu Met Lys Asp Leu Asp
                   485                 490                 495
   Lys Lys Ile Glu Glu Lys Ile Ala Gly Ile Met Lys Gln Tyr Gly Val
               500                 505                 510
   Lys Arg Glu Ser Ile Val Val Asn Lys Glu Lys Asn Ala Ile Ile Tyr
           515                 520                 525
   Pro His Gly Asp His His His Ala Asp Pro Ile Asp Glu His Lys Pro
       530                 535                 540
   Val Gly Ile Gly His Ser His Ser Asn Tyr Glu Leu Phe Lys Pro Glu
   545                 550                 555                 560
   Glu Gly Val Ala Lys Lys Glu Gly Asn Lys Val Tyr Thr Gly Glu Glu
                   565                 570                 575
   Leu Thr Asn Val Val Asn Leu Leu Lys Asn Ser Thr Phe Asn Asn Gln
               580                 585                 590
   Asn Phe Thr Leu Ala Asn Gly Gln Lys Arg Val Ser Phe Ser Phe Pro
           595                 600                 605
   Pro Glu Leu Glu Lys Lys Leu Gly Ile Asn Met Leu Val Lys Leu Ile
       610                 615                 620
   Thr Pro Asp Gly Lys Val Leu Glu Lys Val Ser Gly Lys Val Phe Gly
   625                 630                 635                 640
   Glu Gly Val Gly Asn Ile Ala Asn Phe Glu Leu Asp Gln Pro Tyr Leu
                   645                 650                 655
   Pro Gly Gln Thr Phe Lys Tyr Thr Ile Ala Ser Lys Asp Tyr Pro Glu
               660                 665                 670
   Val Ser Tyr Asp Gly Thr Phe Thr Val Pro Thr Ser Leu Ala Tyr Lys
           675                 680                 685
   Met Ala Ser Gln Thr Ile Phe Tyr Pro Phe His Ala Gly Asp Thr Tyr
       690                 695                 700
   Leu Arg Val Asn Pro Gln Phe Ala Val Pro Lys Gly Thr Asp Ala Leu
   705                 710                 715                 720
   Val Arg Val Phe Asp Glu Phe His Gly Asn Ala Tyr Leu Glu Asn Asn
                   725                 730                 735
   Tyr Lys Val Gly Glu Ile Lys Leu Pro Ile Pro Lys Leu Asn Gln Gly
               740                 745                 750
   Thr Thr Arg Thr Ala Gly Asn Lys Ile Pro Val Thr Phe Met Ala Asn
           755                 760                 765
   Ala Tyr Leu Asp Asn Gln Ser Thr Tyr Ile Val Glu Val Pro Ile Leu
       770                 775                 780
   Glu Lys Glu Asn Gln Thr Asp Lys Pro Ser Ile Leu Pro Gln Phe Lys
   785                 790                 795                 800
   Arg Asn Lys Ala Gln Glu Asn Ser Lys Leu Asp Glu Lys Val Glu Glu
                   805                 810                 815
   Pro Lys Thr Ser Glu Lys Val Glu Lys Glu Lys Leu Ser Glu Thr Gly
               820                 825                 830
   Asn Ser Thr Ser Asn Ser Thr Leu Glu Glu Val Pro Thr Val Asp Pro
           835                 840                 845
   Val Gln Glu Lys Val Ala Lys Phe Ala Glu Ser Tyr Gly Met Lys Leu
       850                 855                 860
   Glu Asn Val Leu Phe Asn Met Asp Gly Thr Ile Glu Leu Tyr Leu Pro
   865                 870                 875                 880
   Ser Gly Glu Val Ile Lys Lys Asn Met Ala Asp Phe Thr Gly Glu Ala
                   885                 890                 895
   Pro Gln Gly Asn Gly Glu Asn Lys Pro Ser Glu Asn Gly Lys Val Ser
               900                 905                 910
   Thr Gly Thr Val Glu Asn Gln Pro Thr Glu Asn Lys Pro Ala Asp Ser
           915                 920                 925
   Leu Pro Glu Ala Pro Asn Glu Lys Pro Val Lys Pro Glu Asn Ser Thr
       930                 935                 940
   Asp Asn Gly Met Leu Asn Pro Glu Gly Asn Val Gly Ser Asp Pro Met
   945                 950                 955                 960
   Leu Asp Pro Ala Leu Glu Glu Ala Pro Ala Val Asp Pro Val Gln Glu
                   965                 970                 975
   Lys Leu Glu Lys Phe Thr Ala Ser Tyr Gly Leu Gly Leu Asp Ser Val
               980                 985                 990
   Ile Phe Asn Met Asp Gly Thr Ile Glu Leu Arg Leu Pro Ser Gly Glu
           995                 1000                1005
   Val Ile Lys Lys Asn Leu Ser Asp Phe Ile Ala
       1010                1015
<210> 56
   <211> 489
   <212> PRT
   <213> S. pneumoniae
<400> 56
   Cys Ala Tyr Ala Leu Asn Gln His Arg Ser Gln Glu Asn Lys Asp Asn
    1               5                  10                  15
   Asn Arg Val Ser Tyr Val Asp Gly Ser Gln Ser Ser Gln Lys Ser Glu
               20                  25                  30
   Asn Leu Thr Pro Asp Gln Val Ser Gln Lys Glu Gly Ile Gln Ala Glu
           35                  40                  45
   Gln Ile Val Ile Lys Ile Thr Asp Gln Gly Tyr Val Thr Ser His Gly
       50                  55                  60
   Asp His Tyr His Tyr Tyr Asn Gly Lys Val Pro Tyr Asp Ala Leu Phe
   65                  70                  75                  80
   Ser Glu Glu Leu Leu Met Lys Asp Pro Asn Tyr Gln Leu Lys Asp Ala
                   85                  90                  95
   Asp Ile Val Asn Glu Val Lys Gly Gly Tyr Ile Ile Lys Val Asp Gly
               100                 105                 110
   Lys Tyr Tyr Val Tyr Leu Lys Asp Ala Ala His Ala Asp Asn Val Arg
           115                 120                 125
   Thr Lys Asp Glu Ile Asn Arg Gln Lys Gln Glu His Val Lys Asp Asn
       130                 135                 140
   Glu Lys Val Asn Ser Asn Val Ala Val Ala Arg Ser Gln Gly Arg Tyr
   145                 150                 155                 160
   Thr Thr Asn Asp Gly Tyr Val Phe Asn Pro Ala Asp Ile Ile Glu Asp
                   165                 170                 175
   Thr Gly Asn Ala Tyr Ile Val Pro His Gly Gly His Tyr His Tyr Ile
               180                 185                 190
   Pro Lys Ser Asp Leu Ser Ala Ser Glu Leu Ala Ala Ala Lys Ala His
           195                 200                 205
   Leu Ala Gly Lys Asn Met Gln Pro Ser Gln Leu Ser Tyr Ser Ser Thr
       210                 215                 220
   Ala Ser Asp Asn Asn Thr Gln Ser Val Ala Lys Gly Ser Thr Ser Lys
   225                 230                 235                 240
   Pro Ala Asn Lys Ser Glu Asn Leu Gln Ser Leu Leu Lys Glu Leu Tyr
                   245                 250                 255
   Asp Ser Pro Ser Ala Gln Arg Tyr Ser Glu Ser Asp Gly Leu Val Phe
               260                 265                 270
   Asp Pro Ala Lys Ile Ile Ser Arg Thr Pro Asn Gly Val Ala Ile Pro
           275                 280                 285
   His Gly Asp His Tyr His Phe Ile Pro Tyr Ser Lys Leu Ser Ala Leu
       290                 295                 300
   Glu Glu Lys Ile Ala Arg Met Val Pro Ile Ser Gly Thr Gly Ser Thr
   305                 310                 315                 320
   Val Ser Thr Asn Ala Lys Pro Asn Glu Val Val Ser Ser Leu Gly Ser
                   325                 330                 335
   Leu Ser Ser Asn Pro Ser Ser Leu Thr Thr Ser Lys Glu Leu Ser Ser
               340                 345                 350
   Ala Ser Asp Gly Tyr Ile Phe Asn Pro Lys Asp Ile Val Glu Glu Thr
           355                 360                 365
   Ala Thr Ala Tyr Ile Val Arg His Gly Asp His Phe His Tyr Ile Pro
       370                 375                 380
   Lys Ser Asn Gln Ile Gly Gln Pro Thr Leu Pro Asn Asn Ser Leu Ala
   385                 390                 395                 400
   Thr Pro Ser Pro Ser Leu Pro Ile Asn Pro Gly Thr Ser His Glu Lys
                   405                 410                 415
   His Glu Glu Asp Gly Tyr Gly Phe Asp Ala Asn Arg Ile Ile Ala Glu
               420                 425                 430
   Asp Glu Ser Gly Phe Val Met Ser His Gly Asp His Asn His Tyr Phe
           435                 440                 445
   Phe Lys Lys Asp Leu Thr Glu Glu Gln Ile Lys Ala Ala Gln Lys His
       450                 455                 460
   Leu Glu Glu Val Lys Thr Ser His Asn Gly Leu Asp Ser Leu Ser Ser
   465                 470                 475                 480
   His Glu Gln Asp Tyr Pro Gly Asn Ala
                   485
<210> 57
   <211> 509
   <212> PRT
   <213> S. pneumoniae
<400> 57
   Met Lys Phe Ser Lys Lys Tyr Ile Ala Ala Gly Ser Ala Val Ile Val
    1               5                  10                  15
   Ser Leu Ser Leu Cys Ala Tyr Ala Leu Asn Gln His Arg Ser Gln Glu
               20                  25                  30
   Asn Lys Asp Asn Asn Arg Val Ser Tyr Val Asp Gly Ser Gln Ser Ser
           35                  40                  45
   Gln Lys Ser Glu Asn Leu Thr Pro Asp Gln Val Ser Gln Lys Glu Gly
       50                  55                  60
   Ile Gln Ala Glu Gln Ile Val Ile Lys Ile Thr Asp Gln Gly Tyr Val
   65                  70                  75                  80
   Thr Ser His Gly Asp His Tyr His Tyr Tyr Asn Gly Lys Val Pro Tyr
                   85                  90                  95
   Asp Ala Leu Phe Ser Glu Glu Leu Leu Met Lys Asp Pro Asn Tyr Gln
               100                 105                 110
   Leu Lys Asp Ala Asp Ile Val Asn Glu Val Lys Gly Gly Tyr Ile Ile
           115                 120                 125
   Lys Val Asp Gly Lys Tyr Tyr Val Tyr Leu Lys Asp Ala Ala His Ala
       130                 135                 140
   Asp Asn Val Arg Thr Lys Asp Glu Ile Asn Arg Gln Lys Gln Glu His
   145                 150                 155                 160
   Val Lys Asp Asn Glu Lys Val Asn Ser Asn Val Ala Val Ala Arg Ser
                   165                 170                 175
   Gln Gly Arg Tyr Thr Thr Asn Asp Gly Tyr Val Phe Asn Pro Ala Asp
               180                 185                 190
   Ile Ile Glu Asp Thr Gly Asn Ala Tyr Ile Val Pro His Gly Gly His
           195                 200                 205
   Tyr His Tyr Ile Pro Lys Ser Asp Leu Ser Ala Ser Glu Leu Ala Ala
       210                 215                 220
   Ala Lys Ala His Leu Ala Gly Lys Asn Met Gln Pro Ser Gln Leu Ser
   225                 230                 235                 240
   Tyr Ser Ser Thr Ala Ser Asp Asn Asn Thr Gln Ser Val Ala Lys Gly
                   245                 250                 255
   Ser Thr Ser Lys Pro Ala Asn Lys Ser Glu Asn Leu Gln Ser Leu Leu
               260                 265                 270
   Lys Glu Leu Tyr Asp Ser Pro Ser Ala Gln Arg Tyr Ser Glu Ser Asp
           275                 280                 285
   Gly Leu Val Phe Asp Pro Ala Lys Ile Ile Ser Arg Thr Pro Asn Gly
       290                 295                 300
   Val Ala Ile Pro His Gly Asp His Tyr His Phe Ile Pro Tyr Ser Lys
   305                 310                 315                 320
   Leu Ser Ala Leu Glu Glu Lys Ile Ala Arg Met Val Pro Ile Ser Gly
                   325                 330                 335
   Thr Gly Ser Thr Val Ser Thr Asn Ala Lys Pro Asn Glu Val Val Ser
               340                 345                 350
   Ser Leu Gly Ser Leu Ser Ser Asn Pro Ser Ser Leu Thr Thr Ser Lys
           355                 360                 365
   Glu Leu Ser Ser Ala Ser Asp Gly Tyr Ile Phe Asn Pro Lys Asp Ile
       370                 375                 380
   Val Glu Glu Thr Ala Thr Ala Tyr Ile Val Arg His Gly Asp His Phe
   385                 390                 395                 400
   His Tyr Ile Pro Lys Ser Asn Gln Ile Gly Gln Pro Thr Leu Pro Asn
                   405                 410                 415
   Asn Ser Leu Ala Thr Pro Ser Pro Ser Leu Pro Ile Asn Pro Gly Thr
               420                 425                 430
   Ser His Glu Lys His Glu Glu Asp Gly Tyr Gly Phe Asp Ala Asn Arg
           435                 440                 445
   Ile Ile Ala Glu Asp Glu Ser Gly Phe Val Met Ser His Gly Asp His
       450                 455                 460
   Asn His Tyr Phe Phe Lys Lys Asp Leu Thr Glu Glu Gln Ile Lys Ala
   465                 470                 475                 480
   Ala Gln Lys His Leu Glu Glu Val Lys Thr Ser His Asn Gly Leu Asp
                   485                 490                 495
   Ser Leu Ser Ser His Glu Gln Asp Tyr Pro Gly Asn Ala
               500                 505
<210> 58
   <211> 1057
   <212> PRT
   <213> S. pneumoniae
<400> 58
   Asp Leu Thr Glu Glu Gln Ile Lys Ala Ala Gln Lys His Leu Glu Glu
    1               5                  10                  15
   Val Lys Thr Ser His Asn Gly Leu Asp Ser Leu Ser Ser His Glu Gln
               20                  25                  30
   Asp Tyr Pro Gly Asn Ala Lys Glu Met Lys Asp Leu Asp Lys Lys Ile
           35                  40                  45
   Glu Glu Lys Ile Ala Gly Ile Met Lys Gln Tyr Gly Val Lys Arg Glu
       50                  55                  60
   Ser Ile Val Val Asn Lys Glu Lys Asn Ala Ile Ile Tyr Pro His Gly
   65                  70                  75                  80
   Asp His His His Ala Asp Pro Ile Asp Glu His Lys Pro Val Gly Ile
                   85                  90                  95
   Gly His Ser His Ser Asn Tyr Glu Leu Phe Lys Pro Glu Glu Gly Val
               100                 105                 110
   Ala Lys Lys Glu Gly Asn Lys Val Tyr Thr Gly Glu Glu Leu Thr Asn
           115                 120                 125
   Val Val Asn Leu Leu Lys Asn Ser Thr Phe Asn Asn Gln Asn Phe Thr
       130                 135                 140
   Leu Ala Asn Gly Gln Lys Arg Val Ser Phe Ser Phe Pro Pro Glu Leu
   145                 150                 155                 160
   Glu Lys Lys Leu Gly Ile Asn Met Leu Val Lys Leu Ile Thr Pro Asp
                   165                 170                 175
   Gly Lys Val Leu Glu Lys Val Ser Gly Lys Val Phe Gly Glu Gly Val
               180                 185                 190
   Gly Asn Ile Ala Asn Phe Glu Leu Asp Gln Pro Tyr Leu Pro Gly Gln
           195                 200                 205
   Thr Phe Lys Tyr Thr Ile Ala Ser Lys Asp Tyr Pro Glu Val Ser Tyr
       210                 215                 220
   Asp Gly Thr Phe Thr Val Pro Thr Ser Leu Ala Tyr Lys Met Ala Ser
   225                 230                 235                 240
   Gln Thr Ile Phe Tyr Pro Phe His Ala Gly Asp Thr Tyr Leu Arg Val
                   245                 250                 255
   Asn Pro Gln Phe Ala Val Pro Lys Gly Thr Asp Ala Leu Val Arg Val
               260                 265                 270
   Phe Asp Glu Phe His Gly Asn Ala Tyr Leu Glu Asn Asn Tyr Lys Val
           275                 280                 285
   Gly Glu Ile Lys Leu Pro Ile Pro Lys Leu Asn Gln Gly Thr Thr Arg
       290                 295                 300
   Thr Ala Gly Asn Lys Ile Pro Val Thr Phe Met Ala Asn Ala Tyr Leu
   305                 310                 315                 320
   Asp Asn Gln Ser Thr Tyr Ile Val Glu Val Pro Ile Leu Glu Lys Glu
                   325                 330                 335
   Asn Gln Thr Asp Lys Pro Ser Ile Leu Pro Gln Phe Lys Arg Asn Lys
               340                 345                 350
   Ala Gln Glu Asn Ser Lys Leu Asp Glu Lys Val Glu Glu Pro Lys Thr
           355                 360                 365
   Ser Glu Lys Val Glu Lys Glu Lys Leu Ser Glu Thr Gly Asn Ser Thr
       370                 375                 380
   Ser Asn Ser Thr Leu Glu Glu Val Pro Thr Val Asp Pro Val Gln Glu
   385                 390                 395                 400
   Lys Val Ala Lys Phe Ala Glu Ser Tyr Gly Met Lys Leu Glu Asn Val
                   405                 410                 415
   Leu Phe Asn Met Asp Gly Thr Ile Glu Leu Tyr Leu Pro Ser Gly Glu
               420                 425                 430
   Val Ile Lys Lys Asn Met Ala Asp Phe Thr Gly Glu Ala Pro Gln Gly
   435 440 445
   Asn Gly Glu Asn Lys Pro Ser Glu Asn Gly Lys Val Ser Thr Gly Thr
   450 455 460
   Val Glu Asn Gln Pro Thr Glu Asn Lys Pro Ala Asp Ser Leu Pro Glu
   465 470 475 480
   Ala Pro Asn Glu Lys Pro Val Lys Pro Glu Asn Ser Thr Asp Asn Gly
   485 490 495
   Met Leu Asn Pro Glu Gly Asn Val Gly Ser Asp Pro Met Leu Asp Pro
   500 505 510
   Ala Leu Glu Glu Ala Pro Ala Val Asp Pro Val Gln Glu Lys Leu Glu
   515 520 525
   Lys Phe Thr Ala Ser Tyr Gly Leu Gly Leu Asp Ser Val Ile Phe Asn
   530 535 540
   Met Asp Gly Thr Ile Glu Leu Arg Leu Pro Ser Gly Glu Val Ile Lys
   545 550 555 560
   Lys Asn Leu Ser Asp Phe Ile Ala Lys Leu Arg Tyr Arg Ser Asn His
   565 570 575
   Trp Val Pro Asp Ser Arg Pro Glu Glu Pro Ser Pro Gln Pro Thr Pro
   580 585 590
   Glu Pro Ser Pro Ser Pro Gln Pro Ala Pro Asn Pro Gln Pro Ala Pro
   595 600 605
   Ser Asn Pro Ile Asp Glu Lys Leu Val Lys Glu Ala Val Arg Lys Val
   610 615 620
   Gly Asp Gly Tyr Val Phe Glu Glu Asn Gly Val Ser Arg Tyr Ile Pro
   625 630 635 640
   Ala Lys Asn Leu Ser Ala Glu Thr Ala Ala Gly Ile Asp Ser Lys Leu
   645 650 655
   Ala Lys Gln Glu Ser Leu Ser His Lys Leu Gly Ala Lys Lys Thr Asp
   660 665 670
   Leu Pro Ser Ser Asp Arg Glu Phe Tyr Asn Lys Ala Tyr Asp Leu Leu
   675 680 685
   Ala Arg Ile His Gln Asp Leu Leu Asp Asn Lys Gly Arg Gln Val Asp
   690 695 700
   Phe Glu Ala Leu Asp Asn Leu Leu Glu Arg Leu Lys Asp Val Ser Ser
   705 710 715 720
   Asp Lys Val Lys Leu Val Asp Asp Ile Leu Ala Phe Leu Ala Pro Ile
   725 730 735
   Arg His Pro Glu Arg Leu Gly Lys Pro Asn Ala Gln Ile Thr Tyr Thr
   740 745 750
   Asp Asp Glu Ile Gln Val Ala Lys Leu Ala Gly Lys Tyr Thr Thr Glu
   755 760 765
   Asp Gly Tyr Ile Phe Asp Pro Arg Asp Ile Thr Ser Asp Glu Gly Asp
   770 775 780
   Ala Tyr Val Thr Pro His Met Thr His Ser His Trp Ile Lys Lys Asp
   785 790 795 800
   Ser Leu Ser Glu Ala Glu Arg Ala Ala Ala Gln Ala Tyr Ala Lys Glu
   805 810 815
   Lys Gly Leu Thr Pro Pro Ser Thr Asp His Gln Asp Ser Gly Asn Thr
   820 825 830
   Glu Ala Lys Gly Ala Glu Ala Ile Tyr Asn Arg Val Lys Ala Ala Lys
   835 840 845
   Lys Val Pro Leu Asp Arg Met Pro Tyr Asn Leu Gln Tyr Thr Val Glu
   850 855 860
   Val Lys Asn Gly Ser Leu Ile Ile Pro His Tyr Asp His Tyr His Asn
   865 870 875 880
   Ile Lys Phe Glu Trp Phe Asp Glu Gly Leu Tyr Glu Ala Pro Lys Gly
   885 890 895
   Tyr Thr Leu Glu Asp Leu Leu Ala Thr Val Lys Tyr Tyr Val Glu His
               900                 905                 910
   Pro Asn Glu Arg Pro His Ser Asp Asn Gly Phe Gly Asn Ala Ser Asp
           915                 920                 925
   His Val Gln Arg Asn Lys Asn Gly Gln Ala Asp Thr Asn Gln Thr Glu
       930                 935                 940
   Lys Pro Ser Glu Glu Lys Pro Gln Thr Glu Lys Pro Glu Glu Glu Thr
   945                 950                 955                 960
   Pro Arg Glu Glu Lys Pro Gln Ser Glu Lys Pro Glu Ser Pro Lys Pro
                   965                 970                 975
   Thr Glu Glu Pro Glu Glu Glu Ser Pro Glu Glu Ser Glu Glu Pro Gln
               980                 985                 990
   Val Glu Thr Glu Lys Val Glu Glu Lys Leu Arg Glu Ala Glu Asp Leu
           995                 1000                1005
   Leu Gly Lys Ile Gln Asp Pro Ile Ile Lys Ser Asn Ala Lys Glu Thr
       1010                1015                1020
   Leu Thr Gly Leu Lys Asn Asn Leu Leu Phe Gly Thr Gln Asp Asn Asn
   1025                1030                1035                1040
   Thr Ile Met Ala Glu Ala Glu Lys Leu Leu Ala Leu Leu Lys Glu Ser
                   1045                1050                1055
   Lys
<210> 59
   <211> 205
   <212> PRT
   <213> S. pneumoniae
<400> 59
   Cys Ala Tyr Ala Leu Asn Gln His Arg Ser Gln Glu Asn Lys Asp Asn
    1               5                  10                  15
   Asn Arg Val Ser Tyr Val Asp Gly Ser Gln Ser Ser Gln Lys Ser Glu
               20                  25                  30
   Asn Leu Thr Pro Asp Gln Val Ser Gln Lys Glu Gly Ile Gln Ala Glu
           35                  40                  45
   Gln Ile Val Ile Lys Ile Thr Asp Gln Gly Tyr Val Thr Ser His Gly
       50                  55                  60
   Asp His Tyr His Tyr Tyr Asn Gly Lys Val Pro Tyr Asp Ala Leu Phe
   65                  70                  75                  80
   Ser Glu Glu Leu Leu Met Lys Asp Pro Asn Tyr Gln Leu Lys Asp Ala
                   85                  90                  95
   Asp Ile Val Asn Glu Val Lys Gly Gly Tyr Ile Ile Lys Val Asp Gly
               100                 105                 110
   Lys Tyr Tyr Val Tyr Leu Lys Asp Ala Ala His Ala Asp Asn Val Arg
           115                 120                 125
   Thr Lys Asp Glu Ile Asn Arg Gln Lys Gln Glu His Val Lys Asp Asn
       130                 135                 140
   Glu Lys Val Asn Ser Asn Val Ala Val Ala Arg Ser Gln Gly Arg Tyr
   145                 150                 155                 160
   Thr Thr Asn Asp Gly Tyr Val Phe Asn Pro Ala Asp Ile Ile Glu Asp
                   165                 170                 175
   Thr Gly Asn Ala Tyr Ile Val Pro His Gly Gly His Tyr His Tyr Ile
               180                 185                 190
   Pro Lys Ser Asp Leu Ser Ala Ser Glu Leu Ala Ala Ala
           195                 200                 205
<210> 60
   <211> 821
   <212> PRT
   <213> S. pneumoniae
<400> 60
   Cys Ala Tyr Glu Leu Gly Leu His Gln Ala Gln Thr Val Lys Glu Asn
   1               5                   10                  15
   Asn Arg Val Ser Tyr Ile Asp Gly Lys Gln Ala Thr Gln Lys Thr Glu
               20                  25                  30
   Asn Leu Thr Pro Asp Glu Val Ser Lys Arg Glu Gly Ile Asn Ala Glu
           35                  40                  45
   Gln Ile Val Ile Lys Ile Thr Asp Gln Gly Tyr Val Thr Ser His Gly
       50                  55                  60
   Asp His Tyr His Tyr Tyr Asn Gly Lys Val Pro Tyr Asp Ala Ile Ile
   65                  70                  75                  80
   Ser Glu Glu Leu Leu Met Lys Asp Pro Asn Tyr Gln Leu Lys Asp Ser
                   85                  90                  95
   Asp Ile Val Asn Glu Ile Lys Gly Gly Tyr Val Ile Lys Val Asn Gly
               100                 105                 110
   Lys Tyr Tyr Val Tyr Leu Lys Asp Ala Ala His Ala Asp Asn Val Arg
           115                 120                 125
   Thr Lys Glu Glu Ile Asn Arg Gln Lys Gln Glu His Ser Gln His Arg
       130                 135                 140
   Glu Gly Gly Thr Ser Ala Asn Asp Gly Ala Val Ala Phe Ala Arg Ser
   145                 150                 155                 160
   Gln Gly Arg Tyr Thr Thr Asp Asp Gly Tyr Ile Phe Asn Ala Ser Asp
                   165                 170                 175
   Ile Ile Glu Asp Thr Gly Asp Ala Tyr Ile Val Pro His Gly Asp His
               180                 185                 190
   Tyr His Tyr Ile Pro Lys Asn Glu Leu Ser Ala Ser Glu Leu Ala Ala
           195                 200                 205
   Ala Glu Ala Phe Leu Ser Gly Arg Glu Asn Leu Ser Asn Leu Arg Thr
       210                 215                 220
   Tyr Arg Arg Gln Asn Ser Asp Asn Thr Pro Arg Thr Asn Trp Val Pro
   225                 230                 235                 240
   Ser Val Ser Asn Pro Gly Thr Thr Asn Thr Asn Thr Ser Asn Asn Ser
                   245                 250                 255
   Asn Thr Asn Ser Gln Ala Ser Gln Ser Asn Asp Ile Asp Ser Leu Leu
               260                 265                 270
   Lys Gln Leu Tyr Lys Leu Pro Leu Ser Gln Arg His Val Glu Ser Asp
           275                 280                 285
   Gly Leu Ile Phe Asp Pro Ala Gln Ile Thr Ser Arg Thr Ala Arg Gly
       290                 295                 300
   Val Ala Val Pro His Gly Asn His Tyr His Phe Ile Pro Tyr Glu Gln
   305                 310                 315                 320
   Met Ser Glu Leu Glu Lys Arg Ile Ala Arg Ile Ile Pro Leu Arg Tyr
                   325                 330                 335
   Arg Ser Asn His Trp Val Pro Asp Ser Arg Pro Glu Glu Pro Ser Pro
               340                 345                 350
   Gln Pro Thr Pro Glu Pro Ser Pro Ser Pro Gln Pro Ala Pro Asn Pro
           355                 360                 365
   Gln Pro Ala Pro Ser Asn Pro Ile Asp Glu Lys Leu Val Lys Glu Ala
       370                 375                 380
   Val Arg Lys Val Gly Asp Gly Tyr Val Phe Glu Glu Asn Gly Val Ser
   385                 390                 395                 400
   Arg Tyr Ile Pro Ala Lys Asn Leu Ser Ala Glu Thr Ala Ala Gly Ile
                   405                 410                 415
   Asp Ser Lys Leu Ala Lys Gln Glu Ser Leu Ser His Lys Leu Gly Ala
               420                 425                 430
   Lys Lys Thr Asp Leu Pro Ser Ser Asp Arg Glu Phe Tyr Asn Lys Ala
           435                 440                 445
   Tyr Asp Leu Leu Ala Arg Ile His Gln Asp Leu Leu Asp Asn Lys Gly
       450                 455                 460
   Arg Gln Val Asp Phe Glu Ala Leu Asp Asn Leu Leu Glu Arg Leu Lys
   465                 470                 475                 480
   Asp Val Ser Ser Asp Lys Val Lys Leu Val Asp Asp Ile Leu Ala Phe
                   485                 490                 495
   Leu Ala Pro Ile Arg His Pro Glu Arg Leu Gly Lys Pro Asn Ala Gln
               500                 505                 510
   Ile Thr Tyr Thr Asp Asp Glu Ile Gln Val Ala Lys Leu Ala Gly Lys
           515                 520                 525
   Tyr Thr Thr Glu Asp Gly Tyr Ile Phe Asp Pro Arg Asp Ile Thr Ser
       530                 535                 540
   Asp Glu Gly Asp Ala Tyr Val Thr Pro His Met Thr His Ser His Trp
   545                 550                 555                 560
   Ile Lys Lys Asp Ser Leu Ser Glu Ala Glu Arg Ala Ala Ala Gln Ala
                   565                 570                 575
   Tyr Ala Lys Glu Lys Gly Leu Thr Pro Pro Ser Thr Asp His Gln Asp
               580                 585                 590
   Ser Gly Asn Thr Glu Ala Lys Gly Ala Glu Ala Ile Tyr Asn Arg Val
           595                 600                 605
   Lys Ala Ala Lys Lys Val Pro Leu Asp Arg Met Pro Tyr Asn Leu Gln
       610                 615                 620
   Tyr Thr Val Glu Val Lys Asn Gly Ser Leu Ile Ile Pro His Tyr Asp
   625                 630                 635                 640
   His Tyr His Asn Ile Lys Phe Glu Trp Phe Asp Glu Gly Leu Tyr Glu
                   645                 650                 655
   Ala Pro Lys Gly Tyr Thr Leu Glu Asp Leu Leu Ala Thr Val Lys Tyr
               660                 665                 670
   Tyr Val Glu His Pro Asn Glu Arg Pro His Ser Asp Asn Gly Phe Gly
           675                 680                 685
   Asn Ala Ser Asp His Val Gln Arg Asn Lys Asn Gly Gln Ala Asp Thr
       690                 695                 700
   Asn Gln Thr Glu Lys Pro Ser Glu Glu Lys Pro Gln Thr Glu Lys Pro
   705                 710                 715                 720
   Glu Glu Glu Thr Pro Arg Glu Glu Lys Pro Gln Ser Glu Lys Pro Glu
                   725                 730                 735
   Ser Pro Lys Pro Thr Glu Glu Pro Glu Glu Glu Ser Pro Glu Glu Ser
               740                 745                 750
   Glu Glu Pro Gln Val Glu Thr Glu Lys Val Glu Glu Lys Leu Arg Glu
           755                 760                 765
   Ala Glu Asp Leu Leu Gly Lys Ile Gln Asp Pro Ile Ile Lys Ser Asn
       770                 775                 780
   Ala Lys Glu Thr Leu Thr Gly Leu Lys Asn Asn Leu Leu Phe Gly Thr
   785                 790                 795                 800
   Gln Asp Asn Asn Thr Ile Met Ala Glu Ala Glu Lys Leu Leu Ala Leu
                   805                 810                 815
   Leu Lys Glu Ser Lys
               820
<210> 61
   <211> 334
   <212> PRT
   <213> S. pneumoniae
<400> 61
   Cys Ala Tyr Glu Leu Gly Leu His Gln Ala Gln Thr Val Lys Glu Asn
    1               5                  10                  15
   Asn Arg Val Ser Tyr Ile Asp Gly Lys Gln Ala Thr Gln Lys Thr Glu
               20                  25                  30
   Asn Leu Thr Pro Asp Glu Val Ser Lys Arg Glu Gly Ile Asn Ala Glu
           35                  40                  45
   Gln Ile Val Ile Lys Ile Thr Asp Gln Gly Tyr Val Thr Ser His Gly
       50                  55                  60
   Asp His Tyr His Tyr Tyr Asn Gly Lys Val Pro Tyr Asp Ala Ile Ile
   65                  70                  75                  80
   Ser Glu Glu Leu Leu Met Lys Asp Pro Asn Tyr Gln Leu Lys Asp Ser
                   85                  90                  95
   Asp Ile Val Asn Glu Ile Lys Gly Gly Tyr Val Ile Lys Val Asn Gly
               100                 105                 110
   Lys Tyr Tyr Val Tyr Leu Lys Asp Ala Ala His Ala Asp Asn Val Arg
           115                 120                 125
   Thr Lys Glu Glu Ile Asn Arg Gln Lys Gln Glu His Ser Gln His Arg
       130                 135                 140
   Glu Gly Gly Thr Ser Ala Asn Asp Gly Ala Val Ala Phe Ala Arg Ser
   145                 150                 155                 160
   Gln Gly Arg Tyr Thr Thr Asp Asp Gly Tyr Ile Phe Asn Ala Ser Asp
                   165                 170                 175
   Ile Ile Glu Asp Thr Gly Asp Ala Tyr Ile Val Pro His Gly Asp His
               180                 185                 190
   Tyr His Tyr Ile Pro Lys Asn Glu Leu Ser Ala Ser Glu Leu Ala Ala
           195                 200                 205
   Ala Glu Ala Phe Leu Ser Gly Arg Glu Asn Leu Ser Asn Leu Arg Thr
       210                 215                 220
   Tyr Arg Arg Gln Asn Ser Asp Asn Thr Pro Arg Thr Asn Trp Val Pro
   225                 230                 235                 240
   Ser Val Ser Asn Pro Gly Thr Thr Asn Thr Asn Thr Ser Asn Asn Ser
                   245                 250                 255
   Asn Thr Asn Ser Gln Ala Ser Gln Ser Asn Asp Ile Asp Ser Leu Leu
               260                 265                 270
   Lys Gln Leu Tyr Lys Leu Pro Leu Ser Gln Arg His Val Glu Ser Asp
           275                 280                 285
   Gly Leu Ile Phe Asp Pro Ala Gln Ile Thr Ser Arg Thr Ala Arg Gly
       290                 295                 300
   Val Ala Val Pro His Gly Asn His Tyr His Phe Ile Pro Tyr Glu Gln
   305                 310                 315                 320
   Met Ser Glu Leu Glu Lys Arg Ile Ala Arg Ile Ile Pro Leu
                   325                 330
<210> 62
   <211> 487
   <212> PRT
   <213> S. pneumoniae
<400> 62
   Arg Tyr Arg Ser Asn His Trp Val Pro Asp Ser Arg Pro Glu Glu Pro
    1               5                  10                  15
   Ser Pro Gln Pro Thr Pro Glu Pro Ser Pro Ser Pro Gln Pro Ala Pro
               20                  25                  30
   Asn Pro Gln Pro Ala Pro Ser Asn Pro Ile Asp Glu Lys Leu Val Lys
           35                  40                  45
   Glu Ala Val Arg Lys Val Gly Asp Gly Tyr Val Phe Glu Glu Asn Gly
       50                  55                  60
   Val Ser Arg Tyr Ile Pro Ala Lys Asn Leu Ser Ala Glu Thr Ala Ala
   65                  70                  75                  80
   Gly Ile Asp Ser Lys Leu Ala Lys Gln Glu Ser Leu Ser His Lys Leu
                   85                  90                  95
   Gly Ala Lys Lys Thr Asp Leu Pro Ser Ser Asp Arg Glu Phe Tyr Asn
               100                 105                 110
   Lys Ala Tyr Asp Leu Leu Ala Arg Ile His Gln Asp Leu Leu Asp Asn
           115                 120                 125
   Lys Gly Arg Gln Val Asp Phe Glu Ala Leu Asp Asn Leu Leu Glu Arg
       130                 135                 140
   Leu Lys Asp Val Ser Ser Asp Lys Val Lys Leu Val Asp Asp Ile Leu
   145                 150                 155                 160
   Ala Phe Leu Ala Pro Ile Arg His Pro Glu Arg Leu Gly Lys Pro Asn
                   165                 170                 175
   Ala Gln Ile Thr Tyr Thr Asp Asp Glu Ile Gln Val Ala Lys Leu Ala
               180                 185                 190
   Gly Lys Tyr Thr Thr Glu Asp Gly Tyr Ile Phe Asp Pro Arg Asp Ile
           195                 200                 205
   Thr Ser Asp Glu Gly Asp Ala Tyr Val Thr Pro His Met Thr His Ser
       210                 215                 220
   His Trp Ile Lys Lys Asp Ser Leu Ser Glu Ala Glu Arg Ala Ala Ala
   225                 230                 235                 240
   Gln Ala Tyr Ala Lys Glu Lys Gly Leu Thr Pro Pro Ser Thr Asp His
                   245                 250                 255
   Gln Asp Ser Gly Asn Thr Glu Ala Lys Gly Ala Glu Ala Ile Tyr Asn
               260                 265                 270
   Arg Val Lys Ala Ala Lys Lys Val Pro Leu Asp Arg Met Pro Tyr Asn
           275                 280                 285
   Leu Gln Tyr Thr Val Glu Val Lys Asn Gly Ser Leu Ile Ile Pro His
       290                 295                 300
   Tyr Asp His Tyr His Asn Ile Lys Phe Glu Trp Phe Asp Glu Gly Leu
   305                 310                 315                 320
   Tyr Glu Ala Pro Lys Gly Tyr Thr Leu Glu Asp Leu Leu Ala Thr Val
                   325                 330                 335
   Lys Tyr Tyr Val Glu His Pro Asn Glu Arg Pro His Ser Asp Asn Gly
               340                 345                 350
   Phe Gly Asn Ala Ser Asp His Val Gln Arg Asn Lys Asn Gly Gln Ala
           355                 360                 365
   Asp Thr Asn Gln Thr Glu Lys Pro Ser Glu Glu Lys Pro Gln Thr Glu
       370                 375                 380
   Lys Pro Glu Glu Glu Thr Pro Arg Glu Glu Lys Pro Gln Ser Glu Lys
   385                 390                 395                 400
   Pro Glu Ser Pro Lys Pro Thr Glu Glu Pro Glu Glu Glu Ser Pro Glu
                   405                 410                 415
   Glu Ser Glu Glu Pro Gln Val Glu Thr Glu Lys Val Glu Glu Lys Leu
               420                 425                 430
   Arg Glu Ala Glu Asp Leu Leu Gly Lys Ile Gln Asp Pro Ile Ile Lys
           435                 440                 445
   Ser Asn Ala Lys Glu Thr Leu Thr Gly Leu Lys Asn Asn Leu Leu Phe
       450                 455                 460
   Gly Thr Gln Asp Asn Asn Thr Ile Met Ala Glu Ala Glu Lys Leu Leu
   465                 470                 475                 480
   Ala Leu Leu Lys Glu Ser Lys
                   485
<210> 63
   <211> 613
   <212> PRT
   <213> S. pneumoniae
<400> 63
   Ala Glu Ala Phe Leu Ser Gly Arg Glu Asn Leu Ser Asn Leu Arg Thr
   1               5                   10                  15
   Tyr Arg Arg Gln Asn Ser Asp Asn Thr Pro Arg Thr Asn Trp Val Pro
               20                  25                  30
   Ser Val Ser Asn Pro Gly Thr Thr Asn Thr Asn Thr Ser Asn Asn Ser
           35                  40                  45
   Asn Thr Asn Ser Gln Ala Ser Gln Ser Asn Asp Ile Asp Ser Leu Leu
       50                  55                  60
   Lys Gln Leu Tyr Lys Leu Pro Leu Ser Gln Arg His Val Glu Ser Asp
   65                  70                  75                  80
   Gly Leu Ile Phe Asp Pro Ala Gln Ile Thr Ser Arg Thr Ala Arg Gly
                   85                  90                  95
   Val Ala Val Pro His Gly Asn His Tyr His Phe Ile Pro Tyr Glu Gln
               100                 105                 110
   Met Ser Glu Leu Glu Lys Arg Ile Ala Arg Ile Ile Pro Leu Arg Tyr
           115                 120                 125
   Arg Ser Asn His Trp Val Pro Asp Ser Arg Pro Glu Glu Pro Ser Pro
       130                 135                 140
   Gln Pro Thr Pro Glu Pro Ser Pro Ser Pro Gln Pro Ala Pro Asn Pro
   145                 150                 155                 160
   Gln Pro Ala Pro Ser Asn Pro Ile Asp Glu Lys Leu Val Lys Glu Ala
                   165                 170                 175
   Val Arg Lys Val Gly Asp Gly Tyr Val Phe Glu Glu Asn Gly Val Ser
               180                 185                 190
   Arg Tyr Ile Pro Ala Lys Asn Leu Ser Ala Glu Thr Ala Ala Gly Ile
           195                 200                 205
   Asp Ser Lys Leu Ala Lys Gln Glu Ser Leu Ser His Lys Leu Gly Ala
       210                 215                 220
   Lys Lys Thr Asp Leu Pro Ser Ser Asp Arg Glu Phe Tyr Asn Lys Ala
   225                 230                 235                 240
   Tyr Asp Leu Leu Ala Arg Ile His Gln Asp Leu Leu Asp Asn Lys Gly
                   245                 250                 255
   Arg Gln Val Asp Phe Glu Ala Leu Asp Asn Leu Leu Glu Arg Leu Lys
               260                 265                 270
   Asp Val Ser Ser Asp Lys Val Lys Leu Val Asp Asp Ile Leu Ala Phe
           275                 280                 285
   Leu Ala Pro Ile Arg His Pro Glu Arg Leu Gly Lys Pro Asn Ala Gln
       290                 295                 300
   Ile Thr Tyr Thr Asp Asp Glu Ile Gln Val Ala Lys Leu Ala Gly Lys
   305                 310                 315                 320
   Tyr Thr Thr Glu Asp Gly Tyr Ile Phe Asp Pro Arg Asp Ile Thr Ser
                   325                 330                 335
   Asp Glu Gly Asp Ala Tyr Val Thr Pro His Met Thr His Ser His Trp
               340                 345                 350
   Ile Lys Lys Asp Ser Leu Ser Glu Ala Glu Arg Ala Ala Ala Gln Ala
           355                 360                 365
   Tyr Ala Lys Glu Lys Gly Leu Thr Pro Pro Ser Thr Asp His Gln Asp
       370                 375                 380
   Ser Gly Asn Thr Glu Ala Lys Gly Ala Glu Ala Ile Tyr Asn Arg Val
   385                 390                 395                 400
   Lys Ala Ala Lys Lys Val Pro Leu Asp Arg Met Pro Tyr Asn Leu Gln
                   405                 410                 415
   Tyr Thr Val Glu Val Lys Asn Gly Ser Leu Ile Ile Pro His Tyr Asp
               420                 425                 430
   His Tyr His Asn Ile Lys Phe Glu Trp Phe Asp Glu Gly Leu Tyr Glu
           435                 440                 445
   Ala Pro Lys Gly Tyr Thr Leu Glu Asp Leu Leu Ala Thr Val Lys Tyr
       450                 455                 460
   Tyr Val Glu His Pro Asn Glu Arg Pro His Ser Asp Asn Gly Phe Gly
   465                 470                 475                 480
   Asn Ala Ser Asp His Val Gln Arg Asn Lys Asn Gly Gln Ala Asp Thr
                   485                 490                 495
   Asn Gln Thr Glu Lys Pro Ser Glu Glu Lys Pro Gln Thr Glu Lys Pro
               500                 505                 510
   Glu Glu Glu Thr Pro Arg Glu Glu Lys Pro Gln Ser Glu Lys Pro Glu
           515                 520                 525
   Ser Pro Lys Pro Thr Glu Glu Pro Glu Glu Glu Ser Pro Glu Glu Ser
       530                 535                 540
   Glu Glu Pro Gln Val Glu Thr Glu Lys Val Glu Glu Lys Leu Arg Glu
   545                 550                 555                 560
   Ala Glu Asp Leu Leu Gly Lys Ile Gln Asp Pro Ile Ile Lys Ser Asn
                   565                 570                 575
   Ala Lys Glu Thr Leu Thr Gly Leu Lys Asn Asn Leu Leu Phe Gly Thr
               580                 585                 590
   Gln Asp Asn Asn Thr Ile Met Ala Glu Ala Glu Lys Leu Leu Ala Leu
           595                 600                 605
   Leu Lys Glu Ser Lys
       610
<210> 64
   <211> 568
   <212> PRT
   <213> S. pneumoniae
<400> 64
   Asp Leu Thr Glu Glu Gln Ile Lys Ala Ala Gln Lys His Leu Glu Glu
    1               5                  10                  15
   Val Lys Thr Ser His Asn Gly Leu Asp Ser Leu Ser Ser His Glu Gln
               20                  25                  30
   Asp Tyr Pro Gly Asn Ala Lys Glu Met Lys Asp Leu Asp Lys Lys Ile
           35                  40                  45
   Glu Glu Lys Ile Ala Gly Ile Met Lys Gln Tyr Gly Val Lys Arg Glu
       50                  55                  60
   Ser Ile Val Val Asn Lys Glu Lys Asn Ala Ile Ile Tyr Pro His Gly
   65                  70                  75                  80
   Asp His His His Ala Asp Pro Ile Asp Glu His Lys Pro Val Gly Ile
                   85                  90                  95
   Gly His Ser His Ser Asn Tyr Glu Leu Phe Lys Pro Glu Glu Gly Val
               100                 105                 110
   Ala Lys Lys Glu Gly Asn Lys Val Tyr Thr Gly Glu Glu Leu Thr Asn
           115                 120                 125
   Val Val Asn Leu Leu Lys Asn Ser Thr Phe Asn Asn Gln Asn Phe Thr
       130                 135                 140
   Leu Ala Asn Gly Gln Lys Arg Val Ser Phe Ser Phe Pro Pro Glu Leu
   145                 150                 155                 160
   Glu Lys Lys Leu Gly Ile Asn Met Leu Val Lys Leu Ile Thr Pro Asp
                   165                 170                 175
   Gly Lys Val Leu Glu Lys Val Ser Gly Lys Val Phe Gly Glu Gly Val
               180                 185                 190
   Gly Asn Ile Ala Asn Phe Glu Leu Asp Gln Pro Tyr Leu Pro Gly Gln
           195                 200                 205
   Thr Phe Lys Tyr Thr Ile Ala Ser Lys Asp Tyr Pro Glu Val Ser Tyr
       210                 215                 220
   Asp Gly Thr Phe Thr Val Pro Thr Ser Leu Ala Tyr Lys Met Ala Ser
   225                 230                 235                 240
   Gln Thr Ile Phe Tyr Pro Phe His Ala Gly Asp Thr Tyr Leu Arg Val
                   245                 250                 255
   Asn Pro Gln Phe Ala Val Pro Lys Gly Thr Asp Ala Leu Val Arg Val
               260                 265                 270
   Phe Asp Glu Phe His Gly Asn Ala Tyr Leu Glu Asn Asn Tyr Lys Val
           275                 280                 285
   Gly Glu Ile Lys Leu Pro Ile Pro Lys Leu Asn Gln Gly Thr Thr Arg
       290                 295                 300
   Thr Ala Gly Asn Lys Ile Pro Val Thr Phe Met Ala Asn Ala Tyr Leu
   305                 310                 315                 320
   Asp Asn Gln Ser Thr Tyr Ile Val Glu Val Pro Ile Leu Glu Lys Glu
                   325                 330                 335
   Asn Gln Thr Asp Lys Pro Ser Ile Leu Pro Gln Phe Lys Arg Asn Lys
               340                 345                 350
   Ala Gln Glu Asn Ser Lys Leu Asp Glu Lys Val Glu Glu Pro Lys Thr
           355                 360                 365
   Ser Glu Lys Val Glu Lys Glu Lys Leu Ser Glu Thr Gly Asn Ser Thr
       370                 375                 380
   Ser Asn Ser Thr Leu Glu Glu Val Pro Thr Val Asp Pro Val Gln Glu
   385                 390                 395                 400
   Lys Val Ala Lys Phe Ala Glu Ser Tyr Gly Met Lys Leu Glu Asn Val
                   405                 410                 415
   Leu Phe Asn Met Asp Gly Thr Ile Glu Leu Tyr Leu Pro Ser Gly Glu
               420                 425                 430
   Val Ile Lys Lys Asn Met Ala Asp Phe Thr Gly Glu Ala Pro Gln Gly
           435                 440                 445
   Asn Gly Glu Asn Lys Pro Ser Glu Asn Gly Lys Val Ser Thr Gly Thr
       450                 455                 460
   Val Glu Asn Gln Pro Thr Glu Asn Lys Pro Ala Asp Ser Leu Pro Glu
   465                 470                 475                 480
   Ala Pro Asn Glu Lys Pro Val Lys Pro Glu Asn Ser Thr Asp Asn Gly
                   485                 490                 495
   Met Leu Asn Pro Glu Gly Asn Val Gly Ser Asp Pro Met Leu Asp Pro
               500                 505                 510
   Ala Leu Glu Glu Ala Pro Ala Val Asp Pro Val Gln Glu Lys Leu Glu
           515                 520                 525
   Lys Phe Thr Ala Ser Tyr Gly Leu Gly Leu Asp Ser Val Ile Phe Asn
       530                 535                 540
   Met Asp Gly Thr Ile Glu Leu Arg Leu Pro Ser Gly Glu Val Ile Lys
   545                 550                 555                 560
   Lys Asn Leu Ser Asp Phe Ile Ala
                   565
<210> 65
   <211> 329
   <212> PRT
   <213> S. pneumoniae
<400> 65
   Asp Leu Thr Glu Glu Gln Ile Lys Ala Ala Gln Lys His Leu Glu Glu
    1               5                  10                  15
   Val Lys Thr Ser His Asn Gly Leu Asp Ser Leu Ser Ser His Glu Gln
               20                  25                  30
   Asp Tyr Pro Gly Asn Ala Lys Glu Met Lys Asp Leu Asp Lys Lys Ile
           35                  40                  45
   Glu Glu Lys Ile Ala Gly Ile Met Lys Gln Tyr Gly Val Lys Arg Glu
       50                  55                  60
   Ser Ile Val Val Asn Lys Glu Lys Asn Ala Ile Ile Tyr Pro His Gly
   65                  70                  75                  80
   Asp His His His Ala Asp Pro Ile Asp Glu His Lys Pro Val Gly Ile
                   85                  90                  95
   Gly His Ser His Ser Asn Tyr Glu Leu Phe Lys Pro Glu Glu Gly Val
               100                 105                 110
   Ala Lys Lys Glu Gly Asn Lys Val Tyr Thr Gly Glu Glu Leu Thr Asn
           115                 120                 125
   Val Val Asn Leu Leu Lys Asn Ser Thr Phe Asn Asn Gln Asn Phe Thr
       130                 135                 140
   Leu Ala Asn Gly Gln Lys Arg Val Ser Phe Ser Phe Pro Pro Glu Leu
   145                 150                 155                 160
   Glu Lys Lys Leu Gly Ile Asn Met Leu Val Lys Leu Ile Thr Pro Asp
                   165                 170                 175
   Gly Lys Val Leu Glu Lys Val Ser Gly Lys Val Phe Gly Glu Gly Val
               180                 185                 190
   Gly Asn Ile Ala Asn Phe Glu Leu Asp Gln Pro Tyr Leu Pro Gly Gln
           195                 200                 205
   Thr Phe Lys Tyr Thr Ile Ala Ser Lys Asp Tyr Pro Glu Val Ser Tyr
       210                 215                 220
   Asp Gly Thr Phe Thr Val Pro Thr Ser Leu Ala Tyr Lys Met Ala Ser
   225                 230                 235                 240
   Gln Thr Ile Phe Tyr Pro Phe His Ala Gly Asp Thr Tyr Leu Arg Val
                   245                 250                 255
   Asn Pro Gln Phe Ala Val Pro Lys Gly Thr Asp Ala Leu Val Arg Val
               260                 265                 270
   Phe Asp Glu Phe His Gly Asn Ala Tyr Leu Glu Asn Asn Tyr Lys Val
           275                 280                 285
   Gly Glu Ile Lys Leu Pro Ile Pro Lys Leu Asn Gln Gly Thr Thr Arg
       290                 295                 300
   Thr Ala Gly Asn Lys Ile Pro Val Thr Phe Met Ala Asn Ala Tyr Leu
   305                 310                 315                 320
   Asp Asn Gln Ser Thr Tyr Ile Val Glu
                   325
<210> 66
   <211> 240
   <212> PRT
   <213> S. pneumoniae
<400> 66
   Glu Val Pro Ile Leu Glu Lys Glu Asn Gln Thr Asp Lys Pro Ser Ile
    1               5                  10                  15
   Leu Pro Gln Phe Lys Arg Asn Lys Ala Gln Glu Asn Ser Lys Leu Asp
               20                  25                  30
   Glu Lys Val Glu Glu Pro Lys Thr Ser Glu Lys Val Glu Lys Glu Lys
           35                  40                  45
   Leu Ser Glu Thr Gly Asn Ser Thr Ser Asn Ser Thr Leu Glu Glu Val
       50                  55                  60
   Pro Thr Val Asp Pro Val Gln Glu Lys Val Ala Lys Phe Ala Glu Ser
   65                  70                  75                  80
   Tyr Gly Met Lys Leu Glu Asn Val Leu Phe Asn Met Asp Gly Thr Ile
                   85                  90                  95
   Glu Leu Tyr Leu Pro Ser Gly Glu Val Ile Lys Lys Asn Met Ala Asp
               100                 105                 110
   Phe Thr Gly Glu Ala Pro Gln Gly Asn Gly Glu Asn Lys Pro Ser Glu
           115                 120                 125
   Asn Gly Lys Val Ser Thr Gly Thr Val Glu Asn Gln Pro Thr Glu Asn
       130                 135                 140
   Lys Pro Ala Asp Ser Leu Pro Glu Ala Pro Asn Glu Lys Pro Val Lys
   145                 150                 155                 160
   Pro Glu Asn Ser Thr Asp Asn Gly Met Leu Asn Pro Glu Gly Asn Val
                   165                 170                 175
   Gly Ser Asp Pro Met Leu Asp Pro Ala Leu Glu Glu Ala Pro Ala Val
               180                 185                 190
   Asp Pro Val Gln Glu Lys Leu Glu Lys Phe Thr Ala Ser Tyr Gly Leu
           195                 200                 205
   Gly Leu Asp Ser Val Ile Phe Asn Met Asp Gly Thr Ile Glu Leu Arg
       210                 215                 220
   Leu Pro Ser Gly Glu Val Ile Lys Lys Asn Leu Ser Asp Phe Ile Ala
   225                 230                 235                 240
<210> 67
   <211> 555
   <212> PRT
   <213> S. pneumoniae
<400> 67
   Asp Ile Asp Ser Leu Leu Lys Gln Leu Tyr Lys Leu Pro Leu Ser Gln
    1               5                  10                  15
   Arg His Val Glu Ser Asp Gly Leu Ile Phe Asp Pro Ala Gln Ile Thr
               20                  25                  30
   Ser Arg Thr Ala Arg Gly Val Ala Val Pro His Gly Asn His Tyr His
           35                  40                  45
   Phe Ile Pro Tyr Glu Gln Met Ser Glu Leu Glu Lys Arg Ile Ala Arg
       50                  55                  60
   Ile Ile Pro Leu Arg Tyr Arg Ser Asn His Trp Val Pro Asp Ser Arg
   65                  70                  75                  80
   Pro Glu Glu Pro Ser Pro Gln Pro Thr Pro Glu Pro Ser Pro Ser Pro
                   85                  90                  95
   Gln Pro Ala Pro Asn Pro Gln Pro Ala Pro Ser Asn Pro Ile Asp Glu
               100                 105                 110
   Lys Leu Val Lys Glu Ala Val Arg Lys Val Gly Asp Gly Tyr Val Phe
           115                 120                 125
   Glu Glu Asn Gly Val Ser Arg Tyr Ile Pro Ala Lys Asn Leu Ser Ala
       130                 135                 140
   Glu Thr Ala Ala Gly Ile Asp Ser Lys Leu Ala Lys Gln Glu Ser Leu
   145                 150                 155                 160
   Ser His Lys Leu Gly Ala Lys Lys Thr Asp Leu Pro Ser Ser Asp Arg
                   165                 170                 175
   Glu Phe Tyr Asn Lys Ala Tyr Asp Leu Leu Ala Arg Ile His Gln Asp
               180                 185                 190
   Leu Leu Asp Asn Lys Gly Arg Gln Val Asp Phe Glu Ala Leu Asp Asn
           195                 200                 205
   Leu Leu Glu Arg Leu Lys Asp Val Ser Ser Asp Lys Val Lys Leu Val
       210                 215                 220
   Asp Asp Ile Leu Ala Phe Leu Ala Pro Ile Arg His Pro Glu Arg Leu
   225                 230                 235                 240
   Gly Lys Pro Asn Ala Gln Ile Thr Tyr Thr Asp Asp Glu Ile Gln Val
                   245                 250                 255
   Ala Lys Leu Ala Gly Lys Tyr Thr Thr Glu Asp Gly Tyr Ile Phe Asp
               260                 265                 270
   Pro Arg Asp Ile Thr Ser Asp Glu Gly Asp Ala Tyr Val Thr Pro His
           275                 280                 285
   Met Thr His Ser His Trp Ile Lys Lys Asp Ser Leu Ser Glu Ala Glu
       290                 295                 300
   Arg Ala Ala Ala Gln Ala Tyr Ala Lys Glu Lys Gly Leu Thr Pro Pro
   305                 310                 315                 320
   Ser Thr Asp His Gln Asp Ser Gly Asn Thr Glu Ala Lys Gly Ala Glu
                   325                 330                 335
   Ala Ile Tyr Asn Arg Val Lys Ala Ala Lys Lys Val Pro Leu Asp Arg
               340                 345                 350
   Met Pro Tyr Asn Leu Gln Tyr Thr Val Glu Val Lys Asn Gly Ser Leu
           355                 360                 365
   Ile Ile Pro His Tyr Asp His Tyr His Asn Ile Lys Phe Glu Trp Phe
       370                 375                 380
   Asp Glu Gly Leu Tyr Glu Ala Pro Lys Gly Tyr Thr Leu Glu Asp Leu
   385                 390                 395                 400
   Leu Ala Thr Val Lys Tyr Tyr Val Glu His Pro Asn Glu Arg Pro His
                   405                 410                 415
   Ser Asp Asn Gly Phe Gly Asn Ala Ser Asp His Val Gln Arg Asn Lys
               420                 425                 430
   Asn Gly Gln Ala Asp Thr Asn Gln Thr Glu Lys Pro Ser Glu Glu Lys
           435                 440                 445
   Pro Gln Thr Glu Lys Pro Glu Glu Glu Thr Pro Arg Glu Glu Lys Pro
       450                 455                 460
   Gln Ser Glu Lys Pro Glu Ser Pro Lys Pro Thr Glu Glu Pro Glu Glu
   465                 470                 475                 480
   Glu Ser Pro Glu Glu Ser Glu Glu Pro Gln Val Glu Thr Glu Lys Val
                   485                 490                 495
   Glu Glu Lys Leu Arg Glu Ala Glu Asp Leu Leu Gly Lys Ile Gln Asp
               500                 505                 510
   Pro Ile Ile Lys Ser Asn Ala Lys Glu Thr Leu Thr Gly Leu Lys Asn
           515                 520                 525
   Asn Leu Leu Phe Gly Thr Gln Asp Asn Asn Thr Ile Met Ala Glu Ala
       530                 535                 540
   Glu Lys Leu Leu Ala Leu Leu Lys Glu Ser Lys
   545                 550                 555
<210> 68
   <211> 428
   <212> PRT
   <213> S. pneumoniae
<400> 68
   Asp Ile Asp Ser Leu Leu Lys Gln Leu Tyr Lys Leu Pro Leu Ser Gln
   1               5                   10                  15
   Arg His Val Glu Ser Asp Gly Leu Ile Phe Asp Pro Ala Gln Ile Thr
               20                  25                  30
   Ser Arg Thr Ala Arg Gly Val Ala Val Pro His Gly Asn His Tyr His
           35                  40                  45
   Phe Ile Pro Tyr Glu Gln Met Ser Glu Leu Glu Lys Arg Ile Ala Arg
       50                  55                  60
   Ile Ile Pro Leu Arg Tyr Arg Ser Asn His Trp Val Pro Asp Ser Arg
   65                  70                  75                  80
   Pro Glu Glu Pro Ser Pro Gln Pro Thr Pro Glu Pro Ser Pro Ser Pro
                   85                  90                  95
   Gln Pro Ala Pro Asn Pro Gln Pro Ala Pro Ser Asn Pro Ile Asp Glu
               100                 105                 110
   Lys Leu Val Lys Glu Ala Val Arg Lys Val Gly Asp Gly Tyr Val Phe
           115                 120                 125
   Glu Glu Asn Gly Val Ser Arg Tyr Ile Pro Ala Lys Asn Leu Ser Ala
       130                 135                 140
   Glu Thr Ala Ala Gly Ile Asp Ser Lys Leu Ala Lys Gln Glu Ser Leu
   145                 150                 155                 160
   Ser His Lys Leu Gly Ala Lys Lys Thr Asp Leu Pro Ser Ser Asp Arg
                   165                 170                 175
   Glu Phe Tyr Asn Lys Ala Tyr Asp Leu Leu Ala Arg Ile His Gln Asp
               180                 185                 190
   Leu Leu Asp Asn Lys Gly Arg Gln Val Asp Phe Glu Ala Leu Asp Asn
           195                 200                 205
   Leu Leu Glu Arg Leu Lys Asp Val Ser Ser Asp Lys Val Lys Leu Val
       210                 215                 220
   Asp Asp Ile Leu Ala Phe Leu Ala Pro Ile Arg His Pro Glu Arg Leu
   225                 230                 235                 240
   Gly Lys Pro Asn Ala Gln Ile Thr Tyr Thr Asp Asp Glu Ile Gln Val
                   245                 250                 255
   Ala Lys Leu Ala Gly Lys Tyr Thr Thr Glu Asp Gly Tyr Ile Phe Asp
               260                 265                 270
   Pro Arg Asp Ile Thr Ser Asp Glu Gly Asp Ala Tyr Val Thr Pro His
           275                 280                 285
   Met Thr His Ser His Trp Ile Lys Lys Asp Ser Leu Ser Glu Ala Glu
       290                 295                 300
   Arg Ala Ala Ala Gln Ala Tyr Ala Lys Glu Lys Gly Leu Thr Pro Pro
   305                 310                 315                 320
   Ser Thr Asp His Gln Asp Ser Gly Asn Thr Glu Ala Lys Gly Ala Glu
                   325                 330                 335
   Ala Ile Tyr Asn Arg Val Lys Ala Ala Lys Lys Val Pro Leu Asp Arg
               340                 345                 350
   Met Pro Tyr Asn Leu Gln Tyr Thr Val Glu Val Lys Asn Gly Ser Leu
           355                 360                 365
   Ile Ile Pro His Tyr Asp His Tyr His Asn Ile Lys Phe Glu Trp Phe
       370                 375                 380
   Asp Glu Gly Leu Tyr Glu Ala Pro Lys Gly Tyr Thr Leu Glu Asp Leu
   385                 390                 395                 400
   Leu Ala Thr Val Lys Tyr Tyr Val Glu His Pro Asn Glu Arg Pro His
                   405                 410                 415
   Ser Asp Asn Gly Phe Gly Asn Ala Ser Asp His Val
               420                 425
<210> 69
   <211> 121
   <212> PRT
   <213> S. pneumoniae
<400> 69
   Gly Leu Tyr Glu Ala Pro Lys Gly Tyr Thr Leu Glu Asp Leu Leu Ala
    1               5                  10                  15
   Thr Val Lys Tyr Tyr Val Glu His Pro Asn Glu Arg Pro His Ser Asp
               20                  25                  30
   Asn Gly Phe Gly Asn Ala Ser Asp His Val Gln Arg Asn Lys Asn Gly
           35                  40                  45
   Gln Ala Asp Thr Asn Gln Thr Glu Lys Pro Ser Glu Glu Lys Pro Gln
       50                  55                  60
   Thr Glu Lys Pro Glu Glu Glu Thr Pro Arg Glu Glu Lys Pro Gln Ser
   65                  70                  75                  80
   Glu Lys Pro Glu Ser Pro Lys Pro Thr Glu Glu Pro Glu Glu Glu Ser
                   85                  90                  95
   Pro Glu Glu Ser Glu Glu Pro Gln Val Glu Thr Glu Lys Val Glu Glu
               100                 105                 110
   Lys Leu Arg Glu Ala Glu Asp Leu Leu
           115                 120
<210> 70
   <211> 132
   <212> PRT
   <213> S. pneumoniae
<400> 70
   Ala Ser Asp His Val Gln Arg Asn Lys Asn Gly Gln Ala Asp Thr Asn
    1               5                  10                  15
   Gln Thr Glu Lys Pro Ser Glu Glu Lys Pro Gln Thr Glu Lys Pro Glu
               20                  25                  30
   Glu Glu Thr Pro Arg Glu Glu Lys Pro Gln Ser Glu Lys Pro Glu Ser
           35                  40                  45
   Pro Lys Pro Thr Glu Glu Pro Glu Glu Glu Ser Pro Glu Glu Ser Glu
       50                  55                  60
   Glu Pro Gln Val Glu Thr Glu Lys Val Glu Glu Lys Leu Arg Glu Ala
   65                  70                  75                  80
   Glu Asp Leu Leu Gly Lys Ile Gln Asp Pro Ile Ile Lys Ser Asn Ala
                   85                  90                  95
   Lys Glu Thr Leu Thr Gly Leu Lys Asn Asn Leu Leu Phe Gly Thr Gln
               100                 105                 110
   Asp Asn Asn Thr Ile Met Ala Glu Ala Glu Lys Leu Leu Ala Leu Leu
           115                 120                 125
   Lys Glu Ser Lys
       130
<210> 71
   <211> 226
   <212> PRT
   <213> S. pneumoniae
<400> 71
   Asp Ile Asp Ser Leu Leu Lys Gln Leu Tyr Lys Leu Pro Leu Ser Gln
    1               5                  10                  15
   Arg His Val Glu Ser Asp Gly Leu Ile Phe Asp Pro Ala Gln Ile Thr
               20                  25                  30
   Ser Arg Thr Ala Arg Gly Val Ala Val Pro His Gly Asn His Tyr His
           35                  40                  45
   Phe Ile Pro Tyr Glu Gln Met Ser Glu Leu Glu Lys Arg Ile Ala Arg
       50                  55                  60
   Ile Ile Pro Leu Arg Tyr Arg Ser Asn His Trp Val Pro Asp Ser Arg
   65                  70                  75                  80
   Pro Glu Glu Pro Ser Pro Gln Pro Thr Pro Glu Pro Ser Pro Ser Pro
                   85                  90                  95
   Gln Pro Ala Pro Asn Pro Gln Pro Ala Pro Ser Asn Pro Ile Asp Glu
               100                 105                 110
   Lys Leu Val Lys Glu Ala Val Arg Lys Val Gly Asp Gly Tyr Val Phe
           115                 120                 125
   Glu Glu Asn Gly Val Ser Arg Tyr Ile Pro Ala Lys Asn Leu Ser Ala
       130                 135                 140
   Glu Thr Ala Ala Gly Ile Asp Ser Lys Leu Ala Lys Gln Glu Ser Leu
   145                 150                 155                 160
   Ser His Lys Leu Gly Ala Lys Lys Thr Asp Leu Pro Ser Ser Asp Arg
                   165                 170                 175
   Glu Phe Tyr Asn Lys Ala Tyr Asp Leu Leu Ala Arg Ile His Gln Asp
               180                 185                 190
   Leu Leu Asp Asn Lys Gly Arg Gln Val Asp Phe Glu Ala Leu Asp Asn
           195                 200                 205
   Leu Leu Glu Arg Leu Lys Asp Val Ser Ser Asp Lys Val Lys Leu Val
               210                 215                 220
   Asp Asp
   225
<210> 72
   <211> 203
   <212> PRT
   <213> S. pneumoniae
<400> 72
   Asp Ile Leu Ala Phe Leu Ala Pro Ile Arg His Pro Glu Arg Leu Gly
   1 5 10 15
   Lys Pro Asn Ala Gln Ile Thr Tyr Thr Asp Asp Glu Ile Gln Val Ala
   20 25 30
   Lys Leu Ala Gly Lys Tyr Thr Thr Glu Asp Gly Tyr Ile Phe Asp Pro
   35 40 45
   Arg Asp Ile Thr Ser Asp Glu Gly Asp Ala Tyr Val Thr Pro His Met
   50 55 60
   Thr His Ser His Trp Ile Lys Lys Asp Ser Leu Ser Glu Ala Glu Arg
   65 70 75 80
   Ala Ala Ala Gln Ala Tyr Ala Lys Glu Lys Gly Leu Thr Pro Pro Ser
   85 90 95
   Thr Asp His Gln Asp Ser Gly Asn Thr Glu Ala Lys Gly Ala Glu Ala
   100 105 110
   Ile Tyr Asn Arg Val Lys Ala Ala Lys Lys Val Pro Leu Asp Arg Met
   115 120 125
   Pro Tyr Asn Leu Gln Tyr Thr Val Glu Val Lys Asn Gly Ser Leu Ile
   130 135 140
   Ile Pro His Tyr Asp His Tyr His Asn Ile Lys Phe Glu Trp Phe Asp
   145 150 155 160
   Glu Gly Leu Tyr Glu Ala Pro Lys Gly Tyr Thr Leu Glu Asp Leu Leu
   165 170 175
   Ala Thr Val Lys Tyr Tyr Val Glu His Pro Asn Glu Arg Pro His Ser
   180 185 190
   Asp Asn Gly Phe Gly Asn Ala Ser Asp His Val
   195 200
<210> 73
   <211> 819
   <212> PRT
   <213> S. pneumoniae
<400> 73
   Cys Ser Tyr Glu Leu Gly Arg His Gln Ala Gly Gln Val Lys Lys Glu
   1 5 10 15
   Ser Asn Arg Val Ser Tyr Ile Asp Gly Asp Gln Ala Gly Gln Lys Ala
   20 25 30
   Glu Asn Leu Thr Pro Asp Glu Val Ser Lys Arg Glu Gly Ile Asn Ala
   35 40 45
   Glu Gln Ile Val Ile Lys Ile Thr Asp Gln Gly Tyr Val Thr Ser His
   50 55 60
   Gly Asp His Tyr His Tyr Tyr Asn Gly Lys Val Pro Tyr Asp Ala Ile
   65 70 75 80
   Ile Ser Glu Glu Leu Leu Met Lys Asp Pro Asn Tyr Gln Leu Lys Asp
   85 90 95
   Ser Asp Ile Val Asn Glu Ile Lys Gly Gly Tyr Val Ile Lys Val Asp
   100 105 110
   Gly Lys Tyr Tyr Val Tyr Leu Lys Asp Ala Ala His Ala Asp Asn Ile
               115                 120                 125
   Arg Thr Lys Glu Glu Ile Lys Arg Gln Lys Gln Glu His Ser His Asn
           130                 135                 140
   His Asn Ser Arg Ala Asp Asn Ala Val Ala Ala Ala Arg Ala Gln Gly
   145                 150                 155                 160
   Arg Tyr Thr Thr Asp Asp Gly Tyr Ile Phe Asn Ala Ser Asp Ile Ile
                   165                 170                 175
   Glu Asp Thr Gly Asp Ala Tyr Ile Val Pro His Gly Asp His Tyr His
               180                 185                 190
   Tyr Ile Pro Lys Asn Glu Leu Ser Ala Ser Glu Leu Ala Ala Ala Glu
           195                 200                 205
   Ala Tyr Trp Asn Gly Lys Gln Gly Ser Arg Pro Ser Ser Ser Ser Ser
       210                 215                 220
   Tyr Asn Ala Asn Pro Val Gln Pro Arg Leu Ser Glu Asn His Asn Leu
   225                 230                 235                 240
   Thr Val Thr Pro Thr Tyr His Gln Asn Gln Gly Glu Asn Ile Ser Ser
                   245                 250                 255
   Leu Leu Arg Glu Leu Tyr Ala Lys Pro Leu Ser Glu Arg His Val Glu
               260                 265                 270
   Ser Asp Gly Leu Ile Phe Asp Pro Ala Gln Ile Thr Ser Arg Thr Ala
           275                 280                 285
   Arg Gly Val Ala Val Pro His Gly Asn His Tyr His Phe Ile Pro Tyr
       290                 295                 300
   Glu Gln Met Ser Glu Leu Glu Lys Arg Ile Ala Arg Ile Ile Pro Leu
   305                 310                 315                 320
   Arg Tyr Arg Ser Asn His Trp Val Pro Asp Ser Arg Pro Glu Gln Pro
                   325                 330                 335
   Ser Pro Gln Ser Thr Pro Glu Pro Ser Pro Ser Leu Gln Pro Ala Pro
               340                 345                 350
   Asn Pro Gln Pro Ala Pro Ser Asn Pro Ile Asp Glu Lys Leu Val Lys
           355                 360                 365
   Glu Ala Val Arg Lys Val Gly Asp Gly Tyr Val Phe Glu Glu Asn Gly
       370                 375                 380
   Val Ser Arg Tyr Ile Pro Ala Lys Asp Leu Ser Ala Glu Thr Ala Ala
   385                 390                 395                 400
   Gly Ile Asp Ser Lys Leu Ala Lys Gln Glu Ser Leu Ser His Lys Leu
                   405                 410                 415
   Gly Ala Lys Lys Thr Asp Leu Pro Ser Ser Asp Arg Glu Phe Tyr Asn
               420                 425                 430
   Lys Ala Tyr Asp Leu Leu Ala Arg Ile His Gln Asp Leu Leu Asp Asn
           435                 440                 445
   Lys Gly Arg Gln Val Asp Phe Glu Val Leu Asp Asn Leu Leu Glu Arg
       450                 455                 460
   Leu Lys Asp Val Ser Ser Asp Lys Val Lys Leu Val Asp Asp Ile Leu
   465                 470                 475                 480
   Ala Phe Leu Ala Pro Ile Arg His Pro Glu Arg Leu Gly Lys Pro Asn
                   485                 490                 495
   Ala Gln Ile Thr Tyr Thr Asp Asp Glu Ile Gln Val Ala Lys Leu Ala
               500                 505                 510
   Gly Lys Tyr Thr Thr Glu Asp Gly Tyr Ile Phe Asp Pro Arg Asp Ile
           515                 520                 525
   Thr Ser Asp Glu Gly Asp Ala Tyr Val Thr Pro His Met Thr His Ser
       530                 535                 540
   His Trp Ile Lys Lys Asp Ser Leu Ser Glu Ala Glu Arg Ala Ala Ala
   545                 550                 555                 560
   Gln Ala Tyr Ala Lys Glu Lys Gly Leu Thr Pro Pro Ser Thr Asp His
                   565                 570                 575
   Gln Asp Ser Gly Asn Thr Glu Ala Lys Gly Ala Glu Ala Ile Tyr Asn
                   580                 585                 590
   Arg Val Lys Ala Ala Lys Lys Val Pro Leu Asp Arg Met Pro Tyr Asn
               595                 600                 605
   Leu Gln Tyr Thr Val Glu Val Lys Asn Gly Ser Leu Ile Ile Pro His
       610                 615                 620
   Tyr Asp His Tyr His Asn Ile Lys Phe Glu Trp Phe Asp Glu Gly Leu
   625                 630                 635                 640
   Tyr Glu Ala Pro Lys Gly Tyr Ser Leu Glu Asp Leu Leu Ala Thr Val
                   645                 650                 655
   Lys Tyr Tyr Val Glu His Pro Asn Glu Arg Pro His Ser Asp Asn Gly
               660                 665                 670
   Phe Gly Asn Ala Ser Asp His Val Arg Lys Asn Lys Ala Asp Gln Asp
           675                 680                 685
   Ser Lys Pro Asp Glu Asp Lys Glu His Asp Glu Val Ser Glu Pro Thr
       690                 695                 700
   His Pro Glu Ser Asp Glu Lys Glu Asn His Ala Gly Leu Asn Pro Ser
   705                 710                 715                 720
   Ala Asp Asn Leu Tyr Lys Pro Ser Thr Asp Thr Glu Glu Thr Glu Glu
                   725                 730                 735
   Glu Ala Glu Asp Thr Thr Asp Glu Ala Glu Ile Pro Gln Val Glu Asn
               740                 745                 750
   Ser Val Ile Asn Ala Lys Ile Ala Asp Ala Glu Ala Leu Leu Glu Lys
           755                 760                 765
   Val Thr Asp Pro Ser Ile Arg Gln Asn Ala Met Glu Thr Leu Thr Gly
       770                 775                 780
   Leu Lys Ser Ser Leu Leu Leu Gly Thr Lys Asp Asn Asn Thr Ile Ser
   785                 790                 795                 800
   Ala Glu Val Asp Ser Leu Leu Ala Leu Leu Lys Glu Ser Gln Pro Ala
                   805                 810                 815
   Pro Ile Gln
<210> 74
   <211> 568
   <212> PRT
   <213> S. pneumoniae
<400> 74
   Glu Asn Ile Ser Ser Leu Leu Arg Glu Leu Tyr Ala Lys Pro Leu Ser
    1               5                  10                  15
   Glu Arg His Val Glu Ser Asp Gly Leu Ile Phe Asp Pro Ala Gln Ile
               20                  25                  30
   Thr Ser Arg Thr Ala Arg Gly Val Ala Val Pro His Gly Asn His Tyr
           35                  40                  45
   His Phe Ile Pro Tyr Glu Gln Met Ser Glu Leu Glu Lys Arg Ile Ala
       50                  55                  60
   Arg Ile Ile Pro Leu Arg Tyr Arg Ser Asn His Trp Val Pro Asp Ser
   65                  70                  75                  80
   Arg Pro Glu Gln Pro Ser Pro Gln Ser Thr Pro Glu Pro Ser Pro Ser
                   85                  90                  95
   Leu Gln Pro Ala Pro Asn Pro Gln Pro Ala Pro Ser Asn Pro Ile Asp
               100                 105                 110
   Glu Lys Leu Val Lys Glu Ala Val Arg Lys Val Gly Asp Gly Tyr Val
           115                 120                 125
   Phe Glu Glu Asn Gly Val Ser Arg Tyr Ile Pro Ala Lys Asp Leu Ser
       130                 135                 140
   Ala Glu Thr Ala Ala Gly Ile Asp Ser Lys Leu Ala Lys Gln Glu Ser
   145                 150                 155                 160
   Leu Ser His Lys Leu Gly Ala Lys Lys Thr Asp Leu Pro Ser Ser Asp
   165 170 175
   Arg Glu Phe Tyr Asn Lys Ala Tyr Asp Leu Leu Ala Arg Ile His Gln
   180 185 190
   Asp Leu Leu Asp Asn Lys Gly Arg Gln Val Asp Phe Glu Val Leu Asp
   195 200 205
   Asn Leu Leu Glu Arg Leu Lys Asp Val Ser Ser Asp Lys Val Lys Leu
   210 215 220
   Val Asp Asp Ile Leu Ala Phe Leu Ala Pro Ile Arg His Pro Glu Arg
   225 230 235 240
   Leu Gly Lys Pro Asn Ala Gln Ile Thr Tyr Thr Asp Asp Glu Ile Gln
   245 250 255
   Val Ala Lys Leu Ala Gly Lys Tyr Thr Thr Glu Asp Gly Tyr Ile Phe
   260 265 270
   Asp Pro Arg Asp Ile Thr Ser Asp Glu Gly Asp Ala Tyr Val Thr Pro
   275 280 285
   His Met Thr His Ser His Trp Ile Lys Lys Asp Ser Leu Ser Glu Ala
   290 295 300
   Glu Arg Ala Ala Ala Gln Ala Tyr Ala Lys Glu Lys Gly Leu Thr Pro
   305 310 315 320
   Pro Ser Thr Asp His Gln Asp Ser Gly Asn Thr Glu Ala Lys Gly Ala
   325 330 335
   Glu Ala Ile Tyr Asn Arg Val Lys Ala Ala Lys Lys Val Pro Leu Asp
   340 345 350
   Arg Met Pro Tyr Asn Leu Gln Tyr Thr Val Glu Val Lys Asn Gly Ser
   355 360 365
   Leu Ile Ile Pro His Tyr Asp His Tyr His Asn Ile Lys Phe Glu Trp
   370 375 380
   Phe Asp Glu Gly Leu Tyr Glu Ala Pro Lys Gly Tyr Ser Leu Glu Asp
   385 390 395 400
   Leu Leu Ala Thr Val Lys Tyr Tyr Val Glu His Pro Asn Glu Arg Pro
   405 410 415
   His Ser Asp Asn Gly Phe Gly Asn Ala Ser Asp His Val Arg Lys Asn
   420 425 430
   Lys Ala Asp Gln Asp Ser Lys Pro Asp Glu Asp Lys Glu His Asp Glu
   435 440 445
   Val Ser Glu Pro Thr His Pro Glu Ser Asp Glu Lys Glu Asn His Ala
   450 455 460
   Gly Leu Asn Pro Ser Ala Asp Asn Leu Tyr Lys Pro Ser Thr Asp Thr
   465 470 475 480
   Glu Glu Thr Glu Glu Glu Ala Glu Asp Thr Thr Asp Glu Ala Glu Ile
   485 490 495
   Pro Gln Val Glu Asn Ser Val Ile Asn Ala Lys Ile Ala Asp Ala Glu
   500 505 510
   Ala Leu Leu Glu Lys Val Thr Asp Pro Ser Ile Arg Gln Asn Ala Met
   515 520 525
   Glu Thr Leu Thr Gly Leu Lys Ser Ser Leu Leu Leu Gly Thr Lys Asp
   530 535 540
   Asn Asn Thr Ile Ser Ala Glu Val Asp Ser Leu Leu Ala Leu Leu Lys
   545 550 555 560
   Glu Ser Gln Pro Ala Pro Ile Gln
   565
<210> 75
   <211> 140
   <212> PRT
   <213> S. pneumoniae
<400> 75
   Val Arg Lys Asn Lys Ala Asp Gln Asp Ser Lys Pro Asp Glu Asp Lys
    1               5                  10                  15
   Glu His Asp Glu Val Ser Glu Pro Thr His Pro Glu Ser Asp Glu Lys
               20                  25                  30
   Glu Asn His Ala Gly Leu Asn Pro Ser Ala Asp Asn Leu Tyr Lys Pro
           35                  40                  45
   Ser Thr Asp Thr Glu Glu Thr Glu Glu Glu Ala Glu Asp Thr Thr Asp
       50                  55                  60
   Glu Ala Glu Ile Pro Gln Val Glu Asn Ser Val Ile Asn Ala Lys Ile
   65                  70                  75                  80
   Ala Asp Ala Glu Ala Leu Leu Glu Lys Val Thr Asp Pro Ser Ile Arg
                   85                  90                  95
   Gln Asn Ala Met Glu Thr Leu Thr Gly Leu Lys Ser Ser Leu Leu Leu
               100                 105                 110
   Gly Thr Lys Asp Asn Asn Thr Ile Ser Ala Glu Val Asp Ser Leu Leu
           115                 120                 125
   Ala Leu Leu Lys Glu Ser Gln Pro Ala Pro Ile Gln
       130                 135                 140
<210> 76
   <211> 3171
   <212> DNA
   <213> S. pneumoniae
<400> 76
   gacttgacag aagagcaaat taaggctgcg caaaaacatt tagaggaagt taaaactagt     60
   cataatggat tagattcttt gtcatctcat gaacaggatt atccaggtaa tgccaaagaa    120
   atgaaagatt tagataaaaa aatcgaagaa aaaattgctg gcattatgaa acaatatggt    180
   gtcaaacgtg aaagtattgt cgtgaataaa gaaaaaaatg cgattattta tccgcatgga    240
   gatcaccatc atgcagatcc gattgatgaa cataaaccgg ttggaattgg tcattctcac    300
   agtaactatg aactgtttaa acccgaagaa ggagttgcta aaaaagaagg gaataaagtt    360
   tatactggag aagaattaac gaatgttgtt aatttgttaa aaaatagtac gtttaataat    420
   caaaacttta ctctagccaa tggtcaaaaa cgcgtttctt ttagttttcc gcctgaattg    480
   gagaaaaaat taggtatcaa tatgctagta aaattaataa caccagatgg aaaagtattg    540
   gagaaagtat ctggtaaagt atttggagaa ggagtaggga atattgcaaa ctttgaatta    600
   gatcaacctt atttaccagg acaaacattt aagtatacta tcgcttcaaa agattatcca    660
   gaagtaagtt atgatggtac atttacagtt ccaacctctt tagcttacaa aatggccagt    720
   caaacgattt tctatccttt ccatgcaggg gatacttatt taagagtgaa ccctcaattt    780
   gcagtgccta aaggaactga tgctttagtc agagtgtttg atgaatttca tggaaatgct    840
   tatttagaaa ataactataa agttggtgaa atcaaattac cgattccgaa attaaaccaa    900
   ggaacaacca gaacggccgg aaataaaatt cctgtaacct tcatggcaaa tgcttatttg    960
   gacaatcaat cgacttatat tgtggaagta cctatcttgg aaaaagaaaa tcaaactgat   1020
   aaaccaagta ttctaccaca atttaaaagg aataaagcac aagaaaactc aaaacttgat   1080
   gaaaaggtag aagaaccaaa gactagtgag aaggtagaaa aagaaaaact ttctgaaact   1140
   gggaatagta ctagtaattc aacgttagaa gaagttccta cagtggatcc tgtacaagaa   1200
   aaagtagcaa aatttgctga aagttatggg atgaagctag aaaatgtctt gtttaatatg   1260
   gacggaacaa ttgaattata tttaccatca ggagaagtca ttaaaaagaa tatggcagat   1320
   tttacaggag aagcacctca aggaaatggt gaaaataaac catctgaaaa tggaaaagta   1380
   tctactggaa cagttgagaa ccaaccaaca gaaaataaac cagcagattc tttaccagag   1440
   gcaccaaacg aaaaacctgt aaaaccagaa aactcaacgg ataatggaat gttgaatcca   1500
   gaagggaatg tggggagtga ccctatgtta gatccagcat tagaggaagc tccagcagta   1560
   gatcctgtac aagaaaaatt agaaaaattt acagctagtt acggattagg cttagatagt   1620
   gttatattca atatggatgg aacgattgaa ttaagattgc caagtggaga agtgataaaa   1680
   aagaatttat ctgatttcat agcgaagctt cgttatcgtt caaaccattg ggtaccagat   1740
   tcaagaccag aagaaccaag tccacaaccg actccagaac ctagtccaag tccgcaacct   1800
   gcaccaaatc ctcaaccagc tccaagcaat ccaattgatg agaaattggt caaagaagct   1860
   gttcgaaaag taggcgatgg ttatgtcttt gaggagaatg gagtttctcg ttatatccca   1920
   gccaagaatc tttcagcaga aacagcagca ggcattgata gcaaactggc caagcaggaa   1980
   agtttatctc ataagctagg agctaagaaa actgacctcc catctagtga tcgagaattt   2040
   tacaataagg cttatgactt actagcaaga attcaccaag atttacttga taataaaggt   2100
   cgacaagttg attttgaggc tttggataac ctgttggaac gactcaagga tgtctcaagt   2160
   gataaagtca agttagtgga tgatattctt gccttcttag ctccgattcg tcatccagaa   2220
   cgtttaggaa aaccaaatgc gcaaattacc tacactgatg atgagattca agtagccaag   2280
   ttggcaggca agtacacaac agaagacggt tatatctttg atcctcgtga tataaccagt   2340
   gatgaggggg atgcctatgt aactccacat atgacccata gccactggat taaaaaagat   2400
   agtttgtctg aagctgagag agcggcagcc caggcttatg ctaaagagaa aggtttgacc   2460
   cctccttcga cagaccatca ggattcagga aatactgagg caaaaggagc agaagctatc   2520
   tacaaccgcg tgaaagcagc taagaaggtg ccacttgatc gtatgcctta caatcttcaa   2580
   tatactgtag aagtcaaaaa cggtagttta atcatacctc attatgacca ttaccataac   2640
   atcaaatttg agtggtttga cgaaggcctt tatgaggcac ctaaggggta tactcttgag   2700
   gatcttttgg cgactgtcaa gtactatgtc gaacatccaa acgaacgtcc gcattcagat   2760
   aatggttttg gtaacgctag cgaccatgtt caaagaaaca aaaatggtca agctgatacc   2820
   aatcaaacgg aaaaaccaag cgaggagaaa cctcagacag aaaaacctga ggaagaaacc   2880
   cctcgagaag agaaaccaca aagcgagaaa ccagagtctc caaaaccaac agaggaacca   2940
   gaagaagaat caccagagga atcagaagaa cctcaggtcg agactgaaaa ggttgaagaa   3000
   aaactgagag aggctgaaga tttacttgga aaaatccagg atccaattat caagtccaat   3060
   gccaaagaga ctctcacagg attaaaaaat aatttactat ttggcaccca ggacaacaat   3120
   actattatgg cagaagctga aaaactattg gctttattaa aggagagtaa g            3171
<210> 77
   <211> 473
   <212> PRT
   <213> S. pneumoniae
<400> 77
   Glu Ala Tyr Trp Asn Gly Lys Gln Gly Ser Arg Pro Ser Ser Ser Ser
    1               5                  10                  15
   Ser Tyr Asn Ala Asn Pro Val Gln Pro Arg Leu Ser Glu Asn His Asn
               20                  25                  30
   Leu Thr Val Thr Pro Thr Tyr His Gln Asn Gln Gly Glu Asn Ile Ser
           35                  40                  45
   Ser Leu Leu Arg Glu Leu Tyr Ala Lys Pro Leu Ser Glu Arg His Val
       50                  55                  60
   Glu Ser Asp Gly Leu Ile Phe Asp Pro Ala Gln Ile Thr Ser Arg Thr
   65                  70                  75                  80
   Ala Arg Gly Val Ala Val Pro His Gly Asn His Tyr His Phe Ile Pro
                   85                  90                  95
   Tyr Glu Gln Met Ser Glu Leu Glu Lys Arg Ile Ala Arg Ile Ile Pro
               100                 105                 110
   Leu Arg Tyr Arg Ser Asn His Trp Val Pro Asp Ser Arg Pro Glu Gln
           115                 120                 125
   Pro Ser Pro Gln Ser Thr Pro Glu Pro Ser Pro Ser Leu Gln Pro Ala
       130                 135                 140
   Pro Asn Pro Gln Pro Ala Pro Ser Asn Pro Ile Asp Glu Lys Leu Val
   145                 150                 155                 160
   Lys Glu Ala Val Arg Lys Val Gly Asp Gly Tyr Val Phe Glu Glu Asn
                   165                 170                 175
   Gly Val Ser Arg Tyr Ile Pro Ala Lys Asp Leu Ser Ala Glu Thr Ala
               180                 185                 190
   Ala Gly Ile Asp Ser Lys Leu Ala Lys Gln Glu Ser Leu Ser His Lys
           195                 200                 205
   Leu Gly Ala Lys Lys Thr Asp Leu Pro Ser Ser Asp Arg Glu Phe Tyr
       210                 215                 220
   Asn Lys Ala Tyr Asp Leu Leu Ala Arg Ile His Gln Asp Leu Leu Asp
   225                 230                 235                 240
   Asn Lys Gly Arg Gln Val Asp Phe Glu Val Leu Asp Asn Leu Leu Glu
   245 250 255
   Arg Leu Lys Asp Val Ser Ser Asp Lys Val Lys Leu Val Asp Asp Ile
   260 265 270
   Leu Ala Phe Leu Ala Pro Ile Arg His Pro Glu Arg Leu Gly Lys Pro
   275 280 285
   Asn Ala Gln Ile Thr Tyr Thr Asp Asp Glu Ile Gln Val Ala Lys Leu
   290 295 300
   Ala Gly Lys Tyr Thr Thr Glu Asp Gly Tyr Ile Phe Asp Pro Arg Asp
   305 310 315 320
   Ile Thr Ser Asp Glu Gly Asp Ala Tyr Val Thr Pro His Met Thr His
   325 330 335
   Ser His Trp Ile Lys Lys Asp Ser Leu Ser Glu Ala Glu Arg Ala Ala
   340 345 350
   Ala Gln Ala Tyr Ala Lys Glu Lys Gly Leu Thr Pro Pro Ser Thr Asp
   355 360 365
   His Gln Asp Ser Gly Asn Thr Glu Ala Lys Gly Ala Glu Ala Ile Tyr
   370 375 380
   Asn Arg Val Lys Ala Ala Lys Lys Val Pro Leu Asp Arg Met Pro Tyr
   385 390 395 400
   Asn Leu Gln Tyr Thr Val Glu Val Lys Asn Gly Ser Leu Ile Ile Pro
   405 410 415
   His Tyr Asp His Tyr His Asn Ile Lys Phe Glu Trp Phe Asp Glu Gly
   420 425 430
   Leu Tyr Glu Ala Pro Lys Gly Tyr Ser Leu Glu Asp Leu Leu Ala Thr
   435 440 445
   Val Lys Tyr Tyr Val Glu His Pro Asn Glu Arg Pro His Ser Asp Asn
   450 455 460
   Gly Phe Gly Asn Ala Ser Asp His Val
   465 470
<210> 78
   <211> 780
   <212> PRT
   <213> S. pneumoniae
<400> 78
   Cys Ala Tyr Ala Leu Asn Gln His Arg Ser Gln Glu Asn Lys Asp Asn
    1               5                  10                  15
   Asn Arg Val Ser Tyr Val Asp Gly Ser Gln Ser Ser Gln Lys Ser Glu
               20                  25                  30
   Asn Leu Thr Pro Asp Gln Val Ser Gln Lys Glu Gly Ile Gln Ala Glu
           35                  40                  45
   Gln Ile Val Ile Lys Ile Thr Asp Gln Gly Tyr Val Thr Ser His Gly
       50                  55                  60
   Asp His Tyr His Tyr Tyr Asn Gly Lys Val Pro Tyr Asp Ala Leu Phe
   65                  70                  75                  80
   Ser Glu Glu Leu Leu Met Lys Asp Pro Asn Tyr Gln Leu Lys Asp Ala
                   85                  90                  95
   Asp Ile Val Asn Glu Val Lys Gly Gly Tyr Ile Ile Lys Val Asp Gly
               100                 105                 110
   Lys Tyr Tyr Val Tyr Leu Lys Asp Ala Ala His Ala Asp Asn Val Arg
           115                 120                 125
   Thr Lys Asp Glu Ile Asn Arg Gln Lys Gln Glu His Val Lys Asp Asn
       130                 135                 140
   Glu Lys Val Asn Ser Asn Val Ala Val Ala Arg Ser Gln Gly Arg Tyr
   145                 150                 155                 160
   Thr Thr Asn Asp Gly Tyr Val Phe Asn Pro Ala Asp Ile Ile Glu Asp
                   165                 170                 175
   Thr Gly Asn Ala Tyr Ile Val Pro His Gly Gly His Tyr His Tyr Ile
               180                 185                 190
   Pro Lys Ser Asp Leu Ser Ala Ser Glu Leu Ala Ala Ala Lys Ala His
           195                 200                 205
   Leu Ala Gly Lys Asn Met Gln Pro Ser Gln Leu Ser Tyr Ser Ser Thr
       210                 215                 220
   Ala Ser Asp Asn Asn Thr Gln Ser Val Ala Lys Gly Ser Thr Ser Lys
   225                 230                 235                 240
   Pro Ala Asn Lys Ser Glu Asn Leu Gln Ser Leu Leu Lys Glu Leu Tyr
                   245                 250                 255
   Asp Ser Pro Ser Ala Gln Arg Tyr Ser Glu Ser Asp Gly Leu Val Phe
               260                 265                 270
   Asp Pro Ala Lys Ile Ile Ser Arg Thr Pro Asn Gly Val Ala Ile Pro
           275                 280                 285
   His Gly Asp His Tyr His Phe Ile Pro Tyr Ser Lys Leu Ser Ala Leu
       290                 295                 300
   Glu Glu Lys Ile Ala Arg Met Val Pro Ile Ser Gly Thr Gly Ser Thr
   305                 310                 315                 320
   Val Ser Thr Asn Ala Lys Pro Asn Glu Val Val Ser Ser Leu Gly Ser
                   325                 330                 335
   Leu Ser Ser Asn Pro Ser Ser Leu Thr Thr Ser Lys Glu Leu Ser Ser
               340                 345                 350
   Ala Ser Asp Gly Tyr Ile Phe Asn Pro Lys Asp Ile Val Glu Glu Thr
           355                 360                 365
   Ala Thr Ala Tyr Ile Val Arg His Gly Asp His Phe His Tyr Ile Pro
       370                 375                 380
   Lys Ser Asn Gln Ile Gly Gln Pro Thr Leu Pro Asn Asn Ser Leu Ala
   385                 390                 395                 400
   Thr Pro Ser Pro Ser Leu Pro Ile Asn Pro Gly Thr Ser His Glu Lys
                   405                 410                 415
   His Glu Glu Asp Gly Tyr Gly Phe Asp Ala Asn Arg Ile Ile Ala Glu
               420                 425                 430
   Asp Glu Ser Gly Phe Val Met Ser His Gly Asp His Asn His Tyr Phe
           435                 440                 445
   Phe Lys Lys Asp Leu Thr Glu Glu Gln Ile Lys Ala Ala Gln Lys His
       450                 455                 460
   Leu Glu Glu Val Lys Thr Ser His Asn Gly Leu Asp Ser Leu Ser Ser
   465                 470                 475                 480
   His Glu Gln Asp Tyr Pro Gly Asn Ala Lys Glu Met Lys Asp Leu Asp
                   485                 490                 495
   Lys Lys Ile Glu Glu Lys Ile Ala Gly Ile Met Lys Gln Tyr Gly Val
               500                 505                 510
   Lys Arg Glu Ser Ile Val Val Asn Lys Glu Lys Asn Ala Ile Ile Tyr
           515                 520                 525
   Pro His Gly Asp His His His Ala Asp Pro Ile Asp Glu His Lys Pro
       530                 535                 540
   Val Gly Ile Gly His Ser His Ser Asn Tyr Glu Leu Phe Lys Pro Glu
   545                 550                 555                 560
   Glu Gly Val Ala Lys Lys Glu Gly Asn Lys Val Tyr Thr Gly Glu Glu
                   565                 570                 575
   Leu Thr Asn Val Val Asn Leu Leu Lys Asn Ser Thr Phe Asn Asn Gln
               580                 585                 590
   Asn Phe Thr Leu Ala Asn Gly Gln Lys Arg Val Ser Phe Ser Phe Pro
           595                 600                 605
   Pro Glu Leu Glu Lys Lys Leu Gly Ile Asn Met Leu Val Lys Leu Ile
       610                 615                 620
   Thr Pro Asp Gly Lys Val Leu Glu Lys Val Ser Gly Lys Val Phe Gly
   625                 630                 635                 640
   Glu Gly Val Gly Asn Ile Ala Asn Phe Glu Leu Asp Gln Pro Tyr Leu
                   645                 650                 655
   Pro Gly Gln Thr Phe Lys Tyr Thr Ile Ala Ser Lys Asp Tyr Pro Glu
               660                 665                 670
   Val Ser Tyr Asp Gly Thr Phe Thr Val Pro Thr Ser Leu Ala Tyr Lys
           675                 680                 685
   Met Ala Ser Gln Thr Ile Phe Tyr Pro Phe His Ala Gly Asp Thr Tyr
       690                 695                 700
   Leu Arg Val Asn Pro Gln Phe Ala Val Pro Lys Gly Thr Asp Ala Leu
   705                 710                 715                 720
   Val Arg Val Phe Asp Glu Phe His Gly Asn Ala Tyr Leu Glu Asn Asn
                   725                 730                 735
   Tyr Lys Val Gly Glu Ile Lys Leu Pro Ile Pro Lys Leu Asn Gln Gly
               740                 745                 750
   Thr Thr Arg Thr Ala Gly Asn Lys Ile Pro Val Thr Phe Met Ala Asn
           755                 760                 765
   Ala Tyr Leu Asp Asn Gln Ser Thr Tyr Ile Val Glu
       770                 775                 780
<210> 79
   <211> 690
   <212> PRT
   <213> S. pneumoniae
<400> 79
   Cys Ala Tyr Glu Leu Gly Leu His Gln Ala Gln Thr Val Lys Glu Asn
   1               5                   10                  15
   Asn Arg Val Ser Tyr Ile Asp Gly Lys Gln Ala Thr Gln Lys Thr Glu
               20                  25                  30
   Asn Leu Thr Pro Asp Glu Val Ser Lys Arg Glu Gly Ile Asn Ala Glu
           35                  40                  45
   Gln Ile Val Ile Lys Ile Thr Asp Gln Gly Tyr Val Thr Ser His Gly
       50                  55                  60
   Asp His Tyr His Tyr Tyr Asn Gly Lys Val Pro Tyr Asp Ala Ile Ile
   65                  70                  75                  80
   Ser Glu Glu Leu Leu Met Lys Asp Pro Asn Tyr Gln Leu Lys Asp Ser
                   85                  90                  95
   Asp Ile Val Asn Glu Ile Lys Gly Gly Tyr Val Ile Lys Val Asn Gly
               100                 105                 110
   Lys Tyr Tyr Val Tyr Leu Lys Asp Ala Ala His Ala Asp Asn Val Arg
           115                 120                 125
   Thr Lys Glu Glu Ile Asn Arg Gln Lys Gln Glu His Ser Gln His Arg
       130                 135                 140
   Glu Gly Gly Thr Ser Ala Asn Asp Gly Ala Val Ala Phe Ala Arg Ser
   145                 150                 155                 160
   Gln Gly Arg Tyr Thr Thr Asp Asp Gly Tyr Ile Phe Asn Ala Ser Asp
                   165                 170                 175
   Ile Ile Glu Asp Thr Gly Asp Ala Tyr Ile Val Pro His Gly Asp His
               180                 185                 190
   Tyr His Tyr Ile Pro Lys Asn Glu Leu Ser Ala Ser Glu Leu Ala Ala
           195                 200                 205
   Ala Glu Ala Phe Leu Ser Gly Arg Glu Asn Leu Ser Asn Leu Arg Thr
       210                 215                 220
   Tyr Arg Arg Gln Asn Ser Asp Asn Thr Pro Arg Thr Asn Trp Val Pro
   225                 230                 235                 240
   Ser Val Ser Asn Pro Gly Thr Thr Asn Thr Asn Thr Ser Asn Asn Ser
                   245                 250                 255
   Asn Thr Asn Ser Gln Ala Ser Gln Ser Asn Asp Ile Asp Ser Leu Leu
               260                 265                 270
   Lys Gln Leu Tyr Lys Leu Pro Leu Ser Gln Arg His Val Glu Ser Asp
           275                 280                 285
   Gly Leu Ile Phe Asp Pro Ala Gln Ile Thr Ser Arg Thr Ala Arg Gly
       290                 295                 300
   Val Ala Val Pro His Gly Asn His Tyr His Phe Ile Pro Tyr Glu Gln
   305                 310                 315                 320
   Met Ser Glu Leu Glu Lys Arg Ile Ala Arg Ile Ile Pro Leu Arg Tyr
                   325                 330                 335
   Arg Ser Asn His Trp Val Pro Asp Ser Arg Pro Glu Glu Pro Ser Pro
               340                 345                 350
   Gln Pro Thr Pro Glu Pro Ser Pro Ser Pro Gln Pro Ala Pro Asn Pro
           355                 360                 365
   Gln Pro Ala Pro Ser Asn Pro Ile Asp Glu Lys Leu Val Lys Glu Ala
       370                 375                 380
   Val Arg Lys Val Gly Asp Gly Tyr Val Phe Glu Glu Asn Gly Val Ser
   385                 390                 395                 400
   Arg Tyr Ile Pro Ala Lys Asn Leu Ser Ala Glu Thr Ala Ala Gly Ile
                   405                 410                 415
   Asp Ser Lys Leu Ala Lys Gln Glu Ser Leu Ser His Lys Leu Gly Ala
               420                 425                 430
   Lys Lys Thr Asp Leu Pro Ser Ser Asp Arg Glu Phe Tyr Asn Lys Ala
           435                 440                 445
   Tyr Asp Leu Leu Ala Arg Ile His Gln Asp Leu Leu Asp Asn Lys Gly
       450                 455                 460
   Arg Gln Val Asp Phe Glu Ala Leu Asp Asn Leu Leu Glu Arg Leu Lys
   465                 470                 475                 480
   Asp Val Ser Ser Asp Lys Val Lys Leu Val Asp Asp Ile Leu Ala Phe
                   485                 490                 495
   Leu Ala Pro Ile Arg His Pro Glu Arg Leu Gly Lys Pro Asn Ala Gln
               500                 505                 510
   Ile Thr Tyr Thr Asp Asp Glu Ile Gln Val Ala Lys Leu Ala Gly Lys
           515                 520                 525
   Tyr Thr Thr Glu Asp Gly Tyr Ile Phe Asp Pro Arg Asp Ile Thr Ser
       530                 535                 540
   Asp Glu Gly Asp Ala Tyr Val Thr Pro His Met Thr His Ser His Trp
   545                 550                 555                 560
   Ile Lys Lys Asp Ser Leu Ser Glu Ala Glu Arg Ala Ala Ala Gln Ala
                   565                 570                 575
   Tyr Ala Lys Glu Lys Gly Leu Thr Pro Pro Ser Thr Asp His Gln Asp
               580                 585                 590
   Ser Gly Asn Thr Glu Ala Lys Gly Ala Glu Ala Ile Tyr Asn Arg Val
           595                 600                 605
   Lys Ala Ala Lys Lys Val Pro Leu Asp Arg Met Pro Tyr Asn Leu Gln
       610                 615                 620
   Tyr Thr Val Glu Val Lys Asn Gly Ser Leu Ile Ile Pro His Tyr Asp
   625                 630                 635                 640
   His Tyr His Asn Ile Lys Phe Glu Trp Phe Asp Glu Gly Leu Tyr Glu
                   645                 650                 655
   Ala Pro Lys Gly Tyr Thr Leu Glu Asp Leu Leu Ala Thr Val Lys Tyr
               660                 665                 670
   Tyr Val Glu His Pro Asn Glu Arg Pro His Ser Asp Asn Gly Phe Gly
           675                 680                 685
   Asn Ala
       690
<210> 80
   <211> 2469
   <212> DNA
   <213> S. pneumoniae
<400> 80
   gtgaagaaaa catatggtta tatcggctca gttgctgcca ttttactagc tactcatatt     60
   ggaagttacc aacttggtaa gcatcatatg ggtctagcaa caaaggacaa tcagattgcc    120
   tatattgatg acagcaaagg taaggcaaaa gcccctaaaa caaacaaaac gatggatcaa    180
   atcagtgctg aagaaggcat ctctgctgaa cagatcgtag tcaaaattac tgaccaaggc    240
   tatgtgacct cacacggtga ccattatcat ttttacaatg ggaaagttcc ttatgatgcg    300
   attattagtg aagagttgtt gatgacggat cctaattacc gttttaaaca atcagacgtt    360
   atcaatgaaa tcttagacgg ttacgttatt aaagtcaatg gcaactatta tgtttacctc    420
   aagccaggta gtaagcgcaa aaacattcga accaaacaac aaattgctga gcaagtagcc    480
   aaaggaacta aagaagctaa agaaaaaggt ttagctcaag tggcccatct cagtaaagaa    540
   gaagttgcgg cagtcaatga agcaaaaaga caaggacgct atactacaga cgatggctat    600
   atttttagtc cgacagatat cattgatgat ttaggagatg cttatttagt acctcatggt    660
   aatcactatc attatattcc taaaaaggat ttgtctccaa gtgagctagc tgctgcacaa    720
   gcctactgga gtcaaaaaca aggtcgaggt gctagaccgt ctgattaccg cccgacacca    780
   gccccaggtc gtaggaaagc cccaattcct gatgtgacgc ctaaccctgg acaaggtcat    840
   cagccagata acggtggcta tcatccagcg cctcctaggc caaatgatgc gtcacaaaac    900
   aaacaccaaa gagatgagtt taaaggaaaa acctttaagg aacttttaga tcaactacac    960
   cgtcttgatt tgaaataccg tcatgtggaa gaagatgggt tgatttttga accgactcaa   1020
   gtgatcaaat caaacgcttt tgggtatgtg gtgcctcatg gagatcatta tcatattatc   1080
   ccaagaagtc agttatcacc tcttgaaatg gaattagcag atcgatactt agctggccaa   1140
   actgaggaca atgactcagg ttcagagcac tcaaaaccat cagataaaga agtgacacat   1200
   acctttcttg gtcatcgcat caaagcttac ggaaaaggct tagatggtaa accatatgat   1260
   acgagtgatg cttatgtttt tagtaaagaa tccattcatt cagtggataa atcaggagtt   1320
   acagctaaac acggagatca tttccactat ataggatttg gagaacttga acaatatgag   1380
   ttggatgagg tcgctaactg ggtgaaagca aaaggtcaag ctgatgagct tgctgctgct   1440
   ttggatcagg aacaaggcaa agaaaaacca ctctttgaca ctaaaaaagt gagtcgcaaa   1500
   gtaacaaaag atggtaaagt gggctatatg atgccaaaag atggtaagga ctatttctat   1560
   gctcgtgatc aacttgattt gactcagatt gcctttgccg aacaagaact aatgcttaaa   1620
   gataagaagc attaccgtta tgacattgtt gacacaggta ttgagccacg acttgctgta   1680
   gatgtgtcaa gtctgccgat gcatgctggt aatgctactt acgatactgg aagttcgttt   1740
   gttatcccac atattgatca tatccatgtc gttccgtatt catggttgac gcgcgatcag   1800
   attgcaacag tcaagtatgt gatgcaacac cccgaagttc gtccggatgt atggtctaag   1860
   ccagggcatg aagagtcagg ttcggtcatt ccaaatgtta cgcctcttga taaacgtgct   1920
   ggtatgccaa actggcaaat tatccattct gctgaagaag ttcaaaaagc cctagcagaa   1980
   ggtcgttttg caacaccaga cggctatatt ttcgatccac gagatgtttt ggccaaagaa   2040
   acttttgtat ggaaagatgg ctcctttagc atcccaagag cagatggcag ttcattgaga   2100
   accattaata aatctgatct atcccaagct gagtggcaac aagctcaaga gttattggca   2160
   aagaaaaata ctggtgatgc tactgatacg gataaaccca aagaaaagca acaggcagat   2220
   aagagcaatg aaaaccaaca gccaagtgaa gccagtaaag aagaaaaaga atcagatgac   2280
   tttatagaca gtttaccaga ctatggtcta gatagagcaa ccctagaaga tcatatcaat   2340
   caattagcac aaaaagctaa tatcgatcct aagtatctca ttttccaacc agaaggtgtc   2400
   caattttata ataaaaatgg tgaattggta acttatgata tcaagacact tcaacaaata   2460
   aacccttaa                                                            2469
<210> 81
   <211> 823
   <212> PRT
   <213> S. pneumoniae
<400> 81
   Val Lys Lys Thr Tyr Gly Tyr Ile Gly Ser Val Ala Ala Ile Leu Leu
    1               5                  10                  15
   Ala Thr His Ile Gly Ser Tyr Gln Leu Gly Lys His His Met Gly Leu
               20                  25                  30
   Ala Thr Lys Asp Asn Gln Ile Ala Tyr Ile Asp Asp Ser Lys Gly Lys
               35                  40                  45
   Ala Lys Ala Pro Lys Thr Asn Lys Thr Met Asp Gln Ile Ser Ala Glu
       50                  55                  60
   Glu Gly Ile Ser Ala Glu Gln Ile Val Val Lys Ile Thr Asp Gln Gly
   65                  70                  75                  80
   Tyr Val Thr Ser His Gly Asp His Tyr His Phe Tyr Asn Gly Lys Val
                   85                  90                  95
   Pro Tyr Asp Ala Ile Ile Ser Glu Glu Leu Leu Met Thr Asp Pro Asn
               100                 105                 110
   Tyr Arg Phe Lys Gln Ser Asp Val Ile Asn Glu Ile Leu Asp Gly Tyr
           115                 120                 125
   Val Ile Lys Val Asn Gly Asn Tyr Tyr Val Tyr Leu Lys Pro Gly Ser
       130                 135                 140
   Lys Arg Lys Asn Ile Arg Thr Lys Gln Gln Ile Ala Glu Gln Val Ala
   145                 150                 155                 160
   Lys Gly Thr Lys Glu Ala Lys Glu Lys Gly Leu Ala Gln Val Ala His
                   165                 170                 175
   Leu Ser Lys Glu Glu Val Ala Ala Val Asn Glu Ala Lys Arg Gln Gly
               180                 185                 190
   Arg Tyr Thr Thr Asp Asp Gly Tyr Ile Phe Ser Pro Thr Asp Ile Ile
           195                 200                 205
   Asp Asp Leu Gly Asp Ala Tyr Leu Val Pro His Gly Asn His Tyr His
       210                 215                 220
   Tyr Ile Pro Lys Lys Asp Leu Ser Pro Ser Glu Leu Ala Ala Ala Gln
   225                 230                 235                 240
   Ala Tyr Trp Ser Gln Lys Gln Gly Arg Gly Ala Arg Pro Ser Asp Tyr
                   245                 250                 255
   Arg Pro Thr Pro Ala Pro Gly Arg Arg Lys Ala Pro Ile Pro Asp Val
               260                 265                 270
   Thr Pro Asn Pro Gly Gln Gly His Gln Pro Asp Asn Gly Gly Tyr His
           275                 280                 285
   Pro Ala Pro Pro Arg Pro Asn Asp Ala Ser Gln Asn Lys His Gln Arg
       290                 295                 300
   Asp Glu Phe Lys Gly Lys Thr Phe Lys Glu Leu Leu Asp Gln Leu His
   305                 310                 315                 320
   Arg Leu Asp Leu Lys Tyr Arg His Val Glu Glu Asp Gly Leu Ile Phe
                   325                 330                 335
   Glu Pro Thr Gln Val Ile Lys Ser Asn Ala Phe Gly Tyr Val Val Pro
               340                 345                 350
   His Gly Asp His Tyr His Ile Ile Pro Arg Ser Gln Leu Ser Pro Leu
           355                 360                 365
   Glu Met Glu Leu Ala Asp Arg Tyr Leu Ala Gly Gln Thr Glu Asp Asn
       370                 375                 380
   Asp Ser Gly Ser Glu His Ser Lys Pro Ser Asp Lys Glu Val Thr His
   385                 390                 395                 400
   Thr Phe Leu Gly His Arg Ile Lys Ala Tyr Gly Lys Gly Leu Asp Gly
                   405                 410                 415
   Lys Pro Tyr Asp Thr Ser Asp Ala Tyr Val Phe Ser Lys Glu Ser Ile
               420                 425                 430
   His Ser Val Asp Lys Ser Gly Val Thr Ala Lys His Gly Asp His Phe
           435                 440                 445
   His Tyr Ile Gly Phe Gly Glu Leu Glu Gln Tyr Glu Leu Asp Glu Val
       450                 455                 460
   Ala Asn Trp Val Lys Ala Lys Gly Gln Ala Asp Glu Leu Ala Ala Ala
   465                 470                 475                 480
   Leu Asp Gln Glu Gln Gly Lys Glu Lys Pro Leu Phe Asp Thr Lys Lys
                   485                 490                 495
   Val Ser Arg Lys Val Thr Lys Asp Gly Lys Val Gly Tyr Met Met Pro
               500                 505                 510
   Lys Asp Gly Lys Asp Tyr Phe Tyr Ala Arg Asp Gln Leu Asp Leu Thr
           515                 520                 525
   Gln Ile Ala Phe Ala Glu Gln Glu Leu Met Leu Lys Asp Lys Lys His
       530                 535                 540
   Tyr Arg Tyr Asp Ile Val Asp Thr Gly Ile Glu Pro Arg Leu Ala Val
   545                 550                 555                 560
   Asp Val Ser Ser Leu Pro Met His Ala Gly Asn Ala Thr Tyr Asp Thr
                   565                 570                 575
   Gly Ser Ser Phe Val Ile Pro His Ile Asp His Ile His Val Val Pro
               580                 585                 590
   Tyr Ser Trp Leu Thr Arg Asp Gln Ile Ala Thr Val Lys Tyr Val Met
           595                 600                 605
   Gln His Pro Glu Val Arg Pro Asp Val Trp Ser Lys Pro Gly His Glu
       610                 615                 620
   Glu Ser Gly Ser Val Ile Pro Asn Val Thr Pro Leu Asp Lys Arg Ala
   625                 630                 635                 640
   Gly Met Pro Asn Trp Gln Ile Ile His Ser Ala Glu Glu Val Gln Lys
                   645                 650                 655
   Ala Leu Ala Glu Gly Arg Phe Ala Thr Pro Asp Gly Tyr Ile Phe Asp
               660                 665                 670
   Pro Arg Asp Val Leu Ala Lys Glu Thr Phe Val Trp Lys Asp Gly Ser
           675                 680                 685
   Phe Ser Ile Pro Arg Ala Asp Gly Ser Ser Leu Arg Thr Ile Asn Lys
       690                 695                 700
   Ser Asp Leu Ser Gln Ala Glu Trp Gln Gln Ala Gln Glu Leu Leu Ala
   705                 710                 715                 720
   Lys Lys Asn Thr Gly Asp Ala Thr Asp Thr Asp Lys Pro Lys Glu Lys
                   725                 730                 735
   Gln Gln Ala Asp Lys Ser Asn Glu Asn Gln Gln Pro Ser Glu Ala Ser
               740                 745                 750
   Lys Glu Glu Lys Glu Ser Asp Asp Phe Ile Asp Ser Leu Pro Asp Tyr
           755                 760                 765
   Gly Leu Asp Arg Ala Thr Leu Glu Asp His Ile Asn Gln Leu Ala Gln
       770                 775                 780
   Lys Ala Asn Ile Asp Pro Lys Tyr Leu Ile Phe Gln Pro Glu Gly Val
   785                 790                 795                 800
   Gln Phe Tyr Asn Lys Asn Gly Glu Leu Val Thr Tyr Asp Ile Lys Thr
                   805                 810                 815
   Leu Gln Gln Ile Asn Pro Pro
               820
<210> 82
   <211> 2472
   <212> DNA
   <213> S. pneumoniae
<400> 82
   gtgaagaaaa catatggtta tatcggctca gttgctgcca ttttactagc tactcatatt    60
   ggaagttacc aacttggtaa gcatcatatg ggtctagcaa caaaggacaa tcagattgcc   120
   tatattgatg atagcaaagg taaggcaaaa gcccctaaaa caaacaaaac gatggatcaa   180
   atcagtgctg aagaaggcat ctctgctgaa cagatcgtag tcaaaattac tgaccaaggt   240
   tatgtgacct cacacggtga ccattatcat ttttacaatg ggaaagttcc ttatgatgcg   300
   attattagtg aagagttgtt gatgacggat cctaattacc attttaaaca atcagacgtt   360
   atcaatgaaa tcttagacgg ttacgttatt aaagtcaatg gcaactatta tgtttacctc   420
   aagccaggta gtaagcgcaa aaacattcga accaaacaac aaattgctga gcaagtagcc   480
   aaaggaacta aagaagctaa agaaaaaggt ttagctcaag tggcccatct cagtaaagaa   540
   gaagttgcgg cagtcaatga agcaaaaaga caaggacgct atactacaga cgatggctat   600
   atttttagtc cgacagatat cattgatgat ttaggagacg cttatttagt acctcatggt   660
   aatcactatc attatattcc taaaaaagat ttgtctccaa gtgagctagc tgctgcacaa   720
   gcttactgga gtcaaaaaca aggtcgaggt gctagaccgt ctgattaccg cccgacacca   780
   gccccaggtc gtaggaaagc tccaattcct gatgtgacgc ctaaccctgg acaaggtcat   840
   cagccagata acggtggcta tcatccagcg cctcctaggc caaatgatgc gtcacaaaac   900
   aaacaccaaa gagatgagtt taaaggaaaa acctttaagg aacttttaga tcaactacac   960
   cgtcttgatt tgaaataccg tcatgtggaa gaagatgggt tgatttttga accgactcaa  1020
   gtgatcaaat caaacgcttt tgggtatgtg gtgcctcatg gagatcatta tcatattatc  1080
   ccaagaagtc agttatcacc tcttgaaatg gaattagcag atcgatactt agccggtcaa  1140
   actgaggaca atgattcagg ttcagatcac tcaaaaccat cagataaaga agtgacacat  1200
   acctttcttg gtcatcgcat caaagcttac ggaaaaggct tagatggtaa accatatgat  1260
   acgagtgatg cttatgtttt tagtaaagaa tccattcatt cagtggataa atcaggagtt  1320
   acagctaaac acggagatca tttccactat ataggatttg gagaacttga acaatatgag  1380
   ttggatgagg tcgctaactg ggtgaaagca aaaggtcaag ctgatgagct tgctgctgct  1440
   ttggatcagg aacaaggcaa agaaaaacca ctctttgaca ctaaaaaagt gagtcgcaaa  1500
   gtaacaaaag atggtaaagt gggctatatt atgccaaaag atggcaagga ctatttctat  1560
   gctcgtgatc aacttgattt gactcagatt gcctttgccg aacaagaact aatgcttaaa  1620
   gataagaacc attaccgtta tgacattgtt gacacaggta ttgagccacg acttgctgta  1680
   gatgtgtcaa gtctgccgat gcatgctggt aatgctactt acgatactgg aagttcgttt  1740
   gttatccctc atattgatca tatccatgtc gttccgtatt catggttgac gcgcgatcag  1800
   attgcaacaa tcaagtatgt gatgcaacac cccgaagttc gtccagatgt atggtctaag  1860
   ccagggcatg aagagtcagg ttcggtcatt ccaaatgtta cgcctcttga taaacgtgct  1920
   ggtatgccaa attggcaaat catccattct gctgaagaag ttcaaaaagc cctagcagaa  1980
   ggtcgttttg caacaccaga cggctatatt ttcgatccac gagatgtttt ggccaaagaa  2040
   acttttgtat ggaaagatgg ctcctttagc atcccaagag cagatggcag ttcattgaga  2100
   accattaata aatctgatct atcccaagct gagtggcaac aagctcaaga gttattggca  2160
   aagaaaaacg ctggtgatgc tactgatacg gataaaccca aagaaaagca acaggcagat  2220
   aagagcaatg aaaaccaaca gccaagtgaa gccagtaaag aagaagaaaa agaatcagat  2280
   gactttatag acagtttacc agactatggt ctagatagag caaccctaga agatcatatc  2340
   aatcaattag cacaaaaagc taatatcgat cctaagtatc tcattttcca accagaaggt  2400
   gtccaatttt ataataaaaa tggtgaatta gtaacttatg atatcaagac gcttcaacaa  2460
   ataaaccctt aa                                                      2472
<210> 83
   <211> 824
   <212> PRT
   <213> S. pneumoniae
<400> 83
   Val Lys Lys Thr Tyr Gly Tyr Ile Gly Ser Val Ala Ala Ile Leu Leu
    1               5                  10                  15
   Ala Thr His Ile Gly Ser Tyr Gln Leu Gly Lys His His Met Gly Leu
               20                  25                  30
   Ala Thr Lys Asp Asn Gln Ile Ala Tyr Ile Asp Asp Ser Lys Gly Lys
           35                  40                  45
   Ala Lys Ala Pro Lys Thr Asn Lys Thr Met Asp Gln Ile Ser Ala Glu
       50                  55                  60
   Glu Gly Ile Ser Ala Glu Gln Ile Val Val Lys Ile Thr Asp Gln Gly
   65                  70                  75                  80
   Tyr Val Thr Ser His Gly Asp His Tyr His Phe Tyr Asn Gly Lys Val
                   85                  90                  95
   Pro Tyr Asp Ala Ile Ile Ser Glu Glu Leu Leu Met Thr Asp Pro Asn
               100                 105                 110
   Tyr His Phe Lys Gln Ser Asp Val Ile Asn Glu Ile Leu Asp Gly Tyr
           115                 120                 125
   Val Ile Lys Val Asn Gly Asn Tyr Tyr Val Tyr Leu Lys Pro Gly Ser
       130                 135                 140
   Lys Arg Lys Asn Ile Arg Thr Lys Gln Gln Ile Ala Glu Gln Val Ala
   145                 150                 155                 160
   Lys Gly Thr Lys Glu Ala Lys Glu Lys Gly Leu Ala Gln Val Ala His
                   165                 170                 175
   Leu Ser Lys Glu Glu Val Ala Ala Val Asn Glu Ala Lys Arg Gln Gly
               180                 185                 190
   Arg Tyr Thr Thr Asp Asp Gly Tyr Ile Phe Ser Pro Thr Asp Ile Ile
           195                 200                 205
   Asp Asp Leu Gly Asp Ala Tyr Leu Val Pro His Gly Asn His Tyr His
       210                 215                 220
   Tyr Ile Pro Lys Lys Asp Leu Ser Pro Ser Glu Leu Ala Ala Ala Gln
   225                 230                 235                 240
   Ala Tyr Trp Ser Gln Lys Gln Gly Arg Gly Ala Arg Pro Ser Asp Tyr
                   245                 250                 255
   Arg Pro Thr Pro Ala Pro Gly Arg Arg Lys Ala Pro Ile Pro Asp Val
               260                 265                 270
   Thr Pro Asn Pro Gly Gln Gly His Gln Pro Asp Asn Gly Gly Tyr His
           275                 280                 285
   Pro Ala Pro Pro Arg Pro Asn Asp Ala Ser Gln Asn Lys His Gln Arg
       290                 295                 300
   Asp Glu Phe Lys Gly Lys Thr Phe Lys Glu Leu Leu Asp Gln Leu His
   305                 310                 315                 320
   Arg Leu Asp Leu Lys Tyr Arg His Val Glu Glu Asp Gly Leu Ile Phe
                   325                 330                 335
   Glu Pro Thr Gln Val Ile Lys Ser Asn Ala Phe Gly Tyr Val Val Pro
               340                 345                 350
   His Gly Asp His Tyr His Ile Ile Pro Arg Ser Gln Leu Ser Pro Leu
           355                 360                 365
   Glu Met Glu Leu Ala Asp Arg Tyr Leu Ala Gly Gln Thr Glu Asp Asn
       370                 375                 380
   Asp Ser Gly Ser Asp His Ser Lys Pro Ser Asp Lys Glu Val Thr His
   385                 390                 395                 400
   Thr Phe Leu Gly His Arg Ile Lys Ala Tyr Gly Lys Gly Leu Asp Gly
                   405                 410                 415
   Lys Pro Tyr Asp Thr Ser Asp Ala Tyr Val Phe Ser Lys Glu Ser Ile
               420                 425                 430
   His Ser Val Asp Lys Ser Gly Val Thr Ala Lys His Gly Asp His Phe
           435                 440                 445
   His Tyr Ile Gly Phe Gly Glu Leu Glu Gln Tyr Glu Leu Asp Glu Val
       450                 455                 460
   Ala Asn Trp Val Lys Ala Lys Gly Gln Ala Asp Glu Leu Ala Ala Ala
   465                 470                 475                 480
   Leu Asp Gln Glu Gln Gly Lys Glu Lys Pro Leu Phe Asp Thr Lys Lys
                   485                 490                 495
   Val Ser Arg Lys Val Thr Lys Asp Gly Lys Val Gly Tyr Ile Met Pro
               500                 505                 510
   Lys Asp Gly Lys Asp Tyr Phe Tyr Ala Arg Asp Gln Leu Asp Leu Thr
           515                 520                 525
   Gln Ile Ala Phe Ala Glu Gln Glu Leu Met Leu Lys Asp Lys Asn His
       530                 535                 540
   Tyr Arg Tyr Asp Ile Val Asp Thr Gly Ile Glu Pro Arg Leu Ala Val
   545                 550                 555                 560
   Asp Val Ser Ser Leu Pro Met His Ala Gly Asn Ala Thr Tyr Asp Thr
                   565                 570                 575
   Gly Ser Ser Phe Val Ile Pro His Ile Asp His Ile His Val Val Pro
               580                 585                 590
   Tyr Ser Trp Leu Thr Arg Asp Gln Ile Ala Thr Ile Lys Tyr Val Met
           595                 600                 605
   Gln His Pro Glu Val Arg Pro Asp Val Trp Ser Lys Pro Gly His Glu
       610                 615                 620
   Glu Ser Gly Ser Val Ile Pro Asn Val Thr Pro Leu Asp Lys Arg Ala
   625                 630                 635                 640
   Gly Met Pro Asn Trp Gln Ile Ile His Ser Ala Glu Glu Val Gln Lys
                   645                 650                 655
   Ala Leu Ala Glu Gly Arg Phe Ala Thr Pro Asp Gly Tyr Ile Phe Asp
               660                 665                 670
   Pro Arg Asp Val Leu Ala Lys Glu Thr Phe Val Trp Lys Asp Gly Ser
           675                 680                 685
   Phe Ser Ile Pro Arg Ala Asp Gly Ser Ser Leu Arg Thr Ile Asn Lys
       690                 695                 700
   Ser Asp Leu Ser Gln Ala Glu Trp Gln Gln Ala Gln Glu Leu Leu Ala
   705                 710                 715                 720
   Lys Lys Asn Ala Gly Asp Ala Thr Asp Thr Asp Lys Pro Lys Glu Lys
                   725                 730                 735
   Gln Gln Ala Asp Lys Ser Asn Glu Asn Gln Gln Pro Ser Glu Ala Ser
               740                 745                 750
   Lys Glu Glu Glu Lys Glu Ser Asp Asp Phe Ile Asp Ser Leu Pro Asp
           755                 760                 765
   Tyr Gly Leu Asp Arg Ala Thr Leu Glu Asp His Ile Asn Gln Leu Ala
       770                 775                 780
   Gln Lys Ala Asn Ile Asp Pro Lys Tyr Leu Ile Phe Gln Pro Glu Gly
   785                 790                 795                 800
   Val Gln Phe Tyr Asn Lys Asn Gly Glu Leu Val Thr Tyr Asp Ile Lys
                   805                 810                 815
   Thr Leu Gln Gln Ile Asn Pro Pro
               820
<210> 84
   <211> 1019
   <212> PRT
   <213> S. pneumoniae
<400> 84
   Cys Ala Tyr Ala Leu Asn Gln His Arg Ser Gln Glu Asn Lys Asp Asn
    1               5                  10                  15
   Asn Arg Val Ser Tyr Val Asp Gly Ser Gln Ser Ser Gln Lys Ser Glu
               20                  25                  30
   Asn Leu Thr Pro Asp Gln Val Ser Gln Lys Glu Gly Ile Gln Ala Glu
           35                  40                  45
   Gln Ile Val Ile Lys Ile Thr Asp Gln Gly Tyr Val Thr Ser His Gly
       50                  55                  60
   Asp His Tyr His Tyr Tyr Asn Gly Lys Val Pro Tyr Asp Ala Leu Phe
   65                  70                  75                  80
   Ser Glu Glu Leu Leu Met Lys Asp Pro Asn Tyr Gln Leu Lys Asp Ala
                   85                  90                  95
   Asp Ile Val Asn Glu Val Lys Gly Gly Tyr Ile Ile Lys Val Asp Gly
               100                 105                 110
   Lys Tyr Tyr Val Tyr Leu Lys Asp Ala Ala His Ala Asp Asn Val Arg
           115                 120                 125
   Thr Lys Asp Glu Ile Asn Arg Gln Lys Gln Glu His Val Lys Asp Asn
       130                 135                 140
   Glu Lys Val Asn Ser Asn Val Ala Val Ala Arg Ser Gln Gly Arg Tyr
   145                 150                 155                 160
   Thr Thr Asn Asp Gly Tyr Val Phe Asn Pro Ala Asp Ile Ile Glu Asp
                   165                 170                 175
   Thr Gly Asn Ala Tyr Ile Val Pro His Arg Gly His Tyr His Tyr Ile
                   180                     185                     190
   Pro Lys Ser Asp Leu Ser Ala Ser Glu Leu Ala Ala Ala Lys Ala His
               195                     200                     205
   Leu Ala Gly Lys Asn Met Gln Pro Ser Gln Leu Ser Tyr Ser Ser Thr
       210                     215                     220
   Ala Ser Asp Asn Asn Thr Gln Ser Val Ala Lys Gly Ser Thr Ser Lys
   225                     230                     235                     240
   Pro Ala Asn Lys Ser Glu Asn Leu Gln Ser Leu Leu Lys Glu Leu Tyr
                   245                     250                     255
   Asp Ser Pro Ser Ala Gln Arg Tyr Ser Glu Ser Asp Gly Leu Val Phe
               260                     265                     270
   Asp Pro Ala Lys Ile Ile Ser Arg Thr Pro Asn Gly Val Ala Ile Pro
           275                     280                     285
   His Gly Asp His Tyr His Phe Ile Pro Tyr Ser Lys Leu Ser Ala Leu
       290                     295                     300
   Glu Glu Lys Ile Ala Arg Met Val Pro Ile Ser Gly Thr Gly Ser Thr
   305                     310                     315                     320
   Val Ser Thr Asn Ala Lys Pro Asn Glu Val Val Ser Ser Leu Gly Ser
                   325                     330                     335
   Leu Ser Ser Asn Pro Ser Ser Leu Thr Thr Ser Lys Glu Leu Ser Ser
               340                     345                     350
   Ala Ser Asp Gly Tyr Ile Phe Asn Pro Lys Asp Ile Val Glu Glu Thr
           355                     360                     365
   Ala Thr Ala Tyr Ile Val Arg His Gly Asp His Phe His Tyr Ile Pro
       370                     375                     380
   Lys Ser Asn Gln Ile Gly Gln Pro Thr Leu Pro Asn Asn Ser Leu Ala
   385                     390                     395                     400
   Thr Pro Ser Pro Ser Leu Pro Ile Asn Pro Gly Thr Ser His Glu Lys
                   405                     410                     415
   His Glu Glu Asp Gly Tyr Gly Phe Asp Ala Asn Arg Ile Ile Ala Glu
               420                     425                     430
   Asp Glu Ser Gly Phe Val Met Ser His Gly Asp His Asn His Tyr Phe
           435                     440                     445
   Phe Lys Lys Asp Leu Thr Glu Glu Gln Ile Lys Ala Ala Gln Lys His
       450                     455                     460
   Leu Glu Glu Val Lys Thr Ser His Asn Gly Leu Asp Ser Leu Ser Ser
   465                     470                     475                     480
   His Glu Gln Asp Tyr Pro Ser Asn Ala Lys Glu Met Lys Asp Leu Asp
                   485                     490                     495
   Lys Lys Ile Glu Glu Lys Ile Ala Gly Ile Met Lys Gln Tyr Gly Val
               500                     505                     510
   Lys Arg Glu Ser Ile Val Val Asn Lys Glu Lys Asn Ala Ile Ile Tyr
           515                     520                     525
   Pro His Gly Asp His His His Ala Asp Pro Ile Asp Glu His Lys Pro
       530                     535                     540
   Val Gly Ile Gly His Ser His Ser Asn Tyr Glu Leu Phe Lys Pro Glu
   545                     550                     555                     560
   Glu Gly Val Ala Lys Lys Glu Gly Asn Lys Val Tyr Thr Gly Glu Glu
                   565                     570                     575
   Leu Thr Asn Val Val Asn Leu Leu Lys Asn Ser Thr Phe Asn Asn Gln
               580                     585                     590
   Asn Phe Thr Leu Ala Asn Gly Gln Lys Arg Val Ser Phe Ser Phe Pro
           595                     600                     605
   Pro Glu Leu Glu Lys Lys Leu Gly Ile Asn Met Leu Val Lys Leu Ile
       610                     615                     620
   Thr Pro Asp Gly Lys Val Leu Glu Lys Val Ser Gly Lys Val Phe Gly
   625                     630                     635                     640
   Glu Gly Val Gly Asn Ile Ala Asn Phe Glu Leu Asp Gln Pro Tyr Leu
                       645                 650                 655
   Pro Gly Gln Thr Phe Lys Tyr Thr Ile Ala Ser Lys Asp Tyr Pro Glu
                   660                 665                 670
   Val Ser Tyr Asp Gly Thr Phe Thr Val Pro Thr Ser Leu Ala Tyr Lys
               675                 680                 685
   Met Ala Ser Gln Thr Ile Phe Tyr Pro Phe His Ala Gly Asp Thr Tyr
           690                 695                 700
   Leu Arg Val Asn Pro Gln Phe Ala Val Pro Lys Gly Thr Asp Ala Leu
   705                 710                 715                 720
   Val Arg Val Phe Asp Glu Phe His Gly Asn Ala Tyr Leu Glu Asn Asn
                       725                 730                 735
   Tyr Lys Val Gly Glu Ile Lys Leu Pro Ile Pro Lys Leu Asn Gln Gly
                   740                 745                 750
   Thr Thr Arg Thr Ala Gly Asn Lys Ile Pro Val Thr Phe Met Ala Asn
               755                 760                 765
   Ala Tyr Leu Asp Asn Gln Ser Thr Tyr Ile Val Glu Val Pro Ile Leu
           770                 775                 780
   Glu Lys Glu Asn Gln Thr Asp Lys Pro Ser Ile Leu Pro Gln Phe Lys
   785                 790                 795                 800
   Arg Asn Lys Ala Gln Glu Asn Ser Lys Phe Asp Glu Lys Val Glu Glu
                       805                 810                 815
   Pro Lys Thr Ser Glu Lys Val Glu Lys Glu Lys Leu Ser Glu Thr Gly
                   820                 825                 830
   Asn Ser Thr Ser Asn Ser Thr Leu Glu Glu Val Pro Thr Val Asp Pro
               835                 840                 845
   Val Gln Glu Lys Val Ala Lys Phe Ala Glu Ser Tyr Gly Met Lys Leu
           850                 855                 860
   Glu Asn Val Leu Phe Asn Met Asp Gly Thr Ile Glu Leu Tyr Leu Pro
   865                 870                 875                 880
   Ser Gly Glu Val Ile Lys Lys Asn Met Ala Asp Phe Thr Gly Glu Ala
                       885                 890                 895
   Pro Gln Gly Asn Gly Glu Asn Lys Pro Ser Glu Asn Gly Lys Val Ser
                   900                 905                 910
   Thr Gly Thr Val Glu Asn Gln Pro Thr Glu Asn Lys Pro Ala Asp Ser
               915                 920                 925
   Leu Pro Glu Ala Pro Asn Glu Lys Pro Val Lys Pro Glu Asn Ser Thr
           930                 935                 940
   Asp Asn Gly Met Leu Asn Pro Glu Gly Asn Val Gly Ser Asp Pro Met
   945                 950                 955                 960
   Leu Asp Pro Ala Leu Glu Glu Ala Pro Ala Val Asp Pro Val Gln Glu
                       965                 970                 975
   Lys Leu Glu Lys Phe Thr Ala Ser Tyr Gly Leu Gly Leu Asp Ser Val
                   980                 985                 990
   Ile Phe Asn Met Asp Gly Thr Ile Glu Leu Arg Leu Pro Ser Gly Glu
               995                 1000                1005
   Val Ile Lys Lys Asn Leu Ser Asp Leu Ile Ala
           1010                1015
<210> 85
   <211> 1019
   <212> PRT
   <213> S. pneumoniae
<400> 85
   Cys Ala Tyr Ala Leu Asn Gln His Arg Ser Gln Glu Asn Lys Asp Asn
    1               5                  10                  15
   Asn Arg Val Ser Tyr Val Asp Gly Ser Gln Ser Ser Gln Lys Ser Glu
               20                  25                  30
   Asn Leu Thr Pro Asp Gln Val Ser Gln Lys Glu Gly Ile Gln Ala Glu
           35                  40                  45
   Gln Ile Val Ile Lys Ile Thr Asp Gln Gly Tyr Val Thr Ser His Gly
       50                  55                  60
   Asp His Tyr His Tyr Tyr Asn Gly Lys Val Pro Tyr Asp Ala Leu Phe
   65                  70                  75                  80
   Ser Glu Glu Leu Leu Met Lys Asp Pro Asn Tyr Gln Leu Lys Asp Ala
                   85                  90                  95
   Asp Ile Val Asn Glu Val Lys Gly Gly Tyr Ile Ile Lys Val Asp Gly
               100                 105                 110
   Lys Tyr Tyr Val Tyr Leu Lys Asp Ala Ala His Ala Asp Asn Val Arg
           115                 120                 125
   Thr Lys Asp Glu Ile Asn Arg Gln Lys Gln Glu His Val Lys Asp Asn
       130                 135                 140
   Glu Lys Val Asn Ser Asn Val Ala Val Ala Arg Ser Gln Gly Arg Tyr
   145                 150                 155                 160
   Thr Thr Asn Asp Gly Tyr Val Phe Asn Pro Ala Asp Ile Ile Glu Asp
                   165                 170                 175
   Thr Gly Asn Ala Tyr Ile Val Pro His Gly Gly His Tyr His Tyr Ile
               180                 185                 190
   Pro Lys Ser Asp Leu Ser Ala Ser Glu Leu Ala Ala Ala Lys Ala His
           195                 200                 205
   Leu Ala Gly Lys Asn Met Gln Pro Ser Gln Leu Ser Tyr Ser Ser Thr
       210                 215                 220
   Ala Ser Asp Asn Asn Thr Gln Ser Val Ala Lys Gly Ser Thr Ser Lys
   225                 230                 235                 240
   Pro Ala Asn Lys Ser Glu Asn Leu Gln Ser Leu Leu Lys Glu Leu Tyr
                   245                 250                 255
   Asp Ser Pro Ser Ala Gln Arg Tyr Ser Glu Ser Asp Gly Leu Val Phe
               260                 265                 270
   Asp Pro Ala Lys Ile Ile Ser Arg Thr Pro Asn Gly Val Ala Ile Pro
           275                 280                 285
   His Gly Asp His Tyr His Phe Ile Pro Tyr Ser Lys Leu Ser Ala Leu
       290                 295                 300
   Glu Glu Lys Ile Ala Arg Arg Val Pro Ile Ser Gly Thr Gly Ser Thr
   305                 310                 315                 320
   Val Ser Thr Asn Ala Lys Pro Asn Glu Val Val Ser Ser Leu Gly Ser
                   325                 330                 335
   Leu Ser Ser Asn Pro Ser Ser Leu Thr Thr Ser Lys Glu Leu Ser Ser
               340                 345                 350
   Ala Ser Asp Gly Tyr Ile Phe Asn Pro Lys Asp Ile Val Glu Glu Thr
           355                 360                 365
   Ala Thr Ala Tyr Ile Val Arg His Gly Asp His Phe His Tyr Ile Pro
       370                 375                 380
   Lys Ser Asn Gln Ile Gly Gln Pro Thr Leu Pro Asn Asn Ser Leu Ala
   385                 390                 395                 400
   Thr Pro Ser Pro Ser Leu Pro Ile Asn Pro Gly Ile Ser His Glu Lys
                   405                 410                 415
   His Glu Glu Asp Gly Tyr Gly Phe Asp Ala Asn Arg Ile Ile Ala Glu
               420                 425                 430
   Asp Glu Ser Gly Phe Ile Met Ser His Gly Asn His Asn His Tyr Phe
           435                 440                 445
   Phe Lys Lys Asp Leu Thr Glu Glu Gln Ile Lys Ala Ala Gln Lys His
       450                 455                 460
   Leu Glu Glu Val Lys Thr Ser His Asn Gly Leu Asp Ser Leu Ser Ser
   465                 470                 475                 480
   His Glu Gln Asp Tyr Pro Gly Asn Ala Lys Glu Met Lys Asp Leu Asp
                   485                 490                 495
   Lys Lys Ile Glu Glu Lys Ile Ala Gly Ile Met Lys Gln Tyr Gly Val
               500                 505                 510
   Lys Arg Glu Ser Ile Val Val Asn Lys Glu Lys Asn Ala Ile Ile Tyr
           515                 520                 525
   Pro His Gly Asp His His His Ala Asp Pro Ile Asp Glu His Lys Pro
       530                 535                 540
   Val Gly Ile Gly His Ser His Ser Asn Tyr Glu Leu Phe Lys Pro Glu
   545                 550                 555                 560
   Glu Gly Val Ala Lys Lys Glu Gly Asn Lys Val Tyr Thr Gly Glu Glu
                   565                 570                 575
   Leu Thr Asn Val Val Asn Leu Leu Lys Asn Ser Thr Phe Asn Asn Gln
               580                 585                 590
   Asn Phe Thr Leu Ala Asn Gly Gln Lys Arg Val Ser Phe Ser Phe Pro
           595                 600                 605
   Pro Glu Leu Glu Lys Lys Leu Gly Ile Asn Met Leu Val Lys Leu Ile
       610                 615                 620
   Thr Pro Asp Gly Lys Val Leu Glu Lys Val Ser Gly Lys Val Phe Gly
   625                 630                 635                 640
   Glu Gly Val Gly Asn Ile Ala Asn Phe Glu Leu Asp Gln Pro Tyr Leu
                   645                 650                 655
   Pro Gly Gln Thr Phe Lys Tyr Thr Ile Ala Ser Lys Asp Tyr Pro Glu
               660                 665                 670
   Val Ser Tyr Asp Gly Thr Phe Thr Val Pro Thr Ser Leu Ala Tyr Lys
           675                 680                 685
   Met Ala Ser Gln Thr Ile Phe Tyr Pro Phe His Ala Gly Asp Thr Tyr
       690                 695                 700
   Leu Arg Val Asn Pro Gln Phe Ala Val Pro Lys Gly Thr Asp Ala Leu
   705                 710                 715                 720
   Val Arg Val Phe Asp Glu Phe His Gly Asn Ala Tyr Leu Glu Asn Asn
                   725                 730                 735
   Tyr Lys Val Gly Glu Ile Lys Leu Pro Ile Pro Lys Leu Asn Gln Gly
               740                 745                 750
   Thr Thr Arg Thr Ala Gly Asn Lys Ile Pro Val Thr Phe Met Ala Asn
           755                 760                 765
   Ala Tyr Leu Asp Asn Gln Ser Thr Tyr Ile Val Glu Val Pro Ile Leu
       770                 775                 780
   Glu Lys Glu Asn Gln Thr Asp Lys Pro Ser Ile Leu Pro Gln Phe Lys
   785                 790                 795                 800
   Arg Asn Lys Ala Gln Glu Asn Ser Lys Leu Asp Glu Lys Val Glu Glu
                   805                 810                 815
   Pro Lys Thr Ser Glu Lys Val Glu Lys Glu Lys Leu Ser Glu Thr Gly
               820                 825                 830
   Asn Ser Thr Ser Asn Ser Thr Leu Glu Glu Val Pro Thr Val Asp Pro
           835                 840                 845
   Val Gln Glu Lys Val Ala Lys Phe Ala Glu Ser Tyr Gly Met Lys Leu
       850                 855                 860
   Glu Asn Val Leu Phe Asn Met Asp Gly Thr Ile Glu Leu Tyr Leu Pro
   865                 870                 875                 880
   Ser Gly Glu Val Ile Lys Lys Asn Met Ala Asp Phe Thr Gly Glu Ala
                   885                 890                 895
   Pro Gln Gly Asn Gly Glu Asn Lys Pro Ser Glu Asn Gly Lys Val Ser
               900                 905                 910
   Thr Gly Thr Val Glu Asn Gln Pro Thr Glu Asn Lys Pro Ala Asp Ser
           915                 920                 925
   Leu Pro Glu Ala Pro Asn Glu Lys Pro Val Lys Pro Glu Asn Ser Thr
       930                 935                 940
   Asp Asn Gly Met Leu Asn Pro Glu Gly Asn Val Gly Ser Asp Pro Met
   945                 950                 955                 960
   Leu Asp Pro Ala Leu Glu Glu Ala Pro Ala Val Asp Pro Val Gln Glu
                   965                 970                 975
   Lys Leu Glu Lys Phe Thr Ala Ser Tyr Gly Leu Gly Leu Asp Ser Val
               980                 985                 990
   Ile Phe Asn Met Asp Gly Thr Ile Glu Leu Arg Leu Pro Ser Gly Glu
           995                 1000                1005
   Val Ile Lys Lys Asn Leu Ser Asp Leu Ile Ala
       1010                1015
<210> 86
   <211> 1019
   <212> PRT
   <213> S. pneumoniae
<400> 86
   Cys Ala Tyr Ala Leu Asn Gln His Arg Ser Gln Glu Asn Lys Asp Asn
   1               5                   10                  15
   Asn Arg Val Ser Tyr Val Asp Gly Ser Gln Ser Ser Gln Lys Ser Glu
               20                  25                  30
   Asn Leu Thr Pro Asp Gln Val Ser Gln Lys Glu Gly Ile Gln Ala Glu
           35                  40                  45
   Gln Ile Val Ile Lys Ile Thr Asp Gln Gly Tyr Val Thr Ser His Gly
       50                  55                  60
   Asp His Tyr His Tyr Tyr Asn Gly Lys Val Pro Tyr Asp Ala Leu Phe
   65                  70                  75                  80
   Ser Glu Glu Leu Leu Met Lys Asp Pro Asn Tyr Gln Leu Lys Asp Ala
                   85                  90                  95
   Asp Ile Val Asn Glu Val Lys Gly Gly Tyr Ile Ile Lys Val Asp Gly
               100                 105                 110
   Lys Tyr Tyr Val Tyr Leu Lys Asp Ala Ala His Ala Asp Asn Val Arg
           115                 120                 125
   Thr Lys Asp Glu Ile Asn Arg Gln Lys Gln Glu His Val Lys Asp Asn
       130                 135                 140
   Glu Lys Val Asn Ser Asn Val Ala Val Ala Arg Ser Gln Gly Arg Tyr
   145                 150                 155                 160
   Thr Thr Asn Asp Gly Tyr Val Phe Asn Pro Ala Asp Ile Ile Glu Asp
                   165                 170                 175
   Thr Gly Asn Ala Tyr Ile Val Pro His Gly Gly His Tyr His Tyr Ile
               180                 185                 190
   Pro Lys Ser Asp Leu Ser Ala Ser Glu Leu Ala Ala Ala Lys Ala His
           195                 200                 205
   Leu Ala Gly Lys Asn Met Gln Pro Ser Gln Leu Ser Tyr Ser Ser Thr
       210                 215                 220
   Ala Ser Asp Asn Asn Thr Gln Ser Val Ala Lys Gly Ser Thr Ser Lys
   225                 230                 235                 240
   Pro Ala Asn Lys Ser Glu Asn Leu Gln Ser Leu Leu Lys Glu Leu Tyr
                   245                 250                 255
   Asp Ser Pro Ser Ala Gln Arg Tyr Ser Glu Ser Asp Gly Leu Val Phe
               260                 265                 270
   Asp Pro Ala Lys Ile Ile Ser Arg Thr Pro Asn Gly Val Ala Ile Pro
           275                 280                 285
   His Gly Asp His Tyr His Phe Ile Pro Tyr Ser Lys Leu Ser Ala Leu
       290                 295                 300
   Glu Glu Lys Ile Ala Arg Met Val Pro Ile Ser Gly Thr Gly Ser Thr
   305                 310                 315                 320
   Val Ser Thr Asn Ala Lys Pro Asn Glu Val Val Ser Ser Leu Gly Ser
                   325                 330                 335
   Leu Ser Ser Asn Pro Ser Ser Leu Thr Thr Ser Lys Glu Leu Ser Ser
   340 345 350
   Ala Ser Asp Gly Tyr Ile Phe Asn Pro Lys Asp Ile Val Glu Glu Thr
   355 360 365
   Ala Thr Ala Tyr Ile Val Arg His Gly Asp His Phe His Tyr Ile Pro
   370 375 380
   Lys Ser Asn Gln Ile Gly Gln Pro Thr Leu Pro Asn Asn Ser Leu Ala
   385 390 395 400
   Thr Pro Ser Pro Ser Leu Pro Ile Asn Pro Gly Thr Ser His Glu Lys
   405 410 415
   His Glu Glu Asp Gly Tyr Gly Phe Asp Ala Asn Arg Ile Ile Ala Glu
   420 425 430
   Asp Glu Ser Gly Phe Val Met Ser His Gly Asp His Asn His Tyr Phe
   435 440 445
   Phe Lys Lys Asp Leu Thr Glu Glu Gln Ile Lys Ala Ala Gln Lys His
   450 455 460
   Leu Glu Glu Val Lys Thr Ser His Asn Gly Leu Asp Ser Leu Ser Ser
   465 470 475 480
   His Glu Gln Asp Tyr Pro Ser Asn Ala Lys Glu Met Lys Asp Leu Asp
   485 490 495
   Lys Lys Ile Glu Glu Lys Ile Ala Gly Ile Met Lys Gln Tyr Gly Val
   500 505 510
   Lys Arg Glu Ser Ile Val Val Asn Lys Glu Lys Asn Ala Ile Ile Tyr
   515 520 525
   Pro His Gly Asp His His His Ala Asp Pro Ile Asp Glu His Lys Pro
   530 535 540
   Val Gly Ile Gly His Ser His Ser Asn Tyr Glu Leu Phe Lys Pro Glu
   545 550 555 560
   Glu Gly Val Ala Lys Lys Glu Gly Asn Lys Val Tyr Thr Gly Glu Glu
   565 570 575
   Leu Thr Asn Val Val Asn Leu Leu Lys Asn Ser Thr Phe Asn Asn Gln
   580 585 590
   Asn Phe Thr Leu Ala Asn Gly Gln Lys Arg Val Ser Phe Ser Phe Pro
   595 600 605
   Pro Glu Leu Glu Lys Lys Leu Gly Ile Asn Met Leu Val Lys Leu Ile
   610 615 620
   Thr Pro Asp Gly Lys Val Leu Glu Lys Val Ser Gly Lys Val Phe Gly
   625 630 635 640
   Glu Gly Val Gly Asn Ile Ala Asn Phe Glu Leu Asp Gln Pro Tyr Leu
   645 650 655
   Pro Gly Gln Thr Phe Lys Tyr Thr Ile Ala Ser Lys Asp Tyr Pro Glu
   660 665 670
   Val Ser Tyr Asp Gly Thr Phe Thr Val Pro Thr Ser Leu Ala Tyr Lys
   675 680 685
   Met Ala Ser Gln Thr Ile Phe Tyr Pro Phe His Ala Gly Asp Thr Tyr
   690 695 700
   Leu Arg Val Asn Pro Gln Phe Ala Val Pro Lys Gly Thr Asp Ala Leu
   705 710 715 720
   Val Arg Val Phe Asp Glu Phe His Gly Asn Ala Tyr Leu Glu Asn Asn
   725 730 735
   Tyr Lys Val Gly Glu Ile Lys Leu Pro Ile Pro Lys Leu Asn Gln Gly
   740 745 750
   Thr Thr Arg Thr Ala Gly Asn Lys Ile Pro Val Thr Phe Met Ala Asn
   755 760 765
   Ala Tyr Leu Asp Asn Gln Ser Thr Tyr Ile Val Glu Val Pro Ile Leu
   770 775 780
   Glu Lys Glu Asn Gln Thr Asp Lys Pro Ser Ile Leu Pro Gln Phe Lys
   785 790 795 800
   Arg Asn Lys Ala Gln Glu Asn Leu Lys Leu Asp Glu Lys Val Glu Glu
   805 810 815
   Pro Lys Thr Ser Glu Lys Val Glu Lys Glu Lys Leu Ser Glu Thr Gly
   820 825 830
   Asn Ser Thr Ser Asn Ser Thr Leu Glu Glu Val Pro Thr Val Asp Pro
   835 840 845
   Val Gln Glu Lys Val Ala Lys Phe Ala Glu Ser Tyr Gly Met Lys Leu
   850 855 860
   Glu Asn Val Leu Phe Asn Met Asp Gly Thr Ile Glu Leu Tyr Leu Pro
   865 870 875 880
   Ser Gly Glu Val Ile Lys Lys Asn Met Ala Asp Phe Thr Gly Glu Ala
   885 890 895
   Pro Gln Gly Asn Gly Glu Asn Lys Pro Ser Glu Asn Gly Lys Val Ser
   900 905 910
   Thr Gly Thr Val Glu Asn Gln Pro Thr Glu Asn Lys Pro Ala Asp Ser
   915 920 925
   Leu Pro Glu Ala Pro Asn Glu Lys Pro Val Lys Pro Glu Asn Ser Thr
   930 935 940
   Asp Asn Gly Met Leu Asn Pro Glu Gly Asn Val Gly Ser Asp Pro Met
   945 950 955 960
   Leu Asp Pro Ala Leu Glu Glu Ala Pro Ala Val Asp Pro Val Gln Glu
   965 970 975
   Lys Leu Glu Lys Phe Thr Ala Ser Tyr Gly Leu Gly Leu Asp Ser Val
   980 985 990
   Ile Phe Asn Met Asp Gly Thr Ile Glu Leu Arg Leu Pro Ser Gly Glu
   995 1000 1005
   Val Ile Lys Lys Asn Leu Ser Asp Leu Ile Ala
   1010 1015
<210> 87
   <211> 1019
   <212> PRT
   <213> S. pneumoniae
<400> 87
   Cys Ala Tyr Ala Leu Asn Gln His Arg Ser Gln Glu Asn Lys Asp Asn
    1               5                  10                  15
   Asn Arg Val Ser Tyr Val Asp Gly Ser Gln Ser Ser Gln Lys Ser Glu
               20                  25                  30
   Asn Leu Thr Pro Asp Gln Val Ser Gln Lys Glu Gly Ile Gln Ala Glu
           35                  40                  45
   Gln Ile Val Ile Lys Ile Thr Asp Gln Gly Tyr Val Thr Ser His Gly
       50                  55                  60
   Asp His Tyr His Tyr Tyr Asn Gly Lys Val Pro Tyr Asp Ala Leu Phe
   65                  70                  75                  80
   Ser Glu Glu Leu Leu Met Lys Asp Pro Asn Tyr Gln Leu Lys Asp Ala
                   85                  90                  95
   Asp Ile Val Asn Glu Val Lys Gly Gly Tyr Ile Ile Lys Val Asp Gly
               100                 105                 110
   Lys Tyr Tyr Val Tyr Leu Lys Asp Ala Ala His Ala Asp Asn Val Arg
           115                 120                 125
   Thr Lys Asp Glu Ile Asn Arg Gln Lys Gln Glu His Val Lys Asp Asn
       130                 135                 140
   Glu Lys Val Asn Ser Asn Val Ala Val Ala Arg Ser Gln Gly Arg Tyr
   145                 150                 155                 160
   Thr Thr Asn Asp Gly Tyr Val Phe Asn Pro Ala Asp Ile Ile Glu Asp
                   165                 170                 175
   Thr Gly Asn Ala Tyr Ile Val Pro His Gly Gly His Tyr His Tyr Ile
               180                 185                 190
   Pro Lys Ser Asp Leu Ser Ala Ser Glu Leu Ala Ala Ala Lys Ala His
           195                 200                 205
   Leu Ala Gly Lys Asn Met Gln Pro Ser Gln Leu Ser Tyr Ser Ser Thr
       210                 215                 220
   Ala Ser Asp Asn Asn Thr Gln Ser Val Ala Lys Gly Ser Thr Ser Lys
   225                 230                 235                 240
   Pro Ala Asn Lys Ser Glu Asn Leu Gln Ser Leu Leu Lys Glu Leu Tyr
                   245                 250                 255
   Asp Ser Pro Ser Ala Gln Arg Tyr Ser Glu Ser Asp Gly Leu Val Phe
               260                 265                 270
   Asp Pro Ala Lys Ile Ile Ser Arg Thr Pro Asn Gly Val Ala Ile Pro
           275                 280                 285
   His Gly Asp His Tyr His Phe Ile Pro Tyr Ser Lys Leu Ser Ala Leu
       290                 295                 300
   Glu Glu Lys Ile Ala Arg Met Val Pro Ile Ser Gly Thr Gly Ser Thr
   305                 310                 315                 320
   Val Ser Thr Asn Ala Lys Pro Asn Glu Val Val Ser Ser Leu Gly Ser
                   325                 330                 335
   Leu Ser Ser Asn Pro Ser Ser Leu Thr Thr Ser Lys Glu Leu Ser Ser
               340                 345                 350
   Ala Ser Asp Gly Tyr Ile Phe Asn Pro Lys Asp Ile Val Glu Glu Thr
           355                 360                 365
   Ala Thr Ala Tyr Ile Val Arg His Gly Asp His Phe His Tyr Ile Pro
       370                 375                 380
   Lys Ser Asn Gln Ile Gly Gln Pro Thr Leu Pro Asn Asn Ser Leu Ala
   385                 390                 395                 400
   Thr Pro Ser Pro Ser Leu Pro Ile Asn Pro Gly Thr Ser His Glu Lys
                   405                 410                 415
   His Glu Glu Asp Gly Tyr Gly Phe Asp Ala Asn Arg Ile Ile Ala Glu
               420                 425                 430
   Asp Glu Ser Gly Phe Val Met Ser His Gly Asp His Asn His Tyr Phe
           435                 440                 445
   Phe Lys Lys Asp Leu Thr Glu Glu Gln Ile Lys Ala Ala Gln Lys His
       450                 455                 460
   Leu Glu Glu Val Lys Thr Ser His Asn Gly Leu Asp Ser Leu Ser Ser
   465                 470                 475                 480
   His Glu Gln Asp Tyr Pro Gly Asn Ala Lys Glu Met Lys Asp Leu Asp
                   485                 490                 495
   Lys Lys Ile Glu Glu Lys Ile Ala Gly Ile Met Lys Gln Tyr Gly Val
               500                 505                 510
   Lys Arg Glu Ser Ile Val Val Asn Lys Glu Lys Asn Ala Ile Ile Tyr
           515                 520                 525
   Pro His Gly Asp His His His Ala Asp Pro Ile Asp Glu His Lys Pro
       530                 535                 540
   Val Gly Ile Gly His Ser His Ser Asn Tyr Glu Leu Phe Lys Pro Glu
   545                 550                 555                 560
   Glu Gly Val Ala Lys Lys Glu Gly Asn Lys Val Tyr Thr Gly Glu Glu
                   565                 570                 575
   Leu Thr Asn Val Val Asn Leu Leu Lys Asn Ser Thr Phe Asn Asn Gln
               580                 585                 590
   Asn Phe Thr Leu Ala Asn Gly Gln Lys Arg Val Ser Phe Ser Phe Pro
           595                 600                 605
   Pro Glu Leu Glu Lys Lys Leu Gly Ile Asn Met Leu Val Lys Leu Ile
       610                 615                 620
   Thr Pro Asp Gly Lys Val Leu Glu Lys Val Ser Gly Lys Val Phe Gly
   625                 630                 635                 640
   Glu Gly Val Gly Asn Ile Ala Asn Phe Glu Leu Asp Gln Pro Tyr Leu
                   645                 650                 655
   Pro Gly Gln Thr Phe Lys Tyr Thr Ile Ala Ser Lys Asp Tyr Pro Glu
               660                 665                 670
   Val Ser Tyr Asp Gly Thr Phe Thr Val Pro Thr Ser Leu Ala Tyr Lys
           675                 680                 685
   Met Ala Ser Gln Thr Ile Phe Tyr Pro Phe His Ala Gly Asp Thr Tyr
       690                 695                 700
   Leu Arg Val Asn Pro Gln Phe Ala Val Pro Lys Gly Thr Asp Ala Leu
   705                 710                 715                 720
   Val Arg Val Phe Asp Glu Phe His Gly Asn Ala Tyr Leu Glu Asn Asn
                   725                 730                 735
   Tyr Lys Val Gly Glu Ile Lys Leu Pro Ile Pro Lys Leu Asn Gln Gly
               740                 745                 750
   Thr Thr Arg Thr Ala Gly Asn Lys Ile Pro Val Thr Phe Met Ala Asn
           755                 760                 765
   Ala Tyr Leu Asp Asn Gln Ser Thr Tyr Ile Val Glu Val Pro Ile Leu
       770                 775                 780
   Glu Lys Glu Asn Gln Thr Asp Lys Pro Ser Ile Leu Pro Gln Phe Lys
   785                 790                 795                 800
   Arg Asn Lys Ala Gln Glu Asn Ser Lys Leu Asp Glu Lys Val Glu Glu
                   805                 810                 815
   Pro Lys Thr Ser Glu Lys Val Glu Lys Glu Lys Leu Ser Glu Thr Gly
               820                 825                 830
   Asn Ser Thr Ser Asn Ser Thr Leu Glu Glu Val Pro Thr Val Asp Pro
           835                 840                 845
   Val Gln Glu Lys Val Ala Lys Phe Ala Glu Ser Tyr Gly Met Lys Leu
       850                 855                 860
   Glu Asn Val Leu Phe Asn Met Asp Gly Thr Ile Glu Leu Tyr Leu Pro
   865                 870                 875                 880
   Ser Gly Glu Val Ile Lys Lys Asn Met Ala Asp Phe Thr Gly Glu Ala
                   885                 890                 895
   Pro Gln Gly Asn Gly Glu Asn Lys Pro Ser Glu Asn Gly Lys Val Ser
               900                 905                 910
   Thr Gly Thr Val Glu Asn Gln Pro Thr Glu Asn Lys Pro Ala Asp Ser
           915                 920                 925
   Leu Pro Glu Ala Pro Asn Glu Lys Pro Val Lys Pro Glu Asn Ser Thr
       930                 935                 940
   Asp Asn Gly Met Leu Asn Pro Glu Gly Asn Val Gly Ser Asp Pro Met
   945                 950                 955                 960
   Leu Asp Pro Ala Leu Glu Glu Ala Pro Ala Val Asp Pro Val Gln Glu
                   965                 970                 975
   Lys Leu Glu Lys Phe Thr Ala Ser Tyr Gly Leu Gly Leu Asp Ser Val
               980                 985                 990
   Ile Phe Asn Met Asp Gly Thr Ile Glu Leu Arg Leu Pro Ser Gly Glu
           995                 1000                1005
   Val Ile Lys Lys Asn Leu Ser Asp Phe Ile Ala
       1010                1015
<210> 88
   <211> 1019
   <212> PRT
   <213> S. pneumoniae
<400> 88
   Cys Ala Tyr Ala Leu Asn Gln His Arg Ser Gln Glu Asn Lys Asp Asn
    1               5                  10                  15
   Asn Arg Val Ser Tyr Val Asp Gly Ser Gln Ser Ser Gln Lys Ser Glu
               20                  25                  30
   Asn Leu Thr Pro Asp Gln Val Ser Gln Lys Glu Gly Ile Gln Ala Glu
               35                  40                  45
   Gln Ile Val Ile Lys Ile Thr Asp Gln Gly Tyr Val Thr Ser His Gly
       50                  55                  60
   Asp His Tyr His Tyr Tyr Asn Gly Lys Val Pro Tyr Asp Ala Leu Phe
   65                  70                  75                  80
   Ser Glu Glu Leu Leu Met Lys Asp Pro Asn Tyr Gln Leu Lys Asp Ala
                   85                  90                  95
   Asp Ile Val Asn Glu Val Lys Gly Gly Tyr Ile Ile Lys Val Asp Gly
               100                 105                 110
   Lys Tyr Tyr Val Tyr Leu Lys Asp Ala Ala His Ala Asp Asn Val Arg
           115                 120                 125
   Thr Lys Asp Glu Ile Asn Arg Gln Lys Gln Glu His Val Lys Asp Asn
       130                 135                 140
   Glu Lys Val Asn Ser Asn Val Ala Val Ala Arg Ser Gln Gly Arg Tyr
   145                 150                 155                 160
   Thr Thr Asn Asp Gly Tyr Val Phe Asn Pro Ala Asp Ile Ile Glu Asp
                   165                 170                 175
   Thr Gly Asn Ala Tyr Ile Val Pro His Arg Gly His Tyr His Tyr Ile
               180                 185                 190
   Pro Lys Ser Asp Leu Ser Ala Ser Glu Leu Ala Ala Ala Lys Ala His
           195                 200                 205
   Leu Ala Gly Lys Asn Met Gln Pro Ser Gln Leu Ser Tyr Ser Ser Thr
       210                 215                 220
   Ala Ser Asp Asn Asn Thr Gln Ser Val Ala Lys Gly Ser Thr Ser Lys
   225                 230                 235                 240
   Pro Ala Asn Lys Ser Glu Asn Leu Gln Ser Leu Leu Lys Glu Leu Tyr
                   245                 250                 255
   Asp Ser Pro Ser Ala Gln Arg Tyr Ser Glu Ser Asp Gly Leu Val Phe
               260                 265                 270
   Asp Pro Ala Lys Ile Ile Ser Arg Thr Pro Asn Gly Val Ala Ile Pro
           275                 280                 285
   His Gly Asp His Tyr His Phe Ile Pro Tyr Ser Lys Leu Ser Ala Leu
       290                 295                 300
   Glu Glu Lys Ile Ala Arg Met Val Pro Ile Ser Gly Thr Gly Ser Thr
   305                 310                 315                 320
   Val Ser Thr Asn Ala Lys Pro Asn Glu Val Val Ser Ser Leu Gly Ser
                   325                 330                 335
   Leu Ser Ser Asn Pro Ser Ser Leu Thr Thr Ser Lys Glu Leu Ser Ser
               340                 345                 350
   Ala Ser Asp Gly Tyr Ile Phe Asn Pro Lys Asp Ile Val Glu Glu Thr
           355                 360                 365
   Ala Thr Ala Tyr Ile Val Arg His Gly Asp His Phe His Tyr Ile Pro
       370                 375                 380
   Lys Ser Asn Gln Ile Gly Gln Pro Thr Leu Pro Asn Asn Ser Leu Ala
   385                 390                 395                 400
   Thr Pro Ser Pro Ser Leu Pro Ile Asn Pro Gly Thr Ser His Glu Lys
                   405                 410                 415
   His Glu Glu Asp Gly Tyr Gly Phe Asp Ala Asn Arg Ile Ile Ala Glu
               420                 425                 430
   Asp Glu Ser Gly Phe Val Met Ser His Gly Asp His Asn His Tyr Phe
           435                 440                 445
   Phe Lys Lys Asp Leu Thr Glu Glu Gln Ile Lys Ala Ala Gln Lys His
       450                 455                 460
   Leu Glu Glu Val Lys Thr Ser His Asn Gly Leu Asp Ser Leu Ser Ser
   465                 470                 475                 480
   His Glu Gln Asp Tyr Pro Ser Asn Ala Lys Glu Met Lys Asp Leu Asp
                   485                 490                 495
   Lys Lys Ile Glu Glu Lys Ile Ala Gly Ile Met Lys Gln Tyr Gly Val
               500                     505                     510
   Lys Arg Glu Ser Ile Val Val Asn Lys Glu Lys Asn Ala Ile Ile Tyr
           515                     520                     525
   Pro His Gly Asp His His His Ala Asp Pro Ile Asp Glu His Lys Pro
       530                     535                     540
   Val Gly Ile Gly His Ser His Ser Asn Tyr Glu Leu Phe Lys Pro Glu
   545                     550                     555                     560
   Glu Gly Val Ala Lys Lys Glu Gly Asn Lys Val Tyr Thr Gly Glu Glu
                   565                     570                     575
   Leu Thr Asn Val Val Asn Leu Leu Lys Asn Ser Thr Phe Asn Asn Gln
               580                     585                     590
   Asn Phe Thr Leu Ala Asn Gly Gln Lys Arg Val Ser Phe Ser Phe Pro
           595                     600                     605
   Pro Glu Leu Glu Lys Lys Leu Gly Ile Asn Met Leu Val Lys Leu Ile
       610                     615                     620
   Thr Pro Asp Gly Lys Val Leu Glu Lys Val Ser Gly Lys Val Phe Gly
   625                     630                     635                     640
   Glu Gly Val Gly Asn Ile Ala Asn Phe Glu Leu Asp Gln Pro Tyr Leu
                   645                     650                     655
   Pro Gly Gln Thr Phe Lys Tyr Thr Ile Ala Ser Lys Asp Tyr Pro Glu
               660                     665                     670
   Val Ser Tyr Asp Gly Thr Phe Thr Val Pro Thr Ser Leu Ala Tyr Lys
           675                     680                     685
   Met Ala Ser Gln Thr Ile Phe Tyr Pro Phe His Ala Gly Asp Thr Tyr
       690                     695                     700
   Leu Arg Val Asn Pro Gln Phe Ala Val Pro Lys Gly Thr Asp Ala Leu
   705                     710                     715                     720
   Val Arg Val Phe Asp Glu Phe His Gly Asn Ala Tyr Leu Glu Asn Asn
                   725                     730                     735
   Tyr Lys Val Gly Glu Ile Lys Leu Pro Ile Pro Lys Leu Asn Gln Gly
               740                     745                     750
   Thr Thr Arg Thr Ala Gly Asn Lys Ile Pro Val Thr Phe Met Ala Asn
           755                     760                     765
   Ala Tyr Leu Asp Asn Gln Ser Thr Tyr Ile Val Glu Val Pro Ile Leu
       770                     775                     780
   Glu Lys Glu Asn Gln Thr Asp Lys Pro Ser Ile Leu Pro Gln Phe Lys
   785                     790                     795                     800
   Arg Asn Lys Ala Gln Glu Asn Ser Lys Phe Asp Glu Lys Val Glu Glu
                   805                     810                     815
   Pro Lys Thr Ser Glu Lys Val Glu Lys Glu Lys Leu Ser Glu Thr Gly
               820                     825                     830
   Asn Ser Thr Ser Asn Ser Thr Leu Glu Glu Val Pro Thr Val Asp Pro
           835                     840                     845
   Val Gln Glu Lys Val Ala Lys Phe Ala Glu Ser Tyr Gly Met Lys Leu
       850                     855                     860
   Glu Asn Val Leu Phe Asn Met Asp Gly Thr Ile Glu Leu Tyr Leu Pro
   865                     870                     875                     880
   Ser Gly Glu Val Ile Lys Lys Asn Met Ala Asp Phe Thr Gly Glu Ala
                   885                     890                     895
   Pro Gln Gly Asn Gly Glu Asn Lys Pro Ser Glu Asn Gly Lys Val Ser
               900                     905                     910
   Thr Gly Thr Val Glu Asn Gln Pro Thr Glu Asn Lys Pro Ala Asp Ser
           915                     920                     925
   Leu Pro Glu Ala Pro Asn Glu Lys Pro Val Lys Pro Glu Asn Ser Thr
       930                     935                     940
   Asp Asn Gly Met Leu Asn Pro Glu Gly Asn Val Gly Ser Asp Pro Met
   945                     950                     955                     960
   Leu Asp Pro Ala Leu Glu Glu Ala Pro Ala Val Asp Pro Val Gln Glu
                   965                 970                 975
   Lys Leu Glu Lys Phe Thr Ala Ser Tyr Gly Leu Gly Leu Asp Ser Val
               980                 985                 990
   Ile Phe Asn Met Asp Gly Thr Ile Glu Leu Arg Leu Pro Ser Gly Glu
           995                 1000                1005
   Val Ile Lys Lys Asn Leu Ser Asp Leu Ile Ala
       1010                1015
<210> 89
   <211> 1019
   <212> PRT
   <213> S. pneumoniae
<400> 89
   Cys Ala Tyr Ala Leu Asn Gln His Arg Ser Gln Glu Asn Lys Asp Asn
    1               5                  10                  15
   Asn Arg Val Ser Tyr Val Asp Gly Ser Gln Ser Ser Gln Lys Ser Glu
               20                  25                  30
   Asn Leu Thr Pro Asp Gln Val Ser Gln Lys Glu Gly Ile Gln Ala Glu
           35                  40                  45
   Gln Ile Val Ile Lys Ile Thr Asp Gln Gly Tyr Val Thr Ser His Gly
       50                  55                  60
   Asp His Tyr His Tyr Tyr Asn Gly Lys Val Pro Tyr Asp Ala Leu Phe
   65                  70                  75                  80
   Ser Glu Glu Leu Leu Met Lys Asp Pro Asn Tyr Gln Leu Lys Asp Ala
                   85                  90                  95
   Asp Ile Val Asn Glu Val Lys Gly Gly Tyr Ile Ile Lys Val Asp Gly
               100                 105                 110
   Lys Tyr Tyr Val Tyr Leu Lys Asp Ala Ala His Ala Asp Asn Val Arg
           115                 120                 125
   Thr Lys Asp Glu Ile Asn Arg Gln Lys Gln Glu His Val Lys Asp Asn
       130                 135                 140
   Glu Lys Val Asn Ser Asn Val Ala Val Ala Arg Ser Gln Gly Arg Tyr
   145                 150                 155                 160
   Thr Thr Asn Asp Gly Tyr Val Phe Asn Pro Ala Asp Ile Ile Glu Asp
                   165                 170                 175
   Thr Gly Asn Ala Tyr Ile Val Pro His Arg Gly His Tyr His Tyr Ile
               180                 185                 190
   Pro Lys Ser Asp Leu Ser Ala Ser Glu Leu Ala Ala Ala Lys Ala His
           195                 200                 205
   Leu Ala Gly Lys Asn Met Gln Pro Ser Gln Leu Ser Tyr Ser Ser Thr
       210                 215                 220
   Ala Ser Asp Asn Asn Thr Gln Ser Val Ala Lys Gly Ser Thr Ser Lys
   225                 230                 235                 240
   Pro Ala Asn Lys Ser Glu Asn Leu Gln Ser Leu Leu Lys Glu Leu Tyr
                   245                 250                 255
   Asp Ser Pro Ser Ala Gln Arg Tyr Ser Glu Ser Asp Gly Leu Val Phe
               260                 265                 270
   Asp Pro Ala Lys Ile Ile Ser Arg Thr Pro Asn Gly Val Ala Ile Pro
           275                 280                 285
   His Gly Asp His Tyr His Phe Ile Pro Tyr Ser Lys Leu Ser Ala Leu
       290                 295                 300
   Glu Glu Lys Ile Ala Arg Met Val Pro Ile Ser Gly Thr Gly Ser Thr
   305                 310                 315                 320
   Val Ser Thr Asn Ala Lys Pro Asn Glu Val Val Ser Ser Leu Gly Ser
                   325                 330                 335
   Leu Ser Ser Asn Pro Ser Ser Leu Thr Thr Ser Lys Glu Leu Ser Ser
               340                 345                 350
   Ala Ser Asp Gly Tyr Ile Phe Asn Pro Lys Asp Ile Val Glu Glu Thr
   355 360 365
   Ala Thr Ala Tyr Ile Val Arg His Gly Asp His Phe His Tyr Ile Pro
   370 375 380
   Lys Ser Asn Gln Ile Gly Gln Pro Thr Leu Pro Asn Asn Ser Leu Ala
   385 390 395 400
   Thr Pro Ser Pro Ser Leu Pro Ile Asn Pro Gly Thr Ser His Glu Lys
   405 410 415
   His Glu Glu Asp Gly Tyr Gly Phe Asp Ala Asn Arg Ile Ile Ala Glu
   420 425 430
   Asp Glu Ser Gly Phe Val Met Ser His Gly Asp His Asn His Tyr Phe
   435 440 445
   Phe Lys Lys Asp Leu Thr Glu Glu Gln Ile Lys Ala Ala Gln Lys His
   450 455 460
   Leu Glu Glu Val Lys Thr Ser His Asn Gly Leu Asp Ser Leu Ser Ser
   465 470 475 480
   His Glu Gln Asp Tyr Pro Ser Asn Ala Lys Glu Met Lys Asp Leu Asp
   485 490 495
   Lys Lys Ile Glu Glu Lys Ile Ala Gly Ile Met Lys Gln Tyr Gly Val
   500 505 510
   Lys Arg Glu Ser Ile Val Val Asn Lys Glu Lys Asn Ala Ile Ile Tyr
   515 520 525
   Pro His Gly Asp His His His Ala Asp Pro Ile Asp Glu His Lys Pro
   530 535 540
   Val Gly Ile Gly His Ser His Ser Asn Tyr Glu Leu Phe Lys Pro Glu
   545 550 555 560
   Glu Gly Val Ala Lys Lys Glu Gly Asn Lys Val Tyr Thr Gly Glu Glu
   565 570 575
   Leu Thr Asn Val Val Asn Leu Leu Lys Asn Ser Thr Phe Asn Asn Gln
   580 585 590
   Asn Phe Thr Leu Ala Asn Gly Gln Lys Arg Val Ser Phe Ser Phe Pro
   595 600 605
   Pro Glu Leu Glu Lys Lys Leu Gly Ile Asn Met Leu Val Lys Leu Ile
   610 615 620
   Thr Pro Asp Gly Lys Val Leu Glu Lys Val Ser Gly Lys Val Phe Gly
   625 630 635 640
   Glu Gly Val Gly Asn Ile Ala Asn Phe Glu Leu Asp Gln Pro Tyr Leu
   645 650 655
   Pro Gly Gln Thr Phe Lys Tyr Thr Ile Ala Ser Lys Asp Tyr Pro Glu
   660 665 670
   Val Ser Tyr Asp Gly Thr Phe Thr Val Pro Thr Ser Leu Ala Tyr Lys
   675 680 685
   Met Ala Ser Gln Thr Ile Phe Tyr Pro Phe His Ala Gly Asp Thr Tyr
   690 695 700
   Leu Arg Val Asn Pro Gln Phe Ala Val Pro Lys Gly Thr Asp Ala Leu
   705 710 715 720
   Val Arg Val Phe Asp Glu Phe His Gly Asn Ala Tyr Leu Glu Asn Asn
   725 730 735
   Tyr Lys Val Gly Glu Ile Lys Leu Pro Ile Pro Lys Leu Asn Gln Gly
   740 745 750
   Thr Thr Arg Thr Ala Gly Asn Lys Ile Pro Val Thr Phe Met Ala Asn
   755 760 765
   Ala Tyr Leu Asp Asn Gln Ser Thr Tyr Ile Val Glu Val Pro Ile Leu
   770 775 780
   Glu Lys Glu Asn Gln Thr Asp Lys Pro Ser Ile Leu Pro Gln Phe Lys
   785 790 795 800
   Arg Asn Lys Ala Gln Glu Asn Ser Lys Phe Asp Glu Lys Val Glu Glu
   805 810 815
   Pro Lys Thr Ser Glu Lys Val Glu Lys Glu Lys Leu Ser Glu Thr Gly
   820 825 830
   Asn Ser Thr Ser Asn Ser Thr Leu Glu Glu Val Pro Thr Val Asp Pro
   835 840 845
   Val Gln Glu Lys Val Ala Lys Phe Ala Glu Ser Tyr Gly Met Lys Leu
   850 855 860
   Glu Asn Val Leu Phe Asn Met Asp Gly Thr Ile Glu Leu Tyr Leu Pro
   865 870 875 880
   Ser Gly Glu Val Ile Lys Lys Asn Met Ala Asp Phe Thr Gly Glu Ala
   885 890 895
   Pro Gln Gly Asn Gly Glu Asn Lys Pro Ser Glu Asn Gly Lys Val Ser
   900 905 910
   Thr Gly Thr Val Glu Asn Gln Pro Thr Glu Asn Lys Pro Ala Asp Ser
   915 920 925
   Leu Pro Glu Ala Pro Asn Glu Lys Pro Val Lys Pro Glu Asn Ser Thr
   930 935 940
   Asp Asn Gly Met Leu Asn Pro Glu Gly Asn Val Gly Ser Asp Pro Met
   945 950 955 960
   Leu Asp Pro Ala Leu Glu Glu Ala Pro Ala Val Asp Pro Val Gln Glu
   965 970 975
   Lys Leu Glu Lys Phe Thr Ala Ser Tyr Gly Leu Gly Leu Asp Ser Val
   980 985 990
   Ile Phe Asn Met Asp Gly Thr Ile Glu Leu Arg Leu Pro Ser Gly Glu
   995 1000 1005
   Val Ile Lys Lys Asn Leu Ser Asp Leu Ile Ala
   1010 1015
<210> 90
   <211> 819
   <212> PRT
   <213> S. pneumoniae
<400> 90
   Cys Ser Tyr Glu Leu Gly Arg His Gln Ala Gly Gln Val Lys Lys Glu
    1               5                  10                  15
   Ser Asn Arg Val Ser Tyr Ile Asp Gly Asp Gln Ala Gly Gln Lys Ala
               20                  25                  30
   Glu Asn Leu Thr Pro Asp Glu Val Ser Lys Arg Glu Gly Ile Asn Ala
           35                  40                  45
   Glu Gln Ile Val Ile Lys Ile Thr Asp Gln Gly Tyr Val Thr Ser His
       50                  55                  60
   Gly Asp His Tyr His Tyr Tyr Asn Gly Lys Val Pro Tyr Asp Ala Ile
   65                  70                  75                  80
   Ile Ser Glu Glu Leu Leu Met Lys Asp Pro Asn Tyr Gln Leu Lys Asp
                   85                  90                  95
   Ser Asp Ile Val Asn Glu Ile Lys Gly Gly Tyr Val Ile Lys Val Asp
               100                 105                 110
   Gly Lys Tyr Tyr Val Tyr Leu Lys Asp Ala Ala His Ala Asp Asn Ile
           115                 120                 125
   Arg Thr Lys Glu Glu Ile Lys Arg Gln Lys Gln Glu His Ser His Asn
       130                 135                 140
   His Asn Ser Arg Ala Asp Asn Ala Val Ala Ala Ala Arg Ala Gln Gly
   145                 150                 155                 160
   Arg Tyr Thr Thr Asp Asp Gly Tyr Ile Phe Asn Ala Ser Asp Ile Ile
                   165                 170                 175
   Glu Asp Thr Gly Asp Ala Tyr Ile Val Pro His Gly Asp His Tyr His
               180                 185                 190
   Tyr Ile Pro Lys Asn Glu Leu Ser Ala Ser Glu Leu Ala Ala Ala Glu
           195                 200                 205
   Ala Tyr Trp Asn Gly Lys Gln Gly Ser Arg Pro Ser Ser Ser Ser Ser
       210                 215                 220
   Tyr Asn Ala Asn Pro Val Gln Pro Arg Leu Ser Glu Asn His Asn Leu
   225                 230                 235                 240
   Thr Val Thr Pro Thr Tyr His Gln Asn Gln Gly Glu Asn Ile Ser Ser
                   245                 250                 255
   Leu Leu Arg Glu Leu Tyr Ala Lys Pro Leu Ser Glu Arg His Val Glu
               260                 265                 270
   Ser Asp Gly Leu Ile Phe Asp Pro Ala Gln Ile Thr Ser Arg Thr Ala
           275                 280                 285
   Arg Gly Val Ala Val Pro His Gly Asn His Tyr His Phe Ile Pro Tyr
       290                 295                 300
   Glu Gln Met Ser Glu Leu Glu Lys Arg Ile Ala Arg Ile Ile Pro Leu
   305                 310                 315                 320
   Arg Tyr Arg Ser Asn His Trp Val Pro Asp Ser Arg Pro Glu Gln Pro
                   325                 330                 335
   Ser Pro Gln Ser Thr Pro Glu Pro Ser Pro Ser Leu Gln Pro Ala Pro
               340                 345                 350
   Asn Pro Gln Pro Ala Pro Ser Asn Pro Ile Asp Glu Lys Leu Val Lys
           355                 360                 365
   Glu Ala Val Arg Lys Val Gly Asp Gly Tyr Val Phe Glu Glu Asn Gly
       370                 375                 380
   Val Ser Arg Tyr Ile Pro Ala Lys Asp Leu Ser Ala Glu Thr Ala Ala
   385                 390                 395                 400
   Gly Ile Asp Ser Lys Leu Ala Lys Gln Glu Ser Leu Ser His Lys Leu
                   405                 410                 415
   Gly Ala Lys Lys Thr Asp Leu Pro Ser Ser Asp Arg Glu Phe Tyr Asn
               420                 425                 430
   Lys Ala Tyr Asp Leu Leu Ala Arg Ile His Gln Asp Leu Leu Asp Asn
           435                 440                 445
   Lys Gly Arg Gln Val Asp Phe Glu Val Leu Asp Asn Leu Leu Glu Arg
       450                 455                 460
   Leu Lys Asp Val Ser Ser Asp Lys Val Lys Leu Val Asp Asp Ile Leu
   465                 470                 475                 480
   Ala Phe Leu Ala Pro Ile Arg His Pro Glu Arg Leu Gly Lys Pro Asn
                   485                 490                 495
   Ala Gln Ile Thr Tyr Thr Asp Asp Glu Ile Gln Val Ala Lys Leu Ala
               500                 505                 510
   Gly Lys Tyr Thr Thr Glu Asp Gly Tyr Ile Phe Asp Pro Arg Asp Ile
           515                 520                 525
   Thr Ser Asp Glu Gly Asp Ala Tyr Val Thr Pro His Met Thr His Ser
       530                 535                 540
   His Trp Ile Lys Lys Asp Ser Leu Ser Glu Ala Glu Arg Ala Ala Ala
   545                 550                 555                 560
   Gln Ala Tyr Ala Lys Glu Lys Gly Leu Thr Pro Pro Ser Thr Asp His
                   565                 570                 575
   Gln Asp Ser Gly Asn Thr Glu Ala Lys Gly Ala Glu Ala Ile Tyr Asn
               580                 585                 590
   Arg Val Lys Ala Ala Lys Lys Val Pro Leu Asp Arg Met Pro Tyr Asn
           595                 600                 605
   Leu Gln Tyr Thr Val Glu Val Lys Asn Gly Ser Leu Ile Ile Pro His
       610                 615                 620
   Tyr Asp His Tyr His Asn Ile Lys Phe Glu Trp Phe Asp Glu Gly Leu
   625                 630                 635                 640
   Tyr Glu Ala Pro Lys Gly Tyr Ser Leu Glu Asp Leu Leu Ala Thr Val
                   645                 650                 655
   Lys Tyr Tyr Val Glu His Pro Asn Glu Arg Pro His Ser Asp Asn Gly
                   660                     665                     670
   Phe Gly Asn Ala Ser Asp His Val Arg Lys Asn Lys Ala Asp Gln Asp
               675                     680                     685
   Ser Lys Pro Asp Glu Asp Lys Glu His Asp Glu Val Ser Glu Pro Thr
           690                     695                     700
   His Pro Glu Ser Asp Glu Lys Glu Asn His Ala Gly Leu Asn Pro Ser
   705                     710                     715                     720
   Ala Asp Asn Leu Tyr Lys Pro Ser Thr Asp Thr Glu Glu Thr Glu Glu
                   725                     730                     735
   Glu Ala Glu Asp Thr Thr Asp Glu Ala Glu Ile Pro Gln Val Glu Asn
               740                     745                     750
   Ser Val Ile Asn Ala Lys Ile Ala Asp Ala Glu Ala Leu Leu Glu Lys
           755                     760                     765
   Val Thr Asp Pro Ser Ile Arg Gln Asn Ala Met Glu Thr Leu Thr Gly
       770                     775                     780
   Leu Lys Ser Ser Leu Leu Leu Gly Thr Lys Asp Asn Asn Thr Ile Ser
   785                     790                     795                     800
   Ala Glu Val Asp Ser Leu Leu Ala Leu Leu Lys Glu Ser Gln Pro Ala
                   805                     810                     815
   Pro Ile Gln
<210> 91
   <211> 820
   <212> PRT
   <213> S. pneumoniae
<400> 91
   Cys Ser Tyr Glu Leu Gly Arg His Gln Ala Gly Gln Val Lys Lys Glu
    1               5                  10                  15
   Ser Asn Arg Val Ser Tyr Ile Asp Gly Asp Gln Ala Gly Gln Lys Ala
               20                  25                  30
   Glu Asn Leu Thr Pro Asp Glu Val Ser Lys Arg Glu Gly Ile Asn Ala
           35                  40                  45
   Glu Gln Ile Val Ile Lys Ile Thr Asp Gln Gly Tyr Val Thr Ser His
       50                  55                  60
   Gly Asp His Tyr His Tyr Tyr Asn Gly Lys Val Pro Tyr Asp Ala Ile
   65                  70                  75                  80
   Ile Ser Glu Glu Leu Leu Met Lys Asp Pro Asn Tyr Gln Leu Lys Asp
                   85                  90                  95
   Ser Asp Ile Val Asn Glu Ile Lys Gly Gly Tyr Val Ile Lys Val Asp
               100                 105                 110
   Gly Lys Tyr Tyr Val Tyr Leu Lys Asp Ala Ala His Ala Asp Asn Ile
           115                 120                 125
   Arg Thr Lys Glu Glu Ile Lys Arg Gln Lys Gln Glu His Ser His Asn
       130                 135                 140
   His Gly Gly Gly Ser Asn Asp Gln Ala Val Val Ala Ala Arg Ala Gln
   145                 150                 155                 160
   Gly Arg Tyr Thr Thr Asp Asp Gly Tyr Ile Phe Asn Ala Ser Asp Ile
                   165                 170                 175
   Ile Glu Asp Thr Gly Asp Ala Tyr Ile Val Pro His Gly Asp His Tyr
               180                 185                 190
   His Tyr Ile Pro Lys Asn Glu Leu Ser Ala Ser Glu Leu Ala Ala Ala
           195                 200                 205
   Glu Ala Tyr Trp Asn Gly Lys Gln Gly Ser Arg Pro Ser Ser Ser Ser
       210                 215                 220
   Ser Tyr Asn Ala Asn Pro Ala Gln Pro Arg Leu Ser Glu Asn His Asn
   225                 230                 235                 240
   Leu Thr Val Thr Pro Thr Tyr His Gln Asn Gln Gly Glu Asn Ile Ser
                   245                 250                 255
   Ser Leu Leu Arg Glu Leu Tyr Ala Lys Pro Leu Ser Glu Arg His Val
               260                 265                 270
   Glu Ser Asp Gly Leu Ile Phe Asp Pro Ala Gln Ile Thr Ser Arg Thr
           275                 280                 285
   Ala Arg Gly Val Ala Val Pro His Gly Asn His Tyr His Phe Ile Pro
       290                 295                 300
   Tyr Glu Gln Met Ser Glu Leu Glu Lys Arg Ile Ala Arg Ile Ile Pro
   305                 310                 315                 320
   Leu Arg Tyr Arg Ser Asn His Trp Val Pro Asp Ser Arg Pro Glu Gln
                   325                 330                 335
   Pro Ser Pro Gln Ser Thr Pro Glu Pro Ser Pro Ser Pro Gln Pro Ala
               340                 345                 350
   Pro Asn Pro Gln Pro Ala Pro Ser Asn Pro Ile Asp Glu Lys Leu Val
           355                 360                 365
   Lys Glu Ala Val Arg Lys Val Gly Asp Gly Tyr Val Phe Glu Glu Asn
       370                 375                 380
   Gly Val Ser Arg Tyr Ile Pro Ala Lys Asp Leu Ser Ala Glu Thr Ala
   385                 390                 395                 400
   Ala Gly Ile Asp Ser Lys Leu Ala Lys Gln Glu Ser Leu Ser His Lys
                   405                 410                 415
   Leu Gly Ala Lys Lys Thr Asp Leu Pro Ser Ser Asp Arg Glu Phe Tyr
               420                 425                 430
   Asn Lys Ala Tyr Asp Leu Leu Ala Arg Ile His Gln Asp Leu Leu Asp
           435                 440                 445
   Asn Lys Gly Arg Gln Val Asp Phe Glu Ala Leu Asp Asn Leu Leu Glu
       450                 455                 460
   Arg Leu Lys Asp Val Pro Ser Asp Lys Val Lys Leu Val Asp Asp Ile
   465                 470                 475                 480
   Leu Ala Phe Leu Ala Pro Ile Arg His Pro Glu Arg Leu Gly Lys Pro
                   485                 490                 495
   Asn Ala Gln Ile Thr Tyr Thr Asp Asp Glu Ile Gln Val Ala Lys Leu
               500                 505                 510
   Ala Gly Lys Tyr Thr Thr Glu Asp Gly Tyr Ile Phe Asp Pro Arg Asp
           515                 520                 525
   Ile Thr Ser Asp Glu Gly Asp Ala Tyr Val Thr Pro His Met Thr His
       530                 535                 540
   Ser His Trp Ile Lys Lys Asp Ser Leu Ser Glu Ala Glu Arg Ala Ala
   545                 550                 555                 560
   Ala Gln Ala Tyr Ala Lys Glu Lys Gly Leu Thr Pro Pro Ser Thr Asp
                   565                 570                 575
   His Gln Asp Ser Gly Asn Thr Glu Ala Lys Gly Ala Glu Ala Ile Tyr
               580                 585                 590
   Asn Arg Val Lys Ala Ala Lys Lys Val Pro Leu Asp Arg Met Pro Tyr
           595                 600                 605
   Asn Leu Gln Tyr Thr Val Glu Val Lys Asn Gly Ser Leu Ile Ile Pro
       610                 615                 620
   His Tyr Asp His Tyr His Asn Ile Lys Phe Glu Trp Phe Asp Glu Gly
   625                 630                 635                 640
   Leu Tyr Glu Ala Pro Lys Gly Tyr Thr Leu Glu Asp Leu Leu Ala Thr
                   645                 650                 655
   Val Lys Tyr Tyr Val Glu His Pro Asn Glu Arg Pro His Ser Asp Asn
               660                 665                 670
   Gly Phe Gly Asn Ala Ser Asp His Val Arg Lys Asn Lys Val Asp Gln
           675                 680                 685
   Asp Ser Lys Pro Asp Glu Asp Lys Glu His Asp Glu Val Ser Glu Pro
       690                 695                 700
   Thr His Pro Glu Ser Asp Glu Lys Glu Asn His Ala Gly Leu Asn Pro
   705 710 715 720
   Ser Ala Asp Asn Leu Tyr Lys Pro Ser Thr Asp Thr Glu Glu Thr Glu
   725 730 735
   Glu Glu Ala Glu Asp Thr Thr Asp Glu Ala Glu Ile Pro Gln Val Glu
   740 745 750
   Asn Ser Val Ile Asn Ala Lys Ile Ala Asp Ala Glu Ala Leu Leu Glu
   755 760 765
   Lys Val Thr Asp Pro Ser Ile Arg Gln Asn Ala Met Glu Thr Leu Thr
   770 775 780
   Gly Leu Lys Ser Ser Leu Leu Leu Gly Thr Lys Asp Asn Asn Thr Ile
   785 790 795 800
   Ser Ala Glu Val Asp Ser Leu Leu Ala Leu Leu Lys Glu Ser Gln Pro
   805 810 815
   Ala Pro Ile Gln
   820
<210> 92
   <211> 816
   <212> PRT
   <213> S. pneumoniae
<400> 92
   Cys Ser Tyr Glu Leu Gly Arg His Gln Ala Gly Gln Asp Lys Lys Glu
    1               5                  10                  15
   Ser Asn Arg Val Ala Tyr Ile Asp Gly Asp Gln Ala Gly Gln Lys Ala
               20                  25                  30
   Glu Asn Leu Thr Pro Asp Glu Val Ser Lys Arg Glu Gly Ile Asn Ala
           35                  40                  45
   Glu Gln Ile Val Ile Lys Ile Thr Asp Gln Gly Tyr Val Thr Ser His
       50                  55                  60
   Gly Asp His Tyr His Tyr Tyr Asn Gly Lys Val Pro Tyr Asp Ala Ile
   65                  70                  75                  80
   Ile Ser Glu Glu Leu Leu Met Lys Asp Pro Asn Tyr Gln Leu Lys Asp
                   85                  90                  95
   Ser Asp Ile Val Asn Glu Ile Lys Gly Gly Tyr Val Ile Lys Val Asn
               100                 105                 110
   Gly Lys Tyr Tyr Val Tyr Leu Lys Asp Ala Ala His Ala Asp Asn Ile
           115                 120                 125
   Arg Thr Lys Glu Glu Ile Lys Arg Gln Lys Gln Glu His Ser His Asn
       130                 135                 140
   His Gly Gly Gly Ser Asn Asp Gln Ala Val Val Ala Ala Arg Ala Gln
   145                 150                 155                 160
   Gly Arg Tyr Thr Thr Asp Asp Gly Tyr Ile Phe Asn Ala Ser Asp Ile
                   165                 170                 175
   Ile Glu Asp Thr Gly Asp Ala Tyr Ile Val Pro His Gly Asn His Phe
               180                 185                 190
   His Tyr Ile Pro Lys Ser Asp Leu Ser Ala Ser Glu Leu Ala Ala Ala
           195                 200                 205
   Gln Ala Tyr Trp Asn Gly Lys Gln Gly Ser Arg Pro Ser Ser Ser Ser
       210                 215                 220
   Ser His Asn Ala Asn Pro Ala Gln Pro Arg Leu Ser Glu Asn His Asn
   225                 230                 235                 240
   Leu Thr Val Thr Pro Thr Tyr His Gln Asn Gln Gly Glu Asn Ile Ser
                   245                 250                 255
   Ser Leu Leu Arg Glu Leu Tyr Ala Lys Pro Leu Ser Glu Arg His Val
               260                 265                 270
   Glu Ser Asp Gly Leu Ile Phe Asp Pro Ala Gln Ile Thr Ser Arg Thr
   275 280 285
   Ala Arg Gly Val Ala Val Pro His Gly Asn His Tyr His Phe Ile Pro
   290 295 300
   Tyr Glu Gln Met Ser Glu Leu Glu Glu Arg Ile Ala Arg Ile Ile Pro
   305 310 315 320
   Leu Arg Tyr Arg Ser Asn His Trp Val Pro Asp Ser Arg Pro Glu Gln
   325 330 335
   Pro Ser Pro Gln Pro Ser Pro Ser Pro Gln Pro Ala Pro Asn Pro Gln
   340 345 350
   Pro Ala Pro Ser Asn Pro Ile Asp Glu Lys Leu Val Lys Glu Ala Val
   355 360 365
   Arg Lys Val Gly Asp Gly Tyr Val Phe Glu Glu Asn Gly Val Ser Arg
   370 375 380
   Tyr Ile Pro Ala Lys Asp Leu Ser Ala Glu Thr Ala Ala Gly Ile Asp
   385 390 395 400
   Ser Lys Leu Ala Lys Gln Glu Ser Leu Ser His Lys Leu Gly Thr Lys
   405 410 415
   Lys Thr Asp Leu Pro Ser Ser Asp Arg Glu Phe Tyr Asn Lys Ala Tyr
   420 425 430
   Asp Leu Leu Ala Arg Ile His Gln Asp Leu Leu Asp Asn Lys Gly Arg
   435 440 445
   Gln Val Asp Phe Glu Ala Leu Asp Asn Leu Leu Glu Arg Leu Lys Asp
   450 455 460
   Val Ser Ser Asp Lys Val Lys Leu Val Glu Asp Ile Leu Ala Phe Leu
   465 470 475 480
   Ala Pro Ile Arg His Pro Glu Arg Leu Gly Lys Pro Asn Ser Gln Ile
   485 490 495
   Thr Tyr Thr Asp Asp Glu Ile Gln Val Ala Lys Leu Ala Gly Lys Tyr
   500 505 510
   Thr Thr Glu Asp Gly Tyr Ile Phe Asp Pro Arg Asp Ile Thr Ser Asp
   515 520 525
   Glu Gly Asp Ala Tyr Val Thr Pro His Met Thr His Ser His Trp Ile
   530 535 540
   Lys Lys Asp Ser Leu Ser Glu Ala Glu Arg Ala Ala Ala Gln Ala Tyr
   545 550 555 560
   Ala Lys Glu Lys Gly Leu Thr Pro Pro Ser Thr Asp His Arg Asp Ser
   565 570 575
   Gly Asn Thr Glu Ala Lys Gly Ala Glu Ala Ile Tyr Asn Arg Val Lys
   580 585 590
   Ala Ala Lys Lys Val Pro Leu Asp Arg Met Pro Tyr Asn Leu Gln Tyr
   595 600 605
   Thr Val Glu Val Lys Asn Gly Ser Leu Ile Ile Pro His Tyr Asp His
   610 615 620
   Tyr His Asn Ile Lys Phe Glu Trp Phe Asp Glu Gly Leu Tyr Glu Ala
   625 630 635 640
   Pro Lys Gly Tyr Thr Leu Glu Asp Leu Leu Ala Thr Val Lys Tyr Tyr
   645 650 655
   Val Glu His Pro Asn Glu Arg Pro His Ser Asp Asn Gly Phe Gly Asn
   660 665 670
   Ala Ser Asp His Val Arg Lys Asn Lys Ala Asp Gln Asp Ser Lys Pro
   675 680 685
   Asp Glu Asp Lys Gly His Asp Glu Val Ser Glu Pro Thr His Pro Glu
   690 695 700
   Ser Asp Glu Lys Glu Asn His Ala Gly Leu Asn Pro Ser Ala Asp Asn
   705 710 715 720
   Leu Tyr Lys Pro Ser Thr Asp Thr Glu Glu Thr Glu Glu Glu Ala Glu
   725 730 735
   Asp Thr Thr Asp Glu Ala Glu Ile Pro Gln Val Glu His Ser Val Ile
                           740                 745                 750
   Asn Ala Lys Ile Ala Asp Ala Glu Ala Leu Leu Glu Lys Val Thr Asp
               755                 760                 765
   Pro Ser Ile Arg Gln Asn Ala Met Glu Thr Leu Thr Gly Leu Lys Ser
           770                 775                 780
   Ser Leu Leu Leu Gly Thr Lys Asp Asn Asn Thr Ile Ser Ala Glu Val
   785                 790                 795                 800
   Asp Ser Leu Leu Ala Leu Leu Lys Lys Ser Gln Pro Ala Pro Ile Gln
                       805                 810                 815
<210> 93
   <211> 816
   <212> PRT
   <213> S. pneumoniae
<400> 93
   Cys Ser Tyr Glu Leu Gly Arg His Gln Ala Gly Gln Asp Lys Lys Glu
    1               5                  10                  15
   Ser Asn Arg Val Ala Tyr Ile Asp Gly Asp Gln Ala Gly Gln Lys Ala
               20                  25                  30
   Glu Asn Leu Thr Pro Asp Glu Val Ser Lys Arg Glu Gly Ile Asn Ala
           35                  40                  45
   Glu Gln Ile Val Ile Lys Ile Thr Asp Gln Gly Tyr Val Thr Ser His
       50                  55                  60
   Gly Asp His Tyr His Tyr Tyr Asn Gly Lys Val Pro Tyr Asp Ala Ile
   65                  70                  75                  80
   Ile Ser Glu Glu Leu Leu Met Lys Asp Pro Asn Tyr Gln Leu Lys Asp
                   85                  90                  95
   Ser Asp Ile Val Asn Glu Ile Lys Gly Gly Tyr Val Ile Lys Val Asn
               100                 105                 110
   Gly Lys Tyr Tyr Val Tyr Leu Lys Asp Ala Ala His Ala Asp Asn Ile
           115                 120                 125
   Arg Thr Lys Glu Glu Ile Lys Arg Gln Arg Gln Glu His Ser His Asn
       130                 135                 140
   His Gly Gly Gly Ser Asn Asp Gln Ala Val Val Ala Ala Arg Ala Gln
   145                 150                 155                 160
   Gly Arg Tyr Thr Thr Asp Asp Gly Tyr Ile Phe Asn Ala Ser Asp Ile
                   165                 170                 175
   Ile Glu Asp Thr Gly Asp Ala Tyr Ile Val Pro His Gly Asn His Phe
               180                 185                 190
   His Tyr Ile Pro Lys Ser Asp Leu Ser Ala Ser Glu Leu Ala Ala Ala
           195                 200                 205
   Gln Ala Tyr Trp Asn Gly Lys Gln Gly Ser Arg Pro Ser Ser Ser Ser
       210                 215                 220
   Ser His Asn Ala Asn Pro Ala Gln Pro Arg Leu Ser Glu Asn His Asn
   225                 230                 235                 240
   Leu Thr Val Thr Pro Thr Tyr His Gln Asn Gln Gly Glu Asn Ile Ser
                   245                 250                 255
   Ser Leu Leu Arg Glu Leu Tyr Ala Lys Pro Leu Ser Glu Arg His Val
               260                 265                 270
   Glu Ser Asp Gly Leu Ile Phe Asp Pro Ala Gln Ile Thr Ser Arg Thr
           275                 280                 285
   Ala Arg Gly Val Ala Val Pro His Gly Asn His Tyr His Phe Ile Pro
       290                 295                 300
   Tyr Glu Gln Met Ser Glu Leu Glu Glu Arg Ile Ala Arg Ile Ile Pro
   305                 310                 315                 320
   Leu Arg Tyr Arg Ser Asn His Trp Val Pro Asp Ser Arg Pro Glu Gln
                   325                 330                 335
   Pro Ser Pro Gln Pro Ser Pro Ser Pro Gln Pro Ala Pro Asn Pro Gln
   340 345 350
   Pro Ala Pro Ser Asn Pro Ile Asp Glu Lys Leu Val Lys Glu Ala Val
   355 360 365
   Arg Lys Val Gly Asp Gly Tyr Val Phe Glu Glu Asn Gly Val Ser Arg
   370 375 380
   Tyr Ile Pro Ala Lys Asp Leu Ser Ala Glu Thr Ala Ala Gly Ile Asp
   385 390 395 400
   Ser Lys Leu Ala Lys Gln Glu Ser Leu Ser His Lys Leu Gly Thr Lys
   405 410 415
   Lys Thr Asp Leu Pro Ser Ser Asp Arg Glu Phe Tyr Asn Lys Ala Tyr
   420 425 430
   Asp Leu Leu Ala Arg Ile His Gln Asp Leu Leu Asp Asn Lys Gly Arg
   435 440 445
   Gln Val Asp Phe Glu Ala Leu Asp Asn Leu Leu Glu Arg Leu Lys Asp
   450 455 460
   Val Ser Ser Asp Lys Val Lys Leu Val Glu Asp Ile Leu Ala Phe Leu
   465 470 475 480
   Ala Pro Ile Arg His Pro Glu Arg Leu Gly Lys Pro Asn Ser Gln Ile
   485 490 495
   Thr Tyr Thr Asp Asp Glu Ile Gln Val Ala Lys Leu Ala Gly Lys Tyr
   500 505 510
   Thr Thr Glu Asp Gly Tyr Ile Phe Asp Pro Arg Asp Ile Thr Ser Asp
   515 520 525
   Glu Gly Asp Ala Tyr Val Thr Pro His Met Thr His Ser His Trp Ile
   530 535 540
   Lys Lys Asp Ser Leu Ser Glu Ala Glu Arg Ala Ala Ala Gln Ala Tyr
   545 550 555 560
   Ala Lys Glu Lys Gly Leu Thr Pro Pro Ser Thr Asp His Gln Asp Ser
   565 570 575
   Gly Asn Thr Glu Ala Lys Gly Ala Glu Ala Ile Tyr Asn Arg Val Lys
   580 585 590
   Ala Ala Lys Lys Val Pro Leu Asp Arg Met Pro Tyr Asn Leu Gln Tyr
   595 600 605
   Thr Val Glu Val Lys Asn Gly Ser Leu Ile Ile Pro His Tyr Asp His
   610 615 620
   Tyr His Asn Ile Lys Phe Glu Trp Phe Asp Glu Gly Leu Tyr Glu Ala
   625 630 635 640
   Pro Lys Gly Tyr Thr Leu Glu Asp Leu Leu Ala Thr Val Lys Tyr Tyr
   645 650 655
   Val Glu His Pro Asn Glu Arg Pro His Ser Asp Asn Gly Phe Gly Asn
   660 665 670
   Ala Ser Asp His Val Arg Lys Asn Lys Ala Asp Gln Asp Ser Lys Pro
   675 680 685
   Asp Glu Asp Lys Gly His Asp Glu Val Ser Glu Pro Thr His Pro Glu
   690 695 700
   Ser Asp Glu Lys Glu Asn His Ala Gly Leu Asn Pro Ser Ala Asp Asn
   705 710 715 720
   Leu Tyr Lys Pro Ser Thr Asp Thr Glu Glu Thr Glu Glu Glu Ala Glu
   725 730 735
   Asp Thr Thr Asp Glu Ala Glu Ile Pro Gln Val Glu His Ser Val Ile
   740 745 750
   Asn Ala Lys Ile Ala Asp Ala Glu Ala Leu Leu Glu Lys Val Thr Asp
   755 760 765
   Pro Ser Ile Arg Gln Asn Ala Met Glu Thr Leu Thr Gly Leu Lys Ser
   770 775 780
   Ser Leu Leu Leu Gly Thr Lys Asp Asn Asn Thr Ile Ser Ala Glu Val
   785 790 795 800
   Asp Ser Leu Leu Ala Leu Leu Lys Lys Ser Gln Pro Ala Pro Ile Gln
   805 810 815
<210> 94
   <211> 816
   <212> PRT
   <213> S. pneumoniae
<400> 94
   Cys Ser Tyr Glu Leu Gly Arg His Gln Ala Gly Gln Asp Lys Lys Glu
   1               5                   10                  15
   Ser Asn Arg Val Ala Tyr Ile Asp Gly Asp Gln Ala Gly Gln Lys Ala
               20                  25                  30
   Glu Asn Leu Thr Pro Asp Glu Val Ser Lys Arg Glu Gly Ile Asn Ala
           35                  40                  45
   Glu Gln Ile Val Ile Lys Ile Thr Asp Gln Gly Tyr Val Thr Ser His
       50                  55                  60
   Gly Asp His Tyr His Tyr Tyr Asn Gly Lys Val Pro Tyr Asp Ala Ile
   65                  70                  75                  80
   Ile Ser Glu Glu Leu Leu Met Lys Asp Pro Asn Tyr Gln Leu Lys Asp
                   85                  90                  95
   Ser Asp Ile Val Asn Glu Ile Lys Gly Gly Tyr Val Ile Lys Val Asn
               100                 105                 110
   Gly Lys Tyr Tyr Val Tyr Leu Lys Asp Ala Ala His Ala Asp Asn Ile
           115                 120                 125
   Arg Thr Lys Glu Glu Ile Lys Arg Gln Lys Gln Glu His Ser His Asn
       130                 135                 140
   His Gly Gly Gly Ser Asn Asp Gln Ala Val Val Ala Ala Arg Ala Gln
   145                 150                 155                 160
   Gly Arg Tyr Thr Thr Asp Asp Gly Tyr Ile Phe Asn Ala Ser Asp Ile
                   165                 170                 175
   Ile Glu Asp Thr Gly Asp Ala Tyr Ile Val Pro Arg Gly Asn His Phe
               180                 185                 190
   His Tyr Ile Pro Lys Ser Asp Leu Ser Ala Ser Glu Leu Ala Ala Ala
           195                 200                 205
   Gln Ala Tyr Trp Asn Gly Lys Gln Gly Ser Arg Pro Ser Ser Ser Ser
       210                 215                 220
   Ser His Asn Ala Asn Pro Ala Gln Pro Arg Leu Ser Glu Asn His Asn
   225                 230                 235                 240
   Leu Thr Val Thr Pro Thr Tyr His Gln Asn Gln Gly Glu Asn Ile Ser
                   245                 250                 255
   Ser Leu Leu Arg Glu Leu Tyr Ala Lys Pro Leu Ser Glu Arg Arg Val
               260                 265                 270
   Glu Ser Asp Gly Leu Ile Phe Asp Pro Ala Gln Ile Thr Ser Arg Thr
           275                 280                 285
   Ala Arg Gly Val Ala Val Pro His Gly Asn His Tyr His Phe Ile Pro
       290                 295                 300
   Tyr Glu Gln Met Ser Glu Leu Glu Glu Arg Ile Ala Arg Ile Ile Pro
   305                 310                 315                 320
   Leu Arg Tyr Arg Ser Asn His Trp Val Pro Asp Ser Arg Pro Glu Gln
                   325                 330                 335
   Pro Ser Pro Gln Pro Ser Pro Ser Pro Gln Pro Ala Pro Asn Pro Gln
               340                 345                 350
   Pro Ala Pro Ser Asn Pro Ile Asp Glu Lys Leu Val Lys Glu Ala Val
           355                 360                 365
   Arg Lys Val Gly Asp Gly Tyr Val Phe Glu Glu Asn Gly Val Ser Arg
       370                 375                 380
   Tyr Ile Pro Ala Lys Asp Leu Ser Ala Glu Thr Ala Ala Gly Ile Asp
   385 390 395 400
   Ser Lys Leu Ala Lys Gln Glu Ser Leu Ser His Lys Leu Gly Thr Lys
   405 410 415
   Lys Thr Asp Leu Pro Ser Ser Asp Arg Glu Phe Tyr Asn Lys Ala Tyr
   420 425 430
   Asp Leu Leu Ala Arg Ile His Gln Asp Leu Leu Asp Asn Lys Gly Arg
   435 440 445
   Gln Val Asp Phe Glu Ala Leu Asp Asn Leu Leu Glu Arg Leu Lys Asp
   450 455 460
   Val Ser Ser Asp Lys Val Lys Leu Val Glu Asp Ile Leu Ala Phe Leu
   465 470 475 480
   Ala Pro Ile Arg His Pro Glu Arg Leu Gly Lys Pro Asn Ser Gln Ile
   485 490 495
   Thr Tyr Thr Asp Asp Glu Ile Gln Val Ala Lys Leu Ala Gly Lys Tyr
   500 505 510
   Thr Thr Glu Asp Gly Tyr Ile Phe Asp Pro Arg Asp Ile Thr Ser Asp
   515 520 525
   Glu Gly Asp Ala Tyr Val Thr Pro His Met Thr His Ser His Trp Ile
   530 535 540
   Lys Lys Asp Ser Leu Ser Glu Ala Glu Arg Ala Ala Ala Gln Ala Tyr
   545 550 555 560
   Ala Lys Glu Lys Gly Leu Thr Pro Pro Ser Thr Asp His Gln Asp Ser
   565 570 575
   Gly Asn Thr Glu Ala Lys Gly Ala Glu Ala Ile Tyr Asn Arg Val Lys
   580 585 590
   Ala Ala Lys Lys Val Pro Leu Asp Arg Met Pro Tyr Asn Leu Gln Tyr
   595 600 605
   Thr Val Glu Val Lys Asn Gly Ser Leu Ile Ile Pro His Tyr Asp His
   610 615 620
   Tyr His Asn Ile Lys Phe Glu Trp Phe Asp Glu Gly Leu Tyr Glu Ala
   625 630 635 640
   Pro Lys Gly Tyr Thr Leu Glu Asp Leu Leu Ala Thr Val Lys Tyr Tyr
   645 650 655
   Val Glu His Pro Asn Glu Arg Pro His Ser Asp Asn Gly Phe Gly Asn
   660 665 670
   Ala Ser Asp His Val Arg Lys Asn Lys Ala Asp Gln Asp Ser Lys Pro
   675 680 685
   Asp Glu Asp Lys Gly His Asp Glu Val Ser Glu Pro Thr His Pro Glu
   690 695 700
   Ser Asp Glu Lys Glu Asn His Ala Gly Leu Asn Pro Ser Ala Asp Asn
   705 710 715 720
   Leu Tyr Lys Pro Ser Thr Asp Thr Glu Glu Thr Glu Glu Glu Ala Glu
   725 730 735
   Asp Thr Thr Asp Glu Ala Glu Ile Pro Gln Val Glu His Ser Val Ile
   740 745 750
   Asn Ala Lys Ile Ala Asp Ala Glu Ala Leu Leu Glu Lys Val Thr Asp
   755 760 765
   Pro Ser Ile Arg Gln Asn Ala Met Glu Thr Leu Thr Gly Leu Lys Ser
   770 775 780
   Ser Leu Leu Leu Gly Thr Lys Asp Asn Asn Thr Ile Ser Ala Glu Val
   785 790 795 800
   Asp Ser Leu Leu Ala Leu Leu Lys Lys Ser Gln Pro Ala Pro Ile Gln
   805 810 815
<210> 95
   <211> 834
   <212> PRT
   <213> S. pneumoniae
<400> 95
   Cys Ser Tyr Glu Leu Gly Arg His Gln Ala Gly Gln Val Lys Lys Glu
    1               5                  10                  15
   Ser Asn Arg Val Ser Tyr Ile Asp Gly Asp Gln Ala Gly Gln Lys Ala
               20                  25                  30
   Glu Asn Leu Thr Pro Asp Glu Val Ser Lys Arg Glu Gly Ile Asn Ala
           35                  40                  45
   Glu Gln Ile Val Ile Lys Ile Thr Asp Gln Gly Tyr Val Thr Ser His
       50                  55                  60
   Gly Asp His Tyr His Tyr Tyr Asn Gly Lys Val Pro Tyr Asp Ala Ile
   65                  70                  75                  80
   Ile Ser Glu Glu Leu Leu Met Lys Asp Pro Asn Tyr Gln Leu Lys Asp
                   85                  90                  95
   Ser Asp Ile Val Asn Glu Ile Lys Gly Gly Tyr Val Ile Lys Val Asp
               100                 105                 110
   Gly Lys Tyr Tyr Val Tyr Leu Lys Asp Ala Ala His Ala Asp Asn Ile
           115                 120                 125
   Arg Thr Lys Glu Glu Ile Lys Arg Gln Lys Gln Glu Arg Ser His Asn
       130                 135                 140
   His Asn Ser Arg Ala Asp Asn Ala Val Ala Ala Ala Arg Ala Gln Gly
   145                 150                 155                 160
   Arg Tyr Thr Thr Asp Asp Gly Tyr Ile Phe Asn Ala Ser Asp Ile Ile
                   165                 170                 175
   Glu Asp Thr Gly Asp Ala Tyr Ile Val Pro His Gly Asp His Tyr His
               180                 185                 190
   Tyr Ile Pro Lys Ser Asp Leu Ser Ala Ser Glu Leu Ala Ala Ala Gln
           195                 200                 205
   Ala Tyr Trp Asn Gly Lys Gln Gly Ser Arg Pro Ser Ser Ser Ser Ser
       210                 215                 220
   His Asn Ala Asn Pro Ala Gln Pro Arg Leu Ser Glu Asn His Asn Leu
   225                 230                 235                 240
   Thr Val Thr Pro Thr Tyr His Gln Asn Gln Gly Glu Asn Ile Ser Ser
                   245                 250                 255
   Leu Leu Arg Glu Leu Tyr Ala Lys Pro Leu Ser Glu Arg His Val Glu
               260                 265                 270
   Ser Asp Gly Leu Ile Phe Asp Pro Ala Gln Ile Thr Ser Arg Thr Ala
           275                 280                 285
   Asn Gly Val Ala Val Pro His Gly Asp His Tyr His Phe Ile Pro Tyr
       290                 295                 300
   Ser Gln Leu Ser Pro Leu Glu Glu Lys Leu Ala Arg Ile Ile Pro Leu
   305                 310                 315                 320
   Arg Tyr Arg Ser Asn His Trp Val Pro Asp Ser Arg Pro Glu Gln Pro
                   325                 330                 335
   Ser Pro Gln Ser Thr Pro Glu Pro Ser Pro Ser Pro Gln Pro Ala Pro
               340                 345                 350
   Asn Pro Gln Pro Ala Pro Ser Asn Pro Ile Asp Glu Lys Leu Val Lys
           355                 360                 365
   Glu Ala Val Arg Lys Val Gly Asp Gly Tyr Val Phe Glu Glu Asn Gly
       370                 375                 380
   Val Pro Arg Tyr Ile Pro Ala Lys Asp Leu Ser Ala Glu Thr Ala Ala
   385                 390                 395                 400
   Gly Ile Asp Ser Lys Leu Ala Lys Gln Glu Ser Leu Ser His Lys Leu
                   405                 410                 415
   Gly Ala Lys Lys Thr Asp Leu Pro Ser Ser Asp Arg Glu Phe Tyr Asn
               420                 425                 430
   Lys Ala Tyr Asp Leu Leu Ala Arg Ile His Gln Asp Leu Leu Asp Asn
           435                 440                 445
   Lys Gly Arg Gln Val Asp Phe Glu Ala Leu Asp Asn Leu Leu Glu Arg
   450 455 460
   Leu Lys Asp Val Ser Ser Asp Lys Val Lys Leu Val Asp Asp Ile Leu
   465 470 475 480
   Ala Phe Leu Ala Pro Ile Arg His Pro Glu Arg Leu Gly Lys Pro Asn
   485 490 495
   Ala Gln Ile Thr Tyr Thr Asp Asp Glu Ile Gln Val Ala Lys Leu Ala
   500 505 510
   Gly Lys Tyr Thr Thr Glu Asp Gly Tyr Ile Phe Asp Pro Arg Asp Ile
   515 520 525
   Thr Ser Asp Glu Gly Asp Ala Tyr Val Thr Pro His Met Thr His Ser
   530 535 540
   His Trp Ile Lys Lys Asp Ser Leu Ser Glu Ala Glu Arg Ala Ala Ala
   545 550 555 560
   Gln Ala Tyr Ala Lys Glu Lys Gly Leu Thr Pro Pro Ser Thr Asp His
   565 570 575
   Gln Asp Ser Gly Asn Thr Glu Ala Lys Gly Ala Glu Ala Ile Tyr Asn
   580 585 590
   Arg Val Lys Ala Ala Lys Lys Val Pro Leu Asp Arg Met Pro Tyr Asn
   595 600 605
   Leu Gln Tyr Thr Val Glu Val Lys Asn Gly Ser Leu Ile Ile Pro His
   610 615 620
   Tyr Asp His Tyr His Asn Ile Lys Phe Glu Trp Phe Asp Glu Gly Leu
   625 630 635 640
   Tyr Glu Ala Pro Lys Gly Tyr Ser Leu Glu Asp Leu Leu Ala Thr Val
   645 650 655
   Lys Tyr Tyr Val Glu His Pro Asn Glu Arg Pro His Ser Asp Asn Gly
   660 665 670
   Phe Gly Asn Ala Ser Asp His Val Gln Arg Asn Lys Asn Gly Gln Ala
   675 680 685
   Asp Thr Asn Gln Thr Glu Lys Pro Asn Glu Glu Lys Pro Gln Thr Glu
   690 695 700
   Lys Pro Glu Glu Asp Lys Glu His Asp Glu Val Ser Glu Pro Thr His
   705 710 715 720
   Pro Glu Ser Asp Glu Lys Glu Asn His Val Gly Leu Asn Pro Ser Ala
   725 730 735
   Asp Asn Leu Tyr Lys Pro Ser Thr Asp Thr Glu Glu Thr Glu Glu Glu
   740 745 750
   Ala Glu Asp Thr Thr Asp Glu Ala Glu Ile Pro Gln Val Glu Tyr Ser
   755 760 765
   Val Ile Asn Ala Lys Ile Ala Glu Ala Glu Ala Leu Leu Glu Lys Val
   770 775 780
   Thr Asp Ser Ser Ile Arg Gln Asn Ala Val Glu Thr Leu Thr Gly Leu
   785 790 795 800
   Lys Ser Ser Leu Leu Leu Gly Thr Lys Asp Asn Asn Thr Ile Ser Ala
   805 810 815
   Glu Val Asp Ser Leu Leu Ala Leu Leu Lys Glu Ser Gln Pro Ala Pro
   820 825 830
   Ile Gln
<210> 96
   <211> 811
   <212> PRT
   <213> S. pneumoniae
<400> 96
   Cys Ser Tyr Glu Leu Gly Arg His Gln Ala Gly Gln Asp Lys Lys Glu
   1               5                   10                  15
   Ser Asn Arg Val Ala Tyr Ile Asp Gly Asp Gln Ala Gly Gln Lys Ala
               20                  25                  30
   Glu Asn Leu Thr Pro Asp Glu Val Ser Lys Arg Glu Gly Ile Asn Ala
           35                  40                  45
   Glu Gln Ile Val Ile Lys Ile Thr Asp Gln Gly Tyr Val Thr Ser His
       50                  55                  60
   Gly Asp His Tyr His Tyr Tyr Asn Gly Lys Val Pro Tyr Asp Ala Ile
   65                  70                  75                  80
   Ile Ser Glu Glu Leu Leu Met Lys Asp Pro Asn Tyr Gln Leu Lys Asp
                   85                  90                  95
   Ser Asp Ile Val Asn Glu Ile Lys Gly Gly Tyr Val Ile Lys Val Asn
               100                 105                 110
   Gly Lys Tyr Tyr Val Tyr Leu Lys Asp Ala Ala His Ala Asp Asn Ile
           115                 120                 125
   Arg Thr Lys Glu Glu Ile Lys Arg Gln Lys Gln Glu His Ser His Asn
       130                 135                 140
   His Gly Gly Gly Ser Asn Asp Gln Ala Val Val Ala Ala Arg Ala Gln
   145                 150                 155                 160
   Gly Arg Tyr Thr Thr Asp Asp Gly Tyr Ile Phe Asn Ala Ser Asp Ile
                   165                 170                 175
   Ile Glu Asp Thr Gly Asp Ala Tyr Ile Val Pro His Gly Asn His Phe
               180                 185                 190
   His Tyr Ile Pro Lys Ser Asp Leu Ser Ala Ser Glu Leu Ala Ala Ala
           195                 200                 205
   Gln Ala Tyr Trp Asn Gly Lys Gln Gly Ser Arg Pro Ser Ser Ser Ser
       210                 215                 220
   Ser His Asn Ala Asn Pro Ala Gln Pro Arg Leu Ser Glu Asn His Asn
   225                 230                 235                 240
   Leu Thr Val Thr Pro Thr Tyr His Gln Asn Gln Gly Glu Asn Ile Ser
                   245                 250                 255
   Ser Leu Leu Arg Glu Leu Tyr Ala Lys Pro Leu Ser Glu Arg His Val
               260                 265                 270
   Glu Ser Asp Gly Leu Ile Phe Asp Pro Ala Gln Ile Thr Ser Arg Thr
           275                 280                 285
   Ala Arg Gly Val Ala Val Pro His Gly Asn His Tyr His Phe Ile Pro
       290                 295                 300
   Tyr Glu Gln Met Ser Glu Leu Glu Glu Arg Ile Ala Arg Ile Ile Pro
   305                 310                 315                 320
   Leu Arg Tyr Arg Ser Asn His Trp Val Pro Asp Ser Arg Pro Glu Gln
                   325                 330                 335
   Pro Ser Pro Gln Pro Ser Pro Ser Pro Gln Pro Ala Pro Asn Pro Gln
               340                 345                 350
   Pro Ala Pro Ser Asn Pro Ile Asp Glu Lys Leu Val Lys Glu Ala Val
           355                 360                 365
   Arg Lys Val Gly Asp Gly Tyr Val Phe Glu Glu Asn Gly Val Ser Arg
       370                 375                 380
   Tyr Ile Pro Ala Lys Asp Leu Ser Ala Glu Thr Ala Ala Gly Ile Asp
   385                 390                 395                 400
   Ser Lys Leu Ala Lys Gln Glu Ser Leu Ser His Lys Leu Gly Thr Lys
                   405                 410                 415
   Lys Thr Asp Leu Pro Ser Ser Asp Arg Glu Phe Tyr Asn Lys Ala Tyr
               420                 425                 430
   Asp Leu Leu Ala Arg Ile His Gln Asp Leu Leu Asp Asn Lys Gly Arg
           435                 440                 445
   Gln Val Asp Phe Glu Ala Leu Asp Asn Leu Leu Glu Arg Leu Lys Asp
       450                 455                 460
   Val Ser Ser Asp Lys Val Lys Leu Val Glu Asp Ile Leu Ala Phe Leu
   465 470 475 480
   Ala Pro Ile Arg His Pro Glu Arg Leu Gly Lys Pro Asn Ser Gln Ile
   485 490 495
   Thr Tyr Thr Asp Asp Glu Ile Gln Val Ala Lys Leu Ala Gly Lys Tyr
   500 505 510
   Thr Thr Glu Asp Gly Tyr Ile Phe Asp Pro Arg Asp Ile Thr Ser Asp
   515 520 525
   Glu Gly Asp Ala Tyr Val Thr Pro His Met Thr His Ser His Trp Ile
   530 535 540
   Lys Lys Asp Ser Leu Ser Glu Ala Glu Arg Ala Ala Ala Gln Ala Tyr
   545 550 555 560
   Ala Lys Glu Lys Gly Leu Thr Pro Pro Ser Thr Asp His Gln Asp Ser
   565 570 575
   Gly Asn Thr Glu Ala Lys Gly Ala Glu Ala Ile Tyr Asn Arg Val Lys
   580 585 590
   Ala Ala Lys Lys Val Pro Leu Asp Arg Met Pro Tyr Asn Leu Gln Tyr
   595 600 605
   Thr Val Glu Val Lys Asn Gly Ser Leu Ile Ile Pro His Tyr Asp His
   610 615 620
   Tyr His Asn Ile Lys Phe Glu Trp Phe Asp Glu Gly Leu Tyr Glu Ala
   625 630 635 640
   Pro Lys Gly Tyr Thr Leu Glu Asp Leu Leu Ala Thr Val Lys Tyr Tyr
   645 650 655
   Val Glu His Pro Asn Glu Arg Pro His Ser Asp Asn Gly Phe Gly Asn
   660 665 670
   Ala Ser Asp His Val Arg Lys Asn Lys Ala Asp Gln Asp Ser Lys Pro
   675 680 685
   Asp Glu Asp Lys Gly His Asp Glu Val Ser Glu Pro Thr His Pro Glu
   690 695 700
   Ser Asp Glu Lys Glu Asn His Ala Gly Leu Asn Pro Ser Ala Asp Asn
   705 710 715 720
   Leu Tyr Lys Pro Ser Thr Asp Thr Glu Glu Thr Glu Glu Glu Ala Glu
   725 730 735
   Asp Thr Thr Asp Glu Ala Glu Ile Pro Gln Val Glu His Ser Val Ile
   740 745 750
   Asn Ala Lys Ile Ala Asp Ala Glu Ala Leu Leu Glu Lys Val Thr Asp
   755 760 765
   Pro Ser Ile Arg Gln Asn Ala Met Glu Thr Leu Thr Gly Leu Lys Ser
   770 775 780
   Ser Leu Leu Leu Gly Thr Lys Asp Asn Asn Thr Ile Ser Ala Glu Val
   785 790 795 800
   Asp Ser Leu Leu Ala Leu Leu Lys Glu Ser Lys
   805 810
<210> 97
   <211> 811
   <212> PRT
   <213> S. pneumoniae
<400> 97
   Cys Ser Tyr Glu Leu Gly Arg His Gln Ala Gly Gln Asp Lys Lys Glu
    1               5                  10                  15
   Ser Asn Arg Val Ala Tyr Ile Asp Gly Asp Gln Ala Gly Gln Lys Ala
               20                  25                  30
   Glu Asn Leu Thr Pro Asp Glu Val Ser Lys Arg Glu Gly Ile Asn Ala
           35                  40                  45
   Glu Gln Ile Val Ile Lys Ile Thr Asp Gln Gly Tyr Val Thr Ser His
       50                  55                  60
   Gly Asp His Tyr His Tyr Tyr Asn Gly Lys Val Pro Tyr Asp Ala Ile
   65                  70                  75                  80
   Ile Ser Glu Glu Leu Leu Met Lys Asp Pro Asn Tyr Gln Leu Lys Asp
                   85                  90                  95
   Ser Asp Ile Val Asn Glu Ile Lys Gly Gly Tyr Val Ile Lys Val Asn
               100                 105                 110
   Gly Lys Tyr Tyr Gly Tyr Leu Lys Asp Ala Ala His Ala Asp Asn Ile
           115                 120                 125
   Arg Thr Lys Glu Glu Ile Lys Arg Gln Lys Gln Glu His Ser His Asn
       130                 135                 140
   His Gly Gly Gly Ser Asn Asp Gln Ala Val Val Ala Ala Arg Ala Gln
   145                 150                 155                 160
   Gly Arg Tyr Thr Thr Asp Asp Gly Tyr Ile Phe Asn Ala Ser Asp Ile
                   165                 170                 175
   Ile Glu Asp Thr Gly Asp Ala Tyr Ile Val Pro His Gly Asn His Phe
               180                 185                 190
   His Tyr Ile Pro Lys Ser Asp Leu Ser Ala Ser Glu Leu Ala Ala Ala
           195                 200                 205
   Gln Ala Tyr Trp Asn Gly Lys Gln Gly Ser Arg Pro Ser Ser Ser Ser
       210                 215                 220
   Ser His Asn Ala Asn Pro Ala Gln Pro Arg Leu Ser Glu Asn His Asn
   225                 230                 235                 240
   Leu Thr Val Thr Pro Thr Tyr His Gln Asn Gln Gly Glu Asn Ile Ser
                   245                 250                 255
   Ser Leu Leu Arg Glu Leu Tyr Ala Lys Pro Leu Ser Glu Arg His Val
               260                 265                 270
   Glu Ser Asp Gly Leu Ile Phe Asp Pro Ala Gln Ile Thr Ser Arg Thr
           275                 280                 285
   Ala Arg Gly Val Ala Val Pro His Gly Asn His Tyr His Phe Ile Pro
       290                 295                 300
   Tyr Glu Gln Met Ser Glu Leu Glu Glu Arg Ile Ala Arg Ile Ile Pro
   305                 310                 315                 320
   Leu Arg Tyr Arg Ser Asn His Trp Val Pro Asp Ser Arg Pro Glu Gln
                   325                 330                 335
   Pro Ser Pro Gln Pro Ser Pro Ser Pro Gln Pro Ala Pro Asn Pro Gln
               340                 345                 350
   Pro Ala Pro Ser Asn Pro Ile Asp Glu Lys Leu Val Lys Glu Ala Val
           355                 360                 365
   Arg Lys Val Gly Asp Gly Tyr Val Phe Glu Glu Asn Gly Val Ser Arg
       370                 375                 380
   Tyr Ile Pro Ala Lys Asp Leu Ser Ala Glu Thr Ala Ala Gly Ile Asp
   385                 390                 395                 400
   Ser Lys Leu Ala Lys Gln Glu Ser Leu Ser His Lys Leu Gly Thr Lys
                   405                 410                 415
   Lys Thr Asp Leu Pro Ser Ser Asp Arg Glu Phe Tyr Asn Lys Ala Tyr
               420                 425                 430
   Asp Leu Leu Ala Arg Ile His Gln Asp Leu Leu Asp Asn Lys Gly Arg
           435                 440                 445
   Gln Val Asp Phe Glu Ala Leu Asp Asn Leu Leu Glu Arg Leu Lys Asp
       450                 455                 460
   Val Ser Ser Asp Lys Val Lys Leu Val Glu Asp Ile Leu Ala Phe Leu
   465                 470                 475                 480
   Ala Pro Ile Arg His Pro Glu Arg Leu Gly Lys Pro Asn Ser Gln Ile
                   485                 490                 495
   Thr Tyr Thr Asp Asp Glu Ile Gln Val Ala Lys Leu Ala Gly Lys Tyr
               500                 505                 510
   Thr Thr Glu Asp Gly Tyr Ile Phe Asp Pro Arg Asp Ile Thr Ser Asp
           515                 520                 525
   Glu Gly Asp Ala Tyr Val Thr Pro His Met Thr His Ser His Trp Ile
       530                 535                 540
   Lys Lys Asp Ser Leu Ser Glu Ala Glu Arg Ala Ala Ala Gln Ala Tyr
   545                 550                 555                 560
   Ala Lys Glu Lys Gly Leu Thr Pro Pro Ser Thr Asp His Gln Asp Ser
                   565                 570                 575
   Gly Asn Thr Glu Ala Lys Gly Ala Glu Ala Ile Tyr Asn Arg Val Lys
               580                 585                 590
   Ala Ala Lys Lys Val Pro Leu Asp Arg Met Pro Tyr Asn Leu Gln Tyr
           595                 600                 605
   Thr Val Glu Val Lys Asn Gly Ser Leu Ile Ile Pro His Tyr Asp His
       610                 615                 620
   Tyr His Asn Ile Lys Phe Glu Trp Phe Asp Glu Gly Leu Tyr Glu Ala
   625                 630                 635                 640
   Pro Lys Gly Tyr Thr Leu Glu Asp Leu Leu Ala Thr Val Lys Tyr Tyr
                   645                 650                 655
   Val Glu His Pro Asn Glu Arg Pro His Ser Asp Asn Gly Phe Gly Asn
               660                 665                 670
   Ala Ser Asp His Val Arg Lys Asn Lys Ala Asp Gln Asp Ser Lys Pro
           675                 680                 685
   Asp Glu Asp Lys Gly His Asp Glu Val Ser Glu Pro Thr His Pro Glu
       690                 695                 700
   Ser Asp Glu Lys Glu Asn His Ala Gly Leu Asn Pro Ser Ala Asp Asn
   705                 710                 715                 720
   Leu Tyr Lys Pro Ser Thr Asp Thr Glu Glu Thr Glu Glu Glu Ala Glu
                   725                 730                 735
   Asp Thr Thr Asp Glu Ala Glu Ile Pro Gln Val Glu His Ser Val Ile
               740                 745                 750
   Asn Ala Lys Ile Ala Asp Ala Glu Ala Leu Leu Glu Lys Val Thr Asp
           755                 760                 765
   Pro Ser Ile Arg Gln Asn Ala Met Glu Thr Leu Thr Gly Leu Lys Ser
       770                 775                 780
   Ser Leu Leu Leu Gly Thr Lys Asp Asn Asn Thr Ile Ser Ala Glu Val
   785                 790                 795                 800
   Asp Ser Leu Leu Ala Leu Leu Lys Glu Ser Lys
                   805                 810
<210> 98
   <211> 811
   <212> PRT
   <213> S. pneumoniae
<400> 98
   Cys Ser Tyr Glu Leu Gly Arg His Gln Ala Gly Gln Asp Lys Lys Glu
    1               5                  10                  15
   Ser Asn Arg Val Ala Tyr Ile Asp Gly Asp Gln Ala Gly Gln Lys Ala
               20                  25                  30
   Glu Asn Leu Thr Pro Asp Glu Val Ser Lys Arg Glu Gly Ile Asn Ala
           35                  40                  45
   Glu Gln Ile Val Ile Lys Ile Thr Asp Gln Gly Tyr Val Thr Ser His
       50                  55                  60
   Gly Asp His Tyr His Tyr Tyr Asn Gly Lys Val Pro Tyr Asp Ala Ile
   65                  70                  75                  80
   Ile Ser Glu Glu Leu Leu Met Lys Asp Pro Asn Tyr Gln Leu Lys Asp
                   85                  90                  95
   Ser Asp Ile Val Asn Glu Ile Lys Gly Gly Tyr Val Ile Lys Val Asn
               100                 105                 110
   Gly Lys Tyr Tyr Val Tyr Leu Lys Asp Ala Ala His Ala Asp Asn Ile
               115                 120                 125
   Arg Thr Lys Glu Glu Ile Lys Arg Gln Lys Gln Glu His Ser His Asn
           130                 135                 140
   His Gly Gly Gly Ser Asn Asp Gln Ala Val Val Ala Ala Arg Ala Gln
   145                 150                 155                 160
   Gly Arg Tyr Thr Thr Asp Asp Gly Tyr Ile Phe Asn Ala Ser Asp Ile
                   165                 170                 175
   Ile Glu Asp Thr Gly Asp Ala Tyr Ile Val Pro His Gly Asn His Phe
               180                 185                 190
   His Tyr Ile Pro Lys Ser Asp Leu Ser Ala Ser Glu Leu Ala Ala Ala
           195                 200                 205
   Gln Ala Tyr Trp Asn Gly Lys Gln Gly Ser Arg Pro Ser Ser Ser Ser
       210                 215                 220
   Ser His Asn Ala Asn Pro Ala Gln Pro Arg Leu Ser Glu Asn His Asn
   225                 230                 235                 240
   Leu Thr Val Thr Pro Thr Tyr His Gln Asn Gln Gly Glu Asn Ile Ser
                   245                 250                 255
   Ser Leu Leu Arg Glu Leu Tyr Ala Lys Pro Leu Ser Glu Arg His Val
               260                 265                 270
   Glu Ser Asp Gly Leu Ile Phe Asp Pro Ala Gln Ile Thr Ser Arg Thr
           275                 280                 285
   Ala Arg Gly Val Ala Val Pro His Gly Asn His Tyr His Phe Ile Pro
       290                 295                 300
   Tyr Glu Gln Met Ser Glu Leu Glu Glu Arg Ile Ala Arg Ile Ile Pro
   305                 310                 315                 320
   Leu Arg Tyr Arg Ser Asn His Trp Val Pro Asp Ser Arg Pro Glu Gln
                   325                 330                 335
   Pro Ser Pro Gln Pro Ser Pro Ser Pro Gln Pro Ala Pro Asn Pro Gln
               340                 345                 350
   Pro Ala Pro Ser Asn Pro Ile Asp Glu Lys Leu Val Lys Glu Ala Val
           355                 360                 365
   Arg Lys Val Gly Asp Gly Tyr Val Phe Glu Glu Asn Gly Val Ser Arg
       370                 375                 380
   Tyr Ile Pro Ala Lys Asp Leu Ser Ala Glu Thr Ala Ala Gly Ile Asp
   385                 390                 395                 400
   Ser Lys Leu Ala Lys Gln Glu Ser Leu Ser His Lys Leu Gly Thr Lys
                   405                 410                 415
   Lys Thr Asp Leu Pro Ser Ser Asp Arg Glu Phe Tyr Asn Lys Ala Tyr
               420                 425                 430
   Asp Leu Leu Ala Arg Ile His Gln Asp Leu Leu Asp Asn Lys Gly Arg
           435                 440                 445
   Gln Val Asp Phe Glu Ala Leu Asp Asn Leu Leu Glu Arg Leu Lys Asp
       450                 455                 460
   Val Ser Ser Asp Lys Val Lys Leu Val Glu Asp Ile Leu Ala Phe Leu
   465                 470                 475                 480
   Ala Pro Ile Arg His Pro Glu Arg Leu Gly Lys Pro Asn Ser Gln Ile
                   485                 490                 495
   Thr Tyr Thr Asp Asp Glu Ile Gln Val Ala Lys Leu Ala Gly Lys Tyr
               500                 505                 510
   Thr Thr Glu Asp Gly Tyr Ile Phe Asp Pro Arg Asp Ile Thr Ser Asp
           515                 520                 525
   Glu Gly Asp Ala Tyr Val Thr Pro His Met Thr His Ser His Trp Ile
       530                 535                 540
   Lys Lys Asp Ser Leu Ser Glu Ala Glu Arg Ala Ala Ala Gln Ala Tyr
   545                 550                 555                 560
   Ala Lys Glu Lys Gly Leu Thr Pro Pro Ser Thr Asp His Gln Asp Ser
                   565                 570                 575
   Gly Asn Thr Glu Ala Lys Gly Ala Glu Ala Ile Tyr Asn Arg Val Lys
                   580                 585                 590
   Ala Ala Lys Lys Val Pro Leu Asp Arg Met Pro Tyr Asn Leu Gln Tyr
               595                 600                 605
   Thr Val Glu Val Lys Asn Gly Ser Leu Ile Ile Pro His Tyr Asp His
       610                 615                 620
   Tyr His Asn Ile Lys Phe Glu Trp Phe Asp Glu Gly Leu Tyr Glu Ala
   625                 630                 635                 640
   Pro Lys Gly Tyr Thr Leu Glu Asp Leu Leu Ala Thr Val Lys Tyr Tyr
                   645                 650                 655
   Val Glu His Pro Asn Glu Arg Pro His Ser Asp Asn Gly Phe Gly Asn
               660                 665                 670
   Ala Ser Asp His Val Arg Lys Asn Lys Ala Asp Gln Asp Ser Lys Pro
           675                 680                 685
   Asp Glu Asp Lys Gly His Asp Glu Val Ser Glu Pro Thr His Pro Glu
       690                 695                 700
   Ser Asp Glu Lys Glu Asn His Ala Gly Leu Asn Pro Ser Ala Asp Asn
   705                 710                 715                 720
   Leu Tyr Lys Pro Ser Thr Asp Thr Glu Glu Thr Glu Glu Glu Ala Glu
                   725                 730                 735
   Asp Thr Thr Asp Glu Ala Glu Ile Pro Gln Val Glu His Ser Val Ile
               740                 745                 750
   Asn Ala Lys Ile Ala Asp Ala Glu Ala Leu Leu Glu Lys Val Thr Asp
           755                 760                 765
   Pro Ser Ile Arg Gln Asn Ala Met Glu Thr Leu Thr Gly Leu Lys Ser
       770                 775                 780
   Ser Leu Leu Leu Gly Thr Lys Asp Asn Asn Thr Ile Ser Ala Glu Val
   785                 790                 795                 800
   Asp Ser Leu Leu Ala Leu Leu Lys Glu Ser Lys
                   805                 810
<210> 99
   <211> 811
   <212> PRT
   <213> S. pneumoniae
<400> 99
   Cys Ser Tyr Glu Leu Gly Arg His Gln Ala Gly Gln Val Lys Lys Glu
    1               5                  10                  15
   Ser Asn Arg Val Ser Tyr Ile Asp Gly Asp Gln Ala Gly Gln Lys Ala
               20                  25                  30
   Glu Asn Leu Thr Pro Asp Glu Val Ser Lys Arg Glu Gly Ile Asn Ala
           35                  40                  45
   Glu Gln Ile Val Ile Lys Ile Thr Asp Gln Gly Tyr Val Thr Ser His
       50                  55                  60
   Gly Asp His Tyr His Tyr Tyr Asn Gly Lys Val Pro Tyr Asp Ala Ile
   65                  70                  75                  80
   Ile Ser Glu Glu Leu Leu Met Lys Asp Pro Asn Tyr Gln Leu Lys Asp
                   85                  90                  95
   Ser Asp Ile Val Asn Glu Ile Lys Gly Gly Tyr Val Ile Lys Val Asp
               100                 105                 110
   Gly Lys Tyr Tyr Val Tyr Leu Lys Asp Ala Ala His Ala Asp Asn Ile
           115                 120                 125
   Arg Thr Lys Glu Glu Ile Lys Arg Gln Lys Gln Glu Arg Ser His Asn
       130                 135                 140
   His Asn Ser Arg Ala Asp Asn Ala Val Ala Ala Ala Arg Ala Gln Gly
   145                 150                 155                 160
   Arg Tyr Thr Thr Asp Asp Gly Tyr Ile Phe Asn Ala Ser Asp Ile Ile
                   165                 170                 175
   Glu Asp Thr Gly Asp Ala Tyr Ile Val Pro His Gly Asp His Tyr His
   180 185 190
   Tyr Ile Pro Lys Ser Asp Leu Ser Ala Ser Glu Leu Ala Ala Ala Gln
   195 200 205
   Ala Tyr Trp Asn Gly Lys Gln Gly Ser Arg Pro Ser Ser Ser Ser Ser
   210 215 220
   His Asn Ala Asn Pro Ala Gln Pro Arg Leu Ser Glu Asn His Asn Leu
   225 230 235 240
   Thr Val Thr Pro Thr Tyr His Gln Asn Gln Gly Glu Asn Ile Ser Ser
   245 250 255
   Leu Leu Arg Glu Leu Tyr Ala Lys Pro Leu Ser Glu Arg His Val Glu
   260 265 270
   Ser Asp Gly Leu Ile Phe Asp Pro Ala Gln Ile Thr Ser Arg Thr Ala
   275 280 285
   Asn Gly Val Ala Val Pro His Gly Asp His Tyr His Phe Ile Pro Tyr
   290 295 300
   Ser Gln Leu Ser Pro Leu Glu Glu Lys Leu Ala Arg Ile Ile Pro Leu
   305 310 315 320
   Arg Tyr Arg Ser Asn His Trp Val Pro Asp Ser Arg Pro Glu Gln Pro
   325 330 335
   Ser Pro Gln Ser Thr Pro Glu Pro Ser Pro Ser Pro Gln Pro Ala Pro
   340 345 350
   Asn Pro Gln Pro Ala Pro Ser Asn Pro Ile Asp Glu Lys Leu Val Lys
   355 360 365
   Glu Ala Val Arg Lys Val Gly Asp Gly Tyr Val Phe Glu Glu Asn Gly
   370 375 380
   Val Pro Arg Tyr Ile Pro Ala Lys Asp Leu Ser Ala Glu Thr Ala Ala
   385 390 395 400
   Gly Ile Asp Ser Lys Leu Ala Lys Gln Glu Ser Leu Ser His Lys Leu
   405 410 415
   Gly Ala Lys Lys Thr Asp Leu Pro Ser Ser Asp Arg Glu Phe Tyr Asn
   420 425 430
   Lys Ala Tyr Asp Leu Leu Ala Arg Ile His Gln Asp Leu Leu Asp Asn
   435 440 445
   Lys Gly Arg Gln Val Asp Phe Glu Ala Leu Asp Asn Leu Leu Glu Arg
   450 455 460
   Leu Lys Asp Val Ser Ser Asp Lys Val Lys Leu Val Asp Asp Ile Leu
   465 470 475 480
   Ala Phe Leu Ala Pro Ile Arg His Pro Glu Arg Leu Gly Lys Pro Asn
   485 490 495
   Ala Gln Ile Thr Tyr Thr Asp Asp Glu Ile Gln Val Ala Lys Leu Ala
   500 505 510
   Gly Lys Tyr Thr Thr Glu Asp Gly Tyr Ile Phe Asp Pro Arg Asp Ile
   515 520 525
   Thr Ser Asp Glu Gly Asp Ala Tyr Val Thr Pro His Met Thr His Ser
   530 535 540
   His Trp Ile Lys Lys Asp Ser Leu Ser Glu Ala Glu Arg Ala Ala Ala
   545 550 555 560
   Gln Ala Tyr Ala Lys Glu Lys Gly Leu Thr Pro Pro Ser Thr Asp His
   565 570 575
   Gln Asp Ser Gly Asn Thr Glu Ala Lys Gly Ala Glu Ala Ile Tyr Asn
   580 585 590
   Arg Val Lys Ala Ala Lys Lys Val Pro Leu Asp Arg Met Pro Tyr Asn
   595 600 605
   Leu Gln Tyr Thr Val Glu Val Lys Asn Gly Ser Leu Ile Ile Pro His
   610 615 620
   Tyr Asp His Tyr His Asn Ile Lys Phe Glu Trp Phe Asp Glu Gly Leu
   625 630 635 640
   Tyr Glu Ala Pro Lys Gly Tyr Ser Leu Glu Asp Leu Leu Ala Thr Val
                   645                 650                 655
   Lys Tyr Tyr Val Glu His Pro Asn Glu Arg Pro His Ser Asp Asn Gly
               660                 665                 670
   Phe Gly Asn Ala Ser Asp His Val Gln Arg Asn Lys Asn Gly Gln Ala
           675                 680                 685
   Asp Thr Asn Gln Thr Glu Lys Pro Asn Glu Glu Lys Pro Gln Thr Glu
       690                 695                 700
   Lys Pro Glu Glu Glu Thr Pro Arg Glu Glu Lys Pro Gln Ser Glu Lys
   705                 710                 715                 720
   Pro Glu Ser Pro Lys Pro Thr Glu Glu Pro Glu Glu Glu Ser Pro Glu
                   725                 730                 735
   Glu Ser Pro Glu Glu Ser Glu Glu Pro Gln Val Glu Thr Glu Lys Val
               740                 745                 750
   Lys Glu Lys Leu Arg Glu Ala Glu Asp Leu Leu Gly Lys Ile Gln Asn
           755                 760                 765
   Pro Ile Ile Lys Ser Asn Ala Lys Glu Thr Leu Thr Gly Leu Lys Asn
       770                 775                 780
   Asn Leu Leu Phe Gly Thr Gln Asp Asn Asn Thr Ile Met Ala Glu Ala
   785                 790                 795                 800
   Glu Lys Leu Leu Ala Leu Leu Lys Glu Ser Lys
                   805                 810
<210> 100
   <211> 805
   <212> PRT
   <213> S. pneumoniae
<400> 100
   Cys Ser Tyr Glu Leu Gly Arg His Gln Ala Gly Gln Asp Lys Lys Glu
    1               5                  10                  15
   Ser Asn Arg Val Ala Tyr Ile Asp Gly Asp Gln Ala Gly Gln Lys Ala
               20                  25                  30
   Glu Asn Leu Thr Pro Asp Glu Val Ser Lys Arg Glu Gly Ile Asn Ala
           35                  40                  45
   Glu Gln Ile Val Ile Lys Ile Thr Asp Gln Gly Tyr Val Thr Ser His
       50                  55                  60
   Gly Asp His Tyr His Tyr Tyr Asn Gly Lys Val Pro Tyr Asp Ala Ile
   65                  70                  75                  80
   Ile Ser Glu Glu Leu Leu Met Lys Asp Pro Asn Tyr Gln Leu Lys Asp
                   85                  90                  95
   Ser Asp Ile Val Asn Glu Ile Lys Gly Gly Tyr Val Ile Lys Val Asn
               100                 105                 110
   Gly Lys Tyr Tyr Val Tyr Leu Lys Asp Ala Ala His Ala Asp Asn Ile
           115                 120                 125
   Arg Thr Lys Glu Glu Ile Lys Arg Gln Lys Gln Glu Arg Ser His Asn
       130                 135                 140
   His Asn Ser Arg Ala Asp Asn Ala Val Ala Ala Ala Arg Ala Gln Gly
   145                 150                 155                 160
   Arg Tyr Thr Thr Asp Asp Gly Tyr Ile Phe Asn Ala Ser Asp Ile Ile
                   165                 170                 175
   Glu Asp Thr Gly Asp Ala Tyr Ile Val Pro His Gly Asp His Tyr His
               180                 185                 190
   Tyr Ile Pro Lys Asn Glu Leu Ser Ala Ser Glu Leu Ala Ala Ala Glu
           195                 200                 205
   Ala Tyr Trp Asn Gly Lys Gln Gly Ser Arg Pro Ser Ser Ser Ser Ser
       210                 215                 220
   Tyr Asn Ala Asn Pro Ala Gln Pro Arg Leu Ser Glu Asn His Asn Leu
   225 230 235 240
   Thr Val Thr Pro Thr Tyr His Gln Asn Gln Gly Glu Asn Ile Ser Ser
   245 250 255
   Leu Leu Arg Glu Leu Tyr Ala Lys Pro Leu Ser Glu Arg His Val Glu
   260 265 270
   Ser Asp Gly Leu Ile Phe Asp Pro Ala Gln Ile Thr Ser Arg Thr Ala
   275 280 285
   Arg Gly Val Ala Val Pro His Gly Asn His Tyr His Phe Ile Pro Tyr
   290 295 300
   Glu Gln Met Ser Glu Leu Glu Lys Arg Ile Ala Arg Ile Ile Pro Leu
   305 310 315 320
   Arg Tyr Arg Ser Asn His Trp Val Pro Asp Ser Arg Pro Glu Glu Pro
   325 330 335
   Ser Pro Gln Pro Thr Pro Glu Pro Ser Pro Ser Pro Gln Pro Ala Pro
   340 345 350
   Ser Asn Pro Ile Asp Glu Lys Leu Val Lys Glu Ala Val Arg Lys Val
   355 360 365
   Gly Asp Gly Tyr Val Phe Glu Glu Asn Gly Val Ser Arg Tyr Ile Pro
   370 375 380
   Ala Lys Asp Leu Ser Ala Glu Thr Ala Ala Gly Ile Asp Ser Lys Leu
   385 390 395 400
   Ala Lys Gln Glu Ser Leu Ser His Lys Leu Gly Ala Lys Lys Thr Asp
   405 410 415
   Leu Pro Ser Ser Asp Arg Glu Phe Tyr Asn Lys Ala Tyr Asp Leu Leu
   420 425 430
   Ala Arg Ile His Gln Asp Leu Leu Asp Asn Lys Gly Arg Gln Val Asp
   435 440 445
   Phe Glu Ala Leu Asp Asn Leu Leu Glu Arg Leu Lys Asp Val Ser Ser
   450 455 460
   Asp Lys Val Lys Leu Val Asp Asp Ile Leu Ala Phe Leu Ala Pro Ile
   465 470 475 480
   Arg His Pro Glu Arg Leu Gly Lys Pro Asn Ala Gln Ile Thr Tyr Thr
   485 490 495
   Asp Asp Glu Ile Gln Val Ala Lys Leu Ala Gly Lys Tyr Thr Thr Glu
   500 505 510
   Asp Gly Tyr Ile Phe Asp Pro Arg Asp Ile Thr Ser Asp Glu Gly Asp
   515 520 525
   Ala Tyr Val Thr Pro His Met Thr His Ser His Trp Ile Lys Lys Asp
   530 535 540
   Ser Leu Ser Glu Ala Glu Arg Ala Ala Ala Gln Ala Tyr Ala Lys Glu
   545 550 555 560
   Lys Gly Leu Thr Pro Pro Ser Thr Asp His Gln Asp Ser Gly Asn Thr
   565 570 575
   Glu Ala Lys Gly Ala Glu Ala Ile Tyr Asn Arg Val Lys Ala Ala Lys
   580 585 590
   Lys Val Pro Leu Asp Arg Met Pro Tyr Asn Leu Gln Tyr Thr Val Glu
   595 600 605
   Val Lys Asn Gly Ser Leu Ile Ile Pro His Tyr Asp His Tyr His Asn
   610 615 620
   Ile Lys Phe Glu Trp Phe Asp Glu Gly Leu Tyr Glu Ala Pro Lys Gly
   625 630 635 640
   Tyr Ser Leu Glu Asp Leu Leu Ala Thr Val Lys Tyr Tyr Val Glu His
   645 650 655
   Pro Asn Glu Arg Pro His Ser Asp Asn Gly Phe Gly Asn Ala Ser Asp
   660 665 670
   His Val Gln Arg Asn Lys Asn Gly Gln Ala Asp Thr Asn Gln Thr Glu
   675 680 685
   Lys Pro Asn Glu Glu Lys Pro Gln Thr Glu Lys Pro Glu Glu Glu Thr
       690                 695                 700
   Pro Arg Glu Glu Lys Pro Gln Ser Glu Lys Pro Glu Ser Pro Lys Pro
   705                 710                 715                 720
   Thr Glu Glu Pro Glu Glu Glu Ser Pro Glu Glu Ser Pro Glu Glu Ser
                   725                 730                 735
   Glu Glu Pro Gln Val Glu Thr Glu Lys Val Lys Glu Lys Leu Arg Glu
               740                 745                 750
   Ala Glu Asp Leu Leu Gly Lys Ile Gln Asn Pro Ile Ile Lys Ser Asn
           755                 760                 765
   Ala Lys Glu Thr Leu Thr Gly Leu Lys Asn Asn Leu Leu Phe Gly Thr
       770                 775                 780
   Gln Asp Asn Asn Thr Ile Met Ala Glu Ala Glu Lys Leu Leu Ala Leu
   785                 790                 795                 800
   Leu Lys Glu Ser Lys
                   805
<210> 101
   <211> 807
   <212> PRT
   <213> S. pneumoniae
<400> 101
   Cys Ser Tyr Glu Leu Gly Arg His Gln Ala Gly Gln Val Lys Lys Glu
    1               5                  10                  15
   Ser Asn Arg Val Ser Tyr Ile Asp Gly Asp Gln Ala Gly Gln Lys Ala
               20                  25                  30
   Glu Asn Leu Thr Pro Asp Glu Val Ser Lys Arg Glu Gly Ile Asn Ala
           35                  40                  45
   Glu Gln Ile Val Ile Lys Ile Thr Asp Gln Gly Tyr Val Thr Ser His
       50                  55                  60
   Gly Asp His Tyr His Tyr Tyr Asn Gly Lys Val Pro Tyr Asp Ala Ile
   65                  70                  75                  80
   Ile Ser Glu Glu Leu Leu Met Lys Asp Pro Asn Tyr Gln Leu Lys Asp
                   85                  90                  95
   Ser Asp Ile Val Asn Glu Ile Lys Gly Gly Tyr Val Ile Lys Val Asp
               100                 105                 110
   Gly Lys Tyr Tyr Val Tyr Leu Lys Asp Ala Ala His Ala Asp Asn Ile
           115                 120                 125
   Arg Thr Lys Glu Glu Ile Lys Arg Gln Lys Gln Glu Arg Ser His Asn
       130                 135                 140
   His Asn Ser Arg Ala Asp Asn Ala Val Ala Ala Ala Arg Ala Gln Gly
   145                 150                 155                 160
   Arg Tyr Thr Thr Asp Asp Gly Tyr Ile Phe Asn Ala Ser Asp Ile Ile
                   165                 170                 175
   Glu Asp Thr Gly Asp Ala Tyr Ile Val Pro His Gly Asn His Phe His
               180                 185                 190
   Tyr Ile Pro Lys Ser Asp Leu Ser Ala Ser Glu Leu Ala Ala Ala Gln
           195                 200                 205
   Ala Tyr Trp Asn Gly Lys Gln Gly Ser Arg Pro Ser Ser Ser Ser Ser
       210                 215                 220
   His Asn Ala Asn Pro Ala Gln Pro Arg Leu Ser Glu Asn His Asn Leu
   225                 230                 235                 240
   Thr Val Thr Pro Thr Tyr His Gln Asn Gln Gly Glu Asn Ile Ser Ser
                   245                 250                 255
   Leu Leu Arg Glu Leu Tyr Ala Lys Pro Leu Ser Glu Arg His Val Glu
               260                 265                 270
   Ser Asp Gly Leu Ile Phe Asp Pro Ala Gln Ile Thr Ser Arg Thr Ala
           275                 280                 285
   Arg Gly Val Ala Val Pro His Gly Asn His Tyr His Phe Ile Pro Tyr
       290                 295                 300
   Ser Gln Met Ser Glu Leu Glu Glu Arg Ile Ala Arg Ile Ile Pro Leu
   305                 310                 315                 320
   Arg Tyr Arg Ser Asn His Trp Val Pro Asp Ser Arg Pro Glu Gln Pro
                   325                 330                 335
   Ser Pro Gln Ser Thr Pro Glu Pro Ser Pro Ser Pro Gln Ser Ala Pro
               340                 345                 350
   Asn Pro Gln Pro Ala Pro Ser Asn Pro Ile Asp Glu Lys Leu Val Lys
           355                 360                 365
   Glu Val Val Arg Lys Val Gly Asp Gly Tyr Val Phe Glu Lys Asn Gly
       370                 375                 380
   Val Ser Arg Tyr Ile Pro Ala Lys Asn Leu Ser Ala Glu Thr Ala Ala
   385                 390                 395                 400
   Gly Ile Asp Ser Lys Leu Ala Lys Gln Glu Ser Leu Ser His Lys Leu
                   405                 410                 415
   Gly Ala Lys Lys Thr Asp Leu Pro Ser Ser Asp Arg Glu Phe Tyr Asn
               420                 425                 430
   Lys Ala Tyr Asp Leu Leu Ala Arg Ile His Gln Asp Leu Leu Asp Asn
           435                 440                 445
   Lys Gly Arg Gln Val Asp Phe Glu Ala Leu Asp Asn Leu Leu Glu Arg
       450                 455                 460
   Leu Glu Asp Val Pro Ser Asp Lys Val Lys Leu Val Asp Asp Ile Leu
   465                 470                 475                 480
   Ala Phe Leu Ala Pro Ile Arg His Pro Glu Arg Leu Gly Lys Pro Asn
                   485                 490                 495
   Ala Gln Ile Thr Tyr Thr Asp Asp Glu Ile Gln Val Ala Lys Leu Ala
               500                 505                 510
   Gly Lys Tyr Thr Thr Glu Asp Gly Tyr Ile Phe Asp Pro Arg Asp Ile
           515                 520                 525
   Thr Ser Asp Glu Gly Asp Ala Tyr Val Thr Pro His Met Thr His Ser
       530                 535                 540
   His Trp Ile Lys Lys Asp Ser Leu Ser Glu Ala Glu Arg Ala Ala Ala
   545                 550                 555                 560
   Gln Ala Tyr Ala Lys Glu Lys Gly Leu Thr Pro Pro Ser Thr Asp His
                   565                 570                 575
   Gln Asp Ser Gly Asn Thr Glu Ala Lys Gly Ala Glu Ala Ile Tyr Asn
               580                 585                 590
   Arg Val Lys Ala Ala Lys Lys Val Pro Leu Asp Arg Met Pro Tyr Asn
           595                 600                 605
   Leu Gln Tyr Thr Val Glu Val Lys Asn Gly Ser Leu Ile Ile Pro His
       610                 615                 620
   Tyr Asp His Tyr His Asn Ile Lys Phe Glu Trp Phe Asp Glu Gly Leu
   625                 630                 635                 640
   Tyr Glu Ala Pro Lys Gly Tyr Thr Leu Glu Asp Leu Leu Ala Thr Val
                   645                 650                 655
   Lys Tyr Tyr Val Glu His Pro Asn Glu Arg Pro His Ser Asp Asn Gly
               660                 665                 670
   Phe Gly Asn Ala Ser Asp His Val Gln Arg Asn Lys Asn Gly Gln Ala
           675                 680                 685
   Asp Thr Asn Gln Thr Glu Lys Pro Ser Glu Glu Lys Pro Gln Thr Glu
       690                 695                 700
   Lys Pro Glu Glu Glu Thr Pro Arg Glu Glu Lys Pro Gln Ser Glu Lys
   705                 710                 715                 720
   Pro Glu Ser Pro Lys Pro Thr Glu Glu Pro Glu Glu Glu Ser Pro Glu
                   725                 730                 735
   Glu Ser Glu Glu Pro Gln Val Glu Thr Glu Lys Val Glu Glu Lys Leu
               740                 745                 750
   Arg Glu Ala Glu Asp Leu Leu Gly Lys Ile Gln Asp Pro Ile Ile Lys
           755                 760                 765
   Ser Asn Ala Lys Glu Thr Leu Thr Gly Leu Lys Asn Asn Leu Leu Phe
       770                 775                 780
   Gly Thr Gln Asp Asn Asn Thr Ile Met Ala Glu Ala Glu Lys Leu Leu
   785                 790                 795                 800
   Ala Leu Leu Lys Glu Ser Lys
                   805
<210> 102
   <211> 821
   <212> PRT
   <213> S. pneumoniae
<400> 102
   Cys Ala Tyr Glu Leu Gly Leu His Gln Ala Gln Thr Val Lys Glu Asn
   1               5                   10                  15
   Asn Arg Val Ser Tyr Ile Asp Gly Lys Gln Ala Thr Gln Lys Thr Glu
               20                  25                  30
   Asn Leu Thr Pro Asp Glu Val Ser Lys Arg Glu Gly Ile Asn Ala Glu
           35                  40                  45
   Gln Ile Val Ile Lys Ile Thr Asp Gln Gly Tyr Val Thr Ser His Gly
       50                  55                  60
   Asp His Tyr His Tyr Tyr Asn Gly Lys Val Pro Tyr Asp Ala Ile Ile
   65                  70                  75                  80
   Ser Glu Glu Leu Leu Met Lys Asp Pro Asn Tyr Gln Leu Lys Asp Ser
                   85                  90                  95
   Asp Ile Val Asn Glu Ile Lys Gly Gly Tyr Val Ile Lys Val Asn Gly
               100                 105                 110
   Lys Tyr Tyr Val Tyr Leu Lys Asp Ala Ala His Ala Asp Asn Val Arg
           115                 120                 125
   Thr Lys Glu Glu Ile Asn Arg Gln Lys Gln Glu His Ser Gln His Arg
       130                 135                 140
   Glu Gly Gly Thr Ser Ala Asn Asp Gly Ala Val Ala Phe Ala Arg Ser
   145                 150                 155                 160
   Gln Gly Arg Tyr Thr Thr Asp Asp Gly Tyr Ile Phe Asn Ala Ser Asp
                   165                 170                 175
   Ile Ile Glu Asp Thr Gly Asp Ala Tyr Ile Val Pro His Gly Asp His
               180                 185                 190
   Tyr His Tyr Ile Pro Lys Asn Glu Leu Ser Ala Ser Glu Leu Ala Ala
           195                 200                 205
   Ala Glu Ala Phe Leu Ser Gly Arg Glu Asn Leu Ser Asn Leu Arg Thr
       210                 215                 220
   Tyr Arg Arg Gln Asn Ser Asp Asn Thr Pro Arg Thr Asn Trp Val Pro
   225                 230                 235                 240
   Ser Val Ser Asn Pro Gly Thr Thr Asn Thr Asn Thr Ser Asn Asn Ser
                   245                 250                 255
   Asn Thr Asn Ser Gln Ala Ser Gln Ser Asn Asp Ile Asp Ser Leu Leu
               260                 265                 270
   Lys Gln Leu Tyr Lys Leu Pro Leu Ser Gln Arg His Val Glu Ser Asp
           275                 280                 285
   Gly Leu Ile Phe Asp Pro Ala Gln Ile Thr Ser Arg Thr Ala Arg Gly
       290                 295                 300
   Val Ala Val Pro His Gly Asn His Tyr His Phe Ile Pro Tyr Glu Gln
   305                 310                 315                 320
   Met Ser Glu Leu Glu Lys Arg Ile Ala Arg Ile Ile Pro Leu Arg Tyr
                   325                 330                 335
   Arg Ser Asn His Trp Val Pro Asp Ser Arg Pro Glu Glu Pro Ser Pro
               340                 345                 350
   Gln Pro Thr Pro Glu Pro Ser Pro Ser Pro Gln Pro Ala Pro Asn Pro
           355                 360                 365
   Gln Pro Ala Pro Ser Asn Pro Ile Asp Glu Lys Leu Val Lys Glu Ala
       370                 375                 380
   Val Arg Lys Val Gly Asp Gly Tyr Val Phe Glu Glu Asn Gly Val Ser
   385                 390                 395                 400
   Arg Tyr Ile Pro Ala Lys Asn Leu Ser Ala Glu Thr Ala Ala Gly Ile
                   405                 410                 415
   Asp Ser Lys Leu Ala Lys Gln Glu Ser Leu Ser His Lys Leu Gly Ala
               420                 425                 430
   Lys Lys Thr Asp Leu Pro Ser Ser Asp Arg Glu Phe Tyr Asn Lys Ala
           435                 440                 445
   Tyr Asp Leu Leu Ala Arg Ile His Gln Asp Leu Leu Asp Asn Lys Gly
       450                 455                 460
   Arg Gln Val Asp Phe Glu Ala Leu Asp Asn Leu Leu Glu Arg Leu Lys
   465                 470                 475                 480
   Asp Val Ser Ser Asp Lys Val Lys Leu Val Asp Asp Ile Leu Ala Phe
                   485                 490                 495
   Leu Ala Pro Ile Arg His Pro Glu Arg Leu Gly Lys Pro Asn Ala Gln
               500                 505                 510
   Ile Thr Tyr Thr Asp Asp Glu Ile Gln Val Ala Lys Leu Ala Gly Lys
           515                 520                 525
   Tyr Thr Thr Glu Asp Gly Tyr Ile Phe Asp Pro Arg Asp Ile Thr Ser
       530                 535                 540
   Asp Glu Gly Asp Ala Tyr Val Thr Pro His Met Thr His Ser His Trp
   545                 550                 555                 560
   Ile Lys Lys Asp Ser Leu Ser Glu Ala Glu Arg Ala Ala Ala Gln Ala
                   565                 570                 575
   Tyr Ala Lys Glu Lys Gly Leu Thr Pro Pro Ser Thr Asp His Gln Asp
               580                 585                 590
   Ser Gly Asn Thr Glu Ala Lys Gly Ala Glu Ala Ile Tyr Asn Arg Val
           595                 600                 605
   Lys Ala Ala Lys Lys Val Pro Leu Asp Arg Met Pro Tyr Asn Leu Gln
       610                 615                 620
   Tyr Thr Val Glu Val Lys Asn Gly Ser Leu Ile Ile Pro His Tyr Asp
   625                 630                 635                 640
   His Tyr His Asn Ile Lys Phe Glu Trp Phe Asp Glu Gly Leu Tyr Glu
                   645                 650                 655
   Ala Pro Lys Gly Tyr Thr Leu Glu Asp Leu Leu Ala Thr Val Lys Tyr
               660                 665                 670
   Tyr Val Glu His Pro Asn Glu Arg Pro His Ser Asp Asn Gly Phe Gly
           675                 680                 685
   Asn Ala Ser Asp His Val Gln Arg Asn Lys Asn Gly Gln Ala Asp Thr
       690                 695                 700
   Asn Gln Thr Glu Lys Pro Ser Glu Glu Lys Pro Gln Thr Glu Lys Pro
   705                 710                 715                 720
   Glu Glu Glu Thr Pro Arg Glu Glu Lys Pro Gln Ser Glu Lys Pro Glu
                   725                 730                 735
   Ser Pro Lys Pro Thr Glu Glu Pro Glu Glu Glu Ser Pro Glu Glu Ser
               740                 745                 750
   Glu Glu Pro Gln Val Glu Thr Glu Lys Val Glu Glu Lys Leu Arg Glu
           755                 760                 765
   Ala Glu Asp Leu Leu Gly Lys Ile Gln Asp Pro Ile Ile Lys Ser Asn
       770                 775                 780
   Ala Lys Glu Thr Leu Thr Gly Leu Lys Asn Asn Leu Leu Phe Gly Thr
   785                 790                 795                 800
   Gln Asp Asn Asn Thr Ile Met Ala Glu Ala Glu Lys Leu Leu Ala Leu
                       805                    810                    815
   Leu Lys Glu Ser Lys
               820

## Description

### FIELD OF THE INVENTION

The present invention is related to antigens, more particularly protein antigens of streptococcus pneumoniaepathogen which are useful as vaccine components for therapy and/or prophylaxis.

### BACKGROUND OF THE INVENTION

S. pneumoniae is an important agent of disease in man especially among infants, the elderly and immunocompromised persons. It is a bacterium frequently isolated from patients with invasive diseases such as bacteraemia/septicaemia, pneumonia, meningitis with high morbidity and mortality throughout the world. Even with appropriate antibiotic therapy, pneumococcal infections still result in many deaths. Although the advent of antimicrobial drugs has reduced the overall mortality from pneumococcal disease, the presence of resistant pneumococcal organisms has become a major problem in the world today. Effective pneumococcal vaccines could have a major impact on the morbidity and mortality associated with S. pneumoniae disease. Such vaccines would also potentially be useful to prevent otitis media in infants and young children.

Efforts to develop a pneumococcal vaccine have generally concentrated on generating immune responses to the pneumococcal capsular polysaccharide. More than 80 pneumococcal capsular serotypes have been identified on the basis of antigenic differences. The currently available pneumococcal vaccine, comprising 23 capsular polysaccharides that most frequently caused disease, has significant shortcomings related primarily to the poor immunogenicity of some capsular polysaccharides, the diversity of the serotypes and the differences in the distribution of serotypes over time, geographic areas and age groups. In particular, the failure of existing vaccines and capsular conjugate vaccines currently in development to protect young children against all serotypes spurres evaluation of other S. pneumoniae components. Although immunogenicity of capsular polysaccharides can be improved, serotype specificity will still represent a major limitation of polysaccharide-based vaccines. The use of a antigenically conserved immunogenic pneumococcal protein antigen, either by itself or in combination with additional components, offers the possibility of a protein-based pneumococcal vaccine.

PCT Publication number WO98/1893 published may 7 1998 entitled "*Streptococcus Pneumoniae* antigens and vaccines" describes certain polypeptides which are claimed to be antigenic. However, no biological activity of these polypeptides is reported.

Therefore their remains an unmet need for Streptococcus antigens that may be used as vaccine components for the prophylaxis and/or therapy of Streptococcus infection.

### SUMMARY OF THE INVENTION

According to one aspect, the present invention provides a vaccine composition comprising an isolated polypeptide having a) greater than 90% identity to a second polypeptide having an amino acid sequence chosen from: SEQ ID NOs: 14, 4, 58, 60, 61, 63, 73 to 75, or 79 or, b) greater than 99% identity to a second polypeptide having a sequence chosen from: SEQ ID NO: 67 or 68, wherein the polypeptide is not SEQ ID NO:5 of NO 00/17370, or SEQ ID NO:194 of WO 00/06737, wherein the polypeptide elicits an anti-streptococcal immune response when administered to an individual; and a pharmaceutically acceptable carrier, diluent or adjuvant; for use in the treatment or prophylaxis of *Streptococcus pneumoniae* infection.

### BRIEF DESCRIPTION OF THE DRAWINGS

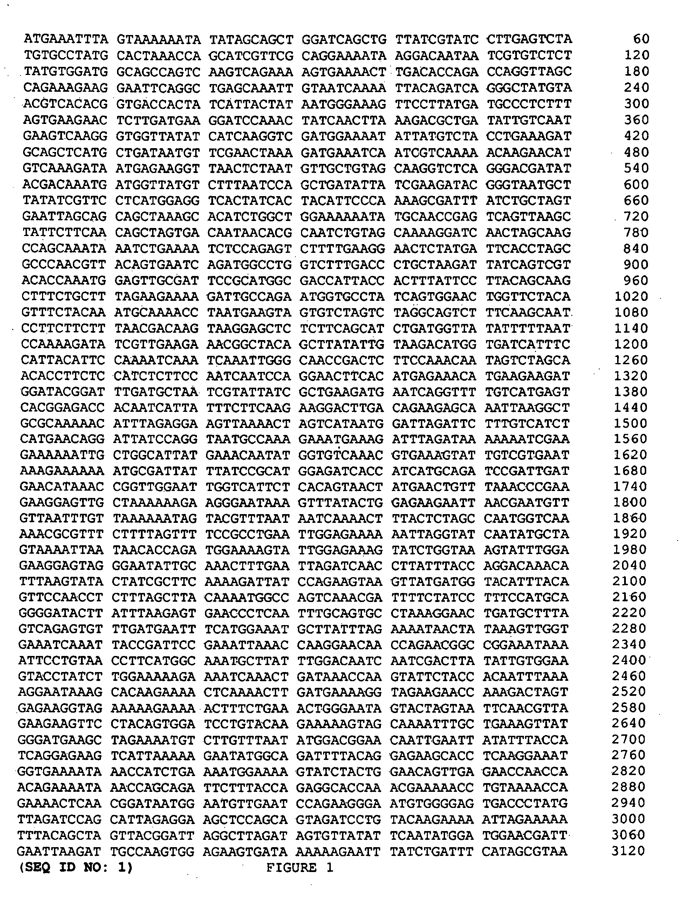

Figure 1 is the DNA sequence of BVH-3 gene; **SEQ ID NO: 1.**

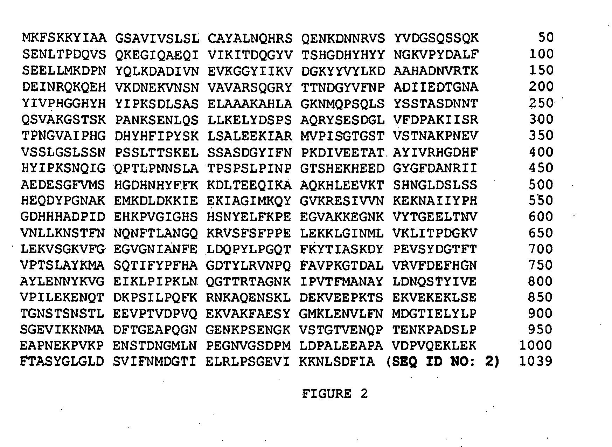

Figure 2 is the amino acid sequence of BVH-3 protein; **SEQ ID NO: 2.**

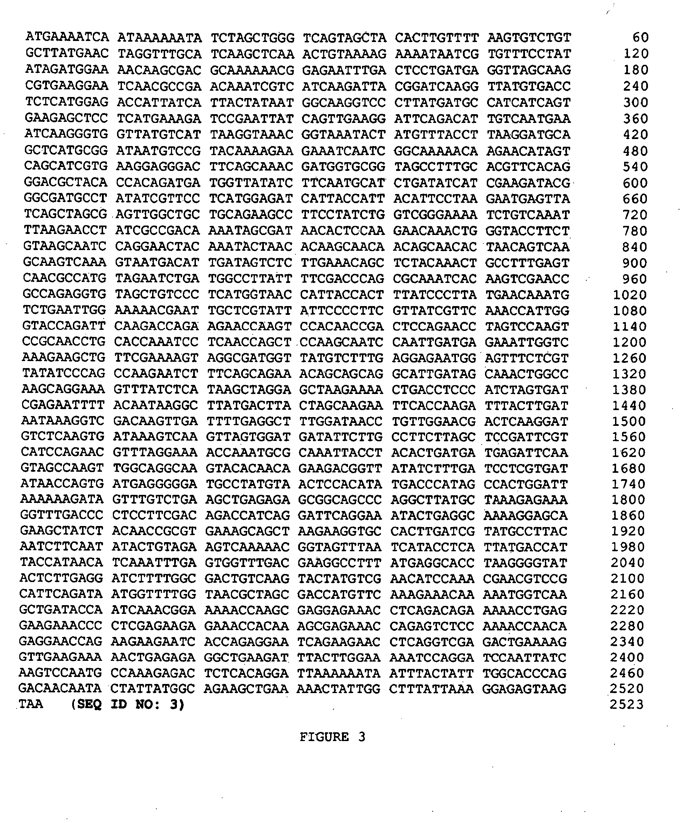

Figure 3 is.the DNA sequence of BVH-11 gene; **SEQ ID NO: 3.**

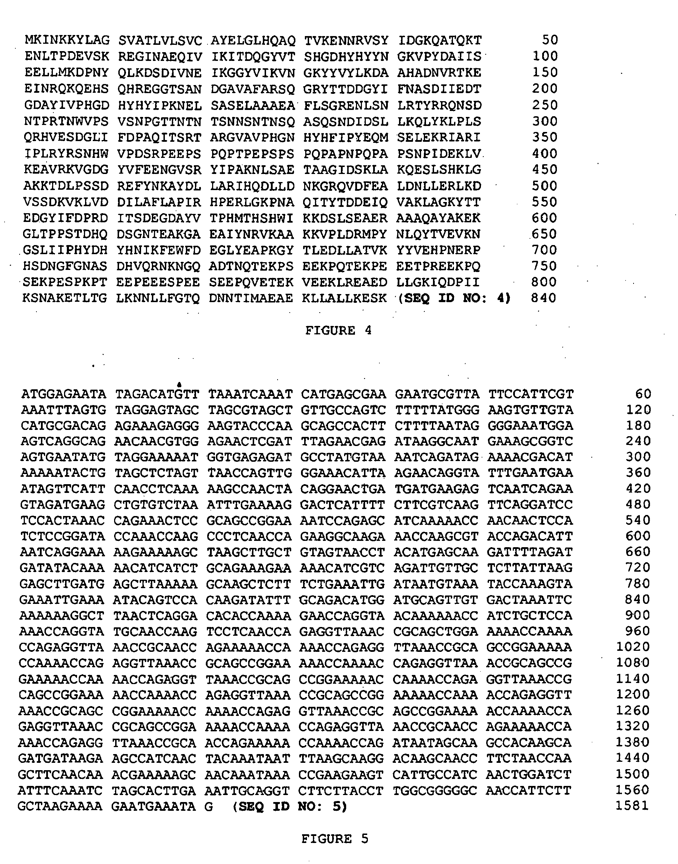

Figure 4 is the amino acid sequence of BVH-11 protein; **SEQ ID NO: 4.**
Figure 5 is the DNA sequence of BVH-28 gene; **SEQ ID NO: 5.**

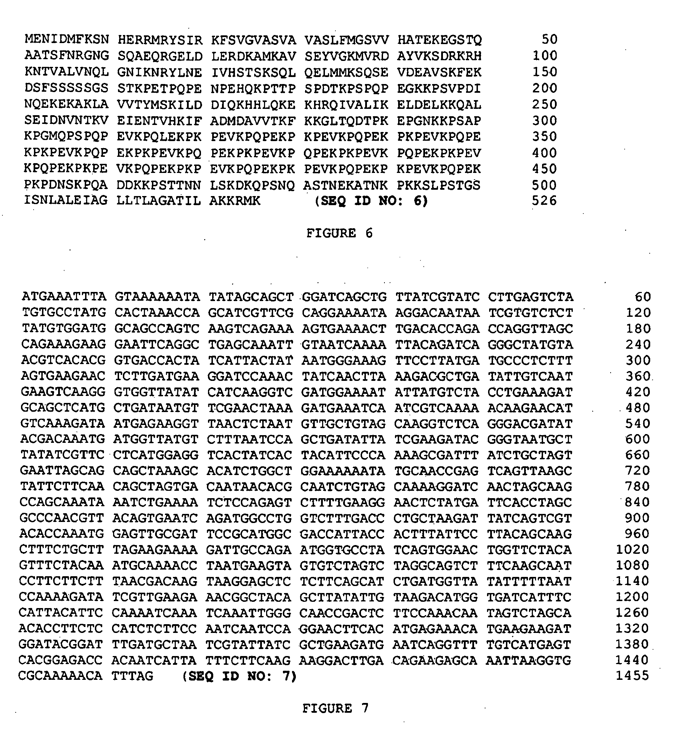

Figure 6 is the amino acid sequence of BVH-28 protein; **SEQ ID NO: 6.**
Figure 7 is the DNA sequence of BVH-3A gene which corresponds to the 5' terminal end of BVH-3; **SEQ ID NO: 7.**

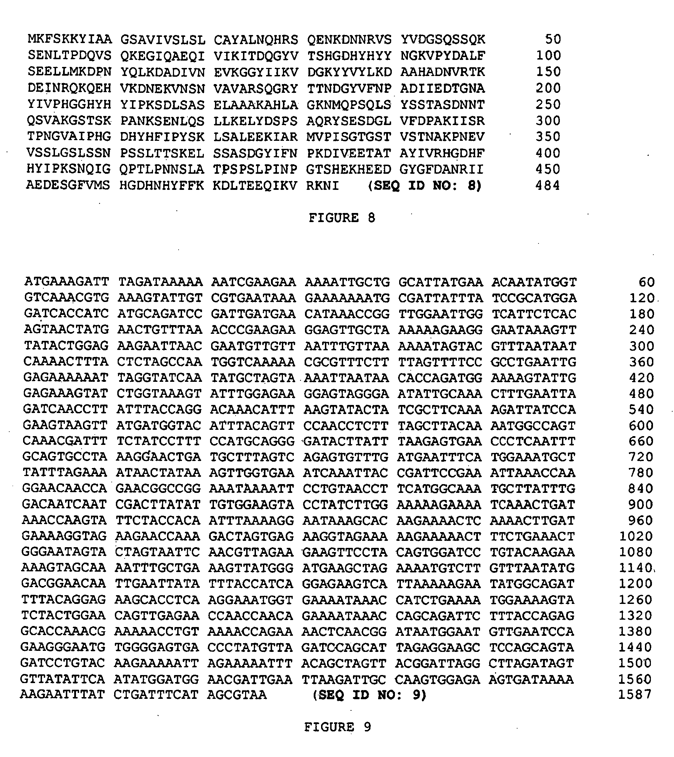

Figure 8 is the amino acid sequence of BVH-3A protein; **SEQ ID NO: 8.**
Figure 9 is the DNA sequence of BVH-3B gene which corresponds to the 3' terminal end of BVH-3; **SEQ ID NO: 9.**

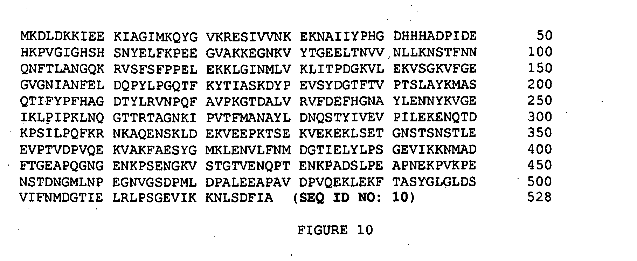

Figure 10 is the amino acid sequence of BVH-3B protein; **SEQ ID NO: 10.**
Figure 11 depicts the comparison of the predicted amino acid sequences of the BVH-3 open reading frames from WU2, RX1, JNR.7/87, SP64, P4241 and A66 S. pneumoniae strains by using the program Clustal W from MacVector sequence analysis software (version 6.5). Underneath the alignment, there is a consensus line where * and . characters indicate identical and similar amino acid residues, respectively.
Figure 12 depicts the comparison of the predicted amino acid sequences of the BVH-11 open reading frames from WU2, Rx1, JNR.7/87, SP64, P4241, A66 and SP63 S. pneumoniae strains by using the program Clustal W from MacVector sequence analysis software (version 6.5). Underneath the alignment, there is a consensus line where * and . characters indicate identical and similar amino acid residues, respectively.
Figure 13 depicts the comparison of the predicted amino acid sequences of the BVH-11 proteins from various S. pneumoniae strains. The degrees of identity (I) and similarity (S) were determined by using the program Clustal W from MacVector sequence analysis software (version 6.5).

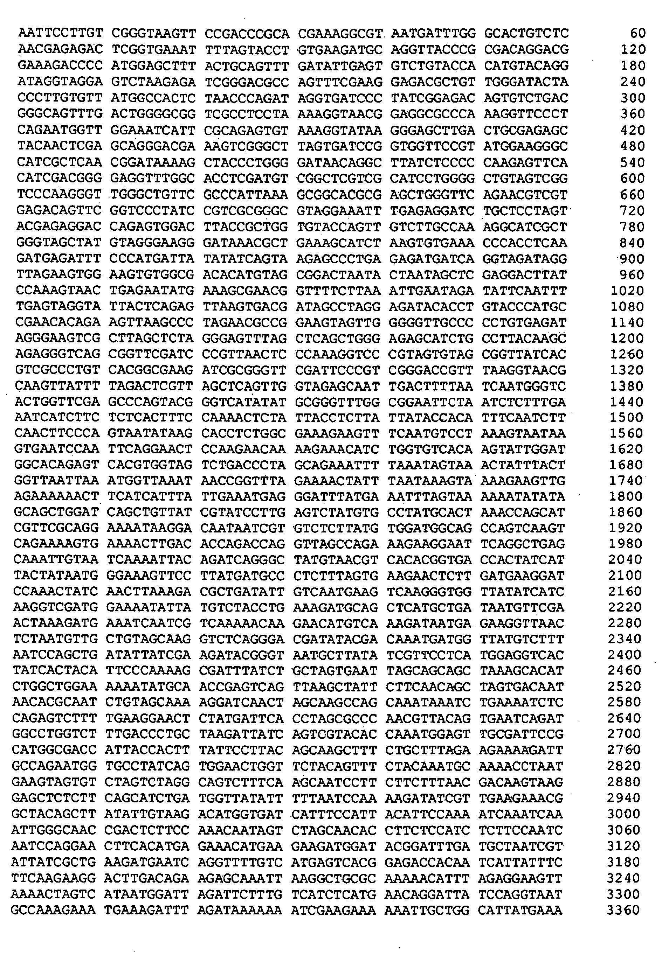

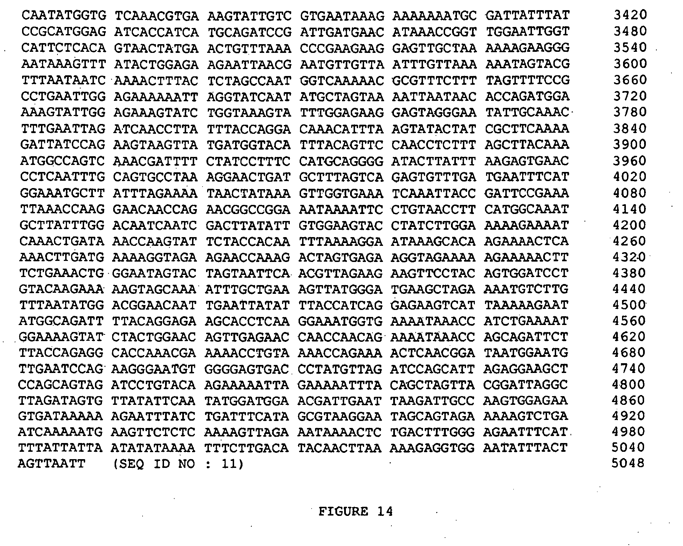

Figure 14 is a DNA sequence containing the complete BVH-3 gene (open reading frame "ORF" at nucleotides 1777 to 4896); **SEQ ID NO: 11.**

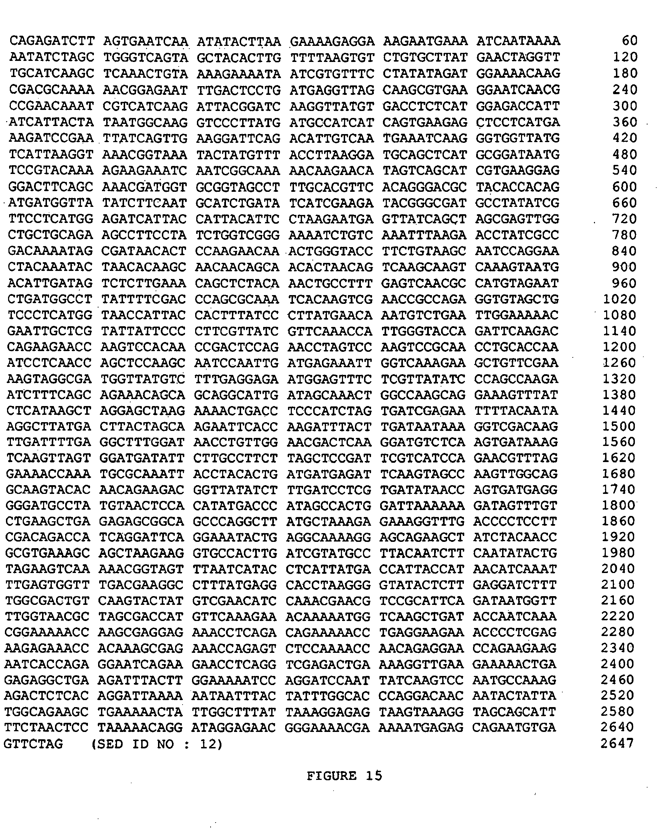

Figure 15 is a DNA sequence containing the complete BVH-11 gene (ORF at nucleotides 45 to 2567); **SEQ ID NO: 12.**

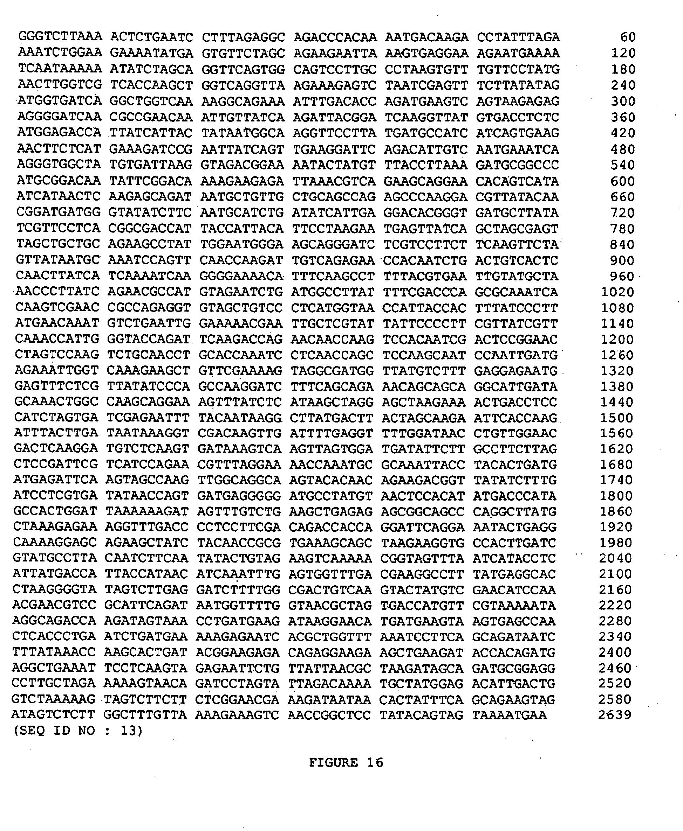

Figure 16 is a DNA sequence containing the complete BVH-11-2 gene (ORF at nucleotides 114 to 2630); **SEQ ID NO: 13.**

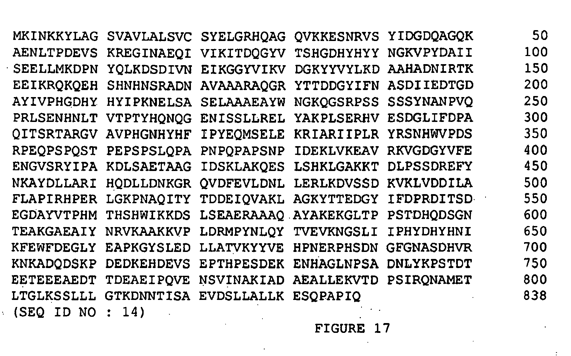

Figure 17 is the amino acid sequence of BVH-11-2 protein; **SEQ ID NO: 14.**

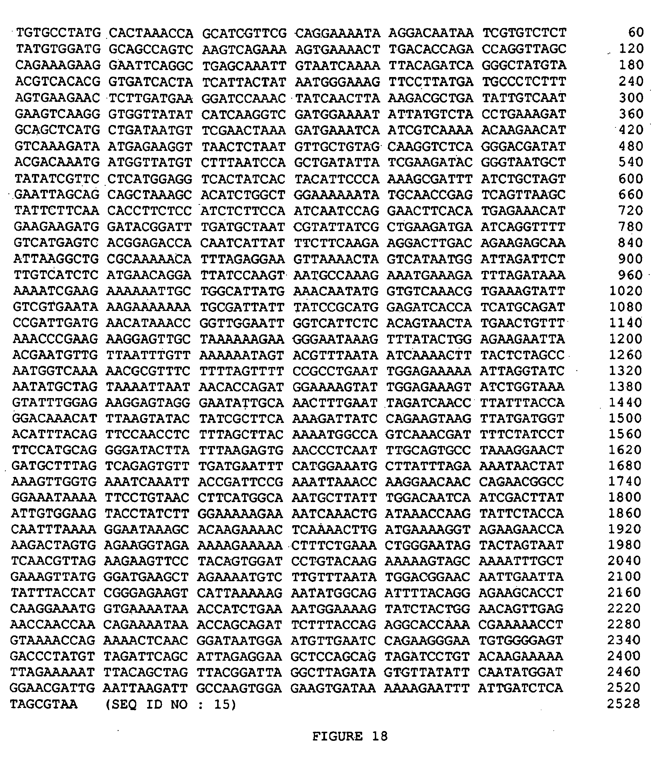

Figure 18 is the DNA sequence of SP63 BVH-3 gene; **SEQ ID NO:15.**

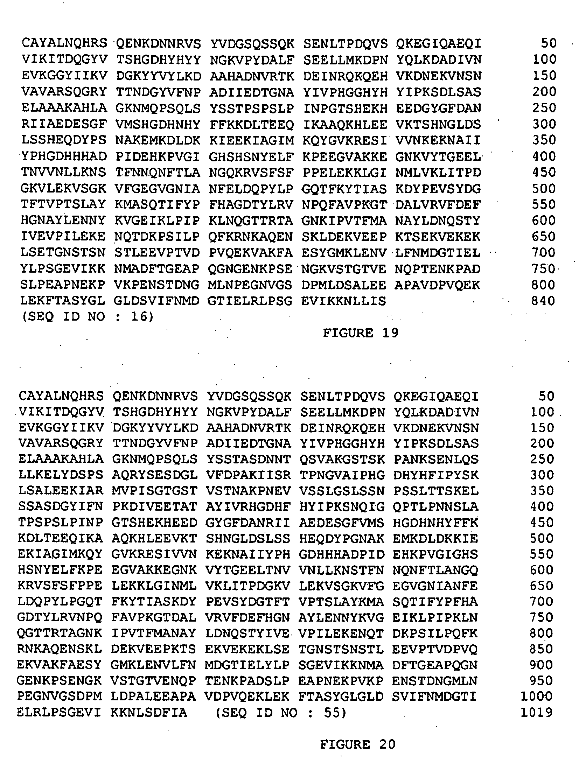

Figure 19 is the amino acid sequence of SP63 BVH-3 protein; **SEQ ID NO: 16.**
Figure 20 is the amino acid sequence of BVH-3M protein; **SEQ ID NO: 55.**

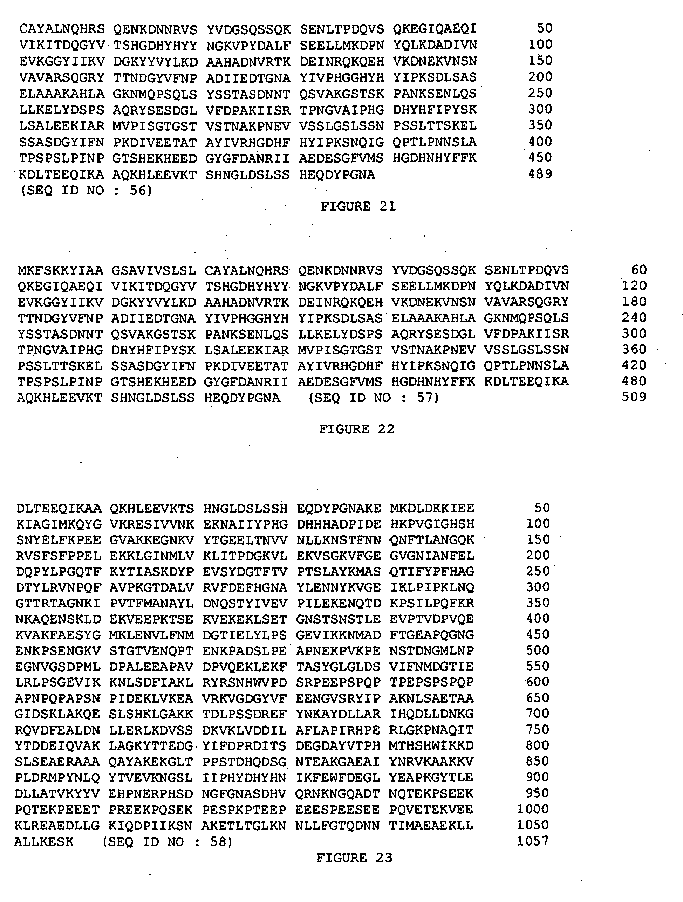

Figure 21 is the amino acid sequence of BVH-3AD protein; **SEQ ID NO: 56.**
Figure 22 is the amino acid sequence of L-BVH-3-AD protein; **SEQ ID NO: 57.**
Figure 23 is the amino acid sequence of NEW12 protein; **SEQ ID NO: 58.**

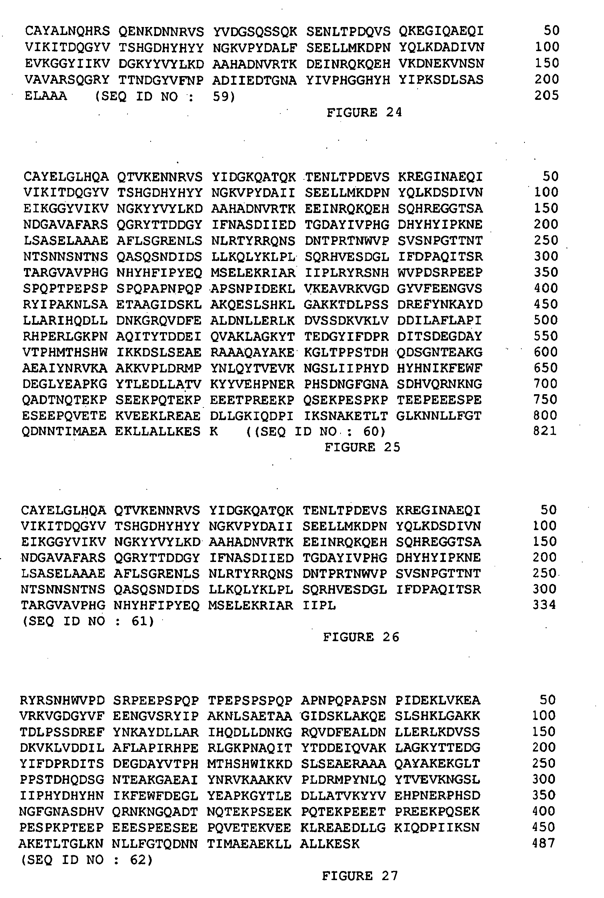

Figure 24 is the amino acid sequence of BVH-3C protein; **SEQ ID NO: 59.**
Figure 25 is the amino acid sequence of BVH-11M protein; **SEQ ID NO: 60.**
Figure 26 is the amino acid sequence of BVH-11A protein; **SEQ ID NO: 61.**
Figure 27 is the amino acid sequence of BVH-11B (also called New13) protein; **SEQ ID NO: 62.**

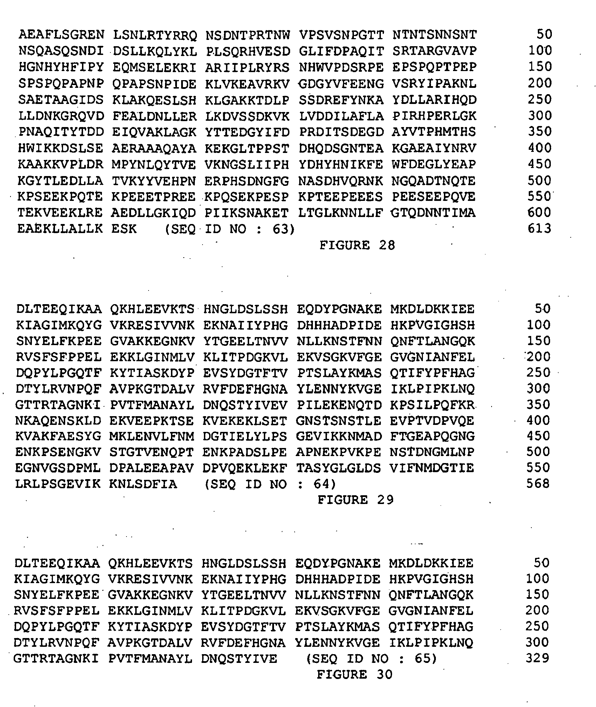

Figure 28 is the amino acid sequence of BVH-11C protein; **SEQ ID NO: 63.**
Figure 29 is the amino acid sequence of NEW1 protein; **SEQ ID NO: 64.**
Figure 30 is the amino acid sequence of NEW2 protein; **SEQ ID NO: 65.**

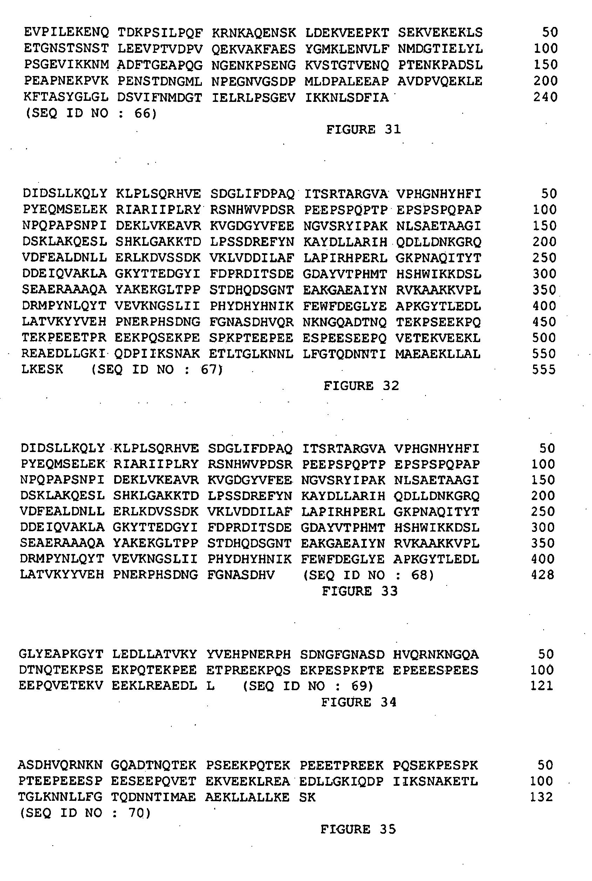

Figure 31 is the amino acid sequence of NEW3 protein; **SEQ ID NO: 66.**
Figure 32 is the amino acid sequence of NEW4 protein; **SEQ ID NO: 67.**
Figure 33 is the amino acid sequence of NEW5 protein; **SEQ ID NO: 68.**
Figure 34 is the amino acid sequence of NEW6 protein; **SEQ ID NO: 69.**
Figure 35 is the amino acid sequence of NEW7 protein; **SEQ ID NO: 70.**

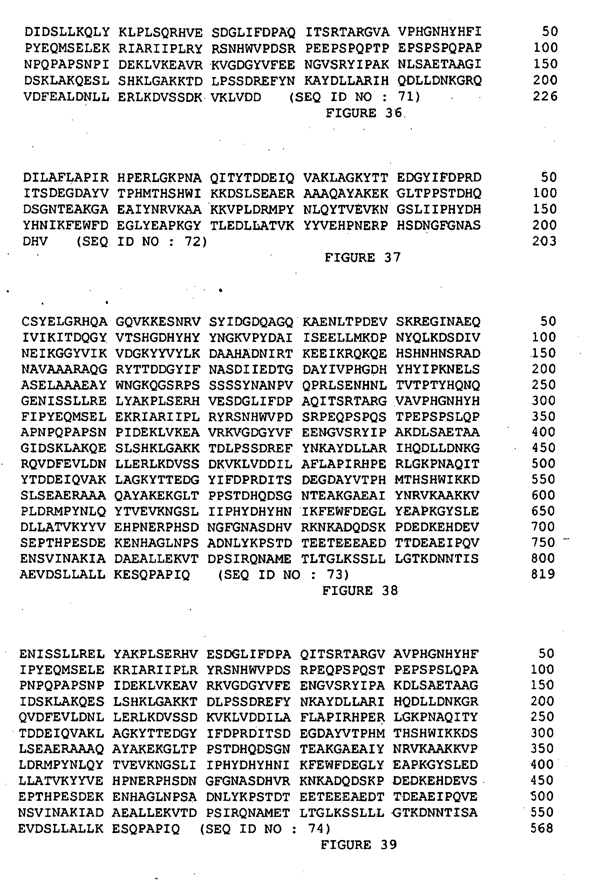

Figure 36 is the amino acid sequence of NEW8 protein; **SEQ ID NO: 71.**
Figure 37 is the amino acid sequence of NEW9 protein; **SEQ ID NO: 72.**
Figure 38 is the amino acid sequence of BVH-11-2M protein; **SEQ ID NO: 73.**
Figure 39 is the amino acid sequence of NEW10 protein; **SEQ ID NO: 74.**

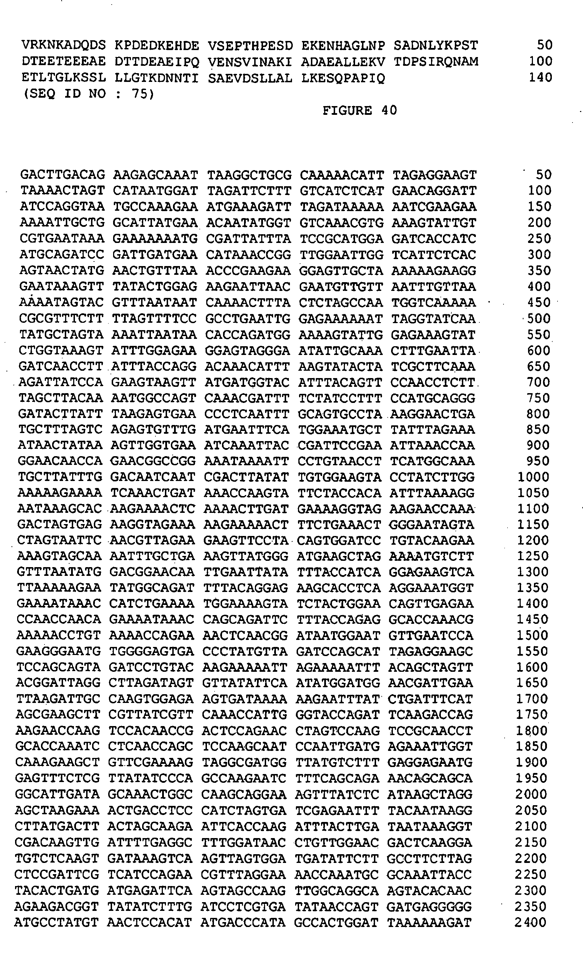

Figure 40 is the amino acid sequence of NEW11 protein; **SEQ ID NO: 75.**

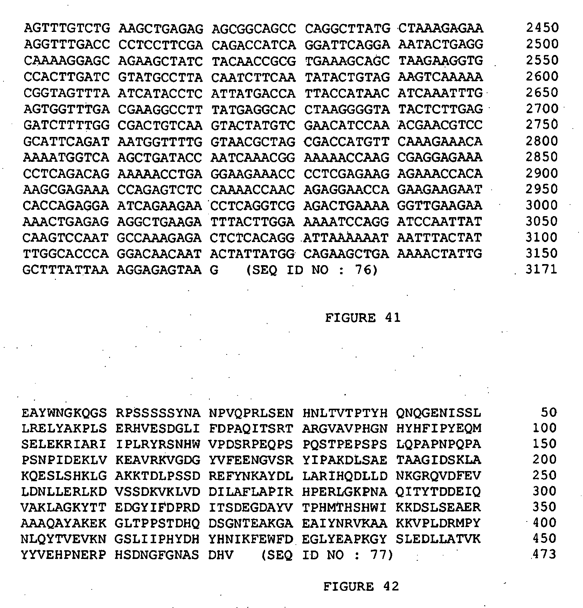

Figure 41 is the DNA sequence of NEW12 gene; **SEQ ID NO: 76.**
Figure 42 is the amino acid sequence of NEW14 protein; **SEQ ID NO: 77.**

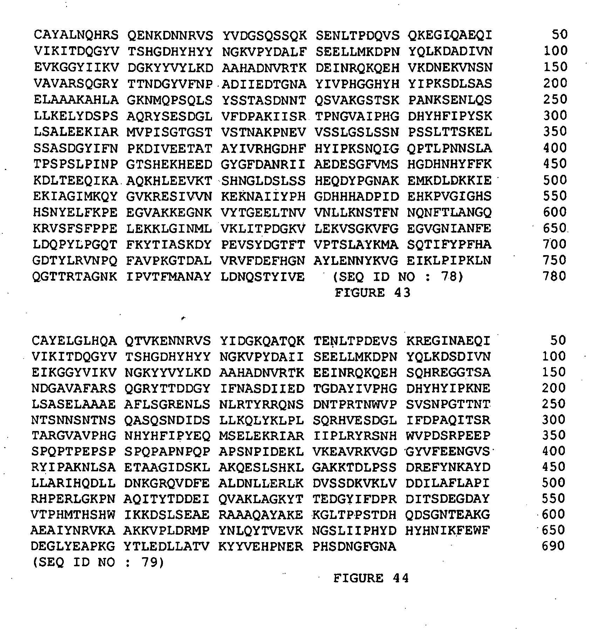

Figure 43 is the amino acid sequence of NEW15 protein; **SEQ ID NO: 78.**
Figure 44 is the amino acid sequence of NEW16 protein; **SEQ ID NO: 79.**

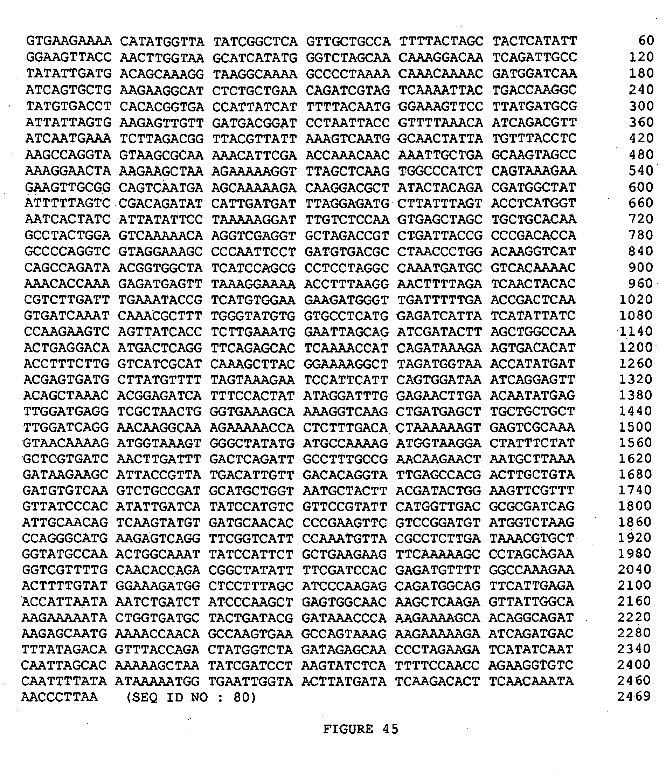

Figure 45 is the DNA sequence of GBS BVH-71 gene; **SEQ ID NO: 80.**

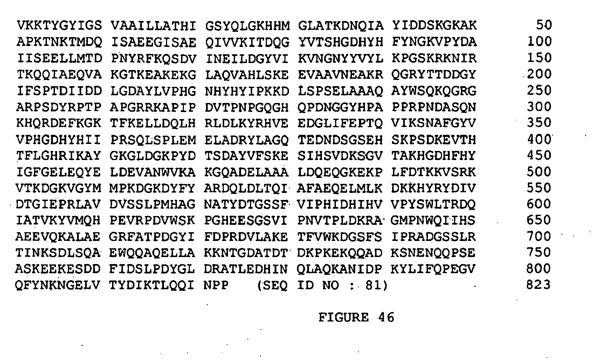

Figure 46 is the amino acid sequence of GBS BVH-71 protein; **SEQ ID NO: 81.**

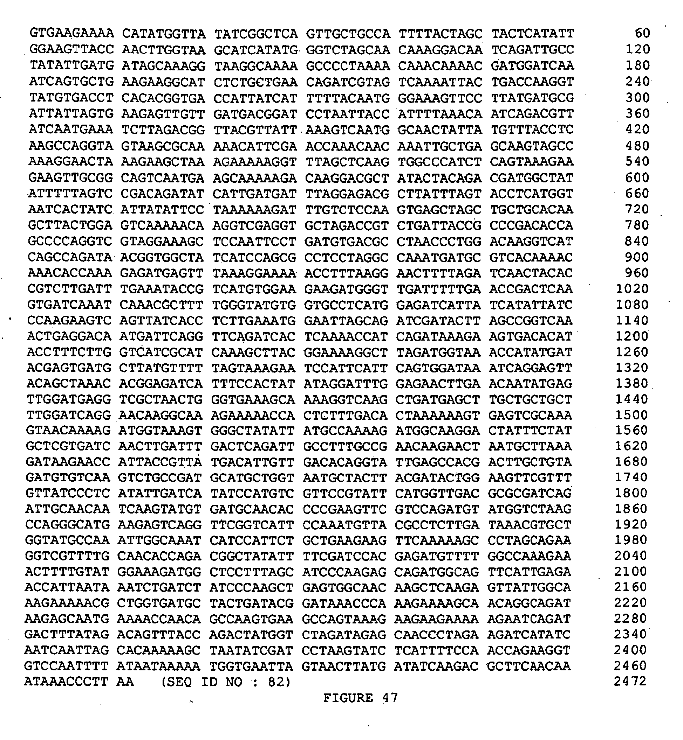

Figure 47 is the DNA sequence of GAS BVH-71 gene; **SEQ ID NO:82.**

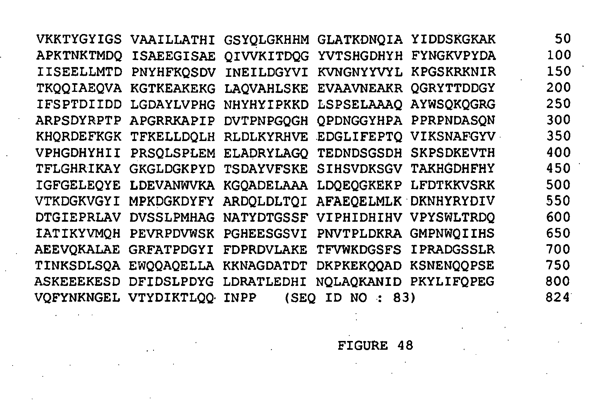

Figure 48 is the amino acid sequence of GAS BVH-71 protein; **SEQ ID NO:83.**

### DETAILED DESCRIPTION OF THE INVENTION

According to one aspect, the present invention provides an isolated polynucleotide encoding a polypeptide having at least 95% identity to a second polypeptide comprising a sequence chosen from **SEQ ID NOs: 4, 14, 58, 60, 61, 63, 73 to 75, 79** or fragments, analogs or derivatives thereof.

According to one aspect, the present invention provides an isolated polynucleotide encoding a polypeptide having at least 95% identity to a second polypeptide comprising sequence **SEQ ID NO: 4** or fragments, analogs or derivatives thereof.

According to one aspect, the present invention provides an isolated polynucleotide encoding a polypeptide having at least 95% identity to a second polypeptide comprising sequence **SEQ ID NO: 14** or fragments, analogs or derivatives thereof.

According to one aspect, the present invention provides an isolated polynucleotide encoding a polypeptide having at least 95% identity to a second polypeptide comprising sequence **SEQ ID NO: 58** or fragments, analogs or derivatives thereof.

According to one aspect, the present invention provides an isolated polynucleotide encoding a polypeptide having at least 95% identity to a second polypeptide comprising sequence **SEQ ID NO: 60** or fragments, analogs or derivatives thereof.

According to one aspect, the present invention provides an isolated polynucleotide encoding a polypeptide having greater than 99% identity to a second polypeptide comprising sequence **SEQ ID NO: 67** or fragments, analogs or derivatives thereof.

According to one aspect, the present invention provides an isolated polynucleotide encoding a polypeptide having greater than 99% identity to a second polypeptide comprising sequence **SEQ ID NO: 68** or fragments, analogs or derivatives thereof.

According to one aspect, the present invention relates to polypeptides characterized by the amino acid sequence chosen from **SEQ ID NOs: 4, 14, 58, 60 to 63, 67, 68, 73 to 75, 77, 79** or fragments, analogs or derivatives thereof.

According to one aspect, the present invention relates to polypeptides characterized by the amino acid sequence chosen from **SEQ ID NOs: 4, 14** or fragments, analogs or derivatives thereof.

According to one aspect, the present invention relates to polypeptides characterized by the amino acid sequence comprising sequence **SEQ ID NO: 4** or fragments, analogs or derivatives thereof.

According to one aspect, the present invention relates to polypeptides characterized by the amino acid sequence comprising sequence **SEQ ID NO: 14** or fragments, analogs or derivatives thereof.

According to one aspect, the present invention relates to polypeptides characterized by the amino acid sequence chosen from **SEQ ID NOs: 58, 60 to 63, 67, 68, 73 to 75, 77, 79** or fragments, analogs or derivatives thereof.

According to one aspect, the present invention relates to polypeptides characterized by the amino acid sequence chosen from **SEQ ID NO: 58, 60, 62, 67, 68, 77** or fragments, analogs or derivatives thereof.

According to one aspect, the present invention relates to polypeptides characterized by the amino acid sequence chosen from **SEQ ID NO: 67, 68, 77** or fragments, analogs or derivatives thereof.

According to one aspect, the present invention relates to polypeptides characterized by the amino acid sequence comprising sequence **SEQ ID NO: 58** or fragments, analogs or derivatives thereof.

According to one aspect, the present invention relates to polypeptides characterized by the amino acid sequence comprising sequence **SEQ ID NO: 67** or fragments, analogs or derivatives thereof.

According to one aspect, the present invention relates to polypeptides characterized by the amino acid sequence comprising sequence **SEQ ID NO: 68** or fragments, analogs or derivatives thereof.

According to one aspect, the present invention relates to polypeptides characterized by the amino acid sequence comprising sequence **SEQ ID NO: 77** or fragments, analogs or derivatives thereof.

In a further embodiment, the present invention also relates to chimeric polypeptides which comprise one or more polypeptides or fragments, analogs or derivatives thereof as described in the present application.

In a further embodiment, the present invention also relates to chimeric polypeptides which comprise one or more polypeptides or fragments, analogs or derivatives thereof as defined in the figures of the present application.

In a further embodiment, the present application also relates to chimeric polypeptides which comprise two or more polypeptides chosen from **SEQ ID NOs: 4, 14, 58, 60 to 63, 67, 68, 73 to 75, 77, 79** or fragments, analogs or derivatives thereof ;provided that the polypeptides or fragments, analogs or derivatives thereof are linked as to form a chimeric polypeptide.

In a further embodiment, the chimeric polypeptide will comprise two or more polypeptides chosen from **SEQ ID NOs** : **58, 60, 62, 67, 68, 77** or fragments, analogs or derivatives thereof; provided that the polypeptides or fragments, analogs or derivatives thereof are linked as to form a chimeric polypeptide.

In a further embodiment, the chimeric polypeptide will comprise two or more polypeptides chosen from **SEQ ID NOs** : **62, 67, 68, 77** or fragments, analogs or derivatives thereof; provided that the polypeptides or fragments, analogs or derivatives thereof are linked as to form a chimeric polypeptide.

In a further embodiment, the chimeric polypeptide will comprise between 2 and 5 polypeptides.

In a further embodiment, the chimeric polypeptide will comprise between 2 and 4 polypeptides.

In a further embodiment, the chimeric polypeptide will comprise between 2 and 3 polypeptides.

In a further embodiment, the chimeric polypeptide will comprise 2 polypeptides.

In a further embodiment, there is provided a chimeric polypeptide of formula (I):

**A-(B)ₘ-(C)ₙ-D** (I)

Wherein;
**m** is 0 or 1,
**n** is 0 or 1,
**A** is chosen from **SEQ ID NOs: 4, 14, 58, 60 to 63, 67, 68, 73 to 75, 77, 79** or fragments, analogs or derivatives thereof;
**B** is chosen from **SEQ ID NOs: 4, 14, 58, 60 to 63, 67, 68, 73 to 75, 77, 79** or fragments, analogs or derivatives thereof;
**C** is chosen from **SEQ ID NOs: 4, 14, 58, 60 to 63, 67, 68, 73 to 75, 77, 79** or fragments, analogs or derivatives thereof; and
**D** is chosen from **SEQ ID NOs: 4, 14, 58, 60 to 63, 67, 68, 73 to 75, 77, 79** or fragments, analogs or derivatives thereof.

In a further embodiment,
**A** is chosen from **SEQ ID NOs : 58, 60, 62, 67, 68, 77** or fragments, analogs or derivatives thereof;
**B** is chosen from **SEQ ID NOs : 58, 60, 62, 67, 68, 77** or fragments, analogs or derivatives thereof;
**C** is chosen from **SEQ ID NOs : 58, 60, 62, 67, 68, 77** or fragments, analogs or derivatives thereof; and
**D** is chosen from **SEQ ID NOs : 58, 60, 62, 67, 68, 77** or fragments, analogs or derivatives thereof.

In one embodiment, chimeric polypeptides of the present invention comprise those wherein the following embodiments are present, either independently or in combination.

In a further embodiment, **A** is **SEQ ID NOs : 58, 62, 67, 68, 77** or fragments, analogs or derivatives thereof.

In a further embodiment, **A** is **SEQ ID NO :58** or fragments, analogs or derivatives thereof.

In a further embodiment, **A** is **SEQ ID NO :62** or fragments, analogs or derivatives thereof.

In a further embodiment, **A** is **SEQ ID NO :67** or fragments, analogs or derivatives thereof.

In a further embodiment, **A** is **SEQ ID NO :68** or fragments, analogs or derivatives thereof.

In a further embodiment, **A** is **SEQ ID NO :77** or fragments, analogs or derivatives thereof.

In a further embodiment, **B** is **SEQ ID NOs : 58, 62, 67, 68, 77** or fragments, analogs or derivatives thereof.

In a further embodiment, **B** is **SEQ ID NO :58** or fragments, analogs or derivatives thereof.

In a further embodiment, **B** is **SEQ ID NO :67** or fragments, analogs or derivatives thereof.

In a further embodiment, **B** is **SEQ ID NO :68** or fragments, analogs or derivatives thereof.

In a further embodiment, **B** is **SEQ ID NO : 77** or fragments, analogs or derivatives thereof.

In a further embodiment, **C** is **SEQ ID NOs : 58, 62, 67, 68, 77** or fragments, analogs or derivatives thereof.

In a further embodiment, **C** is **SEQ ID NO :58** or fragments, analogs or derivatives thereof.

In a further embodiment, **C** is **SEQ ID NO : 62** or fragments, analogs or derivatives thereof.

In a further embodiment, **C** is **SEQ ID NO : 67** or fragments, analogs or derivatives thereof.

In a further embodiment, **C** is **SEQ ID NO : 68** or fragments, analogs or derivatives thereof.

In a further embodiment, **C** is **SEQ ID NO : 77** or fragments, analogs or derivatives thereof.

In a further embodiment, **D** is **SEQ ID NO : 58, 62, 67, 68, 77** or fragments, analogs or derivatives thereof.

In a further embodiment, **D** is **SEQ ID NO :58** or fragments, analogs or derivatives thereof.

In a further embodiment, **D** is **SEQ ID NO :62** or fragments, analogs or derivatives thereof.

In a further embodiment, **D** is **SEQ ID NO :67** or fragments, analogs or derivatives thereof.

In a further embodiment, **D** is **SEQ ID NO :68** or fragments, analogs or derivatives thereof.

In a further embodiment, **D** is **SEQ ID NO :77** or fragments, analogs or derivatives thereof.

In a further embodiment, **m** is 0.

In a further embodiment, **n** is 0.

In a further embodiment, **m** and **n** are 0.

In accordance with the present invention, all nucleotides encoding polypeptides and chimeric polypeptides are within the scope of the present invention.

In a further embodiment, the polypeptides or chimeric polypeptides in accordance with the present invention are antigenic.

In a further embodiment, the polypeptides or chimeric polypeptides in accordance with the present invention can elicit an immune response in an individual.

In a further embodiment, the present invention also relates to polypeptides which are able to raise antibodies having binding specificity to the polypeptides or chimeric polypeptides of the present invention as defined above.

An antibody that " has binding specificity" is an antibody that recognizes and binds the selected polypeptide but which does not substantially recognize and bind other molecules in a sample, e.g., a biological sample, which naturally includes the selected peptide. Specific binding can be measured using an ELISA assay in which the selected polypeptide is used as an antigen.

Unless otherwise defined, all technical and scientific terms used herein have the same meaning as commonly understood by one of ordinary skill in the art to which this invention belongs. All publications, patent applications, patents, and other references mentioned herein are incorporated by reference in their entirety. In case of conflict, the present specification, including definitions, will control. In addition, the materials, methods, and examples are illustrative only and not intended to be limiting.

As used herein, "fragments", "derivatives" or "analogs" of the polypeptides of the invention include those polypeptides in which one or more of the amino acid residues are substituted with a conserved or non-conserved amino acid residue (preferably conserved) and which may be natural or unnatural. In one embodiment, derivatives and analogs of polypeptides of the invention will have about 70% identity with those sequences illustrated in the figures or fragments thereof. That is, 70% of the residues are the same. In a further embodiment, polypeptides will have greater than 75% homology. In a further embodiment, polypeptides will have greater than 80% homology. In a further embodiment, polypeptides will have greater than 85% homology. In a further embodiment, polypeptides will have greater than 90% homology. In a further embodiment, polypeptides will have greater than 95% homology. In a further embodiment, polypeptides will have greater than 99% homology. In a further embodiment, derivatives and analogs of polypeptides of the invention will have fewer than about 20 amino acid residue substitutions, modifications or deletions and more preferably less than 10. Preferred substitutions are those known in the art as conserved i.e. the substituted residues share physical or chemical properties such as hydrophobicity, size, charge or functional groups.

In accordance with the present invention, polypeptides of the invention include both polypeptides and chimeric polypeptides.

Also included are polypeptides which have fused thereto other compounds which alter the polypeptides biological or pharmacological properties i.e. polyethylene glycol (PEG) to increase half-life; leader or secretory amino acid sequences for ease of purification; prepro- and prosequences; and (poly)saccharides.

Furthermore, in those situations where amino acid regions are found to be polymorphic, it may be desirable to vary one or more particular amino acids to more effectively mimic the different epitopes of the different streptococcus strains.

Moreover, the polypeptides of the present invention can be modified by terminal -NH₂ acylation (eg. by acetylation, or thioglycolic acid amidation, terminal carbosy amidation, e.g. with ammonia or methylamine) to provide stability, increased hydrophobicity for linking or binding to a support or other molecule.

Also contemplated are hetero and homo polypeptide multimers of the polypeptide fragments, analogues and derivatives. These polymeric forms include, for example, one or more polypeptides that have been cross-linked with cross-linkers such as avidin/biotin, gluteraldehyde or dimethylsuperimidate. Such polymeric forms also include polypeptides containing two or more tandem or inverted contiguous sequences, produced from multicistronic mRNAs generated by recombinant DNA technology.

Preferably, a fragment, analog or derivative of a polypeptide of the invention will comprise at least one antigenic region i.e. at least one epitope.

In order to achieve the formation of antigenic polymers (i.e. synthetic multimers), polypeptides may be utilized having bishaloacetyl groups, nitroarylhalides, or the like, where the reagents being specific for thio groups. Therefore, the link between two mercapto groups of the different peptides may be a single bond or may be composed of a linking group of at least two, typically at least four, and not more than 16, but usually not more than about 14 carbon atoms.

In a particular embodiment, polypeptide fragments, analogs and derivatives of the invention do not contain a methionine (Met) starting residue. Preferably, polypeptides will not incorporate a leader or secretory sequence (signal sequence). The signal portion of a polypeptide of the invention may be determined according to established molecular biological techniques. In general, the polypeptide of interest may be isolated from a streptococcus culture and subsequently sequenced to determine the initial residue of the mature protein and therefore the sequence of the mature polypeptide.

According to another aspect, there are provided vaccine compositions comprising one or more streptococcus polypeptides of the invention in admixture with a pharmaceutically acceptable carrier diluent or adjuvant. Suitable adjuvants include oils i.e. Freund's complete or incomplete adjuvant; salts i.e. AlK(SO₄)₂, AlNa(SO₄)₂, AlNH₄(SO₄)₂, silica, kaolin, carbon polynucleotides i.e. poly IC and poly AU. Preferred adjuvants include QuilA and Alhydrogel. Vaccines of the invention may be administered parenterally by injection, rapid infusion, nasopharyngeal absorption, dermoabsorption, or bucal or oral. Pharmaceutically acceptable carriers also include tetanus toxoid.

Vaccine compositions of the invention are used for the treatment or prophylaxis of streptococcus infection and/or diseases and symptoms mediated by streptococcus infection as described in P.R. Murray (Ed, in chief),E.J. Baron, M.A. Pfaller, F.C. Tenover and R.H. Yolken. Manual of Clinical Microbiology, ASM Press, Washington, D.C. sixth edition, 1995, 1482p which are herein incorporated by reference. In one embodiment, vaccine compositions of the present invention are used for the treatment or prophylaxis of meningitis, otitis media, bacteremia or pneumonia. In one embodiment, vaccine compositions of the invention are used for the treatment or prophylaxis of *streptococcus* infection and/or diseases and symptoms mediated by *streptococcus* infection, in particular S.pneumoniae, group A *streptococcus (pyogenes),* group B *streptococcus* (GBS or *agalactiae*), *dysgalactiae, uberis, nocardia* as well as *Staphylococcus aureus.* In a further embodiment, the streptococcus infection is S.pneumoniae.

In a particular embodiment, vaccines are administered to those individuals at risk of streptococcus infection such as infants, elderly and immunocompromised individuals.

As used in the present application, the term " individuals" include mammals. In a further embodiment, the mammal is human.

Vaccine compositions are preferably in unit dosage form of about 0.001 to 100 µg/kg (antigen/body weight) and more preferably 0.01 to 10 µg/kg and most preferably 0.1 to 1 µg/kg 1 to 3 times with an interval of about 1 to 6 week intervals between immunizations.

According to another aspect, there are provided polynucleotides encoding polypeptides characterized by the amino acid sequence chosen from **SEQ ID NOs: 4, 14, 58, 60 to 63, 67, 68, 73 to 75, 77, 79** or fragments, analogs or derivatives thereof.

In one embodiment, polynucleotides are those illustrated in **SEQ ID Nos: 3, 12, 13, 76** which may include the open reading frames (ORF), encoding polypeptides of the invention. It will be appreciated that the polynucleotide sequences illustrated in the figures may be altered with degenerate codons yet still encode the polypeptides of the invention. Accordingly the present invention further provides polynucleotides which hybridize to the polynucleotide sequences herein above described (or the complement sequences thereof) having 50% identity between sequences. In one embodiment, at least 70% identity between sequences. In one embodiment, at least 75% identity between sequences. In one embodiment, at least 80% identity between sequences. In one embodiment, at least 85% identity between sequences. In one embodiment, at least 90% identity between sequences. In a further embodiment, polynucleotides are hybridizable under stringent conditions i.e. having at least 95% identity. In a further embodiment, more than 97% identity.

In a further embodiment, polynucleotides are those illustrated in **SEQ ID NOs : 3, 12, 13, 76,** encoding polypeptides of the invention.

In a further embodiment, polynucleotides are those illustrated in **SEQ ID NO :3,** encoding polypeptides of the invention.

In a further embodiment, polynucleotides are those illustrated in **SEQ ID NO :12,** encoding polypeptides of the invention.

In a further embodiment, polynucleotides are those illustrated in **SEQ ID NO :13,** encoding polypeptides of the invention.

In a further embodiment, polynucleotides are those illustrated in **SEQ ID NO :76,** encoding polypeptides of the invention.

As will be readily appreciated by one skilled in the art, polynucleotides include both DNA and RNA.

The present invention also includes polynucleotides complementary to the polynucleotides described in the present application.

In a further aspect, polynucleotides encoding polypeptides of the invention, or fragments, analogs or derivatives thereof, may be used in a DNA immunization method. That is, they can be incorporated into a vector which is replicable and expressible upon injection thereby producing the antigenic polypeptide in vivo. For example polynucleotides may be incorporated into a plasmid vector under the control of the CMV promoter which is functional in eukaryotic cells. Preferably the vector is injected intramuscularly.

According to another aspect, there is provided a process for producing polypeptides of the invention by recombinant techniques by expressing a polynucleotide encoding said polypeptide in a host cell and recovering the expressed polypeptide product. Alternatively, the polypeptides can be produced according to established synthetic chemical techniques i.e. solution phase or solid phase synthesis of oligopeptides which are ligated to produce the full polypeptide (block ligation).

General methods for obtention and evaluation of polynucleotides and polypeptides are described in the following references: Sambrook et al, Molecular Cloning: A Laboratory Manual, 2nd ed, Cold Spring Harbor, N.Y., 1989; Current Protocols in Molecular Biology, Edited by Ausubel F.M. et al., John Wiley and Sons, Inc. New York; PCR Cloning Protocols, from Molecular Cloning to Genetic Engineering, Edited by White B.A., Humana Press, Totowa, New Jersey, 1997, 490 pages; Protein Purification, Principles and Practices, Scopes R.K., Springer-Verlag, New York, 3rd Edition, 1993, 380 pages; Current Protocols in Immunology, Edited by Coligan J.E. et al., John Wiley & Sons Inc., New York which are herein incorporated by reference.

For recombinant production, host cells are transfected with vectors which encode the polypeptide, and then cultured in a nutrient media modified as appropriate for activating promoters, selecting transformants or amplifying the genes. Suitable vectors are those that are viable and replicable in the chosen host and include chromosomal, non-chromosomal and synthetic DNA sequences e.g. bacterial plasmids, phage DNA, baculovirus, yeast plasmids, vectors derived from combinations of plasmids and phage DNA. The polypeptide sequence may be incorporated in the vector at the appropriate site using restriction enzymes such that it is operably linked to an expression control region comprising a promoter, ribosome binding site (consensus region or Shine-Dalgarno sequence), and optionally an operator (control element). One can select individual components of the expression control region that are appropriate for a given host and vector according to established molecular biology principles (Sambrook et al, Molecular Cloning: A Laboratory Manual, 2nd ed, Cold Spring Harbor, N.Y., 1989; Current Protocols in Molecular Biology, Edited by Ausubel F.M. et al., John Wiley and Sons, Inc. New York incorporated herein by reference). Suitable promoters include but are not limited to LTR or SV40 promoter, E.coli lac, tac or trp promoters and the phage lambda P_{L} promoter. Vectors will preferably incorporate an origin of replication as well as selection markers i.e. ampicilin resistance gene. Suitable bacterial vectors include pET, pQE70, pQE60, pQE-9, pbs, pD10 phagescript, psiX174, pbluescript SK, pbsks, pNH8A, pNH16a, pNH18A, pNH46A, ptrc99a, pKK223-3, pKK233-3, pDR540, pRIT5 and eukaryotic vectors pBlueBacIII, pWLNEO, pSV2CAT, pOG44, pXT1, pSG, pSVK3, pBPV, pMSG and pSVL. Host cells may be bacterial i.e. E.coli, Bacillus subtilis, Streptomyces; fungal i.e. Aspergillus niger, Aspergillus nidulins; yeast i.e. Saccharomyces or eukaryotic i.e. CHO, COS.

Upon expression of the polypeptide in culture, cells are typically harvested by centrifugation then disrupted by physical or chemical means (if the expressed polypeptide is not secreted into the media) and the resulting crude extract retained to isolate the polypeptide of interest. Purification of the polypeptide from culture media or lysate may be achieved by established techniques depending on the properties of the polypeptide i.e. using ammonium sulfate or ethanol precipitation , acid extraction, anion or cation exchange chromatography, phosphocellulose chromatography, hydrophobic interaction chromatography, hydroxylapatite chromatography and lectin chromatography. Final purification may be achieved using HPLC.

The polypeptide may be expressed with or without a leader or secretion sequence. In the former case the leader may be removed using post-translational processing (see US 4,431,739; US 4,425,437; and US 4,338,397 incorporated herein by reference) or be chemically removed subsequent to purifying the expressed polypeptide.

According to a further aspect, the streptococcus polypeptides of the invention may be used in a diagnostic test for streptococcus infection, in particular S. pneumoniae infection. Several diagnostic methods are possible, for example detecting streptococcus organism in a biological sample, the following procedure may be followed:
a) obtaining a biological sample from a patient;
b) incubating an antibody or fragment thereof reactive with a streptococcus polypeptide of the invention with the biological sample to form a mixture; and
c) detecting specifically bound antibody or bound fragment in the mixture which indicates the presence of streptococcus.

Alternatively, a method for the detection of antibody specific to a streptococcus antigen in a biological sample containing or suspected of containing said antibody may be performed as follows:
a) obtaining a biological sample from a patient;
b) incubating one or more streptococcus polypeptides of the invention or fragments thereof with the biological sample to form a mixture; and
c) detecting specifically bound antigen or bound fragment in the mixture which indicates the presence of antibody specific to streptococcus.

One of skill in the art will recognize that this diagnostic test may take several forms, including an immunological test such as an enzyme-linked immunosorbent assay (ELISA), a radioimmunoassay or a latex agglutination assay, essentially to determine whether antibodies specific for the protein are present in an organism.

The DNA sequences encoding polypeptides of the invention may also be used to design DNA probes for use in detecting the presence of streptococcus in a biological sample suspected of containing such bacteria. The detection method of this invention comprises:
a) obtaining the biological sample from a patient;
b) incubating one or more DNA probes having a DNA sequence encoding a polypeptide of the invention or fragments thereof with the biological sample to form a mixture; and
c) detecting specifically bound DNA probe in the mixture which indicates the presence of streptococcus bacteria.

The DNA probes of this invention may also be used for detecting circulating streptococcus i.e. S.pneumoniaenucleic acids in a sample, for example using a polymerase chain reaction, as a method of diagnosing streptococcus infections. The probe may be synthesized using conventional techniques and may be immobilized on a solid phase, or may be labelled with a detectable label. A preferred DNA probe for this application is an oligomer having a sequence complementary to at least about 6 contiguous nucleotides of the streptococcus pneumoniae polypeptides of the invention.

Another diagnostic method for the detection of streptococcus in a patient comprises:
a) labelling an antibody reactive with a polypeptide of the invention or fragment thereof with a detectable label;
b) administering the labelled antibody or labelled fragment to the patient; and
c) detecting specifically bound labelled antibody or labelled fragment in the patient which indicates the presence of streptococcus.

A further aspect of the invention is the use of the streptococcus polypeptides of the invention as immunogens for the production of specific antibodies for the diagnosis and in particular the treatment of streptococcus infection. Suitable antibodies may be determined using appropriate screening methods, for example by measuring the ability of a particular antibody to passively protect against streptococcus infection in a test model. One example of an animal model is the mouse model described in the examples herein. The antibody may be a whole antibody or an antigen-binding fragment thereof and may belong to any immunoglobulin class. The antibody or fragment may be of animal origin, specifically of mammalian origin and more specifically of murine, rat or human origin. It may be a natural antibody or a fragment thereof, or if desired, a recombinant antibody or antibody fragment. The term recombinant antibody or antibody fragment means antibody or antibody fragment which was produced using molecular biology techniques. The antibody or antibody fragments may be polyclonal, or preferably monoclonal. It may be specific for a number of epitopes associated with the streptococcus pneumoniae polypeptides but is preferably specific for one.

Without limiting its scope, the present invention also relates to new antigens designated BVH-3, BVH-11, BVH-11-2, BVH-28 and BVH-71. The present invention also relates to truncated polypeptides comprising fragments of the new antigens designated BVH-3, BVH-11, BVH-11-2, BVH-28 and BVH-71. The present invention also relates to chimeric polypeptides comprising fragments of the new antigens designated BVH-3, BVH-11, BVH-11-2, BVH-28 and BVH-71. The following is a reference table summarizing the relation between the antigens of the present invention:

| **Family** | **Nucleotide SEQ ID NO** | **Polypeptide SEQ ID NO** |
|---|---|---|
| BVH-3 | | |
| BVH-3 | 1, 11 | 2 |
| BVH-3A | 7 | 8 |
| BVH-3B | 9 | 10 |
| BVH-3 SP63 | 15 | 16 |
| BVH-3M | | 55 |
| BVH-3AD | | 56 |
| L-BVH-3AD | | 57 |
| Newl2 | 76 | 58 |
| BVH-3C | | 59 |
| New1 | | 64 |
| New2 | | 65 |
| New3 | | 66 |
| New15 | | 78 |
| BVH-11 | | |
| BVH-11-1 | 3, 12 | 4 |
| BVH-11-2 | 13 | 14 |
| BVH-11M | | 60 |
| BVH-11A | | 61 |
| BVH-11B also referred to as NEW13 | | 62 |
| BVH-11C | | 63 |
| New4 | | 67 |
| New5 | | 68 |
| New6 | | 69 |
| New7 | | 70 |
| New8 | | 71 |
| New9 | | 72 |
| BVH-11-2M | | 73 |
| New10 | | 74 |
| New11 | | 75 |
| New12 | 76 | 58 |
| New14 | | 77 |
| New16 | | 79 |
| BVH-28 | | |
| BVH-28 | 5 | 6 |
| BVH-71 | | |
| GBS | 80 | 81 |
| GAS | 82 | 83 |

The invention contains the following embodiments:
1. An isolated polynucleotide encoding a polypeptide having at least 95% identity to a second polypeptide having a sequence chosen from: **SEQ ID NOs: 14, 4, 58, 60, 61, 63, 73 to 75, 77, 79** or fragments, analogs or derivatives thereof.
2. An isolated polynucleotide encoding a polypeptide capable of generating antibodies having binding specificity for a polypeptide having a sequence chosen from: **SEQ ID NOs: 14, 4, 58, 60 to 63, 67, 68, 73 to 75, 77, 79** or fragments, analogs or derivatives thereof.
3. An isolated polynucleotide that is complementary to the polynucleotide of embodiment 1 or 2.
4. A vector comprising the polynucleotide of embodiment 1 or 2, wherein said DNA is operably linked to an expression control region.
5. A host cell transfected with a vector of embodiment 4.
6. A process of producing a polypeptide comprising culturing a host cell according to embodiment 5 under conditions suitable for expression of said polypeptide.
7. An isolated polypeptide having at least 95% identity to a second polypeptide having an amino acid sequence chosen from: **SEQ ID NOs: 14, 4, 58, 60, 61, 63, 73 to 75, 77, 79** or fragments, analogs or derivatives thereof.
8. An isolated polypeptide capable of generating antibodies having binding specificity for a second polypeptide having a sequence chosen from: **SEQ ID NOs: 14, 4, 58, 60 to 63, 67, 68, 73 to 75, 77, 79** or fragments, analogs or derivatives thereof.
9. An isolated polypeptide having an amino acid sequence chosen from **SEQ ID NOs: 14, 4, 58, 60 to 63, 67, 68, 73 to 75, 77, 79** or fragments, analogs or derivatives thereof.
10. An isolated polypeptide according to embodiment 9, wherein the N-terminal Met residue is deleted.
11. An isolated polypeptide according to embodiment 9, wherein the secretory amino acid sequence is deleted.
12. A chimeric polypeptide comprising two or more polypeptides chosen from **SEQ ID NOs: 14, 4, 58, 60 to 63, 67, 68, 73 to 75, 77, 79** or fragments, analogs or derivatives thereof; provided that the polypeptides, or fragments, analogs or derivatives thereof are linked as to form a chimeric polypeptide.
13. A chimeric polypeptide comprising two or more polypeptides chosen from **SEQ ID NOs: 58, 60, 62, 67, 68, 77** or fragments, analogs or derivatives thereof; provided that the polypeptides, or fragments, analogs or derivatives thereof are linked as to form a chimeric polypeptide.
14. A chimeric polypeptide of formula (I):

   **A-(B)ₘ-(C)ₙ-D** (I)

   Wherein;
   **m** is 0 or 1,
   **n** is 0 or 1,
   **A** is chosen from **SEQ ID NOs: 14, 4, 58, 60 to 63, 67, 68, 73 to 75, 77, 79** or fragments, analogs or derivatives thereof;
   **B** is chosen from **SEQ ID NOs: 14, 4, 58, 60 to 63, 67, 68, 73 to 75, 77, 79** or fragments, analogs or derivatives thereof;
   **C** is chosen from **SEQ ID NOs: 14, 4, 58, 60 to 63, 67, 68, 73 to 75, 77, 79** or fragments, analogs or derivatives thereof; and
   **D** is chosen from **SEQ ID NOs: 14, 4, 58, 60 to 63, 67, 68, 73 to 75, 77, 79** or fragments, analogs or derivatives thereof.
15. A chimeric polypeptide of formula (I):

   **A-(B)ₘ-(C)ₙ-D** (I)

   Wherein;
   **m** is 0 or 1,
   **n** is 0 or 1,
   **A** is chosen from **SEQ ID NOs: 58, 60, 62, 67, 68, 77** or fragments, analogs or derivatives thereof;
   **B** is chosen from **SEQ ID NOs: 58, 60, 62, 67, 68, 77** or fragments, analogs or derivatives thereof;
   **C** is chosen from **SEQ ID NOs: 58, 60, 62, 67, 68, 77** or fragments, analogs or derivatives thereof; and
   **D** is chosen from **SEQ ID NOs: 58, 60, 62, 67, 68, 77** or fragments, analogs or derivatives thereof.
16. A vaccine composition comprising a polypeptide according to any one of embodiments 7 to 15 and a pharmaceutically acceptable carrier, diluent or adjuvant.
17. Use of a vaccine composition according to embodiment 16 for the prophylactic or therapeutic treatment of Streptococcal infection in an animal susceptible to or infected with streptococcal infection comprising administering to said animal a prophylactic or therapeutic amount of the composition.

### EXAMPLE 1

This example illustrates the cloning of S. pneumoniae genes.

The coding region of S. pneumoniae gene BVH-3 (**SEQ ID NO: 1**) and the coding region of S. pneumoniae gene BVH-28 (**SEQ ID NO: 5)** were amplified by PCR (DNA Thermal Cycler GeneAmp PCR system 2400 Perkin Elmer, San Jose, CA) from genomic DNA of serogroup 6 S. pneumoniae strain SP64 using the oligos that contained base extensions for the addition of restriction sites BglII (AGATCT) and XbaI (TCTAGA). PCR products were purified from agarose gel using a QIAquick gel extraction kit from QIAgen (Chatsworth, CA), digested BglII-XbaI (Pharmacia Canada Inc, Baie d'Urfé, Canada), extracted with phenol : chloroform and precipitated with ethanol. The Superlinker vector pSL301 (Invitrogen, San Diego, CA) was digested with BglII and XbaI and purified from agarose gel using a QIAquick gel extraction kit from QIAgen (Chatsworth, CA). The BglII-XbaI genomic DNA fragments were ligated to the BglII-XbaI pSL301 vector. The ligated products were transformed into E. coli strain DH5a [f80 *lac*Z DM15 *end*A1 *rec*A1 *hsd*R17 (^{r}K^{-m}K⁺) *sup*E44 *thi*-11⁻ *gyr*A96 *rel*A1 D(*lac*ZYA-*arg*F)U169] (Gibco BRL, Gaithersburg, MD) according to the method of Simanis (Hanahan, D. DNA Cloning, 1985, D.M. Glover (ed), pp. 109-135). Recombinant pSL301 plasmids (rpSL301) containing either BVH-3 or BVH-28 gene were purified using a QIAgen kit (Chatsworth, CA) and DNA inserts were confirmed by nucleotide sequence analysis (Taq Dye Deoxy Terminator Cycle Sequencing kit, ABI, Foster City, CA). Recombinant rpSL301 (rpSL301) were digested with the restriction enzymes BglII (AGATCT) and XhoI (CTCGAG). DNA fragments BglII-XhoI were purified using the QIAquick gel extraction kit from QIAgen (Chatsworth, CA). pET-32c(+) expression vector (Novagen, Madison, WI) containing the thioredoxin-His·Tag sequence was digested with BamHI (GGATCC) and XhoI and gel extracted using the QIAquick gel extraction kit from QIAgen (Chatsworth, CA). The BglII-XhoI DNA fragments were ligated to the BamHI-XhoI pET-32c(+) vector to create the coding sequence for thioredoxin-His·Tag-BVH-3 or thioredoxin-His·Tag-BVH-28 fusion protein. The ligated products were transformed into E. coli strain DH5a [f80 *lac*Z DM15 *end*A1 *rec*A1 *hsd*R17 (^{r}K^{-m}K⁺) *sup*E44 *thi*-11⁻ *gyr*A96 *rel*A1 D(*lac*ZYA-argF)U169] (Gibco BRL, Gaithersburg, MD) according to the method of Simanis (Hanahan, D. DNA Cloning, 1985, D.M. Glover (ed), pp. 109-135). Recombinant pET-32c(+) plasmids were purified using a QIAgen kit (Chatsworth, CA) and the nucleotide sequences at the fusion sites of thioredoxin-His-Tag and DNA insert were verified by DNA sequencing (Taq Dye Deoxy Terminator Cycle Sequencing kit, ABI, Foster City, CA).

### EXAMPLE 2

This example illustrates the cloning of S. pneumoniae protein genes in CMV plasmid pCMV-GH.

The DNA coding region of a S. pneumoniae protein was inserted in phase downstream of a human growth hormone (hGH) gene which was under the transcriptional control of the cytomegalavirus (CMV) promotor in the plasmid vector pCMV-GH (Tang et al., Nature, 1992, 356 :152). The CMV promotor is non functional plasmid in E. coli cells but active upon administration of the plasmid in eukaryotic cells. The vector also incorporated the ampicillin resistance gene.

The coding region of BVH-3 gene (**SEQ ID NO: 1**) and BVH-28 gene (**SEQ ID NO: 5**) were obtained from rpSL301 (see example 1) using restriction enzymes BglII (AGATCT) and XbaI (TCTAGA). The digested products were purified from agarose gel using the QIAquick gel extraction kit from QIAgen (Chatsworth, CA). The pCMV-GH vector (Laboratory of Dr. Stephen A. Johnston, Department of Biochemistry, The University of Texas, Dallas, Texas) containing the human growth hormone to create fusion proteins was digested with BglII and XbaI and purified from agarose gel using the QIAquick gel extraction kit from QIAgen (Chatsworth, CA). The BglII-XbaI DNA fragments were ligated to the BglII-XbaI pCMV-GH vector to create the hGH-BVH-3 or hGH-BVH-28 fusion protein under the control of the CMV promoter. The ligated products were transformed into E. coli strain DH5a[f80 *lac*Z DM15 *end*A1 *rec*A1 *hsd*R17 (^{r}K^{-m}K⁺) *sup*E44 *thi*-11⁻ *gyr*A96 *rel*A1 D(*lac*ZYA-*arg*F)U169] (Gibco BRL, Gaithersburg, MD) according to the method of Simanis (Hanahan, D. DNA Cloning, 1985, D.M. Glover (ed), pp. 109-135). The recombinant pCMV plasmids were purified using a QIAgen kit (QIAgen, Chatsworth, CA).

The coding region of BVH-11 gene (**SEQ ID NO: 3**) was amplified by PCR (DNA Thermal Cycler GeneAmp PCR system 2400 Perkin Elmer, San Jose, CA) from genomic DNA of serogroup 6 S. pneumoniae strain SP64 using the oligos that contained base extensions for the addition of restriction sites BglII (AGATCT) and HindIII (AAGCTT). The PCR product was purified from agarose gel using a QIAquick gel extraction kit from QIAgen (Chatsworth, CA), digested with restriction enzymes (Pharmacia Canada Inc, Baie d'Urfe, Canada), extracted with phenol : chloroform and precipitated with ethanol. The pCMV-GH vector (Laboratory of Dr. Stephen A. Johnston, Department of Biochemistry, The University of Texas, Dallas, Texas) was digested with BglII and HindIII and purified from agarose gel using the QIAquick gel extraction kit from QIAgen (Chatsworth, CA). The BglII-HindIII DNA fragment was ligated to the BglII-HindIII pCMV-GH vector to create the hGH-BVH-11 fusion protein under the control of the CMV promoter. The ligated products were transformed into E. coli strain DH5a [f80 *lac*Z DM15 *end*A1 *rec*A1 *hsd*R17 (^{r}K^{-m}K⁺) *sup*E44 *thi*-11⁻ *gyr*A96 *rel*A1 D(*lac*ZYA-argF)U169] (Gibco BRL, Gaithersburg, MD) according to the method of Simanis (Hanahan, D. DNA Cloning, 1985, D.M. Glover (ed), pp. 109-135). The recombinant pCMV plasmid was purified using a QIAgen kit (Chatsworth, CA) and the nucleotide sequence of the DNA insert was verified by DNA sequencing.

### EXAMPLE 3

This example illustrates the use of DNA to elicit an immune response to S. pneumoniae antigens.

A group of 8 female BALB/c mice (Charles River, St-Constant, Québec, Canada) were immunized by intramuscular injection of 50 µl three times at two- or three-week intervals with 100 µg of recombinant pCMV-GH encoding the BVH-3, BVH-11 or the BVH-28 gene in presence of 50 µg of granulocyte-macrophage colony-stimulating factor (GM-CSF)- expressing plasmid pCMV-GH-GM-CSF (Laboratory of Dr. Stephen A. Johnston, Department of Biochemistry, The University of Texas, Dallas, Texas). As control, a group of mice were injected with 100 µg of pCMV-GH in presence of 50 µg of pCMV-GH-GM-CSF. Blood samples were collected from the orbital prior to each immunization and seven days following the third injection and serum antibody responses were determined by ELISA using thioredoxin-His-Tag-S. pneumoniae fusion protein as coating antigen. DNA immunization with recombinant plasmid pCMV-GH encoding the BVH-3, BVH-11 or the BVH-28 S. pneumoniae protein induced antibody reactive against the respective recombinant protein. The reciprocal antibody titers, defined as the highest serum dilution at which the absorbance values were 0.1 above the background values, were above 4x10³.

### EXAMPLE 4

This example illustrates the production and purification of recombinant S. pneumoniae proteins.

The recombinant pET plasmids containing the BVH-3, BVH-11 or the BVH-28 gene corresponding to the **SEQ ID NO: 1, SEQ ID NO: 3** or the **SEQ ID NO: 5** respectively were transformed by electroporation (Gene. Pulser II apparatus, BIO-RAD Labs, Mississauga, Canada) into E. coli strain AD494 (DE3) *(Dara⁻ leu*7697 D*lac*X74 D*pho*A *Pvu*II *pho*R D*mal*F3 F' [*lac*⁺(*lac*I^{q}) *pro*] *trx*B::Kan) (Novagen, Madison, WI). In this strain of E. coli, the T7 promotor controlling expression of the fusion protein is specifically recognized by the T7 RNA polymerase (present on the lDE3 prophage) whose gene is under the control of the lac promotor which is inducible by isopropyl-ß-d-thio-galactopyranoside (IPTG). The transformant AD494(DE3)/rpET was grown at 37°C with agitation at 250 rpm in LB broth (peptone 10g/L, yeast extract 5g/L, NaCl 10g/L) containing 100µg of ampicillin (Sigma-Aldrich Canada Ltd., Oakville, Canada) per ml until the A₆₀₀ reached a value of 0.6. In order to induce the production of the thioredoxin-His·Tag-BVH-3, thioredoxin-His·Tag-BVH-11 or thioredoxin-His·Tag-BVH-28 fusion protein, the cells were incubated for 2 additional hours in the presence of IPTG at a final concentration of 1 mM. Induced cells from a 100 ml culture were pelleted by centrifugation and frozen at -70°C.

The purification of the fusion proteins from the soluble cytoplasmic fraction of IPTG-induced AD494(DE3)/rpET was done by affinity chromatography based on the properties of the His·Tag sequence (6 consecutive histidine residues) to bind to divalent cations (Ni²⁺) immobilized on the His·Bind metal chelation resin. Briefly, the pelleted cells obtained from a 100mL culture induced with IPTG were resuspended in phosphate-buffered (PBS):500mM NaCl pH7.1, sonicated and spun at 20,000 X g for 20 min to remove debris. The supernatant was filtered (0.22µm pore size membrane) and deposited on a HiTrap® 1mL chelating pre-packed ready-to-use column (Pharmacia Biotech, Baie d'Urfé, Canada). The thioredoxin-His-Tag-S. pneumoniae fusion protein was eluted with 1M imidazole-500mM NaCl-PBS pH7.1. The removal of the salt and imidazole from the sample was done by dialysis against PBS at 4°C. The quantities of fusion protein obtained from the soluble fraction of E. coli was estimated by MicroBCA (Pierce, Rockford, Illinois).

### EXAMPLE 5

This example illustrates the protection of mice against fatal pneumococcal infection by immunization.

Groups of 8 female BALB/c mice (Charles River) were immunized subcutaneously three times at three-week intervals with either 25 µg of affinity purified thioredoxin-His-Tag-BVH-3 fusion protein in presence of 15 µg of QuilA adjuvant (Cedarlane Laboratories Ltd, Hornby, Canada) or, as control, with QuilA adjuvant alone in PBS. Blood samples were collected from the orbital sinus on day 1, 22 and 43 prior to each immunization and seven days (day 50) following the third injection. One week later the mice were challenged with approximately 10⁶ CFU of the type 3 S. pneumoniae strain WU2. Samples of the S. pneumoniae challenge inoculum were plated on chocolate agar plates to determine the CFU and to verify the challenge dose. Deaths were recorded for a period of 14 days and on day 14 post-challenge, the surviving mice were sacrificied and blood samples tested for the presence of S. pneumoniae organisms. The survival data are shown in table 1.

Prechallenge sera were analyzed for the presence of antibodies reactive with S. pneumoniae by standard immunoassays. Elisa and immunoblot analyses indicated that immunization with recombinant S. pneumoniae protein produced in E. coli elicited antibodies reactive with both, recombinant and native pneumococcal protein.

**Table 1. Protection mediated by recombinant BVH-3 protein**

| Immunogen | No. of mice alive : no. of mice dead | Median day of death |
|---|---|---|
| | 14 days post-challenge | |
| BVH-3 | 8 : 0 | >14 |
| none | 0 : 8 | 1 |

All mice immunized with BVH-3 recombinant protein survived to infection while none of the control mice given adjuvant alone survived. There was a significant difference in survival between the two groups of mice (P<0.0001, log rank test for nonparametric analysis of survival curves; P=0.0002, Fisher's exact test). All hemocultures from surviving mice were negative at day 14 post-challenge.

### EXAMPLE 6

This example describes the cloning of BVH-3 and BVH-11 genes from a variety of S. pneumoniae strains and the molecular conservation of these genes.

Molecular analysis of chromosomal DNA from various S. pneumoniae isolates with DNA probes spanning different regions of BVH-3 or BVH-11 revealed the presence of one BVH-3 gene copy and two BVH-11 gene copies. The two BVH-11 gene copies are not identical and the genes were arbitrarily designated BVH-11 (SEQ ID NO:12; ORF at nucleotides 45 to 2567) and BVH-11-2 (SEQ ID NO:13; ORF at nucleotides 114 to 2630).

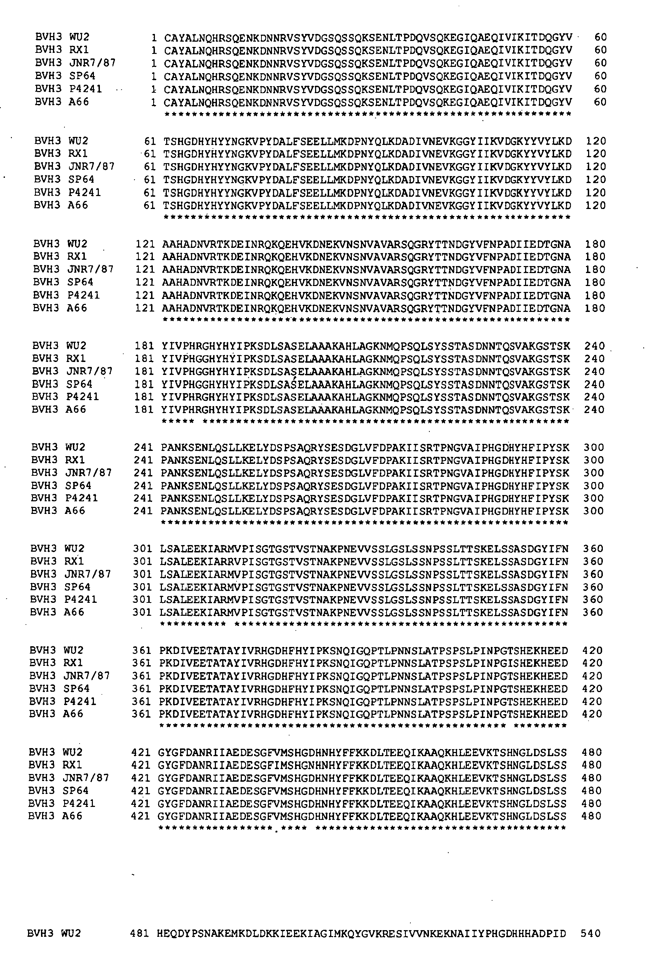

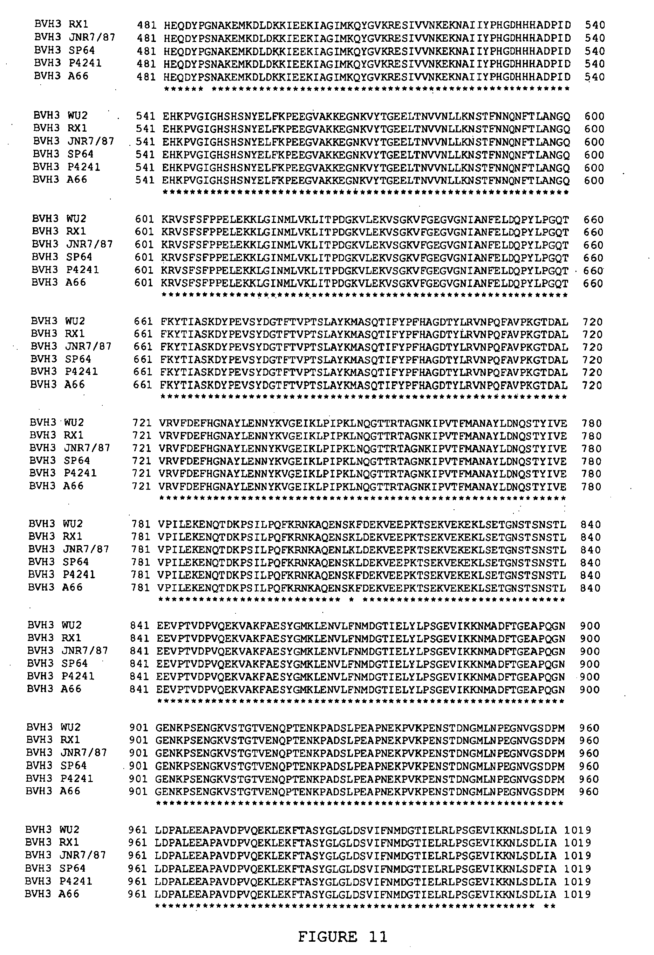

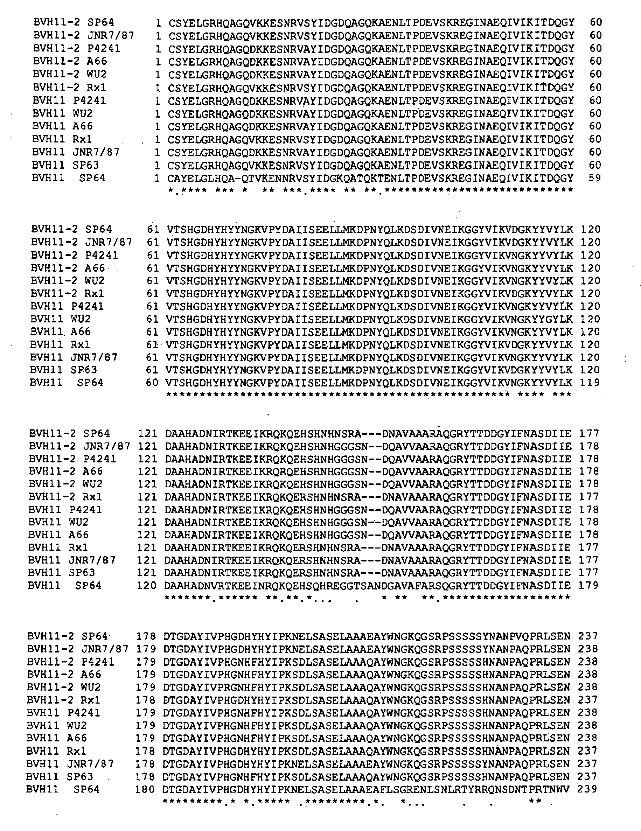

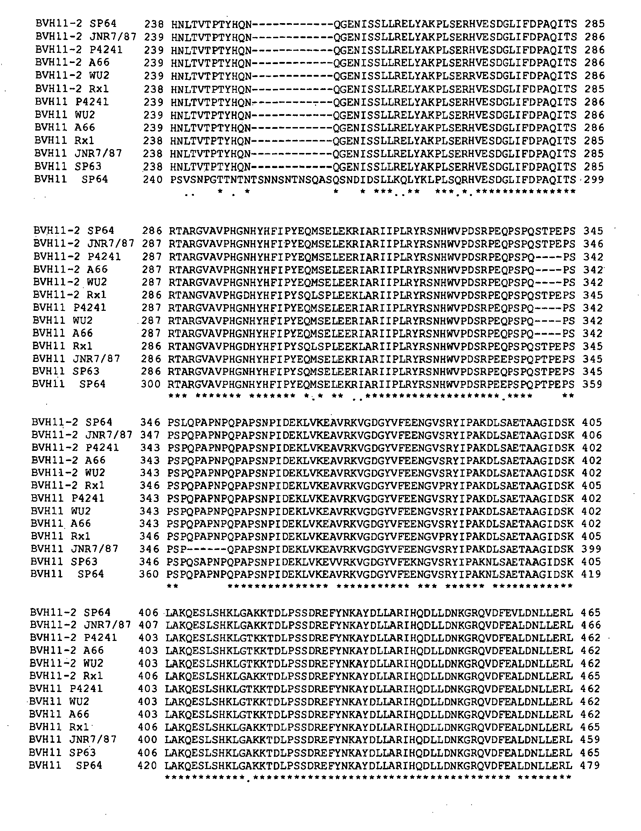

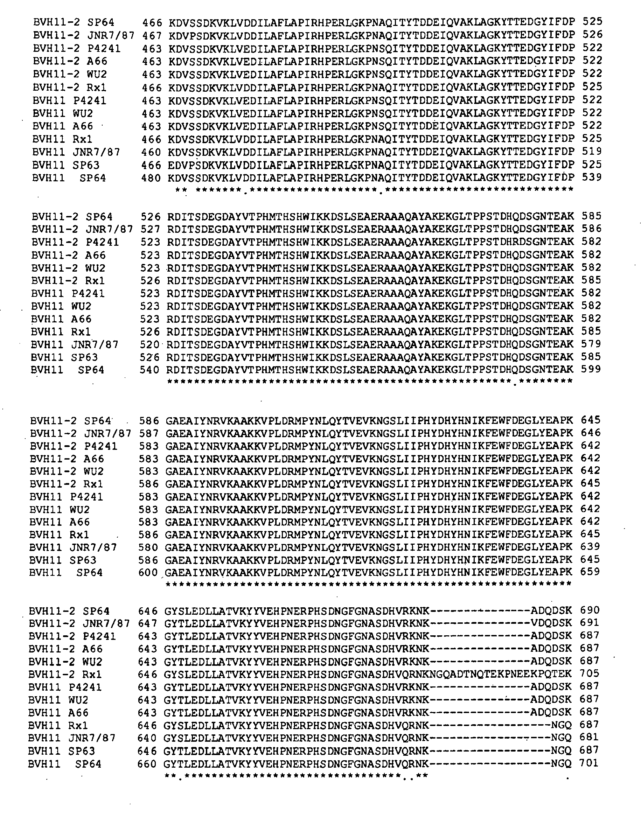

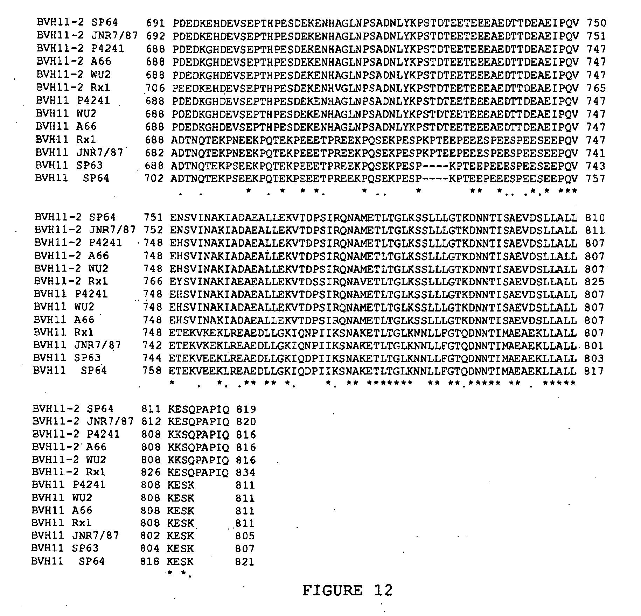

The first amino acids of the BVH-3 and BVH-11 coding regions have the characteristics of leader sequences also known as signal peptides. The consensus signal peptidase cleavage site L-X-X-C of lipoprotein modification/processing sites was present in the sequences. Mature BVH-3, BVH-11 and BVH-11-2 proteins from S. pneumoniae SP64 have 1019, 821 and 819 amino acids, respectively. The regions of S. pneumoniae genes coding for mature BVH-3, termed BVH-3M, (nucleotides 1837 - 4896; SEQ. ID. NO: 11), BVH-11M (nucleotides 102-2567; SEQ. ID. NO: 12) and BVH-11-2M (nucleotides 171-2630; SEQ. ID. NO: 13), were amplified by PCR(DNA Thermal Cycler GeneAmp PCR system 2400 Perkin Elmer, San Jose, CA) from genomic DNA of 6 or 7 S. pneumoniae strains. Serogroup 6 S. pneumoniae SP64 and serogroup 9 SP63 clinical isolates were provided by the laboratoire de la santé publique du Québec, Sainte-Anne-de-Bellevue; serotype 4 strain JNR.7/87 was provided by Andrew Camilli, Tufts University School of Medicine, Boston; Rx1 strain, a nonencapsulated derivative of the type 2 strain D39 and the type 3 strains A66 and WU2 were provided by David E. Briles from University of Alabama, Birmingham and the type 3 clinical isolate P4241 was provided by the centre de recherche en infectiologie du centre hospitalier de l'université Laval, Sainte-Foy. The sets of oligonucleotide primers OCRR479-OCRR480; HAMJ160-OCRR488 and HAMJ160-HAMJ186, that contained base extensions for the addition of restriction sites were used for the amplification of BVH-3, BVH-11 and BVH-11-2 gene, respectively, with the exception of BVH-11 gene from SP64 strain which was amplified using the set of primers consisting of HAMJ487 and OCRR488. Primer sequences are listed below (Table 2). PCR products were purified from agarose gel using a QIAquick gel extraction kit from QIAgen (Chatsworth, CA) and digested BglII-XbaI or BglII-HindIII (Pharmacia Canada Inc, Baie d'Urfé, Canada). Digestions were cleaned using a QIAquick PCR purification kit from QIAgen (Chatsworth, CA). The PCR products were ligated to the BglII-XbaI or BglII-HindIII pSL301 vector. The ligated products were transformed into E. coli strain DH5α [φ80 *lac*Z ΔM15 *end*A1 *rec*A1 *hsd*R17 (^{r}K^{-m}K⁺) *sup*E44 *thi*-1λ⁻ *gyr*A96 *rel*A1 Δ(*lac*ZYA-*arg*F)U169] (Gibco BRL, Gaithersburg, MD) according to the method of Simanis (Hanahan, D. DNA Cloning, 1985, D.M. Glover (ed), pp. 109-135). Recombinant pSL301 plasmids (rpSL301) containing BVH-3, BVH-11 or BVH11-2 were purified using a QIAgen.kit (Chatsworth, CA) and DNA inserts were sequenced (Taq Dye Deoxy Terminator Cycle Sequencing kit, ABI, Foster City, CA). The figures 11 and 12 depict the consensus sequence established from the BVH-3, and BVH-11 deduced amino acid sequences, respectively. Comparison of BVH-3 protein sequences revealed 99 to 100% identity of sequences for all strains with the exception that BVH-3 from serogroup 9.SP63 strain (SEQ. ID. NO: 15 and SEQ. ID. NO: 16) misses a stretch of 177 amino acids corresponding to residues 244 to 420 on BVH-3 protein sequence of S. pneumoniae SP64. Analysis of sequences of additional serogroup 9 strains revealed BVH-3 molecule having the same deletion in 3 out of 4 strains thus suggesting that the 3 strains are members of a S. pneumoniae serogroup 9 clone.

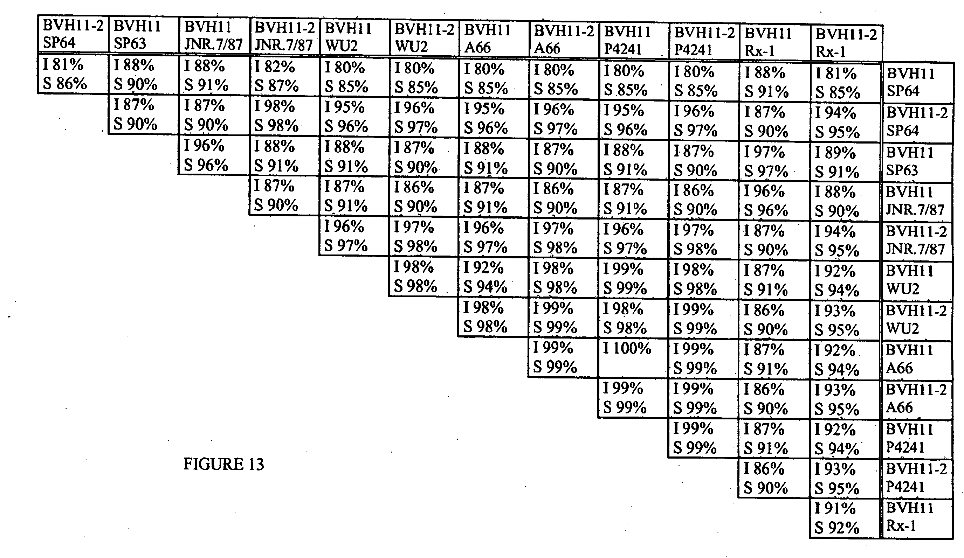

Comparison of 13 BVH-11 nucleotide sequences obtained from 7 S. pneumoniae strains, revealed that the nucleotide sequences are very similar. Computer analysis (MacVector, Clustal W 1.4) using multiple alignment of the predicted BVH-11 protein sequences revealed that these sequences were 75% identical and 82 % homologous on a length of 834 amino acids. Pairwise alignment revealed 80 to 100% identity (Figure 13). The sequences showed great similarity in overall organization. Variability in the primary sequence of these proteins is almost restricted to the last 125 amino acids in the C-terminal portion of the proteins. This region constitutes a domain. Close examination of this domain revealed two groups of sequences. The first 9 sequences from the figure 13 belong to one group while the last 4 sequences belong to another group. A 39% identity value is obtained when the domain sequences of the 13 proteins are compared (MacVector, Clustal W 1.4). The identity value increased to more than 92% when sequences belonging to a same group are compared.

### EXAMPLE 7

This example illustrates the homology of portions of BVH-3 and BVH-11 genes.

Molecular analysis with DNA probes derived from BVH-3 and BVH-11 genes indicated that BVH-3 and BVH-11 were related. In dot blot hybridization studies, DNA probe consisting of either, BVH-3 or BVH-11, gene sequence hybridized to both, BVH-3 and BVH-11 genes thus indicating that BVH-3 and BVH-11 genes shared homologous sequences. Comparison of sequences revealed that the ORFs and the proteins were 43 and 33% identical, respectively. Closer examination revealed that the region corresponding to amino acids 1 to 225 in BVH-3 and 1 to 228 in BVH-11 were 73 and 75% identical at the DNA and protein level, respectively. In contrast, the 3' regions corresponding to amino acids 226 to 1039 from BVH-3 and amino acids 229-840 from BVH-11 were only 34 and 22% identical at the DNA and protein level, respectively. Thus the 5' termini of BVH-3 and BVH-11 genes appear to contain highly conserved sequences while the remaining parts of the genes are highly divergent. These results suggest that BVH-3 and BVH-11 might share similar functions mediated by sequences present in the conserved region whereas BVH-3- and BVH-11-specific functions might be mediated by sequences in the divergent region.

### EXAMPLE 8

This example describes the cloning of truncated BVH-3, BVH-11 and BVH-11-2 genes by polymerase chain reaction (PCR) and the expression of truncated BVH-3 and BVH-11 molecules.

Gene fragments were amplified by PCR using pairs of oligonucleotide engineered to amplify fragments spanning the BVH-3 (**SEQ ID NO: 1** and **SEQ ID NO: 11**), BVH-11 (**SEQ ID NO: 3** and **SEQ ID NO: 12**) or BVH-11-2 (**SEQ ID NO: 13**) gene from S. pneumoniae strain SP64. Each of the primers had a restriction endonuclease site at the 5' end, thereby allowing directional in-frame cloning of the amplified product into the digested plasmid vector (Tables 2 and 3). PCR-amplified products were digested with restriction endonucleases and ligated to either linearized plasmid pSL301 (see example 1), pCMV-GH (see example 2) or pET (Novagen, Madison, WI) expression vector digested likewise or digested with enzymes that produce compatible cohesive ends. Recombinant pSL301 and recombinant pCMV-GH plasmids were digested with restriction enzymes for the in-frame cloning in pET expression vector. Clones were first stabilized in E. coli DH5α before introduction into E. coli BL21 (λDE3) or AD494 (λDE3) for expression of truncated BVH-3 or BVH-11 molecules. Each of the resultant plasmid constructs was confirmed by nucleotide sequence analysis. The recombinant proteins were expressed as N-terminal fusions with the thioredoxin and His-tag or as C-terminal fusions with an His-tag. The expressed recombinant proteins were purified from supernatant fractions obtained from centrifugation of sonicated IPTG-induced E. coli cultures using a His-Bind metal chelation resin (QIAgen, Chatsworth, CA). The gene products generated are listed in the table 3. The gene products corresponding to the N-terminal region including the signal sequence are designated as Lipidated-proteins or lipoproteins (L-proteins). The gene products corresponding to the N-terminal region lacking the signal sequence are identified as protein without signal sequence (w/o ss).

**Table 2. List of PCR oligonucleotide primers**

| Primer | SEQ. ID. | Sequence 5'-3' | Nucleotide position | Restriction sites |
|---|---|---|---|---|
| OCRR 479 | 17 | cagtagatctgtgcctatgcactaaac | SEQ ID 1:61-78 | BglII |
| OCRR 480 | 18 | gatctctagactactgctattccttacgctatg | SEQ ID 11:4909-4887 | XbaI |
| OCRR 497 | 19 | atcactcgagcattacctggataatcctgt | SEQ ID 1 :1525-1506 | XhoI |
| OCRR 498 | 20 | ctgctaagcttatgaaagatttagat | SEQ ID 1 :1534-1548 | HindIII |
| OCRR 499 | 21 | gatactcgagctgctattccttac | SEQ ID 11 :4906-4893 | XhoI |
| HAMJ 172 | 22 | gaatctcgagttaagctgctgctaattc | SEQ ID 1 : 675-661 | XhoI |
| HAMJ 247 | 23 | gacgctcgagcgctatgaaatcagataaattc | SEQ ID 1 :3117-3096 | XhoI |
| HAMJ 248 | 24 | gacgctcgagggcattacctggataatcctgttcatg | SEQ ID 1 : 1527-1501 | XhoI |
| HAMJ 249 | 25 | cagtagatctcttcatcatttattgaaaagagg | SEQ ID 11 : 1749-1771 | BglII |
| HAMJ 278 | 26 | ttatttcttccatatggacttgacagaagagcaaattaag | SEQ ID 1 :1414-1437 | NdeI |
| HAMJ 279 | 27 | cgccaagcttcgctatgaaatcagataaattc | SEQ ID 1 :3117-3096 | HindIII |
| HAMJ 280 | 28 | cgccaagcttttccacaatataagtcgattgatt | SEQ ID 1 :2400-2377 | HindIII |
| HAMJ 281 | 29 | ttatttcttccatatggaagtacctatcttggaaaaagaa | SEQ ID 1 :2398-2421 | NdeI |
| HAMJ 300 | 30 | ttatttcttccatatggtgcctatgcactaaaccagc | SEQ ID 1 :62-82 | NdeI |
| HAMJ 313 | 31 | ataagaatgcggccgettccacaatataagtcgattgatt | SEQ ID 1 :2400-2377 | NotI |
| OCRR 487 | 32 | cagtagatctgtgcttatgaactaggtttgc | SEQ ID 3 :58-79 | BglII |
| OCRR 488 | 33 | gatcaagcttgctgctacctttacttactctc | SEQ ID 12 :2577-2556 | HindIII |
| HAMJ 171 | 34 | ctgagatatccgttatcgttcaaacc | SEQ ID 3 :1060-1075 | EcoRV |
| HAMJ 251 | 35 | ctgcaagcttttaaaggggaataatacg | SEQ ID 3 :1059-1045 | HindIII |
| HAMJ 264 | 36 | cagtagatctgcagaagccttcctatctg | SEQ ID 3 :682-700 | BglII |
| HAMJ 282 | 37 | tcgccaagcttcgttatcgttcaaaccattggg | SEQ ID 3 :1060-1081 | HindIII |
| HAMJ 283 | 38 | | SEQ ID 3 :2520-2492 | NdeI |
| HAMJ 284 | 39 | catgccatggacattgatagtctcttgaaacagc | SEQ ID 3 :856-880 | NcoI |
| HAMJ 285 | 40 | cgccaagcttcttactctcctttaataaagccaatag | SEQ ID 3 :2520-2494 | HindIII |
| HAMJ 286 | 41 | cgacaagcttaacatggtcgctagcgttacc | SEQ ID 3 :2139-2119 | HindIII |
| HAMJ 287 | 42 | cataccatgggcctttatgaggcacctaag | SEQ ID 3 :2014-2034 | NcoI |
| HAMJ 288 | 43 | cgacaagcttaagtaaatcttcagcctctctcag | SEQ ID 3 :2376-2353 | HindIII |
| HAMJ 289 | 44 | gataccatggctagcgaccatgttcaaagaa | SEQ ID 3 :2125-2146 | NcoI |
| HAMJ 290 | 45 | cgccaagcttatcatccactaacttgactttatcac | SEQ ID 3 :1533-1508 | HindIII |
| HAMJ 291 | 46 | cataccatggatattcttgccttcttagctccg | SEQ ID 3 :1531-1554 | NcoI |
| HAMJ 301 | 47 | catgccatggtgcttatgaactaggtttgc | SEQ ID 3 :59-79 | NcoI |
| HAMJ 302 | 48 | cgccaagctttagcgttaccaaaaccattatc | SEQ ID 3 :2128-2107 | HindIII |
| HAMJ 160 | 49 | gtattagatctgttcctatgaacttggtcgtcacca | SEQ ID 13 : 172-196 | BglII |
| HAMJ 186 | 50 | cgcctctagactactgtataggagccgg | SEQ ID 13: 2460-2443 | XbaI |
| HAMJ 292 | 51 | catgccatggaaaacatttcaagccttttacgtg | SEQ ID 11: 754-778 | NcoI |
| HAMJ 293 | 52 | cgacaagcttctgtataggagccggttgactttc | SEQ ID 11 : 2457-2434 | HindIII |
| HAMJ 294 | 53 | catgccatggttcgtaaaaataaggcagaccaag | SEQ ID 11 : 2038-2062 | NcoI |
| HAMJ 297 | 54 | catgccatggaagcctattggaatgggaag | SEQ ID 11 : 622-642 | NcoI |

**Table 3. Lists of truncated BVH-3 and BVH-11 gene products generated from S. pneumoniae SP64**

| PCR-primer sets | Protein designation | Identification (encoded amino acids) | SEQ. ID.NO. | Cloning vector |
|---|---|---|---|---|
| OCRR479-OCRR480 | BVH-3M | BVH-3 w/o ss (21-1039) | 55 | pSL301 |
| OCRR479-OCRR497 | BVH-3AD | BVH-3 N' end w/o ss (21-509) | 56 | pSL301 |
| HAMJ248-HAMJ249 | L-BVH-3AD | BVH-3 N'end (1-509) | 57 | pET-21(+) |
| OCRR498-OCRR499 | BVH-3B | BVH-3 C'end (512-1039) | 10 | pSL301 |
| OCRR479-HAMJ172 | BVH-3C | BVH-3 N'end w/o ss (21-225) | 59 | pET-32 c(+) |
| OCRR487-OCRR488 | BVH-11M | BVH-11 w/o ss (20-840) | 60 | pCMV-GH |
| HAMJ251-OCRR487 | BVH-11A | BVH-11 N'end w/o ss (20-353) | 61 | pET-32 c (+) |
| HAMJ171-OCRR488 | BVH-11B | BVH-11 C'end (354-840) | 62 | pET-32 a(+) |
| HAMJ264-OCRR488 | BVH-11C | BVH-11 C'end (228-840) | 63 | pET-32 a(+) |
| HAMJ278-HAMJ279 | NEW1 | BVH-3 C'end (472-1039) | 64 | pET-21b(+) |
| HAMJ278-HAMJ280 | NEW2 | BVH-3 C'end (472-800) | 65 | pET-21b(+) |
| HAMJ281-HAMJ279 | NEW3 | BVH-3 C'end (800-1039) | 66 | pET-21b(+) |
| HAMJ284-HAMJ285 | NEW4 | BVH-11 C'end (286-840) | 67 | pET-21d(+) |
| HAMJ284-HAMJ286 | NEW5 | BVH-11 internal (286-713) | 68 | pET-21d(+) |
| HAMJ287-HAMJ288 | NEW6 | BVH-11 internal (672-792) | 69 | pET-21d(+) |
| HAMJ285-HAMJ289 | NEW7 | BVH-11 internal (709-840) | 70 | pET-21d(+) |
| HAMJ284-HAMJ290 | NEW8 | BVH-11 internal (286-511) | 71 | pET-21d(+) |
| HAMJ286-HAMJ291 | NEW9 | BVH-11 internal (511-713) | 72 | pET-21d(+) |
| HAMJ160-HAMJ186 | BVH-11-2M | BVH-11-2 w/o ss (20-838) | 73 | pSL301 |
| HAMJ292-HAMJ293 | NEW10 | BVH-11-2 C'end (271-838) | 74 | pET-21d(+) |
| HAMJ293-HAMJ294 | NEW11 | BVH-11-2 C'end (699-838) | 75 | pET-21d(+) |
| HAMJ282-HAMJ283 | BVH-11B | BVH-11 C'end (354-840) | 62 | pET-21b(+) |
| HAMJ286-HAMJ297 | NEW14 | BVH-11-2 internal (227-699) | 77 | pET-21d(+) |
| HAMJ300-HAMJ313 | NEW15 | BVH-3 N'end w/o ss (21-800) | 78 | pET-21b(+) |
| HAMJ301-HAMJ302 | NEW16 | BVH-11 N'end w/o ss (20-709) | 79 | pET-21d(+) |

### EXAMPLE 9

This example describes the isolation of monoclonal antibodies (Mabs) and the use of Mabs to characterize BVH-3, BVH-11 and BVH-11-2 protein epitopes.

Female BALB/c mice (Charles River) were immunized subcutaneously with BVH-3, BVH-11 or BVH-11-2 gene products from S. pneumoniae strain SP64 in presence of 15 µg of QuilA adjuvant (Cedarlane Laboratories Ltd, Hornby, Canada). One set of mice (fusion experiment 1) were immunized on day 1 and 14 with 25 µg of affinity purified thioredoxin-His•Tag-BVH-3M fusion protein. A second group of mice (fusion experiment 2) were immunized three times at three-week intervals with 25 µg of affinity purified thioredoxin-His•Tag-BVH-11M. A third group of mice (fusion experiment 3) were immunized on day 1 and day 15 with 25 µg of affinity purified thioredoxin-His•Tag-BVH-11-2M fusion protein. A fourth group of mice (fusion experiment 4) were immunized on day 1 with 25 µg of affinity purified thioredoxin-His•BVH-11B fusion protein and boosted by intravenous injection on day 16 and on day 37 with recombinant BVH-11B in PBS. Three to four days before fusion, mice were injected intravenously with 25 µg of the respective antigen suspended in PBS alone. Hybridomas were produced by fusion of spleen cells with nonsecreting SP2/0 myeloma cells as previously described by J. Hamel et al. [J. Med. Microbiol., 23, pp163-170 (1987)]. Culture supernatants of hybridomas were initially screened by enzyme-linked-immunoassay according to the procedure described by Hamel et al. (Supra) using plates coated with preparations of purified recombinant proteins or suspensions of heat-killed S. pneumoniae cells. Positive hybridomas selected on the basis of ELISA reactivity with a variety of antigens were then cloned by limiting dilutions, expanded and frozen.

Hybridomas were tested by ELISA or Western immunoblotting against BVH-3 and BVH-11 gene products in order to characterize the epitopes recognized by the Mabs. BVH-3 and BVH-11 shared common epitopes with 6 Mabs (H3-1-F9, H3-1-D4, H3-1-H12, H11-1-E7, H11-1-H10 and H11-1.1-G11) showing reactivities with both proteins (Table 4). BVH-11 and BVH-11-2 molecules from S. pneumoniae SP64 shared common epitopes not present on BVH-3 with Mabs (3A1, 13C11, 10H10, 1D8, 10G9, 10A2, 3E8, 10D7, 2H7 and 6H7) reactive with both, BVH-11 and BVH-11-2, recombinant proteins (Table 5).

**Table 4. Reactivity of BVH-3-immunoreactive Mabs with a panel of BVH-3 and BVH-11 gene products**

| | a.Immunoreactivity with | | | | | | |
|---|---|---|---|---|---|---|---|
| MAbs | BVH-3M 21-1039 | BVH-3A 21-509 | BVH-3B 512-1039 | BVH-3C 21-225 | NEW2 472-800 | NEW3 800-1039 | BVH-11M 20-840 |
| H3-1-F9 | + | + | - | + | - | - | + |
| H3-1-D4 | + | + | - | + | - | - | + |
| H3-1-H12 | + | + | - | + | - | - | + |
| H3-2-G2 | + | + | - | - | - | - | - |
| H3-3-A1 | + | + | - | - | - | - | - |
| H3-4-D3 | + | - | + | - | - | + | - |
| H11-1-E7 | + | + | - | + | - | - | + |
| H11-1-H10 | + | + | - | + | - | - | + |
| H11- 1.1-G11 | + | + | - | + | + | - | + |

**Table 5. Reactivity of Mabs raised against BVH-11-2 protein from S. pneumoniae strain SP64 with a panel of BVH-11 gene products**

| | b.Immunoreactivity with | | | | | | | |
|---|---|---|---|---|---|---|---|---|
| Mabs^{a} | c.BVH-11 products | | | | d.BVH-11-2 products | | | |
| | BVH-11M 20-840 | NEW8 286-511 | NEW9 511-713 | BVH-11B 354-840 | BVH-11-2 20-838 | NEW10 271-838 | NEW11 699-838 | NEW14 227-699 |
| 3A1 | + | + | - | + | + | + | - | + |
| 13C1 | + | + | + | + | + | + | - | + |
| 10H10 | + | + | + | + | + | + | - | + |
| 1D8 | + | + | - | + | + | + | - | + |
| 10G9 | + | - | - | + | + | + | - | + |
| 10A2 | + | - | - | + | + | + | - | + |
| 3E8 | + | - | - | + | + | + | - | + |
| 10D7 | + | - | - | + | + | + | - | + |
| 2H7 | + | ⁻- | - | - | + | - | - | - |
| 6H7 | + | - | - | - | + | - | - | - |
| 3A4 | - | - | - | - | + | + | + | - |
| 14H6 | - | - | - | - | + | + | + | - |
| 7G2 | - | - | - | - | + | + | - | + |
| 13H10 | - | - | - | - | + | - | - | + |
| 7E8 | - | - | - | - | + | - | - | - |
| 7H6 | - | - | - | - | + | - | - | - |

| | | | | | | | | |
|---|---|---|---|---|---|---|---|---|
| ^{a} Mabs listed in this table were not reactive with recombinant BVH-3 molecule | | | | | | | | |

The results obtained from the immunoreactivity studies of the Mabs (Table 4 and Table 5) are in agreement with the protein sequences derived from the respective gene sequences. Indeed the Mabs cross-reactive with BVH-3 and BVH-11 molecules recognized BVH-3C protein corresponding to the conserved region, and BVH-11 and BVH-11-2 specific Mabs were reactive with epitopes located on variable parts of these molecules. BVH-3 and BVH-11, and BVH-11 and BVH-11-2 can be distinguished by their reactivity with Mabs.

### EXAMPLE 10

This example illustrates the simultaneous expression of BVH-3 and BVH-11 gene products by S. pneumoniae.

A standard Western blot technique was used to investigate whether BVH-3 and BVH-11 genes were expressed in S. pneumoniae. S. pneumoniae strain SP64 and SP63 were grown overnight at 37°C in 5% CO₂ on chocolate agar plates, bacteria were suspended in PBS and heat-killed at 56°C for 20 min. For the preparation of antigens, suspensions of S. pneumoniae were treated with sample buffer containing SDS and 2-mercaptoethanol for 5 min at 100°C. Pneumococcal protein antigens were resolved by SDS-PAGE electrophoresis according to the method of Laemmli [Nature, 227, pp. 680-685 (1970)]. After SDS-PAGE, the proteins were transferred electrophoretically from the gel to nitrocellulose paper by the method of Towbin [Proc. Natl. Acad. Sci. USA, 76, pp. 4350-4354 (1979)] and probed with mouse antiserum or monoclonal antibodies. The detection of antigens reactive with the antibodies was performed by indirect enzyme-immunoassay using conjugated-anti-mouse immunoglobulins and a colour substrate. When antiserum raised to recombinant BVH-3 was tested against S. pneumoniae SP64 antigens, two reactive bands having apparent molecular masses of 127 kDa and 99 kDa were detected. Bands having the same apparent molecular masses were also detected when Mabs H3-1-F9, H3-1-D4, H3-1-H12, H11-1-E7, H11-1-H10 and H11-1.1-G11 were used individually as immunological probes. In contrast, Mabs specific for the BVH-3 molecule detected the 127 kDa band only and Mabs specific for BVH-11 detected the 99 kDa band only thus confirming the identity of the 127 and 99 kDa bands as BVH-3 and BVH-11, respectively. These studies provide evidence that BVH-3 and BVH-11 proteins are simultaneously present on S. pneumoniae. Moreover, the results are consistent with our previous observations that BVH-3 and BVH-11 possess epitopes that are common to both proteins and epitopes that are exclusive to either protein.

In S. pneumoniae SP64, mature BVH-3, BVH-11 and BVH-11-2. are proteins of 1019, 821 and 819 amino acids.with predicted molecular mass of 112.5 kDa, 92.4 kDa, and 91.7 kDa, respectively. Although there is a discrepancy between the molecular mass predicted from the sequence and the molecular mass calculated on SDS-PAGE, BVH-3 can be distinguished from BVH-11 by its higher molecular mass. Moreover, BVH-3 molecules from S. pneumoniae strain SP63 have an apparent molecular mass of 112 kDa in SDS-PAGE compared to 127 kDa for BVH-3 of SP64 strain. This data is consistent with the deletion of a stretch of 177 amino acid residues in BVH-3 of S. pneumoniae strain SP63.

### EXAMPLE 11

This example describes the protection conferred in experimental infection of mice vaccinated with recombinant BVH-3 or BVH-11 gene products.

Groups of 7 or 8 female BALB/c mice (Charles River) were immunized subcutaneously three times at three-week intervals with either affinity purified thioredoxin-His•Tag-BVH-3M fusion protein, affinity purified thioredoxin-His•Tag-BVH-11M fusion protein or, as control, with QuilA adjuvant alone in PBS. Twelve to 14 days following the third immunization, the mice were challenged intravenously with S. pneumoniae WU2 strain or intranasally with P4241 strain. Samples of the S. pneumoniae challenge inoculum were plated on chocolate agar plates to determine the CFU and to verify the challenge dose. The challenge dose was approximately 10⁶ CFU. Deaths were recorded for a period of 14 days and on day 14 post-challenge, the surviving mice were sacrificed and blood samples tested for the presence of S. pneumoniae organisms. The survival data are shown in Tables 6 and 7.

**Table 6. Protection mediated by recombinant BVH-3M and BVH-11M proteins in experimental infection with virulent S. pneumoniae WU2**

| Experiment | Immunogen | Alive : dead^{a} | Median days alive |
|---|---|---|---|
| 1 | BVH-3M | 8 : 0 | >14 |
| | none | 0 : 8 | 1 |
| 2 | BVH-11M | 8 : 0 | >14 |
| | none | 0 : 8 | 1 |

| | | | |
|---|---|---|---|
| ^{a} The number of mice alive : the number of mice dead on day 14 post-challenge. | | | |

**Table 7. Protection mediated by recombinant BVH-3M and BVH-11M proteins in experimental pneumonia with virulent S. pneumoniae P4241**

| Experiment | Immunogen | Alive : dead^{a} | Median day alive |
|---|---|---|---|
| 1 | BVH-3M | 6 : 1 | >14 |
| | none | 1 : 7 | 4.5 |
| 2 | BVH-3M | 8 : 0 | >14 |
| | BVH-11M | 8 : 0 | >14 |
| | none | 0 : 8 | 4 |

| | | | |
|---|---|---|---|
| ^{a} The number of mice alive : the number of mice dead on day 14 post-challenge. | | | |

All mice immunized with recombinant BVH-3M or BVH-11M protein survived to infection with WU2 while none of the control mice given adjuvant alone survived. All except one mice immunized with recombinant BVH-3M or BVH-11M protein survived to infection with P4241 while only one control mice given adjuvant alone survived. All hemocultures from surviving mice were negative at day 14 post-challenge. These results clearly indicate that both, BVH-3M and BVH-11M, elicit protective anti-pneumococcal immune responses in mice. The fact that these proteins are highly conserved among S. pneumoniae isolates emphasize the potential of BVH-3 and BVH-11 as universal vaccine candidates. Indeed, the BVH-3 and BVH-11 proteins from serogroup 6 S. pneumoniae strain SP64 elicited protection against pneumococcal infections with strains of different capsular serotypes.

Ideally, a vaccine that could protect against pneumococcal disease, could protect against meningitis, otitis media, bacteremia and pneumonia. BVH-3 and BVH-11 were protective against lethal systemic- and pneumonia-infection models thus suggesting that, in humans, BVH-3- and BVH11-protein-based vaccines could reduce the incidence of a wide spectrum of disease caused by virtually all S. pneumoniae independently of the capsular serotype.

Data from Tables 6 and 7 clearly demonstrate that BVH-3 and BVH-11 were, both, protection-eliciting molecules of S. pneumoniae. It was not known, however, whether protection can be mediated by specific sequences that were not shared on BVH-3 and BVH-11 molecules. Groups of female BALB/c mice (Charles River) were immunized subcutaneously three times at three-week intervals with either affinity purified thioredoxin-His•Tag- BVH-3AD, -BVH-3B or -BVH-3C fusion protein in presence of 15 µg of QuilA adjuvant (Cedarlane Laboratories Ltd, Hornby, Canada). Control mice were immunized with QuilA adjuvant alone in PBS or affinity purified thioredoxin-His•Tag or thioredoxin-His•Tag-fusion protein (His-Thio) in presence of QuilA.

To determine the protective ability of a set of truncated proteins, termed NEW4, NEW5, NEW6, NEW7, NEW8, NEW9, NEW10, NEW11, NEW14 and BVH-11B, groups of female BALB/c mice (Charles River) were immunized subcutaneously two times at three-week intervals with 25 µg of either affinity purified His•Tag-fusion protein in presence of 15 µg of QuilA adjuvant. Ten to 14 days following the last immunization, the mice were challenged with virulent S. pneumoniae. Our results indicate that, BVH-3B, a truncated BVH-3 molecule consisting of amino acids 512-1039, elicited protection against the mouse-virulent strains WU2 and P4241. Similarly, BVH-11B, NEW4 and NEW5 molecules, three truncated BVH-11 molecules consisting of amino acids 354-840, amino acids 286-840 and amino acids 286-713, respectively, elicited protection against experiment intravenous challenge with WU2 and intranasal challenge with P4241. Moreover, vaccination with NEW10 and NEW14, consisting of amino acids 272-838 and amino acids 227-699 from BVH-11-2 molecule also resulted in protection against death with the pneumococcal strains. These results indicate that the region comprising 428 amino acids extending from amino acids 286-713 and amino acids 272-699 on S. pneumoniae SP64 BVH-11 and BVH-11-2 protein sequences, respectively, contains protective epitopes. This region is highly conserved with a global 91% identity and 94% homology among thirteen BVH-11 protein sequences.

**Table 8. Evaluation of protection elicited by vaccination of mice with BVH-3 and BVH-11 gene products**

| | | Challenge with WU2 | | Challenge with P4241 | |
|---|---|---|---|---|---|
| Experiment | Immunogen | Alive : dead^{a} | Median day alive | Alive : dead | Median day alive |
| 1^{b} | None | 0 : 8 | 1.5 | 1 : 7 | 4.5 |
| | NEW4 | 8 : 0 | >14 | 8 : 0 | >14 |
| | NEW5 | 8 : 0 | >14 | 8 : 0 | >14 |
| | NEW7 | 0 : 8 | 2 | 0 : 8 | 5 |
| | BVH-11M | 8 : 0 | >14 | 8 : 0 | >14 |
| 2^{b} | None | 0 : 8 | 1 | 0 : 8 | 4 |
| | NEW5 | 8 : 0 | >14 | 8 : 0 | >14 |
| | NEW8 | 0 : 8 | 1.5 | 0 : 8 | 5.5 |
| | NEW9 | 3 : 5 | 3.5 | 2 : 6 | 7 |
| | BVH-11M | 8 : 0 | >14 | 8 : 0 | >14 |
| 3^{b} | None | 0 : 8 | 1 | 0 : 8 | 4 |
| | NEW6 | 0 : 8 | 1 | 4 : 4 | 10.5^{c} |
| | NEW10 | 8 : 0 | >14 | 8 : 0 | >14 |
| | NEW11 | 0 : 8 | 1.5 | 1 : 7 | 6 |
| | BVH-11M | 8 : 0 | >14 | 8 : 0 | >14 |
| 4^{b} | None | 0 : 8 | 2 | 0 : 8 | 4 |
| | BVH-11B | 7 : 1 | >14 | 8 : 0 | >14 |
| | NEW14 | 8 : 0 | >14 | 8 : 0 | >14 |
| 5 | His-Thio | 0 : 8 | 2 | | |
| | BVH-3AD | 1 : 7 | 2.5 | | |
| | BVH-3B | 5 : 3 | >14 | | |
| 6 | His-Thio | 0 : 8 | 1 | | |
| | BVH-3C | 0 : 8 | 1 | | |

| | | | | | |
|---|---|---|---|---|---|
| ^{a} The number of mice alive : the number of mice dead on day 14 post-challenge. ^{b} The WU2 challenge dose was 10⁵ CFU. ^{c} Mice living longer than 14 days were assigned a survival time of 14 days for the determination of median values. | | | | | |

### EXAMPLE 12

This example described the cloning and expression of a chimeric gene encoding for a chimeric polypeptide corresponding to the carboxy-terminal region of BVH-3 in fusion at the C' end to the carboxy-terminal region of BVH-11 and the additive protection observed after vaccination with a chimeric polypeptide.

It is clear from the studies described above that BVH-3 and BVH-11 are serologically distinct molecules simultaneously present on S. pneumoniae. The results of immunological studies of mice indicate that both proteins are good vaccine candidates. These proteins have the potential to provide protection against all pneumococci, regardless of serotype. Even though the two proteins share epitopes and sequences, they have different characteristics and may serve different biological functions. Thus, immunization against the two proteins may provide a higher level of protection than that imparted by each individually. To examine this, several avenues where full-length or truncated BVH-3 and BVH-11 are administered in combination or in conjugation can be explored. Here we describe the genetic engineering of a BVH-3-BVH-11 fusion gene and protein, termed NEW12 (**SEQ ID NO:76 and SEQ ID NO:58,** respectively), and the potential use of NEW12 protein as a vaccine.

BVH-3 and BVH-11 gene fragments corresponding to the 3'end of the genes were amplified by PCR using pairs of oligonucleotides engineered to amplify fragments spanning nucleotides 1414 to 3117(**SEQ ID NO: 1**) and nucleotides 1060 to 2520 (**SEQ ID NO: 3**) from S. pneumoniae strain SP64 BVH-3 and BVH-11 genes, respectively. The primers used, HAMJ278 and HAMJ279; HAMJ282 and HAMJ283 had a restriction endonuclease site at the 5' end, thereby allowing directional in-frame cloning of the amplified product into the digested pET21b(+) plasmid vector (Table 2). PCR-amplified products were digested with restriction endonucleases and ligated to linearized plasmid pET21b(+) vector digested likewise. The resultant plasmid constructs were confirmed by nucleotide sequence analysis. The recombinant pET21b(+) plasmid containing the NdeI-HindIII BVH-3 PCR product was linearized by digestion with the restriction enzymes HindIII and NotI for the in-frame cloning of the HindIII-NotI DNA fragment obtained from the recombinant pET21(+) vector containing the BVH-11 gene fragment. Clones were first stabilized in E. coli DH5α before introduction into E. coli BL21(λDE3) for expression of a chimeric pneumococcal protein molecule. The recombinant chimeric polypeptide, termed NEW 12, was expressed as C-terminal fusion with an His-tag. The expressed recombinant NEW 12 protein was purified from supernatant fractions obtained from centrifugation of sonicated IPTG-induced E. coli cultures using a His-Bind metal chelation resin (QIAgen, Chatsworth, CA).

According to the same procedure described above, it is possible to construct other chimeric polypeptides, as a result of a simultaneous expression of New 1 and New 4, New 1 and New 5, New 1 and New 10, or New 1 and New 14. The construction can be with New 1 upstream or downstream of New 4, New 5, New 10, BVH-11B or New 14. It is also possible to construct other chimeric polypeptides as a result of a simultaneous expression of more than two fragments of either genes of BVH-3, BVH-11 or BVH-11-2.

Groups of 8 female BALB/c mice (Charles River) were immunized subcutaneously two times at three-week intervals with 25 µg of either affinity purified His•Tag-fusion NEW1, BVH-11B or NEW12 protein in presence of 15 µg of QuilA adjuvant. Ten to 14 days following the last immunization, the mice were challenged with virulent S. pneumoniae. As demonstrated before, NEW1 and BVH-11B molecules comprising amino acids 472 to 1039 from BVH-3 protein and amino acids 354-840 from BVH-11 protein, respectively, correspond to portions of the proteins capable of eliciting a protective immune response. To determine if a chimeric polypeptide would significantly improve the protection compared with those seen for the individual counterparts, the challenge dose was adjusted in a manner that protection was not expected with NEW1 and BVH-11B molecules. Interestingly, the chimeric NEW12 protein, elicited protection against the mouse-virulent strains WU2 and P4241. Seven out of 8 mice immunized with NEW12 were still alive 10 days after the challenge while 28 out of 32 mice immunized with NEW1, BVH-11B, BVH-3M or adjuvant alone were dead by five days post-challenge. Thus, vaccination of mice with NEW12 provided the highest degree of protection against WU2 challenge. These results indicate that immunization with a chimeric polypeptide and possibly a combination of BVH-3 and BVH-11 gene products can provide additional protection to that obtained by administration of BVH-3 or BVH-11 antigens alone.

**Table 9. Evaluation of protection elicited by vaccination of mice with the chimeric NEW12 molecule**

| | Challenge with WU2 | | Challenge with P4241 | |
|---|---|---|---|---|
| Immunogen | Alive : dead^{a} | Median day alive | Alive : dead | Median day alive |
| None | 0 : 8 | 1 | 0 : 8 | 5 |
| NEW1 | 2 : 6 | 2 | 1 : 7 | 8 |
| BVH-11B | 1 : 7 | 3.5 | 8 : 0 | >14 |
| NEW12 | 6 : 2 | >14 | 7 : 1 | >14 |
| BVH-3M | 1 : 7 | 3 | 8 : 1 | >14 |

### EXAMPLE 13

This example illustrates the identification of additional BVH-3 and BVH-11 related sequences in Streptococcus species other than S. pneumoniae.

It was previously shown that BVH-3, BVH-11 and BVH-11-2 are a family of related proteins sharing common sequences. Homology searches were performed with the nucleotide sequence from the conserved region of these genes and compared with GenBank and EMBL sequences using FASTA. The most significant homology was observed with a 2.469-kb gene coding for a calculated 92-kDa protein (**SEQ ID NO: 81**) of unknown function in S. agalactiae also called group B streptococcus or GBS. The gene was designated BVH-71. A protein demonstrating 99.2% identity and 99.5% similarity with that of GBS was also identified in S. pyogenes also called group A streptococcus or GAS (**SEQ ID NO: 83**). The 5' region of the BVH-71 sequences (**SEQ ID NO: 80 and SEQ ID NO: 82**), spanning nucleotides 1 to 717, demonstrated 58 and 60% identity with the conserved regions of BVH-3 (nucleotides 1 to 675) and BVH-11 (nucleotides 1 to 684) genes respectively. The first 239 amino acids of the translated sequences of the GBS and GAS BVH-71 open reading frames are 51 and 54% identical to the first 225 and 228 amino acids of BVH-3 and BVH-11, respectively. In addition to structural similarities, streptococcal BVH-3, BVH-11 and BVH-71 proteins also share antigenic epitopes. A 97-kDa band was revealed on Western blots of GAS or GBS whole cells, using Mab H11-1.1-G11 reactive with the BVH-3 and BVH-11 conserved regions. Similarly, GAS and GBS recombinant BVH-71 proteins were detected in Western immunoblot analysis.

These results indicate that BVH-71, BVH-3 and BVH-11 proteins might share similar functions. Our results also suggest that BVH-71 proteins can be used as protein vaccine components of anti-streptococcus. In a further embodiment BVH-71 proteins can be used as protein vaccine components of anti-GAS or anti-GBS vaccines.

## Claims

1. A vaccine composition comprising an isolated polypeptide having a) greater than 90% identity to a second polypeptide having an amino acid sequence chosen from:
**SEQ ID NOs: 14, 4, 58, 60, 61, 63, 73 to 75, or 79** or, b) greater than 99% identity to a second polypeptide having a sequence chosen from: **SEQ ID NO: 67 or 68,**
wherein the polypeptide is not SEQ ID NO:5 of WO 00/17370, or SEQ ID NO:194 of WO 00/06737, wherein the polypeptide elicits an immune response when administered to an individual; and a pharmaceutically acceptable carrier, diluent or
adjuvant; for use in the treatment or prophylaxis of *Streptococcus pneumoniae* infection.

2. A vaccine composition comprising an isolated polypeptide having a) greater than 90% identity to a second polypeptide having an amino acid sequence chosen from:
**SEQ ID NOs: 14, 4, 58, 60, 61, 63, 73 to 75, or 79** or, b) greater than 99% identity to a second polypeptide having a sequence chosen from: **SEQ ID NO: 67 or 68,**
wherein the polypeptide is not SEQ ID NO:5 of WO 00/17370, SEQ ID NO:4 of WO00/37105, or SEQ ID NO:194 of WO 00/06737, wherein the polypeptide elicits an immune response when administered to an individual; and a pharmaceutically acceptable carrier, diluent or adjuvant; for use in the treatment or prophylaxis of *Streptococcus pneumoniae* infection.

3. A vaccine composition according to claim 1 or claim 2 wherein the isolated polypeptide has greater than 95% identity to a second polypeptide sequence chosen from: **SEQ ID NOs: 14, 4, 58, 60, 61, 63, 73 to 75, or 79.**

4. A vaccine composition according to claim 1 or claim 2 wherein the isolated polypeptide has greater than 99% identity to a second polypeptide sequence chosen from: **SEQ ID NOs: 14, 4, 58, 60, 61, 63, 73 to 75, or 79.**

5. A vaccine composition according to claim 1 or claim 2 wherein the isolated polypeptide has greater than 99% identity to amino acid sequence: **SEQ ID NO: 14.**

6. A vaccine composition according to claim 1 or claim 2 wherein the isolated polypeptide has greater than 99% identity to amino acid sequence: **SEQ ID NO: 73.**

7. A vaccine composition according to claim 1 or claim 2 wherein the isolated polypeptide has an amino acid sequence chosen from: **SEQ ID NOs: 14, 4, 58, 60 to 63, 67, 68, 73 to 75, 77, 79.**

8. A vaccine composition according to claim 7 wherein the isolated polypeptide has the amino acid sequence of SEQ ID NO:4 or SEQ ID NO:14 from which the N-terminal Met residue is deleted.

9. A vaccine composition according to claim 7 wherein the isolated polypeptide has the amino acid sequence of SEQ ID NO:4 or SEQ ID NO:14 from which the secretory amino acid sequence is deleted.

10. A vaccine composition according to any one of claims 1 to 9 for use in the treatment or prophylaxis of meningitis, otitis media, bacteremia, or pneumonia.

11. A vaccine composition according to any one of claims 1 to 10 for use in the treatment or prophylaxis of *Streptococcus pneumoniae* infection in a human.

12. A vaccine composition according to claim 11 wherein the vaccine is formulated for use in infants, elderly or immunocompromised patients.

13. A vaccine composition according to any one of claims 1-12 in unit dose form wherein the unit dose is 0.001-100µg/kg (antigen/body weight).

14. A vaccine composition according to claim any one of claims 1-12 in unit dose form wherein the unit dose is 0.1-10µg/kg (antigen/body weight).

15. A vaccine composition according to any one of claims 13-14 wherein the unit dose is to be administered 1 to 3 times with an interval of 1 to 6 weeks between immunizations.

## Patentansprüche

1. Vakzinzusammensetzung, umfassend ein isoliertes Polypeptid mit a) mehr als 90% Identität zu einem zweiten Polypeptid, welches eine Aminosäuresequenz, ausgewählt aus SEQ ID NOs: 14, 4, 58, 60, 61, 63, 73 bis 75 oder 79, hat; oder b) mehr als 99% Identität zu einem zweiten Polypeptid, welches eine Sequenz, ausgewählt aus SEQ ID NO: 67 oder 68, hat; wobei das Polypeptid nicht SEQ ID NO: 5 von WO 00/17370 oder SEQ ID NO: 194 von WO 00/06737 ist; wobei das Polypeptid eine Immunreaktion hervorruft, wenn es einem Individuum verabreicht wird; und einen pharmazeutisch annehmbaren Träger, ein Verdünnungsmittel oder ein Adjuvans zur Verwendung bei der therapeutischen oder prophylaktischen Behandlung einer Streptococcus pneumoniae Infektion.

2. Vakzinzusammensetzung, umfassend ein isoliertes Polypeptid mit a) mehr als 90% Identität zu einem zweiten Polypeptid, welches eine Aminosäuresequenz, ausgewählt aus SEQ ID NOs: 14, 4, 58, 60, 61, 63, 73 bis 75 oder 79, hat; oder b) mehr als 99% Identität zu einem zweiten Polypeptid, welches eine Sequenz, ausgewählt aus SEQ ID NO: 67 oder 68, hat; wobei das Polypeptid nicht SEQ ID NO: 5 von WO 00/17370, SEQ ID NO: 4 von WO 00/37105 oder SEQ ID NO: 194 von WO 00/06737 ist; wobei das Polypeptid eine Immunreaktion hervorruft, wenn es einem Individuum verabreicht wird; und einen pharmazeutisch annehmbaren Träger, ein Verdünnungsmittel oder ein Adjuvans zur Verwendung bei der therapeutischen oder prophylaktischen Behandlung einer Streptococcus pneumoniae Infektion.

3. Vakzinzusammensetzung gemäß Anspruch 1 oder 2, wobei das isolierte Polypeptid mehr als 95% Identität zu einer zweiten Polypeptidsequenz, ausgewählt aus SEQ ID NOs: 14, 4, 58, 60, 61, 63, 73 bis 75 oder 79, hat.

4. Vakzinzusammensetzung gemäß Anspruch 1 oder 2, wobei das isolierte Polypeptid mehr als 99% Identität zu einer zweiten Polypeptidsequenz, ausgewählt aus SEQ ID NOs: 14, 4, 58, 60, 61, 63, 73 bis 75 oder 79, hat.

5. Vakzinzusammensetzung gemäß Anspruch 1 oder 2, wobei das isolierte Polypeptid mehr als 99% Identität zur Aminosäuresequenz SEQ ID NO: 14 hat.

6. Vakzinzusammensetzung gemäß Anspruch 1 oder 2, wobei das isolierte Polypeptid mehr als 99% Identität zur Aminosäuresequenz SEQ ID NO: 73 hat.

7. Vakzinzusammensetzung gemäß Anspruch 1 oder 2, wobei das isolierte Polypeptid eine Aminosäuresequenz, ausgewählt aus SEQ ID NOs: 14, 4, 58, 60 bis 63, 67, 68, 73 bis 75, 77, 79, hat.

8. Vakzinzusammensetzung gemäß Anspruch 7, wobei das isolierte Polypeptid die Aminosäuresequenz von SEQ ID NO: 4 oder SEQ ID NO: 14 mit einer Deletion des N-terminalen Met hat.

9. Vakzinzusammensetzung gemäß Anspruch 7, wobei das isolierte Polypeptid die Aminosäuresequenz von SEQ ID NO: 4 oder SEQ ID NO: 14 mit einer Deletion der sekretorischen Aminosäuresequenz hat.

10. Vakzinzusammensetzung gemäß einem der Ansprüche 1 bis 9 zur Verwendung bei der therapeutischen oder prophylaktischen Behandlung von Meningitis, Otitis media, Bakteriämie oder Pneumonie.

11. Vakzinzusammensetzung gemäß einem der Ansprüche 1 bis 10 zur Verwendung bei der therapeutischen oder prophylaktischen Behandlung einer Streptococcus pneumoniae Infektion eines Menschen.

12. Vakzinzusammensetzung gemäß Anspruch 11, wobei das Vakzin zur Verwendung bei Kleinkindern, Senioren oder immungeschwächten Patienten formuliert ist.

13. Vakzinzusammensetzung gemäß einem der Ansprüche 1-12 in Form von Einzeldosen, wobei die Einzeldosis 0.001-100 µg/kg (Antigen/Körpergewicht) aufweist.

14. Vakzinzusammensetzung gemäß einem der Ansprüche 1-12 in Form von Einzeldosen, wobei die Einzeldosis 0.1-10 µg/kg (Antigen/Körpergewicht) aufweist.

15. Vakzinzusammensetzung gemäß einem der Ansprüche 13-14, wobei die Einzeldosis 1- bis 3-mal mit einem Intervall von 1 bis 6 Wochen zwischen den Immunisierungen verabreicht wird.

## Revendications

1. Composition vaccinale comprenant un polypeptide isolé présentant a) plus de 90 % d'identité avec un second polypeptide ayant une séquence d'acides aminés choisie parmi : SEQ ID NO : 14, 4, 58, 60, 61, 63, 73 à 75, ou 79 ou, b) plus de 99 % d'identité avec un second polypeptide ayant une séquence choisie parmi : SEQ ID NO : 67 ou 68, dans laquelle le polypeptide n'est pas SEQ ID NO : 5 du document WO 00/17370, ou SEQ ID NO : 194 du document WO 00/06737, dans laquelle le polypeptide déclenche une réponse immunitaire lorsqu'il est administré à un individu ; et un vecteur, un diluant ou un adjuvant pharmaceutiquement acceptable ; pour son utilisation dans le traitement ou la prophylaxie d'une infection à *Streptococcus pneumoniae.*

2. Composition vaccinale comprenant un polypeptide isolé présentant a) plus de 90 % d'identité avec un second polypeptide ayant une séquence d'acides aminés choisie parmi : SEQ ID NO : 14, 4, 58, 60, 61, 63, 73 à 75, ou 79 ou, b) plus de 99 % d'identité avec un second polypeptide ayant une séquence choisie parmi : SEQ ID NO : 67 ou 68, dans laquelle le polypeptide n'est pas SEQ ID NO : 5 du document WO 00/17370, SEQ ID NO : 4 du document WO 00/37105, ou SEQ ID NO : 194 du document WO 00/06737, dans laquelle le polypeptide déclenche une réponse immunitaire lorsqu'il est administré à un individu ; et un vecteur, un diluant ou un adjuvant pharmaceutiquement acceptable ; pour son utilisation dans le traitement ou la prophylaxie d'une infection à *Streptococcus pneumoniae.*

3. Composition vaccinale selon la revendication 1 ou la revendication 2, dans laquelle le polypeptide isolé présente plus de 95 % d'identité avec une seconde séquence polypeptidique choisie parmi : SEQ ID NO : 14, 4, 58, 60, 61, 63, 73 à 75, ou 79.

4. Composition vaccinale selon la revendication 1 ou la revendication 2, dans laquelle le polypeptide isolé présente plus de 99 % d'identité avec une seconde séquence polypeptidique choisie parmi : SEQ ID NO : 14, 4, 58, 60, 61, 63, 73 à 75, ou 79.

5. Composition vaccinale selon la revendication 1 ou la revendication 2, dans laquelle le polypeptide isolé présente plus de 99 % d'identité avec la séquence d'acides aminés : SEQ ID NO : 14.

6. Composition vaccinale selon la revendication 1 ou la revendication 2, dans laquelle le polypeptide isolé présente plus de 99 % d'identité avec la séquence d'acides aminés : SEQ ID NO : 73.

7. Composition vaccinale selon la revendication 1 ou la revendication 2, dans laquelle le polypeptide isolé a une séquence d'acides aminés choisie parmi : SEQ ID NO : 14, 4, 58, 60 à 63, 67, 68, 73 à 75, 77, 79.

8. Composition vaccinale selon la revendication 7, dans laquelle le polypeptide isolé a la séquence d'acides aminés de SEQ ID NO : 4 ou de SEQ ID NO : 14 de laquelle le résidu Met N-terminal est délété.

9. Composition vaccinale selon la revendication 7, dans laquelle le polypeptide isolé a la séquence d'acides aminés de SEQ ID NO : 4 ou SEQ ID NO : 14 de laquelle la séquence d'acides aminés sécrétoire est délétée.

10. Composition vaccinale selon l'une quelconque des revendications 1 à 9, pour son utilisation dans le traitement ou la prophylaxie de la méningite, de l'otite moyenne, d'une bactériémie ou d'une pneumonie.

11. Composition vaccinale selon l'une quelconque des revendications 1 à 10, pour son utilisation dans le traitement ou la prophylaxie d'une infection à *Streptococcus pneumoniae* chez un être humain.

12. Composition vaccinale selon la revendication 11, dans laquelle le vaccin est formulé pour son utilisation chez les nourrissons, les personnes âgées ou les patients immunocompromis.

13. Composition vaccinale selon l'une quelconque des revendications 1 à 12 sous forme de dose unitaire, dans laquelle la dose unitaire est de 0,001 à 100 µg/kg (antigène/poids corporel).

14. Composition vaccinale selon l'une quelconque des revendications 1 à 12 sous forme de dose unitaire, dans laquelle la dose unitaire est de 0,1 à 10 µg/kg (antigène/poids corporel).

15. Composition vaccinale selon l'une quelconque des revendications 13 à 14, dans laquelle la dose unitaire doit être administrée 1 à 3 fois avec un intervalle de 1 à 6 semaines entre les immunisations.
